(19) Europäisches Patentamt
European Patent Office
Office européen des brevets

(11) EP 4 538 274 A1

(12) EUROPEAN PATENT APPLICATION
published in accordance with Art. 153(4) EPC

(43) Date of publication:
**16.04.2025 Bulletin 2025/16**

(21) Application number: **23819248.8**

(22) Date of filing: **09.06.2023**

(51) International Patent Classification (IPC):
***C07D 471/04*** *(2006.01)* ***C07D 209/44*** *(2006.01)*
***A61K 31/4035*** *(2006.01)* ***A61P 35/04*** *(2006.01)*

(52) Cooperative Patent Classification (CPC):
**A61K 31/4035; A61P 35/04; C07D 209/44; C07D 471/04**

(86) International application number:
**PCT/CN2023/099307**

(87) International publication number:
**WO 2023/237085 (14.12.2023 Gazette 2023/50)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:
**10.06.2022 CN 202210654509**
**16.09.2022 CN 202211126072**
**28.10.2022 CN 202211340578**
**09.11.2022 CN 202211397717**
**13.01.2023 CN 202310040632**
**07.04.2023 CN 202310363656**

(71) Applicant: **Betta Pharmaceuticals Co., Ltd**
**Yuhang**
**Hangzhou**
**Zhejiang 311100 (CN)**

(72) Inventors:
- **WU, Hao**
  **Hangzhou, Zhejiang 311100 (CN)**
- **WU, Wenmao**
  **Hangzhou, Zhejiang 311100 (CN)**
- **ZHANG, Zhan**
  **Hangzhou, Zhejiang 311100 (CN)**
- **XU, Yanjie**
  **Hangzhou, Zhejiang 311100 (CN)**
- **YUAN, Ding**
  **Hangzhou, Zhejiang 311100 (CN)**
- **LU, Lu**
  **Hangzhou, Zhejiang 311100 (CN)**
- **WANG, Wei**
  **Hangzhou, Zhejiang 311100 (CN)**
- **ZHU, Xiaoguan**
  **Hangzhou, Zhejiang 311100 (CN)**
- **ZHANG, Yong**
  **Hangzhou, Zhejiang 311100 (CN)**
- **ZHANG, Jian**
  **Hangzhou, Zhejiang 311100 (CN)**
- **LIU, Xianglai**
  **Hangzhou, Zhejiang 311100 (CN)**
- **ZHOU, Bojun**
  **Hangzhou, Zhejiang 311100 (CN)**
- **ZHOU, Quan**
  **Hangzhou, Zhejiang 311100 (CN)**
- **LAN, Hong**
  **Beijing 100176 (CN)**
- **WANG, Jiabing**
  **Beijing 100176 (CN)**
- **DING, Lieming**
  **Hangzhou, Zhejiang 311100 (CN)**

(74) Representative: **Zwicker, Jörk**
**ZSP Patentanwälte PartG mbB**
**Hansastraße 32**
**80686 München (DE)**

# (54) HPK1 INHIBITOR AND MEDICAL USE THEREOF

(57) The present invention relates to a compound represented by general formula (I), a stereoisomer, a tautomer, a deuterated compound or a pharmaceutically acceptable salt thereof, which has therapeutic activity against cancer. The present invention also relates to a method for preparing the compounds and a pharmaceutical composition containing same.

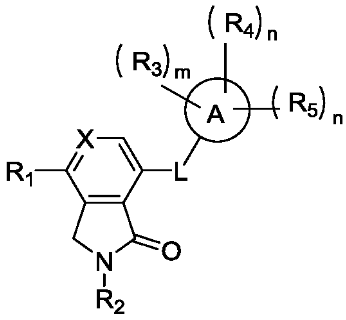

(I)

## Description

### Technical Field

The present invention relates to the field of medical technology, and in particular to a compound of formula (I), its stereoisomers, tautomers, deuterated substances or pharmaceutically acceptable salts, a preparation method thereof, a pharmaceutical composition containing the compound, and its use as a drug for treating cancer.

### Background

Hematopoietic progenitor kinase 1 (HPK1), also known as mitogen-activated protein kinase kinase kinase kinase 1 (MAP4K1), is a member of the serine/threonine kinase subfamily Ste20, whose family members also include MAP4K2 (GCK), MAP4K3 (GLK), MAP4K4 (HGK), MAP4K5 (KHS) and MAP4K6 (MINK). HPK1 is a negative regulator of the activation response of B cells, T cells, and dendritic cells. Inhibiting its expression can specifically enhance the body's anti-tumor immunity. It is mainly expressed in hematopoietic cells, such as T cells, B cells, dendritic cells, macrophages, mast cells, and neutrophils.

In T cells, HPK1 regulates T cell activation through the TCR signaling pathway. After TCR activation, HPK1 interacts with T cell receptor protein and is phosphorylated by tyrosine kinases Zap70 and Lck. It also phosphorylates SLP-76 receptor protein, negatively regulating TCR signaling and thus inhibiting T cell activation and proliferation. Studies have found that HPK1 can participate in many signal cascades, including the MAKP signaling pathway, Fas-induced apoptosis pathway and NF-κB signaling pathway. Moreover, HPK1 can inhibit AP-1, which plays a role in promoting cell proliferation, inhibiting differentiation, and promoting tumor cell invasion and metastasis during tumor formation and development.

Therefore, drugs targeting HPK1 have become one of the current hot areas in drug research and development, and some of them have entered the clinical stage.

However, there are currently no marketed drugs targeting the hematopoietic progenitor cell kinase (HPK1) target. The present invention provides a small molecule HPK1 inhibitor with a novel structure and good anti-tumor activity.

### Summary

The present invention provides a compound represented by general formula (I), its stereoisomers, tautomers, deuterated substances or pharmaceutically acceptable salts:

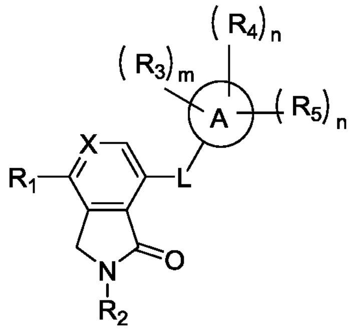

wherein,

- ring A is selected from the group consisting of $C_{5-14}$ bicarbocyclyl, 5-14 membered biheterocyclyl, $C_{10-18}$ bicyclic aryl and 10-18 membered bicyclic heteroaryl;
- L is selected from the group consisting of a bond, NH, O and S;
- $R_1$ is selected from the group consisting of H, halogen, $C_{1-6}$ alkyl, $C_{1-6}$ alkoxy, haloalkyl, hydroxyalkyl, $C_{2-6}$ alkenyl, $C_{2-6}$ alkynyl, $C_{3-14}$ cycloalkyl, 3-14 membered heterocyclyl, $C_{6-18}$ aryl and 6-18 membered heteroaryl; wherein the $C_{1-6}$ alkyl, $C_{1-6}$ alkoxy, haloalkyl, hydroxyalkyl, $C_{3-14}$ cycloalkyl, 3-14 membered heterocyclyl, $C_{6-18}$ aryl and 6-18 membered heteroaryl can be optionally substituted with one or more $R_a$; $R_a$ is independently selected from the group consisting of H, hydroxyl, cyano, halogen, $C_{1-6}$ alkyl, $C_{1-6}$ haloalkyl, -$C_{0-3}$ alkylene-$OR_b$, -OC(=O)$C_{1-6}$ alkyl, -$C_{0-3}$ alkylene-$SR_b$, -$C_{0-3}$ alkylene-N$(R_b)_2$, -$C_{0-3}$ alkylene-S(=O)$R_b$, -$C_{0-3}$ alkylene-S$(=O)_2R_3$, -$C_{0-3}$ alkylene-$SR_b$, -$C_{0-3}$ alkylene-S$(R_b)_5$, -$C_{0-3}$ alkylene-C(=O)$R_b$, -$C_{0-3}$ alkylene-C(=O)$OR_b$, -$C_{0-3}$ alkylene-C(=O)N$(R_b)_2$, $C_{2-6}$ alkenyl, $C_{2-6}$ alkynyl, -$C_{0-3}$ alkylene-$C_{3-14}$ cycloalkyl, -$C_{0-3}$ alkylene-(3-14 membered heterocyclyl), -$C_{0-3}$ alkylene-$C_{6-18}$ aryl and

$-C_{0-3}$ alkylene-(5-18 membered heteroaryl), wherein the $C_{1-6}$ alkyl, $C_{1-6}$ haloalkyl, $C_{2-6}$ alkenyl, $C_{2-6}$ alkynyl, $-C_{0-3}$ alkylene-$C_{3-14}$ cycloalkyl, $-C_{0-3}$ alkylene-(3-14 membered heterocyclyl), $-C_{0-3}$ alkylene-$C_{6-18}$ aryl and $-C_{0-3}$ alkylene-(5-18 membered heteroaryl) can be optionally substituted with one or more $R_b$; each $R_b$ is independently selected from the group consisting of H, halogen, hydroxyl, cyano, $C_{1-6}$ alkyl, $-(CH_2)_{0-3}$ $C_{1-6}$ alkoxy, $C_{3-6}$ cycloalkyl and $C_{1-6}$ haloalkyl;
$R_2$ is selected from the group consisting of H, $C_{1-6}$ alkyl and haloalkyl;
$R_3$ is selected from the group consisting of H, halogen, cyano, $C_{1-6}$ alkyl, $C_{1-6}$ alkoxy, haloalkyl, hydroxyl, hydroxyalkyl, $C_{2-6}$ alkenyl, $C_{2-6}$ alkynyl, $-(CH_2)_{0-3}N(R_b)_2$, $-(CH_2)_{0-3}-C_{3-14}$ cycloalkyl, $-(CH_2)_{0-3}$-3-14 membered heterocyclyl, $-(CH_2)_{0-3}-C_{6-18}$ aryl and $-(CH_2)_{0-3}$-5-18 membered heteroaryl, wherein the $C_{1-6}$ alkyl, $C_{1-6}$ alkoxy, haloalkyl, hydroxyalkyl, $C_{2-6}$ alkenyl, $C_{2-6}$ alkynyl, $-(CH_2)_{0-3}-C_{3-14}$ cycloalkyl, $-(CH_2)_{0-3}$-3-14 membered heterocyclyl, $-(CH_2)_{0-3}-C_{6-18}$ aryl and $-(CH_2)_{0-3}$-5-18 membered heteroaryl can be optionally substituted with one or more $R_c$;
$R_c$ is independently selected from the group consisting of halogen, $C_{1-6}$ alkyl, $C_{1-6}$ alkoxy, $C_{2-6}$ alkenyl, $C_{2-6}$ alkynyl, haloalkyl, cyano, amino, nitro, hydroxyl, hydroxyalkyl, $-C_{0-3}$ alkylene-C(=O)$R_b$, $-C_{0-3}$ alkylene-C(=O)N$(R_b)_2$, $-(CH_2)_{0-3}N(R_b)_2$, $-O(CH_2)_{0-3}-C_{3-14}$ cycloalkyl, $-O(CH_2)_{0-3}$-3-14 membered heterocyclyl, $C_{3-14}$ cycloalkyl, 3-14 membered heterocyclyl, $C_{6-18}$ aryl and 5-18 membered heteroaryl;
$R_4$ and $R_5$ are independently selected from the group consisting of $C_{1-6}$ alkyl, $C_{1-6}$ alkoxy, haloalkyl, hydroxyalkyl, $-(CH_2)_{1-3}N(R_b)_2$, $C_{2-6}$ alkenyl, $C_{3-14}$ cycloalkyl, 3-14 membered heterocyclyl, $C_{6-18}$ aryl and 6-18 membered heteroaryl, wherein the $C_{1-6}$ alkyl, $C_{1-6}$ alkoxy, haloalkyl, hydroxyalkyl, $-(CH_2)_{1-3}N(R_b)_2$, $C_{2-6}$ alkenyl, $C_{3-14}$ cycloalkyl, 3-14 membered heterocyclyl, $C_{6-18}$ aryl and 6-18 membered heteroaryl can be optionally substituted with one or more $R_d$;
$R_4$ or $R_5$, together with directly connected atoms on ring A, respectively form $C_{3-14}$ cycloalkyl, 3-14 membered heterocyclyl, $C_{6-18}$ aryl or 6-18 membered heteroaryl, wherein the $C_{3-14}$ cycloalkyl, 3-14 membered heterocyclyl, $C_{6-18}$ aryl and 6-18 membered heteroaryl can be optionally substituted with one or more $R_d$; or
$R_4$ and $R_5$, together with directly connected atoms on ring A, form $C_{3-14}$ cycloalkyl, 3-14 membered heterocyclyl, $C_{6-18}$ aryl or 6-18 membered heteroaryl; wherein the $C_{3-14}$ cycloalkyl, 3-14 membered heterocyclyl, $C_{6-18}$ aryl and 6-18 membered heteroaryl can be optionally substituted with one or more $R_d$;
$R_d$ is selected from the group consisting of H, halogen, $C_{1-6}$ alkyl, $C_{3-6}$ cycloalkyl, $C_{1-6}$ haloalkyl, cyano, amino, nitro, hydroxy and hydroxyalkyl;
X is $CR_6$ or N;
$R_6$ is selected from the group consisting of H, halogen, $C_{1-6}$ alkyl, $C_{3-6}$ cycloalkyl and $C_{1-6}$ haloalkyl;
m is 0, 1, 2, 3 or 4.
n is 0, 1 or 2.

In some embodiments, $R_3$ in formula (I) is selected from the group consisting of H, halogen, cyano, $C_{1-6}$ Alkyl, $C_{1-6}$ Alkoxy, haloalkyl, hydroxyl, hydroxyalkyl, $-(CH_2)_{0-3}N(R_b)_2$ ,$-(CH_2)_{0-3}-C_{3-14}$ Cycloalkyl, $-(CH_2)_{0-3}$-3-14 membered heterocyclyl, $-(CH_2)_{0-3}-C_{6-18}$ Aryl and $-(CH_2)_{0-3}$-5-18 membered heteroaryl, wherein the $C_{1-6}$ Alkyl, $C_{1-6}$ Alkoxy, haloalkyl, hydroxyalkyl, $-(CH_2)_{0-3}-C_{3-14}$ Cycloalkyl, $-(CH_2)_{0-3}$-3-14 membered heterocyclyl, $-(CH_2)_{0-3}-C_{6-18}$ Aryl or $-(CH_2)_{0-3}$-5-18 membered heteroaryl can be optionally substituted by one or more $R_c$;

$R_c$ is independently selected from the group consisting of halogen, $C_{1-6}$ Alkyl, $C_{1-6}$ Alkoxy, haloalkyl, cyano, amino, nitro, hydroxyl, hydroxyalkyl, $-C_{0-3}$Alkylene-C(=O)$R_b$, $-C_{0-3}$Alkylene-C(=O)N$(R_b)_2$, $-(CH_2)_{0-3}N(R_b)_2$, $-O(CH_2)_{0-3}C_{3-14}$Cycloalkyl, $-O(CH_2)_{0-3}$-3-14 membered heterocyclyl, $C_{3-14}$ Cycloalkyl, 3-14 membered heterocyclyl, $C_{6-18}$ aryl and 5-18 membered heteroaryl.

In some embodiments, each $R_b$ in formula (I) is independently H, halogen, hydroxyl, cyano, $C_{1-6}$ Alkyl, $C_{3-6}$ Cycloalkyl or $C_{1-6}$ Haloalkyl;

The $R_3$ is selected from the group consisting of H, halogen, cyano, $C_{1-6}$ Alkyl, $C_{1-6}$ Alkoxy, haloalkyl, hydroxyl, hydroxyalkyl, $-(CH_2)_{0-3}N(R_b)_2$,$-(CH_2)_{0-3}-C_{3-14}$Cycloalkyl, $-(CH_2)_{0-3}$-3-14 membered heterocyclyl, $-(CH_2)_{0-3}-C_{6-18}$ Aryl and $-(CH_2)_{0-3}$-5-18 membered heteroaryl, wherein the $C_{1-6}$ Alkyl, $C_{1-6}$ Alkoxy, haloalkyl, hydroxyalkyl, $-(CH_2)_{0-3}-C_{3-14}$ Cycloalkyl, $-(CH_2)_{0-3}$-3-14 membered heterocyclyl, $-(CH_2)_{0-3}-C_{6-18}$ Aryl or $-(CH_2)_{0-3}$-5-18 membered heteroaryl can be optionally substituted with one or more $R_c$;
$R_c$ is independently selected from the group consisting of halogen, $C_{1-6}$ Alkyl, $C_{1-6}$ Alkoxy, haloalkyl, cyano, amino, nitro, hydroxyl, hydroxyalkyl, $-C_{0-3}$Alkylene-C(=O)$R_b$,$-C_{0-3}$ Alkylene-C(=O)N$(R_b)_2$, $-(CH_2)_{0-3}N(R_b)_2$, $-O(CH_2)_{0-3}C_{3-14}$Cycloalkyl, $-O(CH_2)_{0-3}$-3-14 membered heterocyclyl, $C_{3-14}$ Cycloalkyl, 3-14 membered heterocyclyl, $C_{6-18}$ aryl and 5-18 membered heteroaryl;
n is 1 or 2.

In some embodiments, each $R_b$ in Formula (I) is independently selected from the group consisting of H, halogen, hydroxyl, cyano, $C_{1-6}$ Alkyl, $C_{3-6}$ Cycloalkyl and $C_{1-6}$ Haloalkyl;

$R_3$ is selected from the group consisting of H, halogen, $C_{1-6}$ Alkyl, $C_{1-6}$ Alkoxy, haloalkyl, hydroxyl, hydroxyalkyl, $-(CH_2)_{0-3}N(R_b)_2$, $-(CH_2)_{0-3}-C_{3-14}$ Cycloalkyl, $-(CH_2)_{0-3}$-3-14 membered heterocyclyl, $-(CH_2)_{0-3}-C_{6-18}$Aryl and $-(CH_2)_{0-3}$-5-18 membered heteroaryl, wherein the $C_{1-6}$ Alkyl, $C_{1-6}$Alkoxy, haloalkyl, hydroxyalkyl, $-(CH_2)_{0-3}-C_{3-14}$Cycloalkyl, $-(CH_2)_{0-3}$-3-14 membered heterocyclyl, $-(CH_2)_{0-3}-C_{6-18}$ Aryl or $-(CH_2)_{0-3}$-5-18 membered heteroaryl can be optionally substituted with one or more $R_c$;

$R_c$ is independently selected from the group consisting of halogen, $C_{1-6}$ Alkyl, $C_{1-6}$ Alkoxy, haloalkyl, cyano, amino, nitro, hydroxyl, hydroxyalkyl, $-C_{0-3}$Alkylene-C(=O)$R_b$, $-C_{0-3}$ Alkylene-C(=O)N$(R_b)_2$, $-(CH_2)_{0-3}N(R_b)_2$, $-O(CH_2)_{0-3}C_{3-14}$Cycloalkyl, $-O(CH_2)_{0-3}$-3-14 membered heterocyclyl, $C_{3-14}$ Cycloalkyl, 3-14 membered heterocyclyl, $C_{6-18}$ aryl and 5-18 membered heteroaryl;

n is 1 or 2.

In some embodiments, $R_4$ or $R_5$ is in formula (I) with the atom directly connected to the A ring form $C_{3-14}$ Cycloalkyl, 3-14 membered heterocyclyl, $C_{6-18}$ Aryl or 6-18 membered heteroaryl, wherein the $C_{3-14}$ Cycloalkyl, 3-14 membered heterocyclyl, $C_{6-18}$ Aryl or 6-18 membered heteroaryl is optionally further substituted by one or more $R_d$; wherein the $R_d$ is selected from the group consisting of H, halogen, $C_{1-6}$ Alkyl, $C_{3-6}$ Cycloalkyl, $C_{1-6}$ haloalkyl, cyano, amino, nitro, hydroxy and hydroxyalkyl.

In some embodiments, L in formula (I) is a bond or NH, preferably NH.

In some embodiments, $R_1$ in formula (I) is selected from the group consisting of $C_{6-18}$ Aryl and 6-18 membered heteroaryl, wherein the $C_{6-18}$ Aryl or 6-18 membered heteroaryl is optionally further substituted by one or more $R_a$; $R_a$ is independently selected from the group consisting of H, hydroxyl, cyano, halogen, $C_{1-6}$ Alkyl, $C_{1-6}$ Haloalkyl, $-C_{0-3}$ Alkylene-O$R_b$, -OC(=O)$C_{1-6}$ Alkyl, $-C_{0-3}$ Alkylene-N$(R_b)_2$ ,$-C_{0-3}$ Alkylene-C(=O)$R_b$ ,$-C_{0-3}$ Alkylene-C(=O)O$R_b$ ,$-C_{0-3}$ Alkylene-C(=O)N$(R_b)_2$ ,$C_{2-6}$ Alkenyl and $C_{2-6}$ Alkynyl; each $R_b$ is selected from the group consisting of H, halogen, hydroxyl, cyano, $C_{1-6}$ Alkyl, $-(CH_2)_{0-3}$ $C_{1-6}$ Alkoxy, $C_{3-6}$ Cycloalkyl and $C_{1-6}$ Haloalkyl.

In some embodiments, $R_1$ in formula (I) is selected from the group consisting of

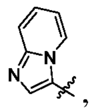,

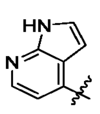, 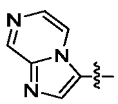, 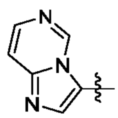, 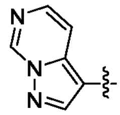, 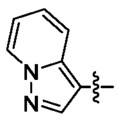, 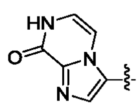, 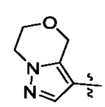, 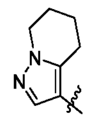, 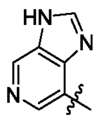,

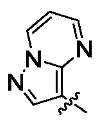 and 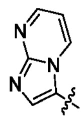,

wherein the

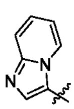, 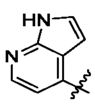, 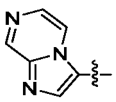, 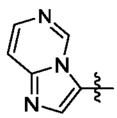, 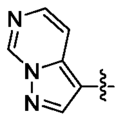, 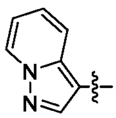,

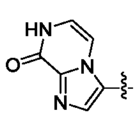, 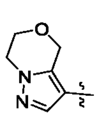, 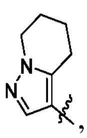, 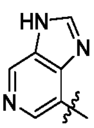, 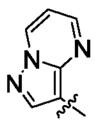 or 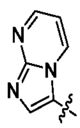

is substituted with one or more $R_a$. $R_a$ is independently selected from the group consisting of H, hydroxyl, cyano, halogen, $C_{1-6}$ Alkyl, $C_{1-6}$ Haloalkyl, $-C_{0-3}$ Alkylene-O$R_b$, -OC(=O)$C_{1-6}$ Alkyl, $-C_{0-3}$ Alkylene-N$(R_b)_2$, $-C_{0-3}$ Alkylene-C(=O)$R_b$, $-C_{0-3}$

Alkylene-C(=O)OR$_b$, -C$_{0-3}$ Alkylene-C(=O)N(R$_b$)$_2$, C$_{2-6}$ Alkenyl and C$_{2-6}$ Alkynyl; wherein each R$_b$ is independently selected from the group consisting of H, halogen, hydroxyl, cyano, C$_{1-6}$ Alkyl, -(CH$_2$)$_{0-3}$C$_{1-6}$ Alkoxy, C$_{3-6}$Cycloalkyl and C$_{1-6}$ Haloalkyl.

In some embodiments, R$_1$ in formula (I) is selected from the group consisting of

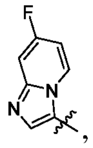,

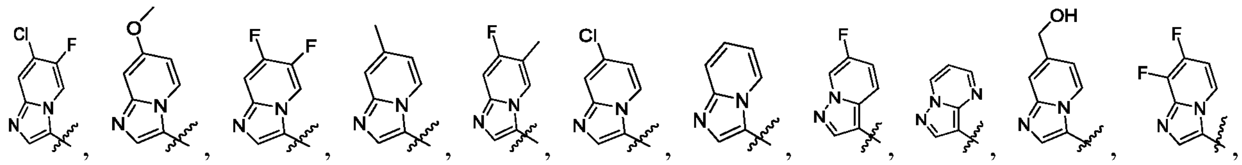,

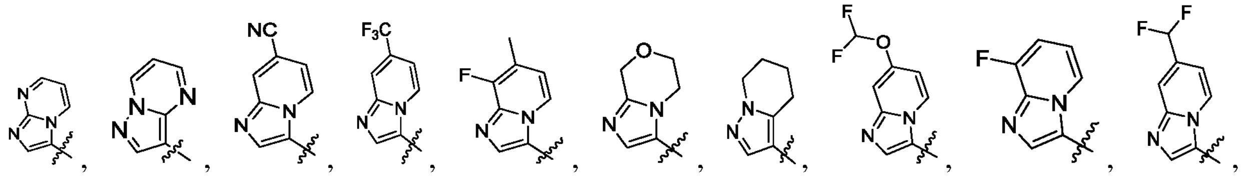,

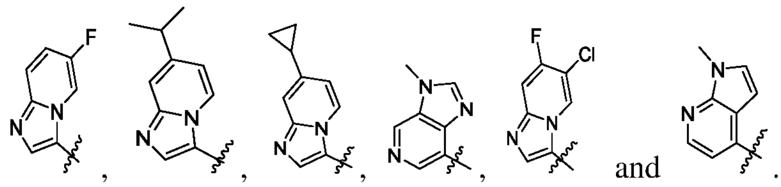.

In some embodiments, R$_1$ in formula (I) is

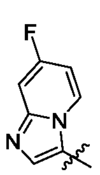 or 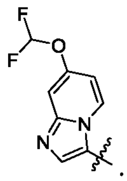.

**In** some embodiments, R$_2$ in formula (I) is H.

In some embodiments, Ring A in Formula (I) is selected from the group consisting of C$_{8-10}$ Bicarbocyclyl, 8-10 membered biheterocycloalkyl, C$_{10-12}$ Bicyclic aryl and $_{10-12}$ membered bicyclic heteroaryl, preferably selected from the group consisting of

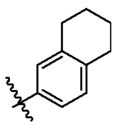, 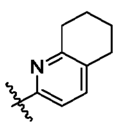,

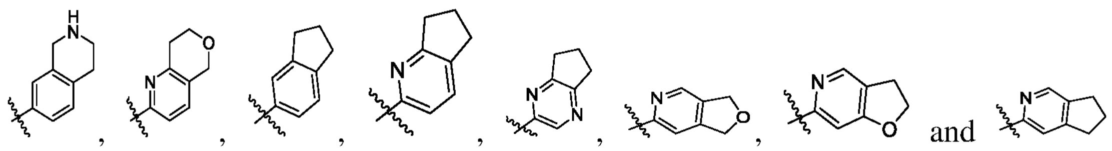.

In some embodiments, Ring A in Formula (I) is selected from the group consisting of C$_{8-10}$ Bicarbocyclyl, 8-10

membered biheterocycloalkyl, $C_{10-12}$ Bicyclic aryl and 10-12 membered bicyclic heteroaryl, preferably selected from the group consisting of

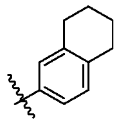, 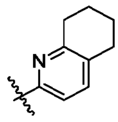,

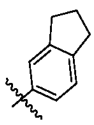, 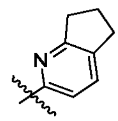, 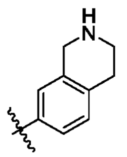 and 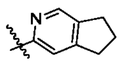.

In some embodiments, $R_3$ in formula (I) is selected from the group consisting of H, halogen, cyano, $C_{1-6}$ Alkyl, $C_{1-6}$ Alkoxy, haloalkyl, hydroxyl, hydroxyalkyl, $C_{2-6}$ Alkenyl, $C_{2-6}$ Alkynyl, $-(CH_2)_{0-3}-C_{3-14}$ Cycloalkyl, $-(CH_2)_{0-3}$-3-14 membered heterocyclyl and $-(CH_2)_{0-3}$ $N(R_b)_2$, wherein the $C_{1-6}$ Alkyl, $C_{1-6}$ Alkoxy, $-(CH_2)_{0-3}-C_{3-14}$ Cycloalkyl, $-(CH_2)_{0-3}$-3-14 membered heterocyclyl, haloalkyl, hydroxyalkyl, $C_{2-6}$ Alkenyl or $C_{2-6}$ Alkynyl can be optionally substituted with one or more $R_c$, the $R_c$ is independently selected from the group consisting of halogen, $C_{1-6}$ Alkyl, $C_{1-6}$ Alkoxy, haloalkyl, cyano, amino, nitro, hydroxyl, hydroxyalkyl, $C_{2-6}$ Alkenyl, $C_{2-6}$ Alkynyl, $-C_{0-3}$ Alkylene-$C(=O)R_b$, $-C_{0-3}$ Alkylene-$C(=O)N(R_b)_2$, $-(CH_2)_{0-3}N(R_b)_2$, $-O(CH_2)_{0-3}C_{3-14}$Cycloalkyl, $-O(CH_2)_{0-3}$-3-14 membered heterocyclyl, $C_{3-14}$Cycloalkyl, 3-14 membered heterocyclyl, $C_{6-18}$ aryl and 5-18 membered heteroaryl; each $R_b$ is independently selected from the group consisting of H, halogen, hydroxyl, cyano, $C_{1-6}$ Alkyl, $-(CH_2)_{0-3}C_{1-6}$ Alkoxy, $C_{3-6}$ Cycloalkyl and $C_{1-6}$ Haloalkyl.

In some embodiments, $R_3$ in formula (I) is H, $-(CH_2)_{0-3}N(R_b)_2$, cyano, $C_{2-6}$ alkenyl, $C_{2-6}$ alkynyl, $-(CH_2)_{0-3}$-3-14 membered heterocyclyl or $C_{1-6}$ alkyl, and wherein each $R_b$ is independently H, halogen, hydroxyl, cyano, $C_{1-6}$ alkyl, $C_{3-6}$ cycloalkyl, $-(CH_2)_{0-3}C_{1-6}$ alkoxy or $C_{1-6}$ haloalkyl.

In some embodiments, wherein the $R_4$, together with directly connected atoms on ring A, form a $C_{3-14}$ cycloalkyl, a 3-14 membered heterocyclyl, a $C_{6-18}$ aryl, or a 6-18 membered heteroaryl.

In some embodiments, Formula (I) is Formula (IA):

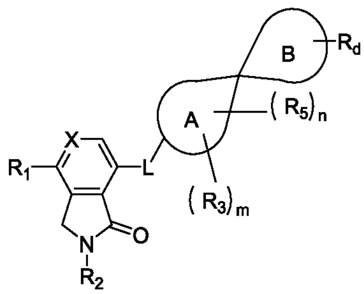

(IA)

wherein,

the ring B is selected from the group consisting of $C_{3-14}$ Cycloalkyl, 3-14 membered heterocycloalkyl, $C_{6-18}$ Aryl and 6-18 membered heteroaryl;

the definitions of $R_1$, $R_2$, $R_3$, $R_5$, X, L, A ring, $R_d$, m and n are the same as those described in formula (I).

In some embodiments, Ring A in Formula (IA) is selected from the group consisting of $C_{8-10}$ Bicarbocyclyl, 8-10 membered biheterocycloalkyl, $C_{10-12}$ Bicyclic aryl and 10-12 membered bicyclic heteroaryl.

In some embodiments, Ring A in Formula (IA) is selected from the group consisting of

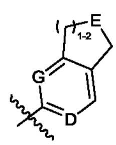, 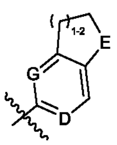 and 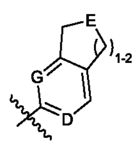,

wherein the D, E and G are each independently selected from the group consisting of C, N and O.

In some embodiments, Ring A in Formula (IA) is selected from the group consisting of

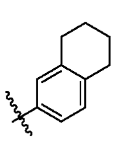, 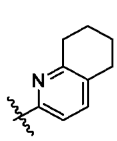, 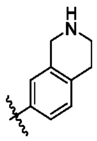, 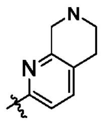, 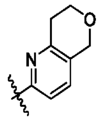, 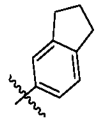, 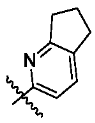, 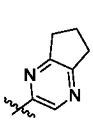, 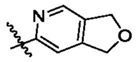,

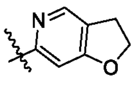, 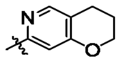 and 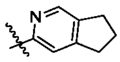.

In some embodiments,

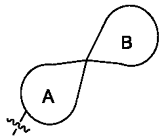

in formula (IA) is selected from the group consisting of

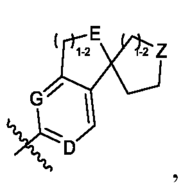, 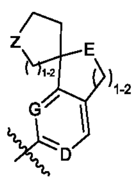, 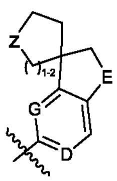, 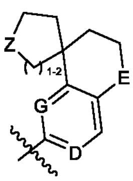, 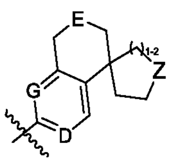 and 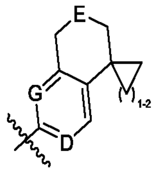,

wherein the D, E, G and Z are each independently selected from the group consisting of C, N and O.

In some embodiments,

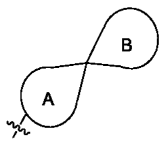

in formula (IA) is selected from the group consisting of

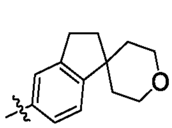, 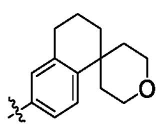, 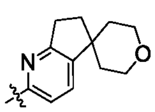, 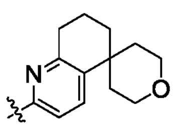, 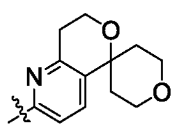, 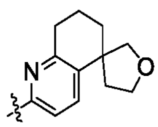,

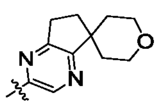, 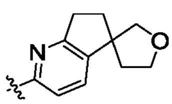, 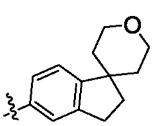, 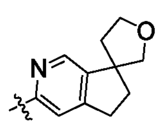, 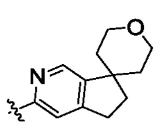, 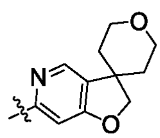,

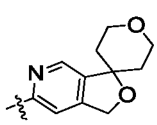, 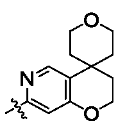, 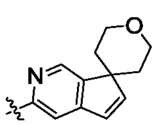, 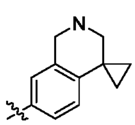, 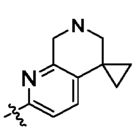, 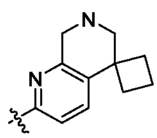, 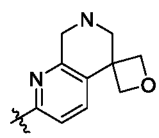,

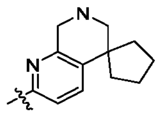, 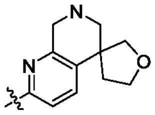, 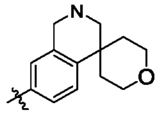, 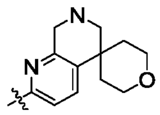 and 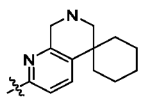.

In some embodiments, $R_3$ in formula (IA) is selected from the group consisting of H, halogen, $C_{1-6}$ alkyl, $C_{1-6}$ alkoxy, haloalkyl, $C_{2-6}$ alkenyl, $C_{2-6}$ alkynyl, $-(CH_2)_{0-3}-C_{3-14}$ cycloalkyl, $-(CH_2)_{0-3}$-3-14 membered heterocyclyl and $-(CH_2)_{0-3}N(R_b)_2$ , wherein the $C_{1-6}$ alkyl, $C_{1-6}$ alkoxy, $-(CH_2)_{0-3}-C_{3-14}$ cycloalkyl, $-(CH_2)_{0-3}$-3-14 membered heterocyclyl, haloalkyl or hydroxyalkyl can be optionally substituted with one or more $R_c$;

$R_c$ is independently selected from the group consisting of halogen, $C_{1-6}$ alkyl, $C_{1-6}$ alkoxy, haloalkyl, cyano, amino, nitro, hydroxy, hydroxyalkyl, $-C_{0-3}$ alkylene-C( = O)$R_b$, $-C_{0-3}$ alkylene-C( = O)N(R $_b$)$_2$, $-(CH_2)_{0-3}N(R_b)_2$, $-O(CH_2)_{0-3}$ $C_{3-14}$ cycloalkyl, $-O(CH_2)$ $_{0-3}$-3-14 membered heterocyclyl, $C_{3-14}$ cycloalkyl, 3-14 membered heterocyclyl, $C_{6-18}$ aryl and 5-18 membered heteroaryl, and each $R_b$ is independently selected from the group consisting of H, halogen, hydroxyl, cyano, $C_{1-6}$ alkyl, $-(CH_2)_{0-3}$ $C_{1-6}$ alkoxy, $C_{3-6}$ cycloalkyl and $C_{1-6}$ haloalkyl;

m is 0, 1, 2 or 3.

In some embodiments, $R_3$ in formula (IA) is selected from the group consisting of H, $-(CH_2)_{0-3}$ $N(R_b)_2$, cyano, $C_{2-6}$ alkenyl, $C_{2-6}$ alkynyl, $-(CH_2)_{0-3}$-3-14 membered heterocyclyl and $C_{1-6}$ alkyl, and wherein each $R_b$ is independently H, halogen, hydroxyl, cyano, $C_{1-6}$ alkyl, $C_{3-6}$ cycloalkyl, $-(CH_2)_{0-3}$ $C_{1-6}$ alkoxy or $C_{1-6}$ haloalkyl.

In some embodiments, $R_5$ in formula (IA) is $C_{1-6}$ alkyl, and n is 0 or 1.

In some embodiments, L in Formula (IA) is NH.

In some embodiments, $R_1$ in formula (IA) is selected from the group consisting of

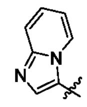,

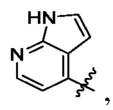, 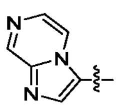, 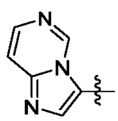, 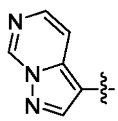, 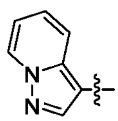, 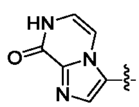, 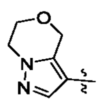, 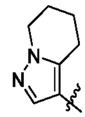, 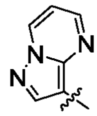

and

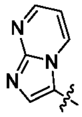,

wherein the

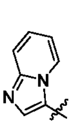, 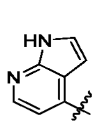, 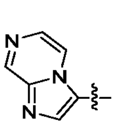, 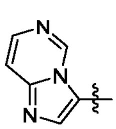, 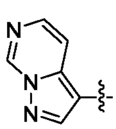, 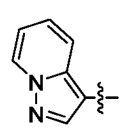, 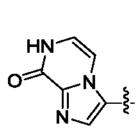,

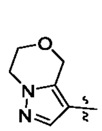, 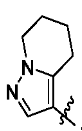, 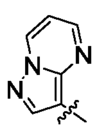 or 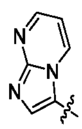

is further substituted by one or more $R_a$, $R_a$ is independently selected from the group consisting of H, hydroxyl, cyano, halogen, $C_{1-6}$ alkyl, $C_{1-6}$ haloalkyl, $-C_{0-3}$ alkylene-$OR_b$, -OC(= O)$C_{1-6}$ alkyl, $-C_{0-3}$ alkylene-$N(R_b)_2$, $-C_{0-3}$ alkylene-C(=O) $R_b$, $-C_{0-3}$ alkylene- C(=O)$OR_b$, $-C_{0-3}$ alkylene-C(=O)$N(R_b)_2$, $C_{2-6}$ alkenyl and $C_{2-6}$ alkynyl, each $R_b$ is independently selected from the group consisting of H, halogen, hydroxyl, cyano, $C_{1-6}$ alkyl, $-(CH_2)_{0-3}C_{1-6}$ alkoxy, $C_{3-6}$ cycloalkyl and $C_{1-6}$ haloalkyl.

In some embodiments, $R_1$ in formula (IA) is selected from the group consisting of

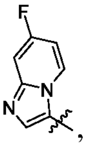,

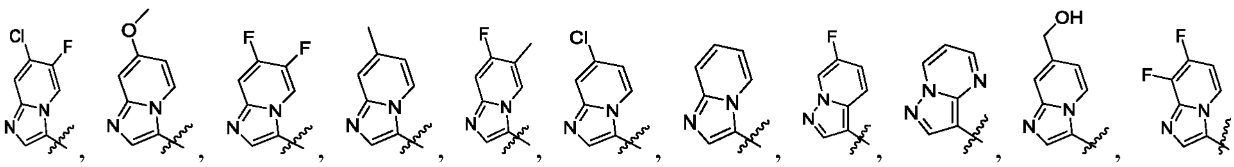, , , , , , , , , , ,

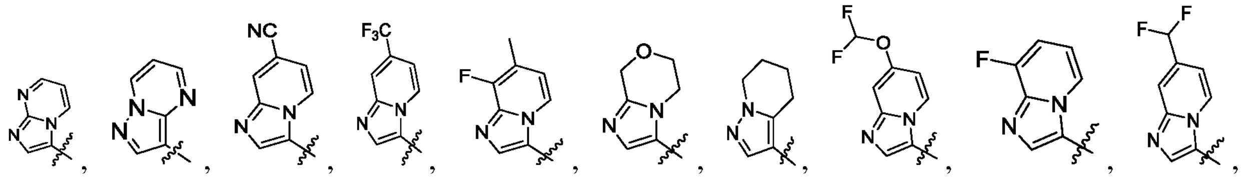, , , , , , , , , ,

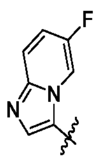, 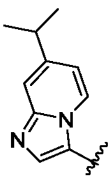, 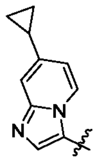, 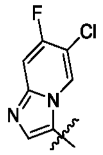 and 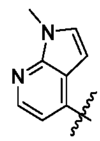.

In some embodiments, $R_2$ in formula (IA) is H.

In some embodiments,

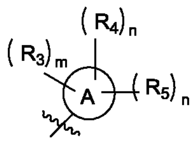

in formula (I) is selected from the group consisting of

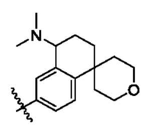, 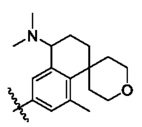, 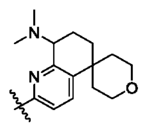, 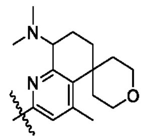, 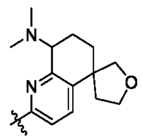, 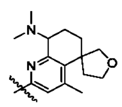, 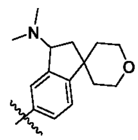,

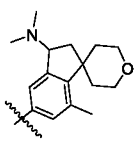, 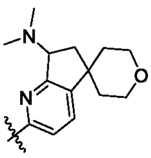, 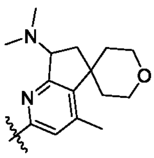, 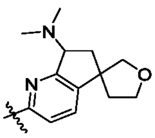, 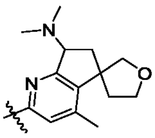, 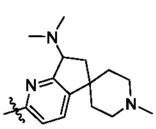, 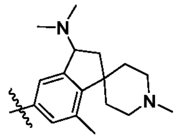,

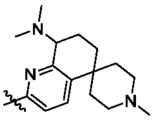, 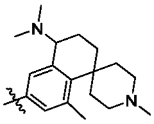, 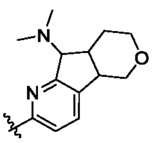, 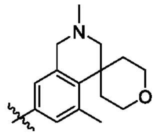, 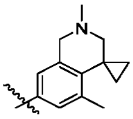, 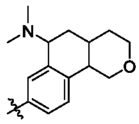, 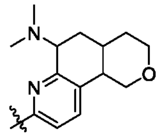,

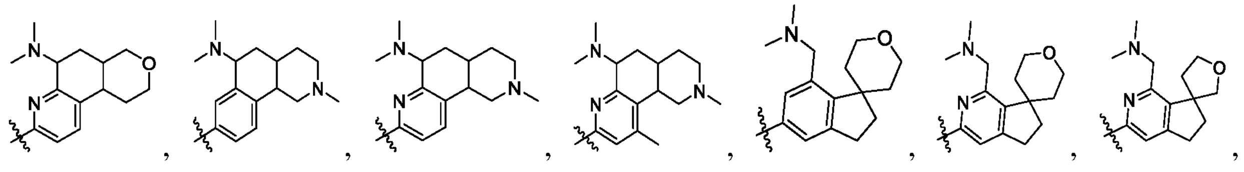
, , , , , , ,

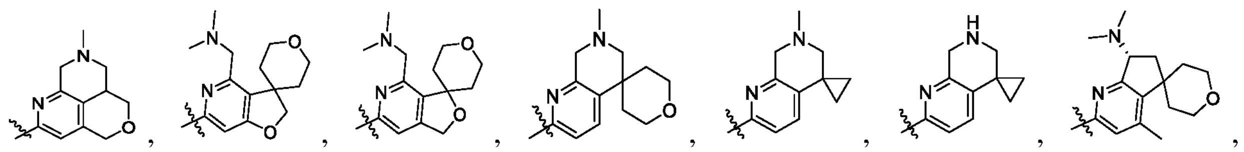
, , , , , , ,

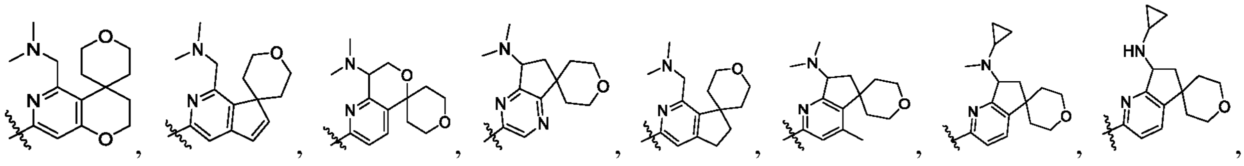
, , , , , , , ,

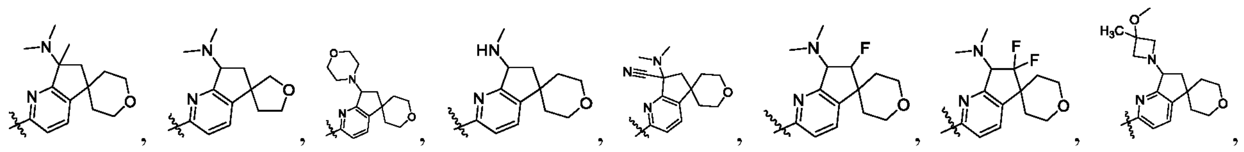
, , , , , , , ,

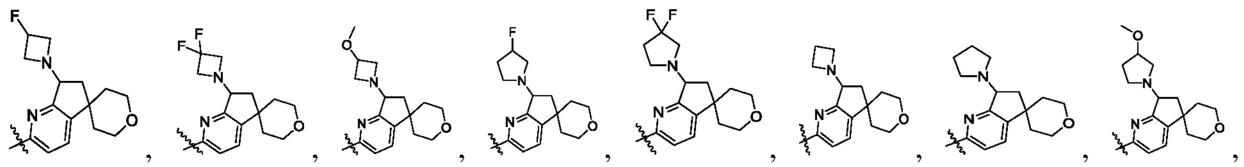
, , , , , , , ,

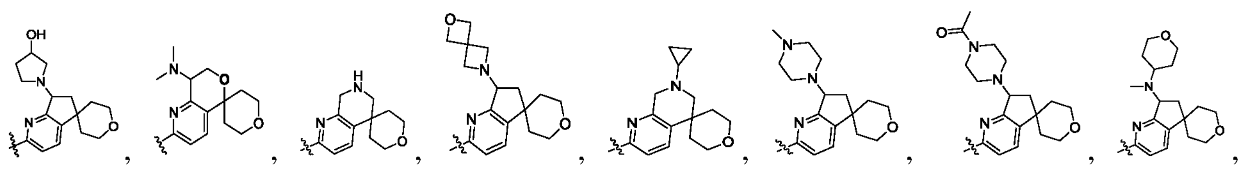
, , , , , , , ,

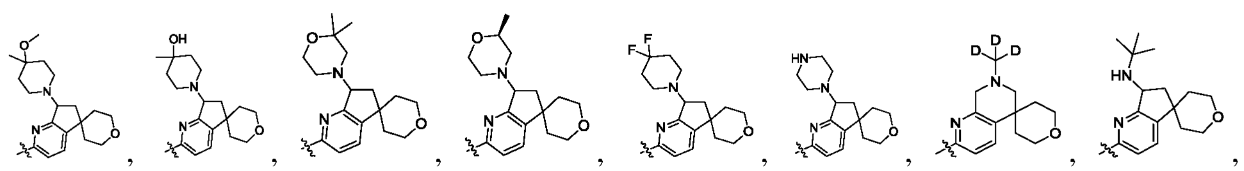
, , , , , , , ,

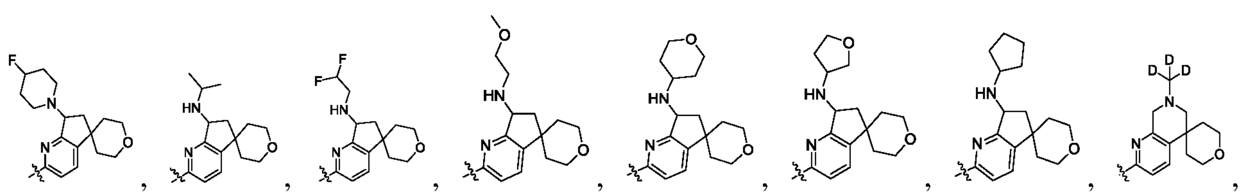
, , , , , , , ,

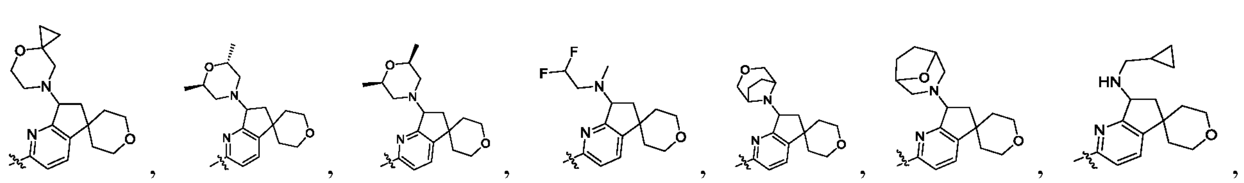
, , , , , , ,

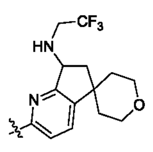, 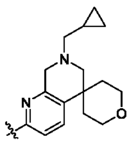, 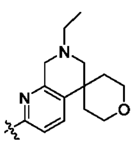, 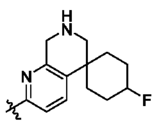, 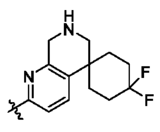, 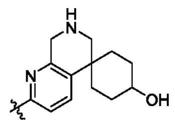, 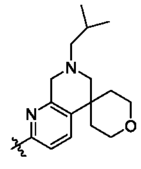,

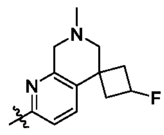, 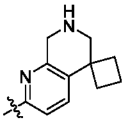 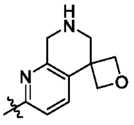, 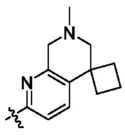, 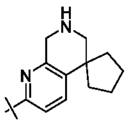, 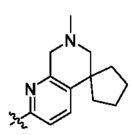, 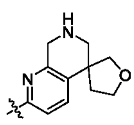, 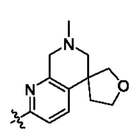,

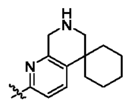, 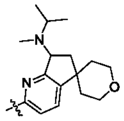, 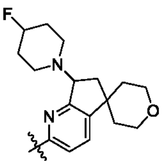, 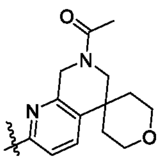, 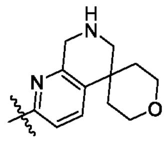, 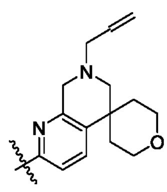, 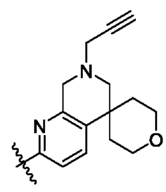,

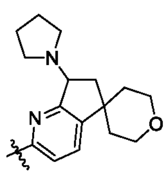 and 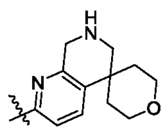.

In some embodiments, formula (I) is selected from the group consisting of:

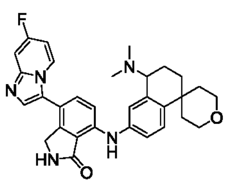
1

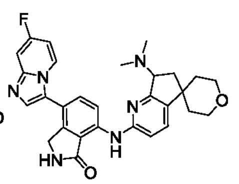
2

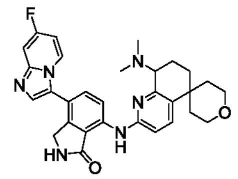
3

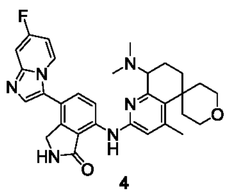
4

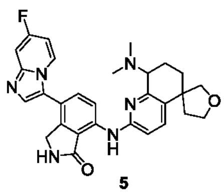
5

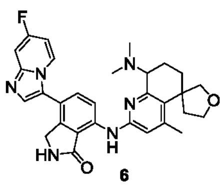
6

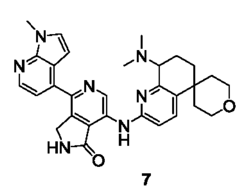
7

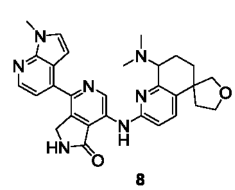
8

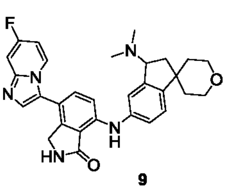
9

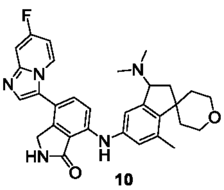
10

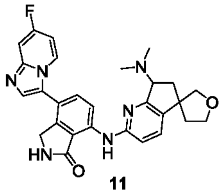
11

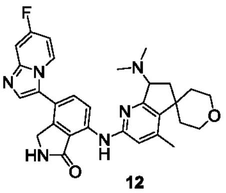
12

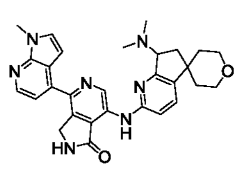
13

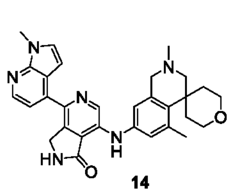
14

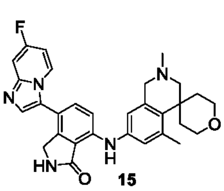
15

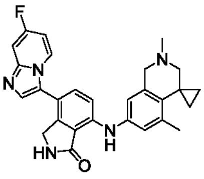
16
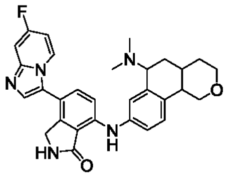
17
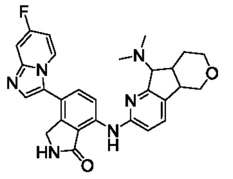
18
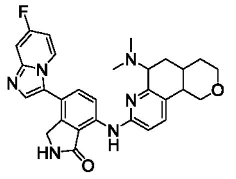
19
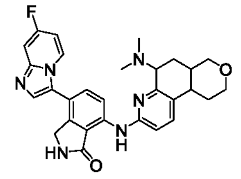
20

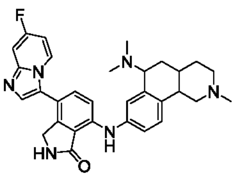
21
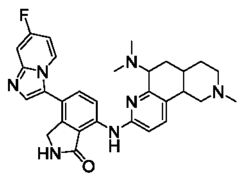
22
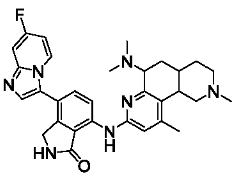
23
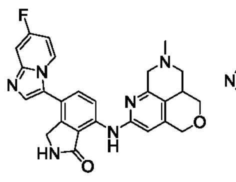
24
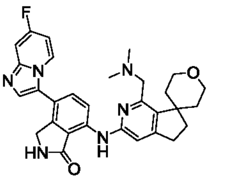
25

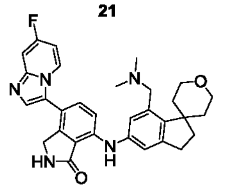
26
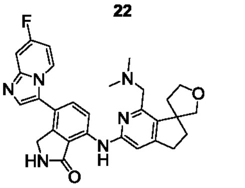
27
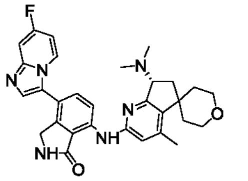
28
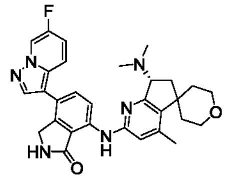
29
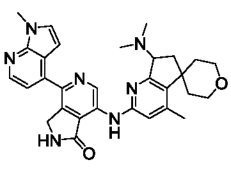
30

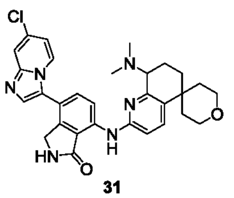
31
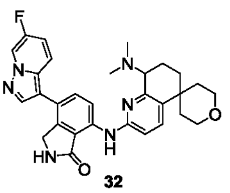
32
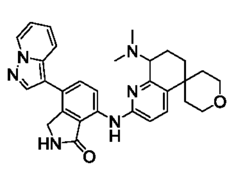
33
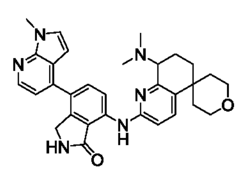
34
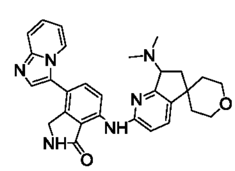
35

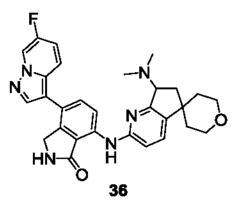
36
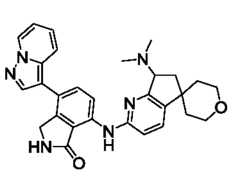
37
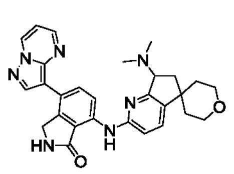
38
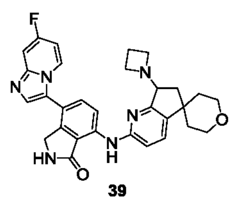
39
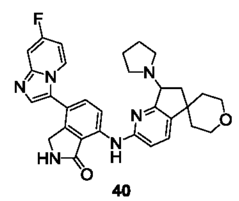
40

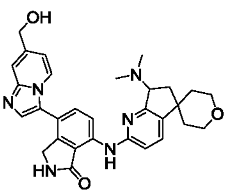
41
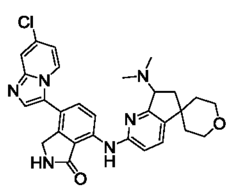
42
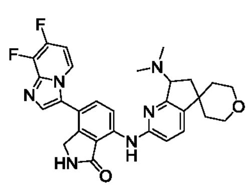
43
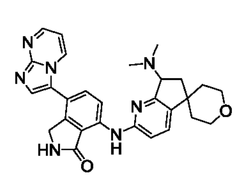
44
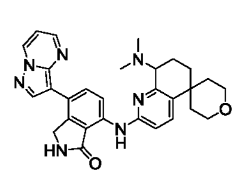
45

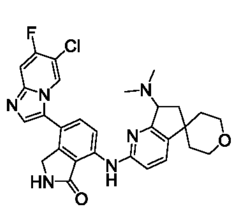
46
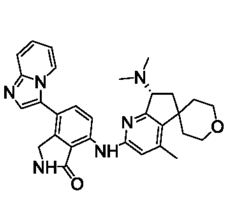
47
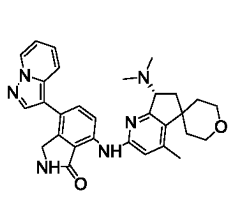
48
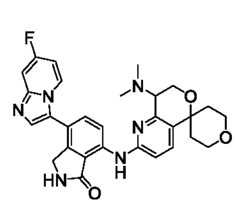
49
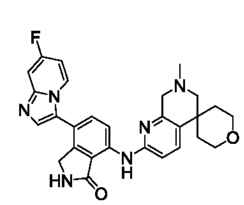
50

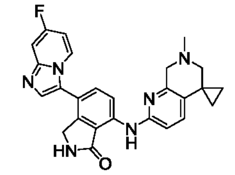
51

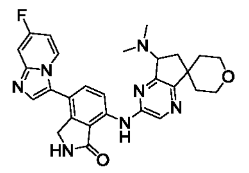
52

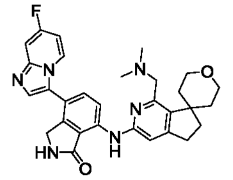
53

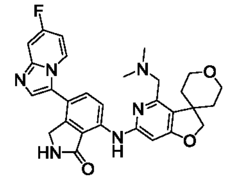
54

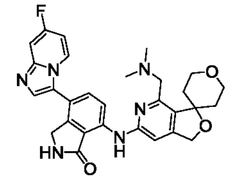
55

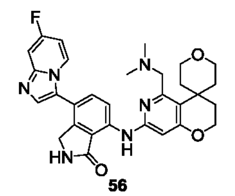
56

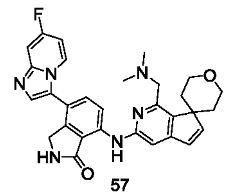
57

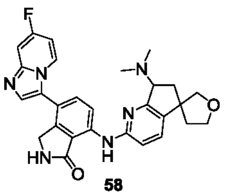
58

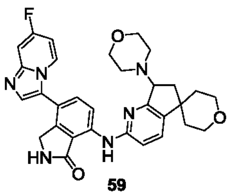
59

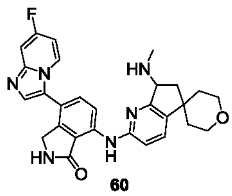
60

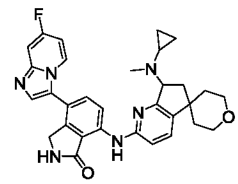
61

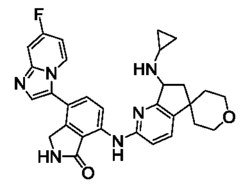
62

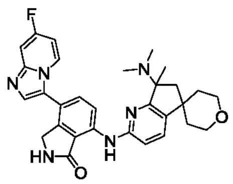
63

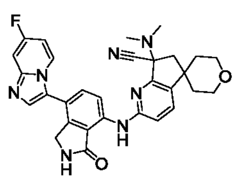
64

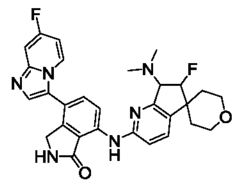
65

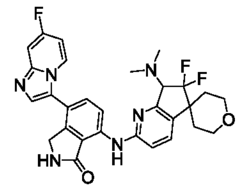
66

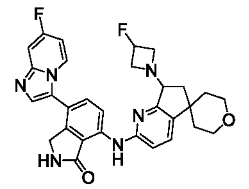
67

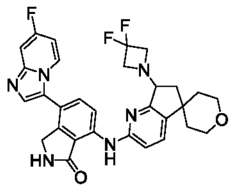
68

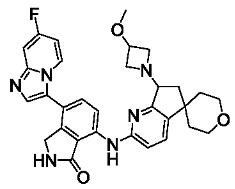
69

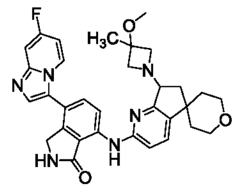
70

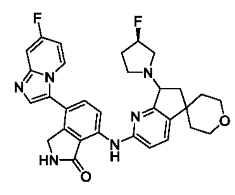
71

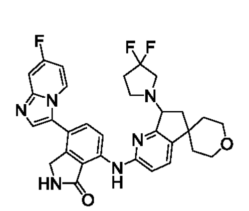
72

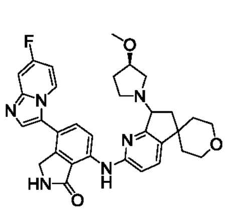
73

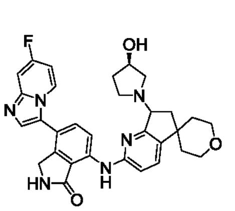
74

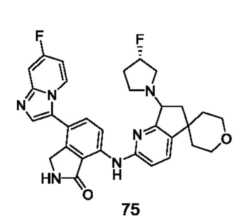
75

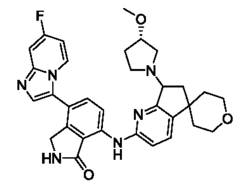
76

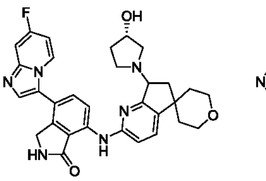
77

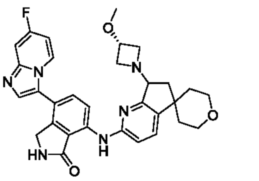
78

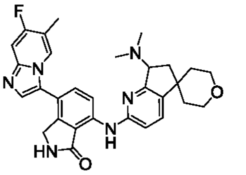
79

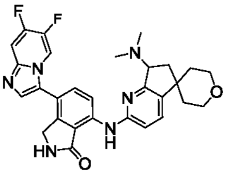
80

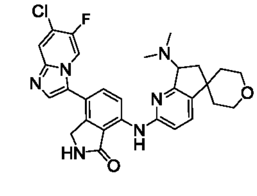
81

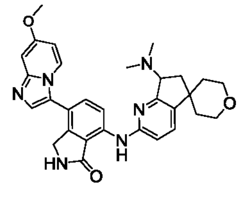
82

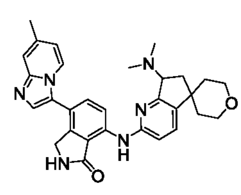
83

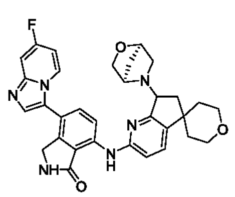
84

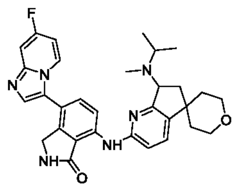
85

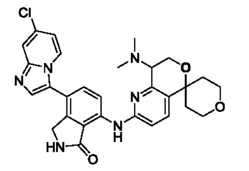

86

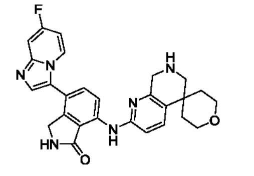

87

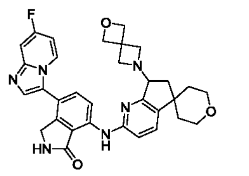

88

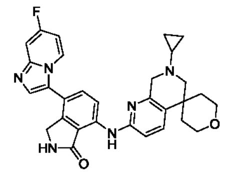

89

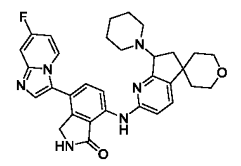

90

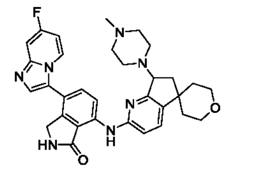

91

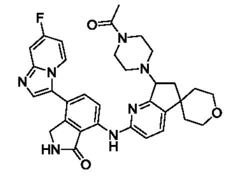

92

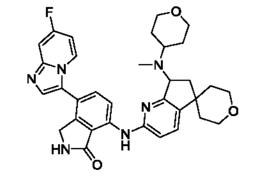

93

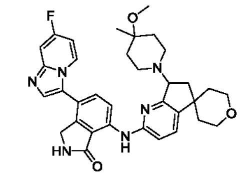

94

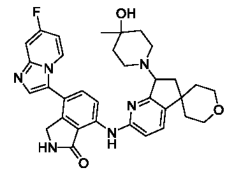

95

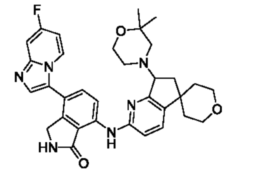

96

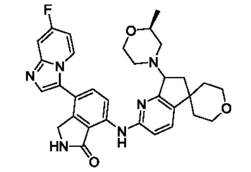

97

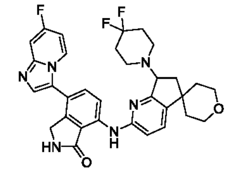

98

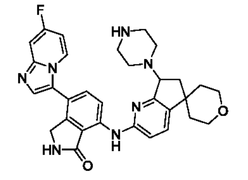

99

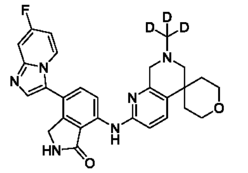

100

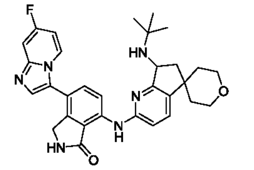

101

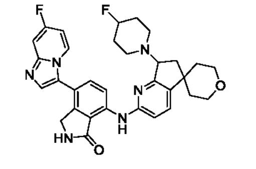

102

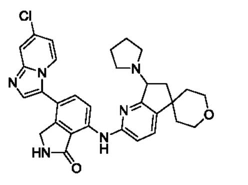

103

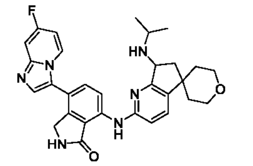

104

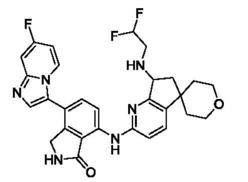

105

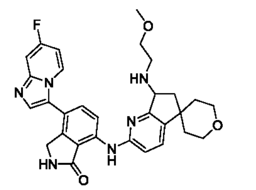

106

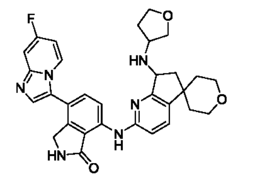

107

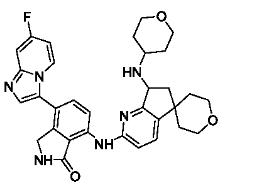

108

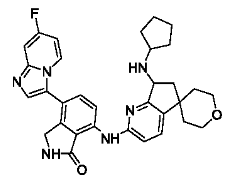

109

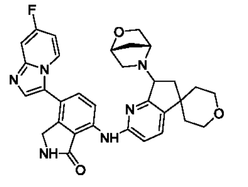

110

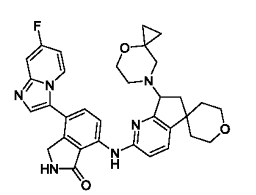

111

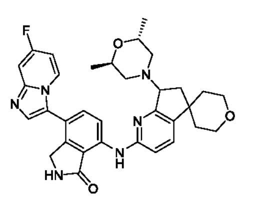

112

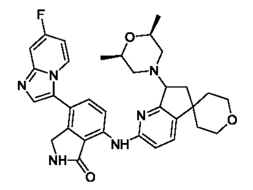

113

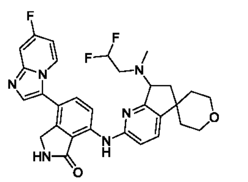

114

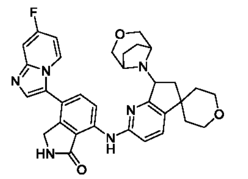

115

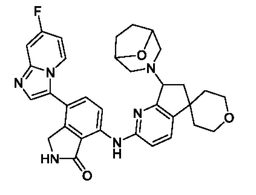

116

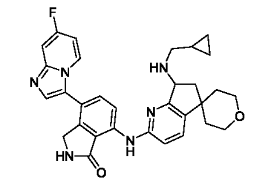

117

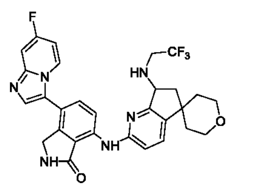

118

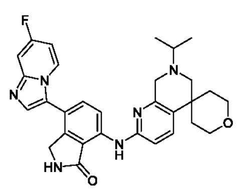

119

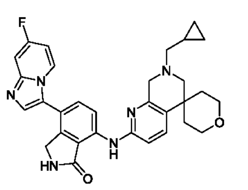

120

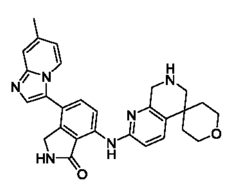
121

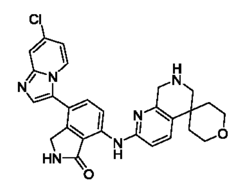
122

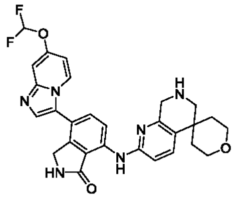
123

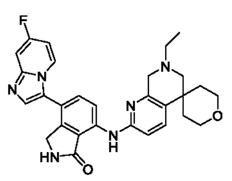
124

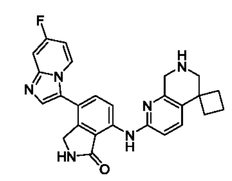
125

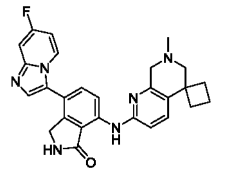
126

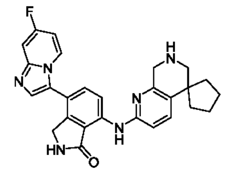
127

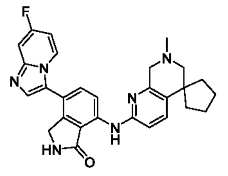
128

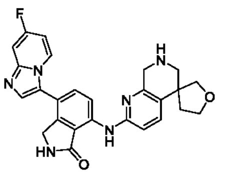
129

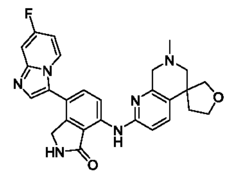
130

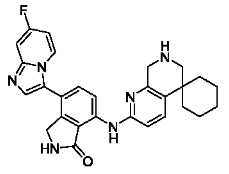
131

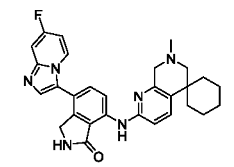
132

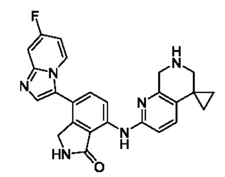
133

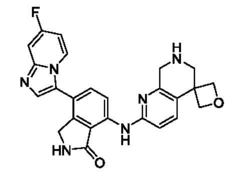
134

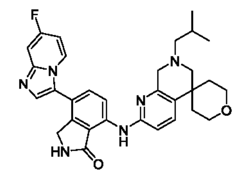
135

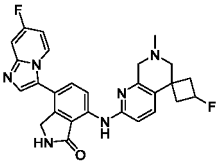
136

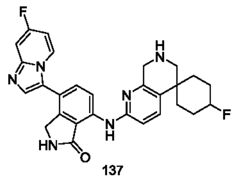
137

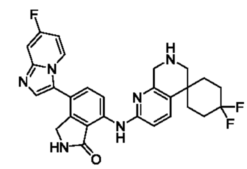
138

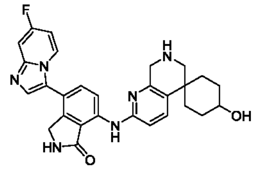
139

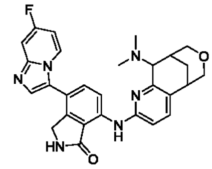
140

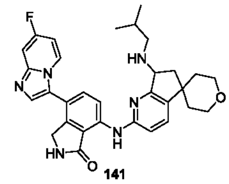
141

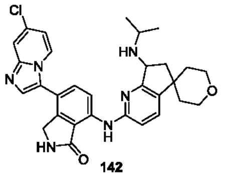
142

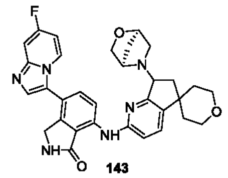
143

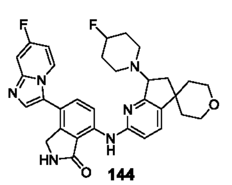
144

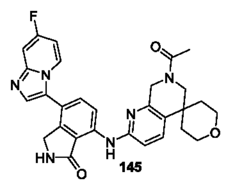
145

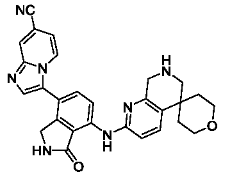
146

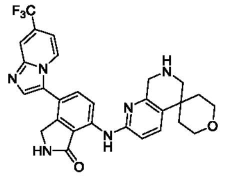
147

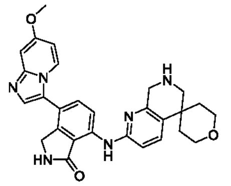
148

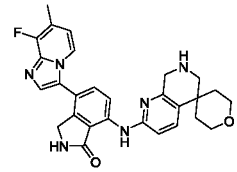
149

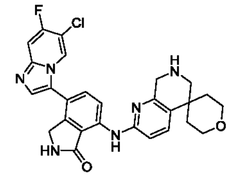
150

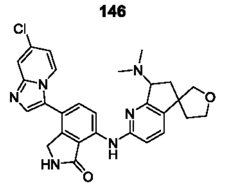
151

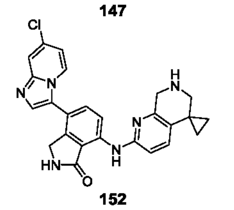
152

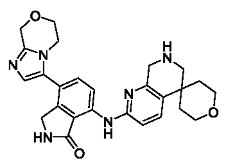
153

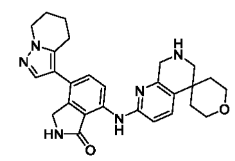
154

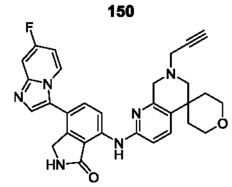
155

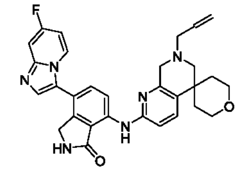
156

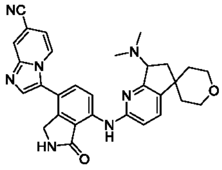
157

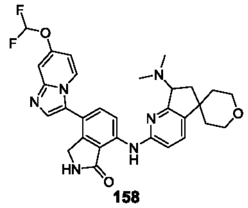
158

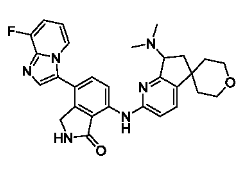
159

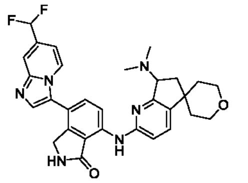
160

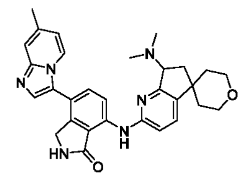
161

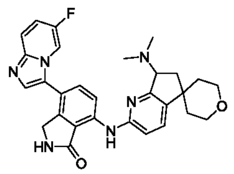
162

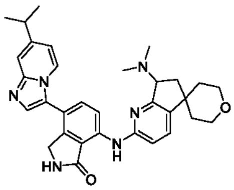
163

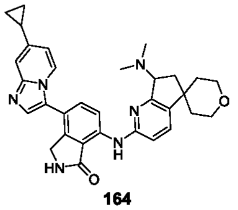
164

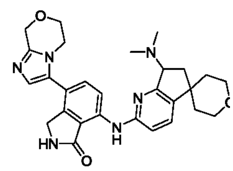
165

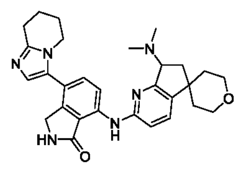
166

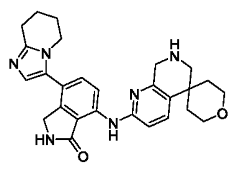
167

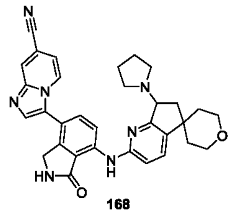
168

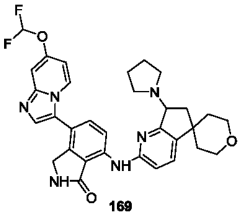
169

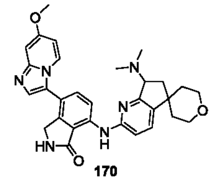
170

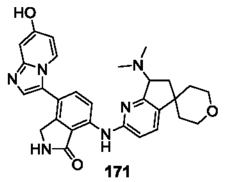
171

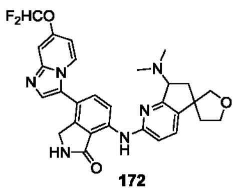
172

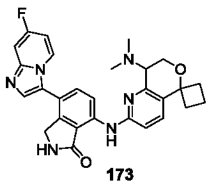
173

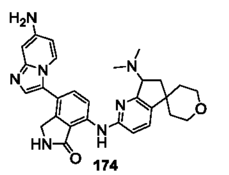
174

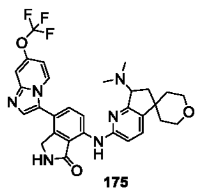
175

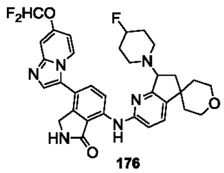
176

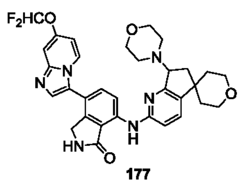
177

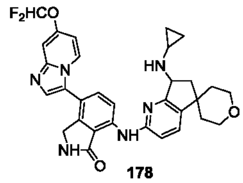
178

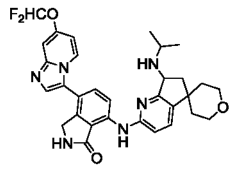
179

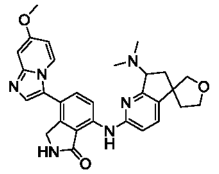
180

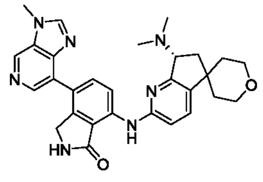
181

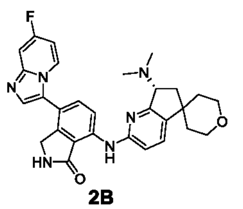
2B

and

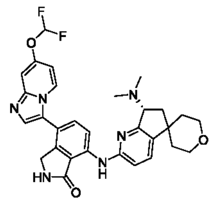
158A

.

On the other hand, the present invention provides the synthesis of the compound described in claim formula (I) or its stereoisomer, tautomer, deuterated substance, or pharmaceutically acceptable salt, wherein the intermediate compound is selected from the group consisting of:

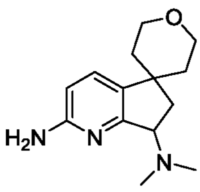 M3 , 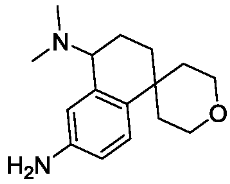 M4 , 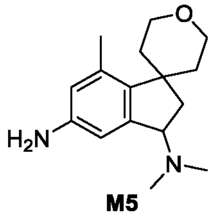 M5 , 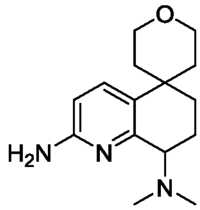 M7 , 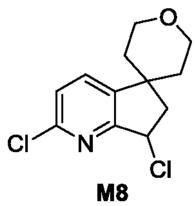 M8 ,

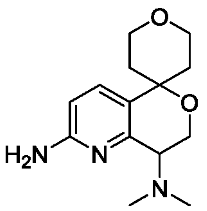 M11 , 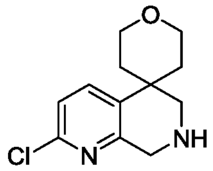 M12 , 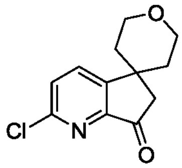 M15-3 , 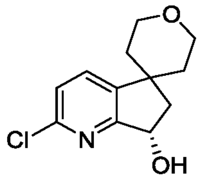 M15-4 and 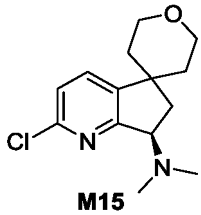 M15 .

The compound of the present invention can effectively inhibit the function of HPK1 protein, induce the secretion of IL2, IFNγ and other cytokines in T cells, and activate the immune system. On the other hand, this type of compound has excellent oral absorption in various animal models. It can significantly inhibit the growth of tumor cells in mouse tumor models, and its efficacy is much higher than that of HPK1 inhibitors currently in the clinical stage. Secondly, the compound of the present invention has the advantages of strong selectivity, low toxicity, and large safety window, and is expected to achieve better clinical efficacy in the future.

The present invention also provides a pharmaceutical composition, wherein the pharmaceutical composition contains a therapeutically effective amount of at least one compound represented by formula (I), its stereoisomer, tautomer, deuterated substance or Medicinal salt.

The present invention provides the use of the compound represented by structural formula (I), its stereoisomer, tautomer, deuterated substance or pharmaceutically acceptable salt, or its pharmaceutical composition in the preparation of medicines.

The present invention further provides preferred technical solutions for the application: Preferably, the application is in the preparation of drugs for the treatment and/or prevention of cancer.

Preferably, the application is the application of preparing drugs for treating diseases mediated by HPK1. Preferably, the disease is cancer.

Preferably, the cancer is selected from the group consisting of breast cancer, multiple myeloma, bladder cancer, endometrial cancer, gastric cancer, cervical cancer, rhabdomyosarcoma, non-small cell lung cancer, small cell lung cancer, polymorphic lung cancer, ovarian cancer, esophageal cancer Carcinoma, melanoma, colorectal cancer, hepatoma, head and neck tumors, cholangiocarcinoma, myelodysplastic syndrome, glioblastoma, prostate cancer, thyroid cancer, Schwann cell tumor, lung squamous cell carcinoma, lichenoid keratosis, synovial sarcoma, skin cancer, pancreatic cancer, testicular cancer, and liposarcoma.

The present invention also provides a method for treating and/or preventing diseases, which includes administering to a treatment subject a therapeutically effective amount of at least any compound represented by structural formula (I) or a pharmaceutical composition containing the same.

The present invention also provides a method for treating and/or preventing diseases mediated by HPK1, which includes administering to a treatment subject a therapeutically effective amount of at least any compound represented by structural formula (I) or a pharmaceutical composition containing the same.

The present invention also provides a method for treating cancer, which includes administering a therapeutically effective amount of at least any compound represented by structural formula (I) or a pharmaceutical composition containing the same to a treatment subject.

Preferably, in the above method, the HPK1-mediated disease is cancer.

Preferably, in the above method, the cancer is selected from the group consisting of breast cancer, multiple myeloma, bladder cancer, endometrial cancer, gastric cancer, cervical cancer, rhabdomyosarcoma, non-small cell lung cancer, small cell lung cancer, polymorphic Lung cancer, ovarian cancer, esophageal cancer, melanoma, colorectal cancer, hepatoma, head and neck tumors, hepatocholangiocarcinoma, myelodysplastic syndrome, malignant glioma, prostate cancer, thyroid cancer, Xuwang cell tumour, lung squamous cell carcinoma, lichenoid keratosis, synovial sarcoma, skin cancer, pancreatic cancer, testicular cancer and liposarcoma.

Unless otherwise stated, general chemical terms used in the structural formulas have their ordinary meanings.

For example, unless otherwise stated, the term "halogen" as used herein refers to fluorine, chlorine, bromine or iodine.

In the present invention, unless otherwise stated, "alkyl" includes linear or branched monovalent saturated hydrocarbon groups. For example, alkyl groups include methyl, ethyl, propyl, isopropyl, n-butyl, isobutyl, sec-butyl, tert-butyl, n-pentyl, 3-(2-methyl)butyl, 2 -Pentyl, 2-methylbutyl, neopentyl, n-hexyl, 2-hexyl, 2-methylpentyl, etc. Similarly, the "$_{1-6}$" in "$C_{1-6}$ alkyl" refers to a group containing 1, 2, 3, 4, 5 or 6 carbon atoms in a straight chain or branched form..

"Alkoxy" refers to the oxygen ether form of the aforementioned linear or branched alkyl, that is, -O-alkyl.

The term "alkylene" refers to a divalent alkyl linking group. Alkylene formally refers to an alkane in which two C-H bonds are replaced by the alkylene's point of attachment to the rest of the compound. Similarly, "$C_{1-3}$ " in $C_{1-3}$ alkylene refers to an alkylene group containing 1, 2 or 3 carbon atoms, including but not limited to methylene, 1,2-ethylene, 1,3-propylene or 1,2-isopropylene.

The term "haloalkyl" refers to an alkyl group in which one or more H has been replaced by a halogen atom.

The term "oxo" or "oxo group" refers to an oxygen atom in the form of a divalent substituent, which forms a carbonyl group when attached to C, and forms a sulfoxide or sulfone group or an N-oxide group when attached to a heteroatom.

In the present invention, unless otherwise specified, the term "aromatic ring", "aromatic ring" or "aromatic heterocycle" refers to a polyunsaturated carbon ring or heterocycle with aromatic characteristics (having (4n+2) delocalized $\pi$ electrons, where n is an integer).

The term "aryl", as used herein, unless otherwise specified, refers to unsubstituted or substituted monocyclic or condensed ring aromatic groups comprising carbon ring atoms. Preferably, $C_{6-18}$ aryl is a monocyclic or bicyclic aromatic ring group, and more preferably, aryl is $C_{6-10}$. Preferred are phenyl and naphthyl. Most preferred is phenyl. The aryl ring may be fused to a heteroaryl, heterocyclyl or cycloalkyl group, wherein the ring attached to the parent structure is an aryl ring, non-limiting examples include but are not limited to benzocyclopentyl.

The term "heterocyclyl" refers to a ring system having at least one cyclized alkyl or cyclized alkenyl group containing a heteroatom selected from the group consisting of N, O and S.The heterocyclic group may include a single ring or multiple rings (eg, having 2, 3 or 4 fused rings, spiro rings, bridged rings, etc.). The heterocyclic group can be connected to other parts of the compound through a ring-forming carbon atom or a ring-forming heteroatom. A 3-14-membered heterocyclic group is preferred, wherein the "3-14-membered" heterocyclic group refers to a heterocyclic group consisting of 3-14 C, N, O or S ring atoms; a 3-8-membered heterocyclic group is more preferred, and a 3-6-membered heterocyclic group is even more preferred. The nitrogen or sulfur heteroatoms may be selectively oxidized, and the nitrogen heteroatoms may be selectively quaternized. Examples of such heterocyclic groups include, but are not limited to, azetidinyl, pyrrolidinyl, piperidinyl, piperazinyl, oxopiperazinyl, oxopiperidinyl, tetrahydrofuranyl, dioxolanyl, tetrahydroimidazolyl, tetrahydrothiazolyl, tetrahydrooxazolyl, tetrahydropyranyl, morpholinyl, thiomorpholinyl, thiomorpholinyl sulfoxide, thiomorpholinyl sulfone, and tetrahydrooxadiazolyl. The heterocyclyl group may be fused to an aryl, heteroaryl or cycloalkyl ring, wherein the ring attached to the parent structure is the heterocyclyl group.

The term "heteroaryl", in the present invention, unless otherwise specified, refers to a monocyclic or polycyclic (for example, having 2, 3 or 4 fused rings, spiro rings, bridged rings, etc.) aromatic heterocycle having at least one heteroatom, wherein the heteroatom is selected from the group consisting of N, O and S, and wherein the nitrogen or sulfur heteroatom can be selectively oxidized, and the nitrogen heteroatom can be selectively quaternized. Preferred is a 5-18-membered heteroaryl group, wherein the "5-18-membered" in the 5-18-membered heteroaryl group refers to a heteroaryl group containing 5 to 18 C, N, O or S atoms forming a ring. More preferred is a 5-10-membered heteroaryl group; even more preferred is a 5-6-membered heteroaryl group.

Examples of heteroaryl groups include, but are not limited to, thienyl, furanyl, imidazolyl, isoxazolyl, oxazolyl, pyrazolyl, pyrrolyl, thiazolyl, thiadiazolyl, triazolyl, pyridinyl, pyridazinyl, indolyl, azaindolyl, indazolyl, benzimidazolyl, benzofuranyl, benzothienyl, benzisoxazolyl, benzothiazolyl, benzothiazolyl, benzothiadiazolyl, benzotriazolyladenine, quinolinyl, or isoquinolinyl. The heteroaryl group may be fused to an aryl, heterocyclyl or cycloalkyl ring, wherein the ring attached to the parent structure is the heteroaryl ring.

The term "cycloalkyl" refers to a ring system having at least one cyclized alkyl group. Preferred is $C_{3-14}$ cycloalkyl, wherein "$C_{3-14}$ " means that the cycloalkyl may have 3, 4, 5, 6, 7, 8, 9, 10, 11, 12, 13 or 14 ring atoms. Cycloalkyl groups can include monocyclic and polycyclic rings (eg, having 2, 3, or 4 fused rings, spiro rings, bridged rings, etc.). In some embodiments, cycloalkyl includes but is not limited to cyclopropyl, cyclobutyl, cyclopentyl, etc.; the cycloalkyl can also be fused to an aryl, heterocyclic or heteroaryl ring, wherein the ring connected to the parent structure is a cycloalkyl.

The term "bicarbocyclic group" refers to a monovalent or polyvalent, non-aromatic, saturated or partially unsaturated ring that does not contain heteroatoms, including a bicyclic ring of 5 to 14 carbon atoms, one of which may be aromatic, but the bicarbocyclic group as a whole does not have aromaticity. Preferred are bicyclic carbocycles of 7-12 atoms. Bicyclic carbocycles of 7-12 atoms may be bicyclic [4,5], [5,5], [5,6] or [6,6] systems, while bicyclic carbocycles of 9 or 10 atoms may be bicyclic [5,6] or [6,6] systems. Suitable bicyclic carbocyclyl groups include, but are not limited to, fused bicyclic groups, spiro bicyclic groups and bridged bicyclic groups. Examples further include, but are in no way limited

to, fused bicyclo[3.3.0]octanyl, fused bicyclo[3.1.0]hexanyl, 1,2,3,4,4a,5,8,8a-octahydronaphthyl, bicyclo[2.2.1]heptanyl, and the like. One or more ring hydrogen atoms are independently optionally replaced with one or more substituents described herein.

The term "biheterocyclyl" refers to a bicyclic ring system in which one or more atoms in the ring are independently optionally substituted with heteroatoms, the ring may be fully saturated or contain one or more unsaturations, one of the rings may be aromatic, but the biheterocyclyl as a whole is not aromatic. The heterocyclic ring system may be attached to the main structure at any heteroatom or carbon atom which results in the formation of a stable compound. One or more ring hydrogen atoms are independently optionally replaced with one or more substituents described herein. Biheterocyclyl groups include, but are not limited to, fused heterobicyclyls, spiro heterobicyclyls and bridged heterobicyclyls. In some embodiments, each ring system of the bicyclic heterocyclic group contains a ring consisting of 3-7 atoms, i.e., 1-6 carbon atoms and 1-3 heteroatoms selected from the group consisting of N, O, P and S, wherein S or P is optionally substituted by one or more oxygen atoms to obtain groups such as SO, $SO_2$, PO, $PO_2$ . In some embodiments, the biheterocyclyl includes heterocyclyl-fused heterocyclyl, heterocyclyl-fused carbocyclyl, heterocyclyl-fused heteroaryl or heterocyclyl-fused aryl, connected to the main structure through the carbocyclyl, heteroaryl or heterocyclyl.

The term "bicyclic aryl" refers to a bicyclic carbocyclic ring system, wherein the bicyclic aromatic ring system is fused to form a ring, the bicyclic aryl ring system as a whole is aromatic, wherein each ring system contains 5 to 9 atoms. Naphthyl may be included. One or more ring hydrogen atoms are independently optionally replaced with one or more substituents described herein.

The term "bicyclic heteroaryl" is a bicyclic ring system wherein the bicyclic heteroaromatic ring systems are fused to form a ring. In which, the bicyclic heteroaromatic ring system is aromatic as a whole, and one or more atoms on the ring are independently optionally substituted by heteroatoms (the heteroatoms are selected from the group consisting of N, O, P and S, where S or P is optionally replaced by one or more oxygen atoms to obtain groups such as SO, $SO_2$, PO, $PO_2$). The bicyclic heteroaromatic system may be attached to the main structure at any heteroatom or carbon atom which forms a stable compound. The bicyclic heteroaromatic system radical can be a bicyclic ring consisting of 7 to 15 atoms, or a bicyclic ring consisting of 7 to 10 atoms. Bicyclic rings having 7-10 atoms may be bicyclic [4,5], [5,5], [5,6] or [6,6] systems. One or more ring hydrogen atoms are independently optionally replaced with one or more substituents described herein.

The term "substituted" means that one or more hydrogen atoms in the group are replaced by the same or different substituents, respectively. Typical substituents include, but are not limited to, halogen (F, Cl, Br or I), $C_{1-8}$ Alkyl, $C_{3-12}$ Cycloalkyl, $-OR^1$ ,$-SR^1$, =O, =S, $-C(O)R^1$, $-C(S)R^1$, $=NR^1$, $-C(O)OR^1$, $-C(S)OR^1$, $-NR^1R^2$ ,$-C(O)NR^1R^2$, cyano, nitro, $-S(O)_2R^1$,$-O-S(O_2)OR^1$, $-O-S(O)_2R^1$, $-OP(O)(OR^1)(OR^2)$; where $R^1$ and $R^2$ Independently selected from the group consisting of -H, $C_{1-6}$ Alkyl, $C_{1-6}$ Haloalkyl and $C_{3-6}$ Cycloalkyl. In some embodiments, the substituents are independently selected from the group consisting of -F, -Cl, -Br, -I, -OH, trifluoromethoxy, ethoxy, propoxy, isopropoxy, n-butoxy base, isobutoxy, tert-butoxy, $-SCH_3$, $-SC_2H_5$, formaldehyde group, $-C(OCH_3)$, cyano, nitro, $-CF_3$, $-OCF_3$, amino, dimethylamino, methylthio, sulfonyl and acetyl groups.

Examples of substituted alkyl groups include, but are not limited to, 2,3-dihydroxypropyl, 2-aminoethyl, 2-hydroxyethyl, pentachloroethyl, trifluoromethyl, methoxymethyl, pentafluoroethyl, phenylmethyl, dioxolylmethyl and piperazinylmethyl.

Examples of substituted alkoxy include, but are not limited to, 2-hydroxyethoxy, 2-fluoroethoxy, 2,2-difluoroethoxy, 2-methoxyethoxy, 2-aminoethoxy, 2,3-dihydroxypropoxy, cyclopropylmethoxy, aminomethoxy, trifluoromethoxy, 2-diethylaminoethoxy, 2-ethoxycarbonylethoxy, 3- Hydroxypropoxy.

When the number of a linking group is 0, for example $-(CH_2)_0-$ indicates that the linking group is a bond, $(R_3)0$ indicates that there is no $R_3$ substitution at that position, and $(R_5)_0$ indicates that there is no $R_5$ substitution at that position.

The term "pharmaceutically acceptable salts" refers to salts prepared from pharmaceutically acceptable non-toxic bases or acids.

When the compound provided by the present invention is an acid, its corresponding salt can be conveniently prepared from pharmaceutically acceptable non-toxic bases, including inorganic bases and organic bases. Salts derived from inorganic bases include aluminum, ammonium, calcium, copper (supervalent and subvalent), ferric, ferrous, lithium, magnesium, manganese (supervalent and subvalent), potassium, sodium, zinc and the like. Particularly preferred are the ammonium, calcium, magnesium, potassium and sodium salts. Non-toxic organic bases from which pharmaceutically acceptable salts can be derived include primary, secondary, and tertiary amines, including cyclic amines and substituted amines, such as naturally occurring and synthetic substituted amines. Other pharmaceutically acceptable non-toxic organic bases capable of forming salts include ion exchange resins and arginine, betaine, caffeine, choline, N', N'-dibenzylethylenediamine, diethylamine, 2-diethylaminoethanol, 2-dimethylaminoethanol, ethanolamine, ethylenediamine, N-ethylmorpholine, N-ethylpiperidine, reduced glucosamine, glucosamine, histidine, isopropylamine, lysine, methylglucamine, morpholine, piperazine, piperidine, polyamine resins, procaine, chloroprocaine, purine, theobromine, triethylamine, trimethylamine, tripropylamine, tromethamine, and the like.

When the compound provided by the present invention is a base, its corresponding salt can be conveniently prepared from pharmaceutically acceptable non-toxic acids, including inorganic acids and organic acids. Such acids

include, for example, acetic acid, benzenesulfonic acid, benzoic acid, camphorsulfonic acid, citric acid, ethanesulfonic acid, formic acid, fumaric acid, gluconic acid, glutamic acid, hydrobromic acid, hydrochloric acid, isethionic acid, lactic acid, maleic acid, malic acid, mandelic acid, methanesulfonic acid, mucic acid, nitric acid, pamoic acid, pantothenic acid, phosphoric acid, succinic acid, sulfuric acid, oxalic acid, propionic acid, glycolic acid, hydroiodic acid, perchloric acid, cyclamic acid, salicylic acid, 2-naphthalenesulfonic acid, saccharinic acid, trifluoroacetic acid, tartaric acid and p-toluenesulfonic acid, etc. Preferably, citric acid, hydrobromic acid, formic acid, hydrochloric acid, maleic acid, phosphoric acid, sulfuric acid and tartaric acid. More preferably, formic acid and hydrochloric acid.

Prodrugs of the compounds of the present invention are included in the protection scope of the present invention. Generally, the prodrug refers to a functional derivative that is readily converted into the desired compound in vivo. For example, any pharmaceutically acceptable salt, ester, salt of an ester or other derivative of the compound of the present application, which, after administration to a recipient, can directly or indirectly provide the compound of the present application or a pharmaceutically active metabolite or residue thereof.

The compounds described herein may contain one or more asymmetric centers and may thereby give rise to diastereomers and optical isomers. The present invention includes all possible diastereomers and their racemic mixtures, their substantially pure resolved enantiomers, all possible geometric isomers, and pharmaceutically acceptable salts thereof.

When the compound represented by formula (I) exists in tautomerism, unless otherwise stated, the present invention includes any possible tautomerism and pharmaceutically acceptable salts thereof, and mixtures thereof.

When compounds of formula (I) are substituted with heavier isotopes such as deuterium, they may afford certain therapeutic advantages resulting from greater metabolic stability, for example increased in vivo half-life or reduced dosage requirements.

The term "pharmaceutical composition" refers to a mixture of one or more compounds of the present application or pharmaceutically acceptable salts thereof and pharmaceutically acceptable excipients. The purpose of a pharmaceutical composition is to facilitate administration of a compound of the present application to an organism.

In the present disclosure, "a", "an", "the", "at least one" and "one or more" are used interchangeably. Thus, for example, a mixture comprising "a" pharmaceutically acceptable excipient composition can be interpreted to mean that the pharmaceutical composition includes "one or more" pharmaceutically acceptable excipients.

The term "pharmaceutically acceptable excipients" refers to those excipients that have no significant irritation to organisms and do not impair the biological activity and properties of the active compound. Suitable excipients are well known to those skilled in the art, such as carbohydrates, waxes, water-soluble and/or water-swellable polymers, hydrophilic or hydrophobic materials, gelatin, oils, solvents, water, and the like.

The pharmaceutical composition of the present invention can be prepared by combining the compound of the present application with suitable pharmaceutically acceptable excipients, for example, it can be formulated into solid, semi-solid, liquid or gaseous preparations, such as tablets, pills, capsules, powders, granules, ointments, emulsions, suspensions, suppositories, injections, inhalants, gels, microspheres and aerosols.

Typical routes of administration of the compounds of the invention or their pharmaceutically acceptable salts or their pharmaceutical compositions include, but are not limited to, oral, rectal, topical, inhalation, parenteral, sublingual, intravaginal, intranasal, intraocular, intraperitoneal, intramuscular, subcutaneous, and intravenous administration.

The term "treat" generally refers to obtaining a desired pharmacological and/or physiological effect. The effect may be therapeutic in terms of partial or complete stabilization or cure of the disease and/or due to side effects of the disease. As used herein, "treatment" encompasses any treatment of a disease in a patient that: (a) inhibits the symptoms of the disease, i.e., arrests its development; or (b) alleviates the symptoms of the disease, i.e., causes regression of the disease or symptoms.

The term "effective amount" means an amount of a compound of the present invention that (i) treats or prevents a specific disease, condition or disorder, (ii) alleviates, ameliorates or eliminates one or more symptoms of a specific disease, condition or disorder, or (iii) prevents or delays the onset of one or more symptoms of a specific disease, condition or disorder described herein. The amount of a compound of the present application that constitutes a "therapeutically effective amount" varies depending on the compound, the disease state and its severity, the mode of administration, and the age of the mammal to be treated, but can be routinely determined by one skilled in the art based on his or her own knowledge and this disclosure.

**Synthesis plan 1:**

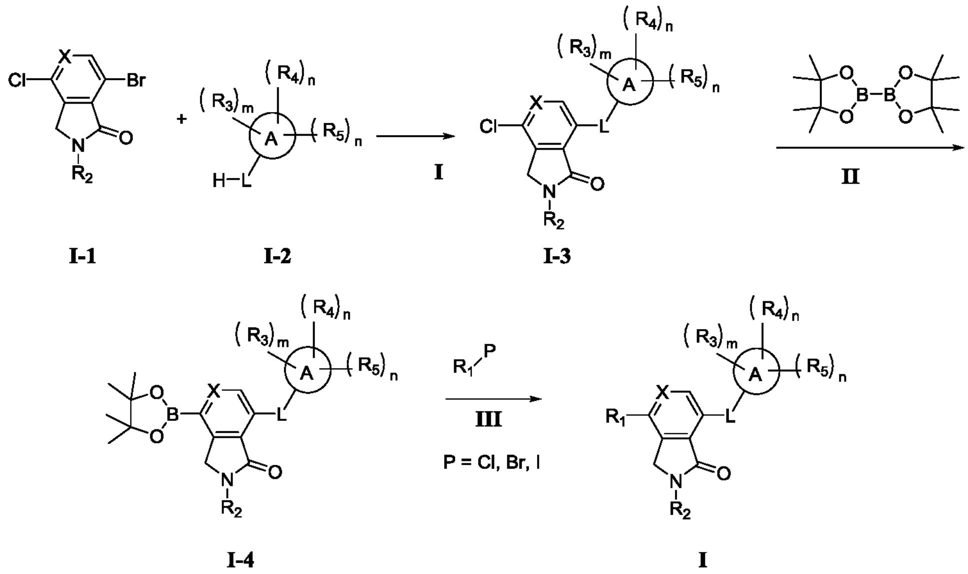


I-1 I-2 I-3

I-4 I

Step I: Compounds I-1 and I-2 are subjected to a Buchwald coupling reaction in the presence of a Pd catalyst such as $Pd_2(dba)_3$ to obtain compound I-3;

Step II: Compound I-3 is subjected to borylation with

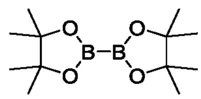

in the presence of a Pd catalyst such as $Pd_2(dba)_3$ to obtain I-4;

Step III: Compound I-4 reacts with

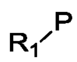

in the presence of a metal catalyst such as $PdCl_2$(dppf). Under the action of $CH_2Cl_2$ or $PdCl_2$ (dppf), $R_1$ is introduced through Suzuki coupling reaction to obtain compound I, wherein P is Cl, Br or I.

In order to make the above content clearer and clearer, the present invention will use the following examples to further illustrate the technical solution of the present invention. The following examples are only used to illustrate specific implementations of the present invention so that those skilled in the art can understand the present invention, but are not used to limit the scope of the present invention. In the specific embodiments of the present invention, technical means or methods that are not specifically described are conventional technical means or methods in the art.

Unless otherwise stated, all temperatures herein refer to degrees Celsius.

The following abbreviations are used in the examples:

DMF: N,N-dimethylformamide;
NBS: N-bromosuccinimide;
THF: tetrahydrofuran;
[$PdCl_2$(dppf)]: [1,1'-bis(diphenylphosphine)ferrocene]palladium dichloride;
[$PdCl_2$(dppf)]$CH_2Cl_2$: [1,1'-bis(diphenylphosphine)ferrocene] palladium dichloride dichloromethane complex;
EA: ethyl acetate;
PE: petroleum ether;
DCM: dichloromethane;
MeOH: methanol;
$Pd_2(dba)_3$: Tris(dibenzylideneacetone)dipalladium;

Sphos: 2-dicyclohexylphosphonium-2',6'-dimethoxybiphenyl;
BPO: (di)benzoyl peroxide;
DMAP: 4-dimethylaminopyridine;
MsCl: Methanesulfonyl chloride;
AIBN: azobisisobutyronitrile;
TMSCN: trimethylsilyl cyanide;
TBAF: tetrabutylammonium fluoride;
LDA: lithium diisopropylamide;
TBAF: tetrabutylammonium fluoride;
LiHMDS: lithium bistrimethylsilylamide;
TMSCl: trimethylsilyl chloride;
XantPhos: 4,5-bisdiphenylphosphine-9,9-dimethylxanthene;
Dioxane: dioxane;
RT: room temperature;
$(Boc)_2O$: di-tert-butyl dicarbonate;
DIBAL-H: diisobutylaluminum hydride;
RuPhos Pd G3: Methanesulfonic acid (2-dicyclohexylphosphino-2',6'-diisopropoxy-1,1'-biphenyl) (2-amino-1,1'-biphenyl-2-yl)palladium(II);
i-PrOH: isopropyl alcohol;
PPA: polyphosphoric acid;
$B_2Pin_2$: Pinacol diborate;
Noyori catalyst(ss): (S,S)-N-(p-toluenesulfonyl)-1,2-diphenylethanediamine (p-cumylbenzene) ruthenium(II) chloride;
$ikB_2$: Tert-butyl carbamate;
TFA: trifluoroacetic acid;
TFAA: trifluoroacetic anhydride;
MTBE: methyl tert-butyl ether;
$Ti(OEt)_4$ : Ethyl titanate;
DMSO: dimethyl sulfoxide;
Solutol/HS15/Solutol HS15: Polyethylene glycol-15 hydroxystearate.

Synthesis of intermediate M1:

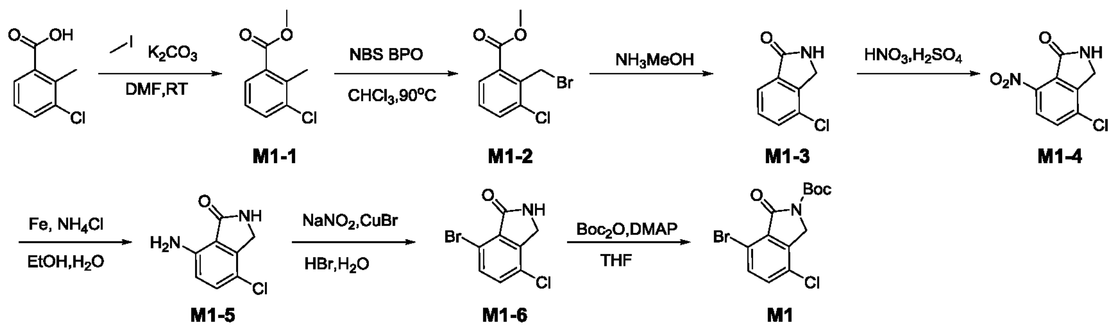


Step 1: Synthesis of Compound M1-1

At room temperature, 3-chloro-2-methylbenzoic acid (200.00 g) and potassium carbonate (405.09 g) were added to DMF (1300.00 mL), and iodomethane (80.28 mL) was added dropwise under an ice-water bath, and the mixture was reacted at room temperature for 16 h. Add 2000mL water to the reaction solution, extract with 1000mL EA, repeat 3 times, combine the organic phases, wash the organic phase six times with saturated sodium chloride aqueous solution, dry over anhydrous sodium sulfate, filter, and concentrate to obtain the target compound M1-1 ( 213.00g, yield 98.41%).

Step 2: Synthesis of Compound M1-2

At room temperature, compound M1-1 (103.00g), NBS (109.22g), and BPO (2.70g) were added to chloroform

(700.00mL), and the reaction was carried out at 90°C for 16h. Add 1000mL water to the reaction solution, extract with 1000mL DCM, repeat 3 times, combine the organic phases, wash the organic phase 3 times with saturated sodium chloride aqueous solution, dry over anhydrous sodium sulfate, filter, and concentrate to obtain the target compound M1-2 (145.00g , yield 98.63%).

Step 3: Synthesis of compound M1-3

Under an ice-water bath, compound M1-2 (350.00g) was added to a solution of ammonia in methanol (7M, 1000.00 mL), and the reaction was carried out under an ice-water bath for 1 hour. The reaction solution was concentrated to obtain a solid, which was washed three times with water and dried to obtain the target compound M1-3 (213.80 g, yield 96.05%).ESI-MS m/z:167.9[M+H] +.

Step 4: Synthesis of compound M1-4

At room temperature, compound M1-3 (100.00g) was added to concentrated sulfuric acid (600.00mL). Under ice-water bath conditions, concentrated nitric acid (50.00mL) was added dropwise, stirred under ice-water bath for 1 hour, and then stirred at room temperature for 1 hour.. The reaction solution was slowly poured into ice water, filtered, the filter cake was washed with water three times, and the filter cake was dried to obtain the target compound M1-4 (116.08g, yield 91.51%).ESI-MS m/z:213.0[M+H] +.

Step 5: Synthesis of Compound M1-5

Compound M1-4 (244.00 g) and ammonium chloride (368.68 g) were added to ethanol (2500.00 mL) and water (500.00 mL), iron powder (320.51 g) was added in batches at 65°C, and the mixture was reacted at 65°C for 2 h. The reaction solution was filtered while hot, and the filter cake was washed six times with EA. The filtrate was concentrated and water was added. Solid precipitated and filtered. The filter cake was washed three times with water and dried. Then, it was slurried with PE/EA=3/1, filtered, and dried to obtain the target compound M1-5. ESI-MS m/z:183.0[M+H] +.

Step 6: Synthesis of Compound M1-6

Compound M1-5 (129.00g) was added to hydrogen bromide (48% aqueous solution, 900.00mL), and a solution of sodium nitrite (73.11g) in water (600.00mL) was added dropwise at -10°C. The reaction was carried out for 1 hour, CuBr (111.47g) was added, the temperature was raised to 80°C, and stirred for 1 hour. The reaction solution was poured into ice water, the product was precipitated, filtered, washed three times with water, and the filter cake was dried to obtain the target compound M1-6 (143.00g, yield 82.12%).ESI-MS m/z:247.9[M+H] +.

Step 7: Synthesis of Compound M1

Compound M1-6 (143.00 g) and DMAP (7.09 g) were added to THF (1300.00 mL), and $(Boc)_2O$ (159.94 mL) was added dropwise under an ice-water bath, and the mixture was reacted at room temperature for 1 h.The reaction solution was added to water (1000.00 mL) to quench, and extracted with 8000 mL of EA, repeated 3 times, the organic phases were combined, washed 3 times with saturated aqueous sodium chloride solution, dried over anhydrous sodium sulfate, filtered, concentrated, and then pulped with PE, filtered, and the filter cake was dried to obtain the target compound M1 (185.00 g, yield 92.00%).ESI-MS m/z:289.9[M+H] +. Synthesis of intermediate M2:

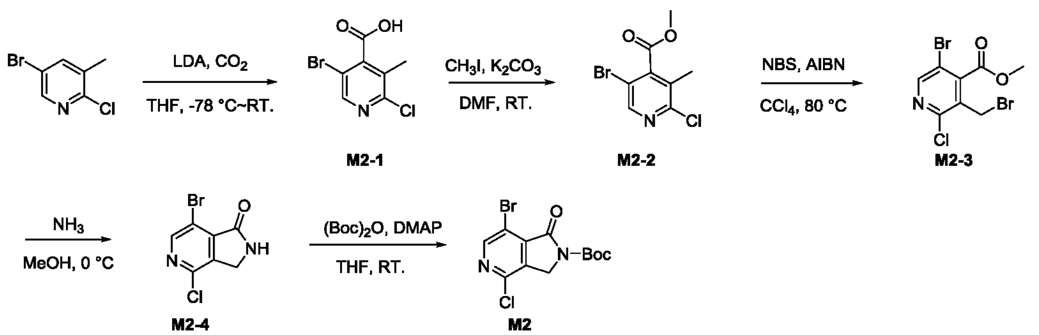

Step 1: Synthesis of Compound M2-1

At room temperature, 2-chloro-3-methyl-5-bromopyridine (5.00 g) was dissolved in THF (50 mL), and then LDA (14.53 mL, 2 M) was slowly added dropwise at -78 °C under the protection of $N_2$. The reaction was kept warm for 40 min, and then $CO_2$ was slowly introduced. The temperature was then slowly raised to room temperature for 40 min to stop the reaction. The reaction was quenched with saturated sodium bicarbonate solution, EA was added, the liquids were separated, the aqueous phase was retained, the organic phase was back-extracted with water twice, the aqueous phases were combined, the pH was adjusted to 4 with HCl, and then extracted 5 times with DCM/i-PrOH=3:1, the organic phases were combined, the organic phases were dried over anhydrous sodium sulfate, and concentrated to obtain the target compound M2-1 (2.83 g, yield 46.66%). ESI-MS m/z:250.5[M+H] +.

Step 2: Synthesis of compound M2-2

At room temperature, compound M2-1 (2.83 g) was dissolved in DMF (30 mL), potassium carbonate (3.12 g) was added, and $CH_3I$ (2.41 g) was slowly added dropwise under nitrogen protection, and then reacted at room temperature for 16 h, and then the reaction was stopped. The reaction was quenched by adding water, and the mixture was extracted three times with EA. The organic phases were combined, washed with saturated sodium chloride, dried over anhydrous sodium sulfate, and concentrated. The concentrate was separated by column chromatography (PE:EA=20:1) to obtain the target compound M2-2 (1.80 g, yield 60.23%).ESI-MS m/z:265.5[M+H] +.

Step 3: Synthesis of compound M2-3

At room temperature, compound M2-2 (1.50 g) was dissolved in $CCl_4$ (20 mL), and NBS (2.02 g) and AIBN (90.00 mg) were added. Under nitrogen protection, the mixture was reacted at 80°C for 16 h, and the reaction was stopped. The reaction was quenched by adding water, and the mixture was extracted three times with DCM. The organic phases were combined, washed with saturated sodium chloride, dried over anhydrous sodium sulfate, and concentrated. The concentrate was separated by column chromatography (PE:EA=20:1) to obtain the target compound M2-3 (1.80 g, yield 92.30%). ESI-MS m/z:344.4[M+H] $^+$.

Step 4: Synthesis of compound M2-4

Compound M2-3 (1.80 g) was dissolved in ammonia in methanol (20 mL) at room temperature, reacted at room temperature for 2 h, and then stopped the reaction. The reaction solution was filtered, and the filter cake was washed with methanol and dried to obtain the target compound M2-4 (1.28g, yield 98.52%). ESI-MS m/z:397.2[M+H] $^+$.

Step 5: Synthesis of Compound M2

At room temperature, compound M2-4 (1.28 g) was dissolved in 1,4-dioxane (60 mL), DMAP (70 mg) was added, and then di-tert-butyl dicarbonate (1.40 g) was slowly added dropwise. The mixture was reacted at room temperature for 2 h, and the reaction was stopped. The reaction was quenched by adding water, and the mixture was extracted three times with EA. The organic phases were combined, washed with saturated sodium chloride, dried over anhydrous sodium sulfate, and concentrated. The concentrate was separated by column chromatography (PE:EA =20:1) to obtain the target compound M2 (1.57 g, yield 87.75%).ESI-MS m/z:347.3[M+H] +.

Synthesis of intermediate M3

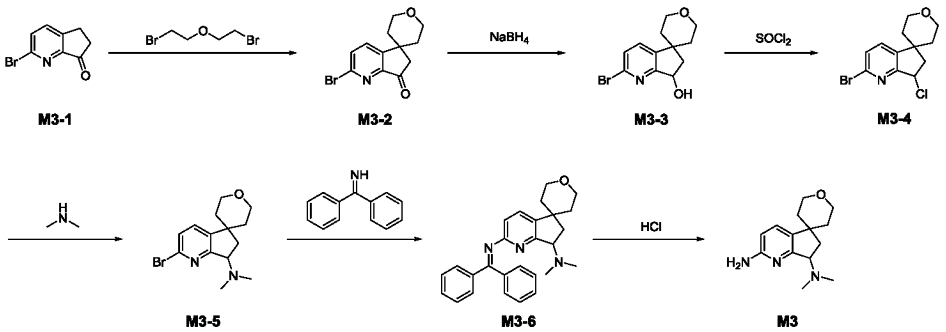

M3-1 M3-2 M3-3 M3-4
M3-5 M3-6 M3

Step 1: Synthesis of compound M3-2

Under nitrogen protection and -78°C conditions, LiHMDS (4.95 mL, 1M / THF) was added dropwise to a solution of M3-1 (1 g) in THF (10 mL) within 30 minutes. After stirring for 30 minutes, TMSC1 (0.66 mL) was added and stirred for 5 minutes. LiHMDS (9.67 mL, 1M/THF) was added dropwise within 30 minutes. The temperature was raised to -30°C to -20°C, stirred for 30 minutes, bis (2-bromoethyl) ether (1.09 g) was added, the temperature was gradually raised to room temperature, and stirred overnight. The reaction solution was quenched with saturated ammonium chloride solution, concentrated under reduced pressure to remove part of the solvent, extracted with EA, combined the organic layers, washed with 1M HCl, water and saturated brine in sequence, dried over anhydrous sodium sulfate, concentrated under reduced pressure, and purified by column chromatography (PE:EA=70:30-65:35) to give compound M3-2 (246 mg, 18% yield).ESI-MS m/z:281.99[M+H]+.

Step 2: Synthesis of compound M3-3

Under ice bath, sodium borohydride (99 mg) was added portionwise to a solution of M3-2 in MeOH (6 mL) and THF (2 mL), the ice bath was removed, and the mixture was reacted at room temperature for 1 hour. The reaction mixture was quenched with water, concentrated under reduced pressure to remove part of the solvent, extracted with EA, and the organic layers were combined, washed with saturated brine, dried over anhydrous sodium sulfate, and concentrated under reduced pressure to give compound M3-3 (217 mg, 88% yield).ESI-MS m/z:283.98[M+H]+.

Step 3: Synthesis of Compound M3-4

Under ice bath, thionyl chloride (0.28 mL) was added dropwise to a DCM (5 mL) solution of M3-3, the ice bath was removed, the temperature was raised to 40°C, and the reaction was carried out for 1 hour. Concentrate under reduced pressure, add a small amount of methanol to dissolve, adjust the pH to alkaline with saturated sodium bicarbonate solution, extract with EA, combine the organic layers, wash with saturated brine, dry over anhydrous sodium sulfate, concentrate under reduced pressure, and purify by column chromatography (PE:EA=85:15) to obtain compound M3-4 (148 mg, 64% yield). ESI-MS m/z:301.96[M+H]+.

Step 4: Synthesis of Compound M3-5

At room temperature, M3-4 (148 mg), potassium carbonate (203 mg) and potassium iodide (16 mg) were dissolved in acetonitrile (5 mL), dimethylamine (2.45 mL, 2M/THF) was added, the temperature was raised to 80°C, and the reaction was carried out for 3 hours.The mixture was cooled and filtered, and the filtrate was concentrated under reduced pressure and purified by column chromatography (DCM:MeOH=95:5) to obtain compound M3-5 (85 mg, 56% yield).

ESI-MS m/z:311.02[M+H]+.

Step 5: Synthesis of Compound M3-6

At room temperature, M3-5 (85 mg), benzophenone imine (59 mg), $Pd_2(dba)_3$ (25 mg), XantPhos (32 mg) and cesium carbonate (267 mg) were dissolved in 1,4-Dioxane (5 mL), replaced with nitrogen, heated to 100°C, and reacted for 6 hours. The mixture was cooled and filtered, and the filtrate was concentrated under reduced pressure and purified by column chromatography (DCM:MeOH=92:8) to obtain compound M3-6 (86 mg, 77% yield). ESI-MS m/z:412.25[M+H]+.

Step 6: Synthesis of Compound M3

At room temperature, M3-6 (86 mg) was dissolved in MeOH (2 mL), hydrochloric acid (0.52 mL, 4M/1,4-Dioxane) was added, the temperature was raised to 50°C, and the reaction was carried out for 1 hour. The mixture was cooled and concentrated under reduced pressure. A small amount of methanol was added to dissolve the mixture. The pH was adjusted to alkaline with saturated sodium bicarbonate solution. The mixture was extracted with EA. The organic layers were combined, washed with saturated brine, dried over anhydrous sodium sulfate, concentrated under reduced pressure, and purified by column chromatography (PE:EA=92:8, 1‰ ammonia water) to give compound M3 (45 mg, 87% yield). ESI-MS m/z:248.15[M+H]+. Synthesis of intermediate M4:

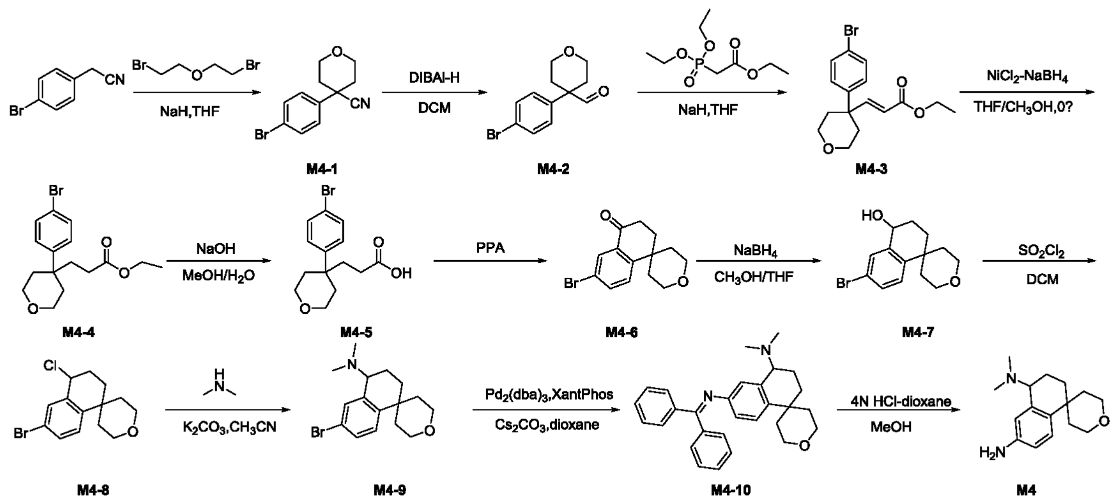


Step 1: Synthesis of compound M4-1

NaH (4.62 g, 60% content) was added to DMSO (200 ml) in batches. Under the protection of $N_2$, the temperature was controlled at 15-20°C, and the ether (50 ml) solution of the raw materials p-bromophenylacetonitrile (10.3 g) and 2,2'-dibromodiethyl ether (12.79 g) was added dropwise for 2 hours. After the dripping is completed, the temperature is raised to room temperature and reacted for 12 hours. Detection reactions are fully processed. The reaction solution was slowly added dropwise to ice water to precipitate a solid, which was filtered, washed with water, filtered, and dried to obtain the target product M4-1 (14.0 g, yield 100%). ESI-MS m/z:266.1[M+H]+.

Step 2: Synthesis of compound M4-2

The raw material M4-1 (6.0 g) was dissolved in DCM (100.00 mL), and the temperature was lowered to -78°C under the protection of $N_2$, and DIBAL-H (27.05 mL) was added dropwise. After the addition was completed, the temperature was kept at -65°C for 2 hours. Then slowly raise the temperature to 0°C and stir for 5 minutes. The temperature was controlled at 0°C, and a 15% aqueous solution of HCl (9.6 ml) was added dropwise, followed by reaction at RT for 2 hours. The reaction solution was diluted with water and DCM, extracted with DCM (100 ml) three times, the organic phase was washed with saturated brine, dried over anhydrous sodium sulfate, and the concentrate was separated by column chromatography (PE:EA=5:1) to obtain the target product M4-2 (3.26 g, yield 53.7%).

Step 3: Synthesis of compound M4-3

Under $N_2$ protection, the raw material triethyl phosphonoacetate (3.30 g) was dissolved in THF (25 mL), and NaH (0.61 g, 60%) was added in batches, and then reacted at RT for 1 hour. The temperature was lowered to 0°C, and a solution of M4-2 (3.30 g) in THF (8 ml) was added dropwise. After the addition was complete, the mixture was reacted at RT for 2 hours. The reaction solution was diluted with water (30 ml) and EA (25 ml), stirred and separated into layers, the organic phase was separated, washed with saturated brine, dried over anhydrous sodium sulfate, and the concentrate was separated by column chromatography (PE:EA=5:1) to obtain the target product M4-3 (3.73 g, yield 89.7%). ESI-MS m/z:340.2[M+H]+.

Step 4: Synthesis of Compound M4-4

Add raw material M4-3 (4.0 g) and nickel chloride hexahydrate (2.80 g) to THF (40.00 mL) and MeOH (10.00 mL). Add sodium borohydride (1.78 g) in batches under ice bath. The color turns black after addition. Keep the temperature at 0°C for 30 minutes, and naturally raise to RT for reaction for 30 minutes. After treatment, the reaction solution was quenched with water, extracted with EA (50 ml) twice, the organic phase was dried over anhydrous sodium sulfate, and the concentrate was separated by column chromatography (PE: EA = 5: 1) to obtain the target product M4-4 (705 mg, yield 17.5%). ESI-MS m/z:342.2[M+H]+.

Step 5: Synthesis of Compound M4-5

The raw material M4-4 (705 mg) was dissolved in THF (6.00 mL) and MeOH (2.00 mL), and sodium hydroxide (0.25 g) in water (5.00 mL) was added and reacted at RT for 2 hours. The reaction solution was rotary evaporated to remove the organic solvent, and diluted HCl was added to adjust the pH to 4 to precipitate a white solid, which was filtered, washed with water, and dried to obtain the target product M4-5 (545 mg, yield 84.2%). ESI-MS m/z:314.2[M+H]+.

Step 6: Synthesis of Compound M4-6

The raw material M4-5 (520 mg) was added to polyphosphoric acid (5.00 mL), heated to 90 degrees for 0.5 hours, ice water and EA were added, and then the pH was adjusted to 8-9 with saturated sodium bicarbonate. The organic phase was separated and dried over anhydrous sodium sulfate. The concentrate was separated by column chromatography (PE: EA = 3: 1) to obtain the target product M4-6 (485 mg, yield 98.9%). ESI-MS m/z:296.2[M+H]+.

Step 7: Synthesis of Compound M4-7

The raw material M4-6 (435.00 mg) was dissolved in methanol (10.00 mL) and tetrahydrofuran (3.00 mL), the temperature was lowered to 0 degrees, sodium borohydride (223.00 mg) was added in portions, and then reacted at RT for 0.5 hours. The reaction solution was rotary evaporated to remove part of the solvent, extracted with water and EA (15 ml) three times, dried over anhydrous sodium sulfate, spin-dried, and dehydrated with DCM three times to obtain the target product M4-7, which was directly used in the next reaction (404 mg, yield 92.2%). ESI-MS m/z:298.2[M+H]+.

Step 8: Synthesis of Compound M4-8

The raw material M4-7 (405.00 mg) was dissolved in dichloromethane (12.00 mL), and dichlorothionyl (0.49 mL) was added dropwise under ice bath, and then heated at 40 degrees to react for 30 minutes. The reaction solution was diluted with DCM, and water (5 ml) was added. The pH was adjusted to 8-9 with saturated sodium bicarbonate. The product was extracted with DCM (15 mL) three times and dried over anhydrous sodium sulfate. The concentrate was separated by column chromatography (PE: EA = 20: 1) to obtain the target product M4-8 (301 mg, yield 70.0%). ESI-MS m/z:315.1[M+H]+.

Step 9: Synthesis of Compound M4-9

The raw material M4-8 (300.00 mg) was dissolved in acetonitrile (7.00 mL), and potassium iodide (31.56 mg), potassium carbonate (394.11 mg), and dimethylamine (2M) (2571.12 mg) were added. The mixture was then sealed and heated at 80 degrees for 8 hours. The reaction solution was cooled to room temperature, filtered, washed with EA, the filtrate was spin-dried, and the concentrate was separated by column chromatography (PE:EA=1:1) to obtain the target product M4-9 (263 mg, yield 85.3%). ESI-MS m/z:324.2[M+H]+.

Step 10: Synthesis of Compound M4-10

Under $N_2$ protection, raw material M4-9 (300.00 mg), benzophenone imine (184.44 mg), cesium carbonate (602.90 mg), 4,5-bis(diphenylphosphino)-9,9-dimethyloxanthene (26.79 mg) were added to 1,4-dioxane (8.00 mL), $N_2$ was bubbled, and the mixture was heated at 100 degrees for 12 hours. The reaction solution was directly filtered, the filter cake was washed with EA, the filtrate was spin-dried, and the concentrate was separated by column chromatography (DCM:CH 3 OH=20:1) to obtain the target product M4-10 (253 mg, yield 64.4%). ESI-MS m/z:425.6[M+H]+.

Step 11: Synthesis of Compound M4

The raw material M4-10 (304.00 mg, 95%) was dissolved in methanol (5.00 mL), and hydrochloric acid (4 M dioxane solution) (0.51 mL, 4.00 mol/L) was added, and the mixture was reacted at RT for 1 hour. The reaction solution was dried by rotation, and then a small amount of methanol (0.5 ml) was added, and EA (10 ml) was added to precipitate a solid, which was filtered and the filter cake was washed with EA. The solid was dissolved in water, and the pH was adjusted to 8 with saturated sodium bicarbonate. DCM:$CH_3OH$=10:1 was used for extraction (15 ml) 4 times. The organic phase was dried over anhydrous sodium sulfate and dried to obtain the target product M4 (118 mg, yield 66.6%). ESI-MS m/z:261.4 [M+H]+.

Synthesis of intermediate M5:

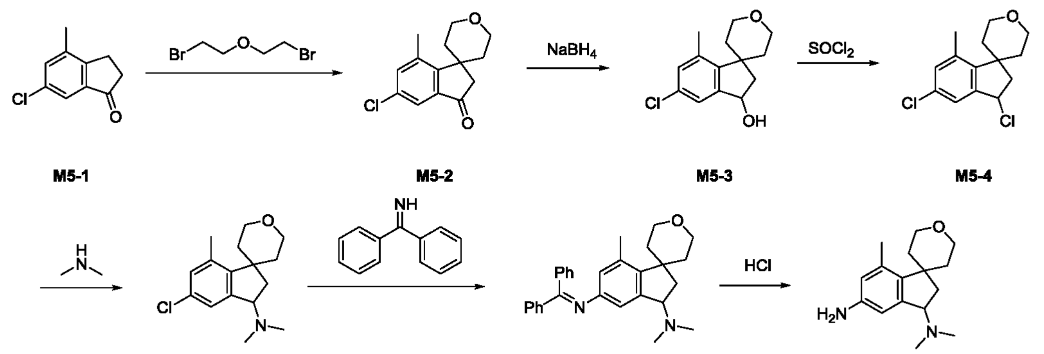


M5-1 M5-2 M5-3 M5-4

M5-5 M5-6 M5

Step 1: Synthesis of compound M5-2

Under nitrogen protection and -78°C conditions, LiHMDS (23.25 mL, 1M / THF) solution of M5-1 (4 g) was added dropwise within 30 minutes. After stirring for 30 minutes, TMSCl (3.09 mL) was added, stirred for 5 minutes, and LiHMDS (55.36 mL, 1M / THF) was added dropwise within 30 minutes. The temperature was raised to -30°C to -20°C, stirred for 30 minutes, bis (2-bromoethyl) ether (5.39 g) was added, and the temperature was gradually raised to room temperature and stirred overnight. The reaction solution was quenched with saturated ammonium chloride solution, concentrated under reduced pressure to remove part of the solvent, extracted with EA, combined the organic layers, washed with 1M HCl, water and saturated brine in sequence, dried over anhydrous sodium sulfate, concentrated under reduced pressure, and purified by column chromatography (PE:EA=75:25-65:35) to give compound M5-2 (1.63 g, 29% yield).

Step 2: Synthesis of compound M5-3

Under ice bath, sodium borohydride (0.74 g) was added portionwise to a solution of M5-2 (1.63 g) in MeOH (10 mL) and THF (6 mL), the ice bath was removed, and the reaction was carried out at room temperature for 1 hour. The reaction mixture was quenched with water, concentrated under reduced pressure to remove part of the solvent, extracted with EA, and the organic layers were combined, washed with saturated brine, dried over anhydrous sodium sulfate, and concentrated under reduced pressure to obtain compound M5-3 (1.60 g, 97% yield). $^1H$ NMR(500MHz, $CDCl_3$) δ7.22(s,1H), 7.07(s,1H), 5.15(t,J=6.3Hz,1H), 4.03-3.92(m,2H), 3.67-3.61(m,2H), 2.6-2.61(m,1H), 2.57-2.51(m,1H), 2.38-2.31(m,1H), 2.05-2.01(m,1H), 1.54-1.51(m,1H), 1.26-1.22 (m,1H).

Step 3: Synthesis of Compound M5-4

To a solution of M5-3 (1.76 g) in DCM (20 mL) was added dropwise thionyl chloride (2.53 mL) under ice-bath. The ice-bath was removed and the temperature was raised to 40°C to react for 1 hour. Concentrate under reduced pressure, add a small amount of methanol to dissolve, adjust the pH to alkaline with saturated sodium bicarbonate solution, extract with EA, combine the organic layers, wash with saturated brine, dry over anhydrous sodium sulfate, concentrate under reduced pressure, and purify by column chromatography (PE:EA=85:15) to obtain compound M5-4 (1.72 g, 91% yield).

Step 4: Synthesis of Compound M5-5

At room temperature, M5-4 (1.72 g), potassium carbonate (1.75 g) and potassium iodide (0.11 g) were dissolved in acetonitrile (20 mL), dimethylamine (4.77 mL, 2M/THF) was added, the temperature was raised to 80°C, and the mixture was reacted for 3 hours. The mixture was cooled and filtered, and the filtrate was concentrated under reduced pressure and purified by column chromatography (DCM:MeOH=96:4) to obtain compound M5-5 (1.18 g, 66% yield). ESI-MS m/z:280.2[M+H]+.

Step 5: Synthesis of Compound M5-6

At room temperature, M5-5 (300 mg), benzophenone imine (251 mg), RuPhos Pd G3 (77 mg) and cesium carbonate (904 mg) were dissolved in xylene (10 mL), the atmosphere was replaced with nitrogen, the temperature was raised to 120°C, and the mixture was reacted for 6 hours. The mixture was cooled and filtered, and the filtrate was concentrated under reduced pressure and purified by column chromatography (DCM:MeOH=93:7) to obtain compound M5-6 (303 mg, 77% yield). ESI-MS m/z:425.3[M+H]+.

Step 6: Synthesis of Compound M5

At room temperature, M5-6 (363 mg) was dissolved in MeOH (5 mL), hydrochloric acid (5 mL, 4M/1,4-Dioxane) was added, the temperature was raised to 50°C, and the reaction was carried out for 2 hours. The mixture was cooled and concentrated under reduced pressure. A small amount of methanol was added to dissolve the mixture. The pH was adjusted to alkaline with saturated sodium bicarbonate solution. The mixture was extracted with EA. The organic layers were combined, washed with saturated brine, dried over anhydrous sodium sulfate, concentrated under reduced pressure, and purified by column chromatography (PE:EA=92:8, 1‰ ammonia water) to give compound M5 (186 mg, 83% yield). ESI-MS m/z:261.3[M+H]+.

Synthesis of intermediate M6:

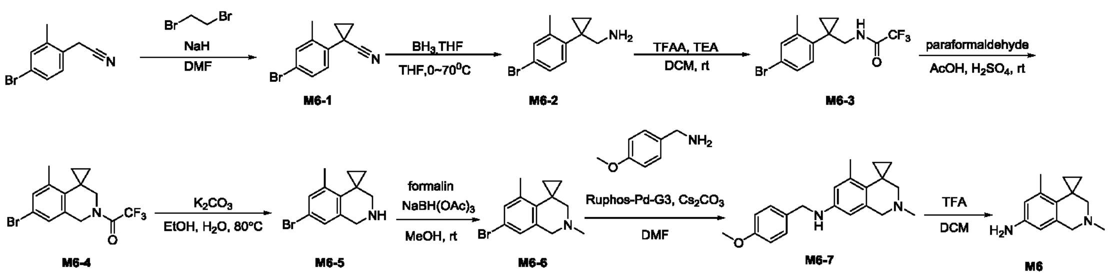


Step 1: Synthesis of compound M6-1

At room temperature, add NaH (2.86 g) into DMF (100.00 mL), then replace with $N_2$, add dropwise 2-(4-bromo-2-methylphenyl)acetonitrile (5.00 g) dissolved in 10 mL of DMF solution under ice-water bath, continue to react at this temperature for 15 min, add dropwise 1,2-dibromoethane (6.71 g) dissolved in 10 mL of DMF solution, remove the ice-water bath, and react at room temperature for 2 h. The reaction solution was added into 300 mL of ice water, and then extracted three times with 150 mL. The organic phases were combined, washed with saturated sodium chloride, and concentrated. The concentrate was separated and purified by column chromatography (PE:EA=95:5-90:10) to obtain the target product M6-1 (4.25 g, 75.63%). ESI-MS m/z:236.0[M+H]+.

Step 2: Synthesis of compound M6-2

At room temperature, compound M6-1 (26.84 g) was dissolved in THF/tetrahydrofuran (270.00 mL), replaced with nitrogen, and $BH_3$ was slowly added dropwise under nitrogen protection and ice-water bath conditions. THF (568.38 mL) was added, and the reaction was continued for 15 min. The ice-water bath was removed, and the reaction was refluxed for 5 h. The reaction solution was cooled, and the pH was adjusted to 2-3 with 1M HCl, and then the reaction solution was refluxed for 1h. Then the pH was adjusted to 8-9 with saturated sodium bicarbonate, and then extracted with EA for 3 times, the organic phases were combined, washed with saturated brine, dried over anhydrous sodium sulfate, and the organic

phase was concentrated to obtain the target product M6-2 (28.80g). ESI-MS m/z:240.0[M+H]+.

Step 3: Synthesis of compound M6-3

At room temperature, compound M6-2 (25.50 g) was dissolved in dichloromethane (300.00 mL). Under nitrogen protection, triethylamine (44.28 mL) was added in an ice-water bath, and then TFAA (22.14 mL) was slowly added dropwise. The mixture was reacted at room temperature for 3 h. The reaction solution was added into a certain amount of ice water, extracted with DCM for 3 times, the organic phases were combined, washed with saturated brine, dried over anhydrous sodium sulfate, and the organic phase was concentrated to obtain a crude product. The sample was mixed and subjected to column chromatography (PE:EA=95:5-91:9) to obtain the target product (20.60 g, yield 57.71%). ESI-MS m/z:336.1[M+H]+.

Step 4: Synthesis of compound M6-4

At room temperature, compound M6-3 (15.60 g) was dissolved in HOAc (206.00 mL). Under nitrogen protection, concentrated sulfuric acid (138.00 mL) was slowly added dropwise in an ice-water bath. The mixture was kept warm for 0.5 h, and then paraformaldehyde (2.09 g) was added in batches. The mixture was returned to room temperature and reacted for 16 h. The reaction solution was slowly poured into a certain amount of ice water, extracted with EA three times, the organic phases were combined, washed with saturated sodium bicarbonate solution and saturated brine, and the organic phase was concentrated to obtain a crude product. The sample was mixed and column chromatography (PE:EA=100:0~91:9) was performed to obtain the target product M6-4 (6.00 g, yield 37.13%). ESI-MS m/z:348.0 [M+H]+.

Step 5: Synthesis of Compound M6-5

At room temperature, compound M6-4 (8.20 g) was dissolved in EtOH (147.00 mL) and $H_2O$ (49.00 mL), potassium carbonate (16.28 g) was added, and the mixture was reacted at 80°C for 1 h.The reaction solution was cooled to room temperature, and ethanol was removed by rotary evaporation. The mixture was then extracted with EA three times, washed with saturated brine three times, dried over anhydrous sodium sulfate, and the organic phase was concentrated to obtain the target product M6-5 (5.70 g, yield 95.98%). ESI-MS m/z:252.0[M+H]+.

Step 6: Synthesis of Compound M6-6

At room temperature, compound M6-5 (5.70 g) was dissolved in methanol (57.00 mL) and HOAc (38.00 mL). Under nitrogen protection, 37% formaldehyde aqueous solution (2.75 g) was slowly added dropwise in an ice-water bath. The mixture was kept warm for 1 hour, and then sodium triacetoxyborohydride (14.37 g) was added in batches. The mixture was then returned to room temperature for 2 hours. The pH of the reaction solution was adjusted to 8-9 with a saturated sodium bicarbonate solution, and then extracted with EA 2-3 times. The organic phases were combined, washed with saturated sodium bicarbonate and saturated brine, and the organic phases were concentrated to obtain a crude product. The crude product was purified by column chromatography (DCM: MeOH = 100: 0-95: 5) to obtain the target product M6-6 (4.80 g, 79.77%).

ESI-MS m/z:266.1[M+H]+.

Step 7: Synthesis of Compound M6-7

At room temperature, compound M6-6 (1.00 g), p-methoxybenzylamine (1.03 g), Ruphos-Pd-G3 (0.31 g), and cesium carbonate (2.45 g) were added to DMF (15.00 mL), and then reacted at 90°C for 24 h under $N_2$ protection conditions, and the reaction was stopped. The reaction solution was directly concentrated to obtain a crude product, which was separated by column chromatography (DCM: MeOH = 96:4 to 90:10) to obtain the target product M6-7 (0.72 g, yield 59.43%). ESI-MS m/z:323.26[M+H]+.

Step 8: Synthesis of Compound M6

At room temperature, compound M6-7 (720.00 mg) was added to dichloromethane (20.00 mL), and then trifluoroacetic acid (5.0 mL) was added in an ice-water bath under $N_2$ protection conditions, and the reaction was refluxed for 1 h to stop the reaction. The reaction solvent was removed by rotary evaporation, 10 mL of $H_2O$ and 10 mL of DCM were added to the reaction solution, and then NaOH was added to adjust the pH to 12, and then extracted three times with 25 mL of DCM. The organic phases were combined and washed with saturated sodium chloride. The organic phases were

concentrated to obtain a crude product, and the crude product was separated by column chromatography (DCM: MeOH = 96:4 ~ 90:10) to obtain the target product M6 (350.00 mg, yield 77.49%). ESI-MS m/z:203.22[M+H]+.

Synthesis of intermediate M7:

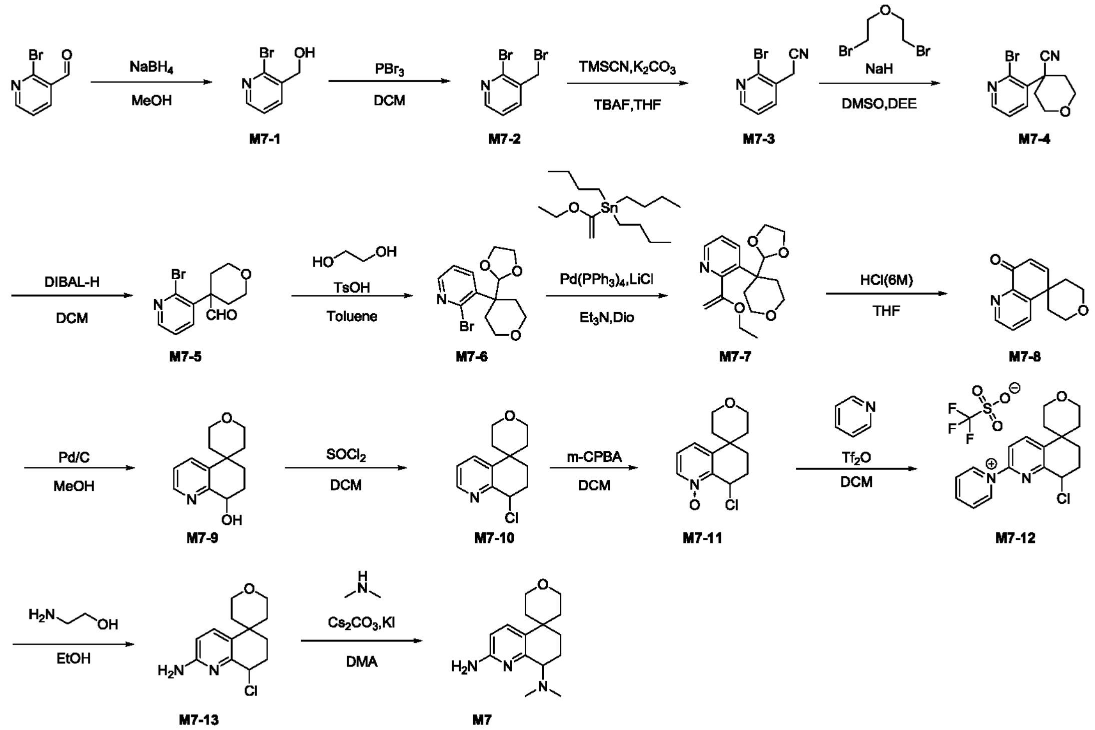


Step 1: Synthesis of intermediate M7-1

At room temperature, add the compound 2-bromo-3-pyridinecarboxaldehyde (15.00 g) to MeOH (200.00 mL), and then under $N_2$ protection conditions, control the temperature at 0-5°C and slowly add sodium borohydride (3.66 g) in batches. After the addition is complete, remove the ice-water bath and react at room temperature at 25°C for 1 hour. The reaction solution was quenched by adding 150 mL of water to remove methanol, and then extracted with 100 mL of EA for 5 times, washed with 100 mL of saturated sodium chloride for 2 times, dried over anhydrous sodium sulfate, and the organic phase was filtered and directly concentrated to dryness to obtain the target product M7-1 (14.20 g, yield 93.67%). ESI-MS m/z:188.0[M+H]+.

Step 2: Synthesis of intermediate M7-2

At room temperature, compound M7-1 (14.20 g) was added to DCM (350.00 mL), and then $PBr_3$ (6.40 mL) was slowly added in batches while controlling the temperature at 0-5°C under the protection of $N_2$. After the addition was completed, the ice-water bath was removed and the reaction was carried out at room temperature at 25°C for 18 hours. The reaction solution was added to 500 mL of ice water to quench, and 30% NaOH was added to adjust the pH to 12-13, then extracted three times with 250 mL of DCM, washed twice with 100 mL of saturated sodium chloride, dried over anhydrous sodium sulfate, filtered, and the organic phase was directly concentrated and dried to obtain the target product M7-2 (18.00 g, yield 94.99%). ESI-MS m/z:250.9[M+H]+.

Step 3: Synthesis of intermediate M7-3

At room temperature, compound M7-2 (18.00 g), TMSCN (12.81 g), and potassium carbonate (19.83 g) were added to THF (200.00 mL), and then under $N_2$ protection conditions, the temperature was controlled at 0-5°C, and TBAF (7.17 mL, 1M-THF) was slowly added in batches. After the addition was completed, the ice water bath was removed and the reaction was carried out at room temperature at 25°C for 14 hours. The reaction solution was added to 500 mL of ice

water to quench, and then extracted with 150 mL of EA 4 times, washed with 100 mL of saturated sodium chloride twice, dried over anhydrous sodium sulfate, filtered, and the organic phase was directly concentrated. The concentrate was separated by column chromatography (PE: EA = 10: 1 to 60: 40) to obtain the target product M7-3 (12.10 g, yield 85.61%). ESI-MS m/z:197.0 [M+H]+.

Step 4: Synthesis of intermediate M7-4

At room temperature, compound NaH (60%) was added to DMSO (240.00 mL), and then under $N_2$ protection conditions, the temperature was controlled at 18-25°C, and a solution of M7-3 (12.10 g) and 2,2'-dibromodiethyl ether dissolved in ether (60.00 mL) and DMSO (15.00 mL) was slowly added dropwise in batches to the reaction system, and the reaction was carried out at room temperature at 25°C for 3 hours. The reaction solution was added to 500 mL of ice water to quench, then extracted 4 times with 150 mL of EA, washed 5 times with 150 mL of saturated sodium chloride, dried over anhydrous sodium sulfate, filtered, and the organic phase was directly concentrated. The concentrate was separated by column chromatography (PE: EA = 10: 1 to 60: 40) to obtain the target product M7-4 (13.20 g, yield 77.89%). ESI-MS m/z:267.2[M+H]+.

Step 5: Synthesis of intermediate M7-5

At room temperature, compound M7-4 (6.00 g) was added to anhydrous DCM (100.00 mL), and then under $N_2$ protection conditions, the temperature was controlled at -78°C, and DIBAL-H (24.33 mL, 1M/Hexane) was slowly added dropwise in batches. The temperature was controlled at -70 to -60°C for 3 hours, and the reaction was stopped. The temperature was raised to room temperature, 10 mL of 15% HCl was added to the reaction solution to quench, saturated sodium bicarbonate was added to adjust the pH to 8-9, and then extracted 4 times with 60 mL of DCM, washed twice with 100 mL of saturated sodium chloride, dried over anhydrous sodium sulfate, filtered, and the organic phase was directly concentrated and dried to obtain a crude product. The crude product was separated by column chromatography (PE: EA = 10: 1-40: 60) to obtain the target product M7-5 (4.00 g, yield 65.93%). ESI-MS m/z:270.2[M+H]+.

Step 6: Synthesis of intermediate M7-6

At room temperature, compound M7-5 (4.00 g), p-toluenesulfonic acid (0.26 g), and ethylene glycol (1.84 g) were added to toluene (50.00 mL), and then the mixture was allowed to react for 3 hours under reflux conditions using a water separator to separate water from toluene, and the reaction was stopped. After cooling to room temperature, the reaction solution was directly concentrated and dried to obtain a crude product, which was separated by column chromatography (PE:EA=10:1-40:60) to obtain the target product M7-6 (4.50 g, yield 52.58%). ESI-MS m/z:314.2[M+H]+.

Step 7: Synthesis of intermediate M7-7

At room temperature, compound M7-6 (4.50 g), tributyl(1-ethoxyethylene)tin (6.72 g), LiCl (1.82 g), triethylamine (4.35 g), and $Pd(PPh_3)$ 4 (1.66 g) were added to 1,4-dioxane (80.00 mL), and then the reaction was carried out at 110°C under nitrogen protection for 14 hours, and the reaction was stopped. The mixture was cooled to room temperature, and 200 mL of 5% KF aqueous solution was added to the reaction solution to quench for 0.5 h, then extracted with 60 mL of DCM for 5 times, washed with 100 mL of saturated sodium chloride for 2 times, dried over anhydrous sodium sulfate, filtered, and the organic phase was directly concentrated and dried to give a crude product. The crude product was separated by column chromatography (PE: EA = 10: 1 ~ 40: 60) to give the target product M7-7 (2.30 g, yield 52.58%). ESI-MS m/z:306.4[M+H]+.

Step 8: Synthesis of Intermediate M7-8

At room temperature, compound M7-7 (2.30 g) was added to 6M HCl (10.00 mL) and DCM (10.00 mL), and then the reaction was carried out at 50°C for 1 h under $N_2$ protection conditions to stop the reaction. The reaction solution was added to 100 mL of ice water to quench, and 30% NaOH was added to adjust the pH to 12-13, then extracted three times with 50 mL of DCM, washed twice with 100 mL of saturated sodium chloride, dried over anhydrous sodium sulfate, filtered, and the organic phase was directly concentrated and dried to obtain a crude product. The crude product was separated by column chromatography (DCM: MeOH = 96:4-90:10) to obtain the target product M7-8 (0.9 g, yield 55.51%). ESI-MS m/z:216.3[M+H]+.

Step 9: Synthesis of intermediate M7-9

At room temperature, compound M7-8 (1.80 g) and 10% palladium on carbon (0.18 g) were added to methanol (40.00 mL), and then the mixture was reacted at 25°C for 34 h under $H_2$ conditions, and the reaction was stopped. Celite was added to filter the palladium carbon, and then washed with 20 mL of methanol 5 times. The combined reaction liquid was directly concentrated and dried to obtain a crude product. The crude product was separated by column chromatography (DCM:EA=10:1-50:50) to obtain the target product M7-9 (1.70 g, yield 52.58%). ESI-MS m/z:220.3[M+H]+.

Step 10: Synthesis of intermediate M7-10

At room temperature, compound M7-9 (1.70 g) was added to DCM (40.00 mL), and then $SOCl_2$ (2.48 mL) was slowly added in batches under the protection of $N_2$, while the temperature was controlled at 0-5°C. After the addition was completed, the ice water bath was removed, and the reaction was carried out at room temperature at 25°C for 2 h to stop the reaction. The reaction solution was added to 50 mL of ice water to quench, saturated sodium bicarbonate was added to adjust the pH to 8-9, and then extracted with 50 mL of DCM for 3 times, washed with 100 mL of saturated sodium chloride for 2 times, dried over anhydrous sodium sulfate, filtered, and the organic phase was directly concentrated and dried to obtain a crude product. The crude product was separated by column chromatography (DCM: MeOH = 95:5-93:7) to obtain the target product M7-10 (1.40 g, yield 86.09%). ESI-MS m/z:238.7[M+H]+.

Step 11: Synthesis of intermediate M7-11

At room temperature, compound M7-10 (1.43 g) was added to DCM (50.00 mL), and then m-chloroperbenzoic acid (2.44 g, 85%) was slowly added in batches under $N_2$ protection conditions. The reaction was carried out at room temperature of 25°C for 1 h, and the reaction was stopped. The reaction solution was added to 100 mL of saturated sodium bicarbonate to quench, then extracted 4 times with 30 mL of DCM, washed twice with 100 mL of saturated sodium chloride, dried over anhydrous sodium sulfate, filtered, and the organic phase was directly concentrated and dried to obtain a crude product. The crude product was separated by column chromatography (DCM: MeOH = 95:5 to 93:7) to obtain the target product M7-11 (1.50 g, yield 98.28%). ESI-MS m/z:254.7[M+H]+.

Step 12: Synthesis of intermediate M7-12

At room temperature, compound M7-11 (1.30 g) and pyridine (2.06 mL) were added to DCM (40.00 mL), and then trifluoromethanesulfonic anhydride (1.72 mL) was slowly added in batches under $N_2$ protection conditions. The reaction was carried out at room temperature of 25°C for 1 h, and the reaction was stopped. The reaction solution was concentrated to obtain a crude product, which was then slurried with 10 mL of ether, filtered, and the solid was directly drained to obtain the target product M7-12 (2.0 g, yield 123.59%). ESI-MS m/z:254.7[M+H]+.

Step 13: Synthesis of intermediate M7-13

At room temperature, compound M7-12 (2.00 g) and ethanolamine (11.91 mL) were added to ethanol (15.00 mL), and then reacted at room temperature (25°C) for 3 h under $N_2$ protection conditions, and then the reaction was stopped. The reaction solution was added into 20 mL of saturated sodium bicarbonate to quench, and a large amount of light yellow solid precipitated. After filtration, the solid was directly dried to obtain the target product M7-13 (1.10 g, yield 68.72%). ESI-MS m/z:253.8[M+H]+.

Step 14: Synthesis of intermediate M7

At room temperature, compound M7-13 (1.05 g), KI (137.97 mg), cesium carbonate (4.06 g) and dimethylamine (16.62 mL, 2 M/THF) were added to DMA (15.00 mL), and then the reaction was carried out at 100° C for 4 h under $N_2$ protection conditions, and the reaction was stopped. The reaction solution was directly concentrated to obtain a crude product, which was separated by column chromatography (DCM:MeOH=95:5-90:10) to obtain the target product M7 (1.05 g, yield 96.70%). ESI-MS m/z:262.1[M+H]+.

Synthesis of intermediate M8:

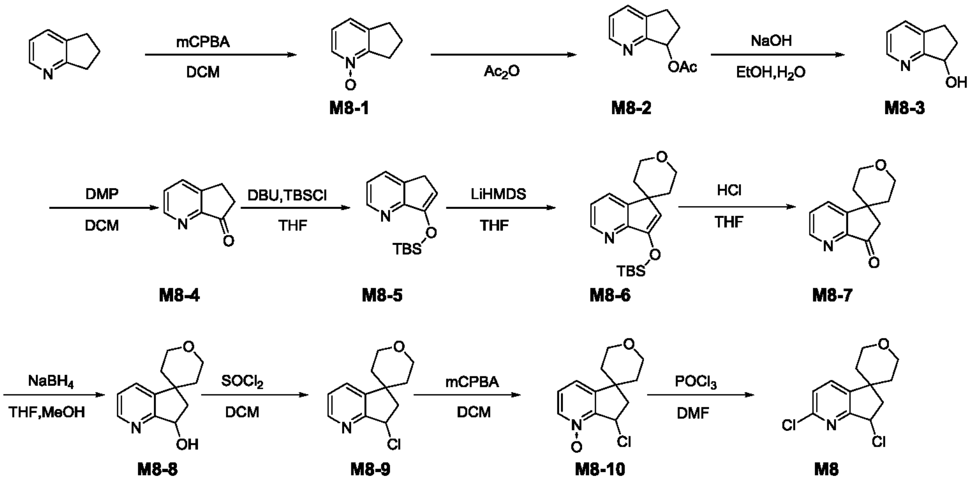


Step 1: Synthesis of compound M8-1

The temperature was controlled at 15-30°C in an ice-water bath. To a DCM solution (1500 mL) of compound 6,7-dihydro-5H-cyclopenta[b]pyridine (240.0 g) was added mCPBA (490.7 g, 85%) in batches. After the addition was complete, the reaction was stirred at room temperature for 1.5 hours. The reaction solution was directly purified by column chromatography to obtain compound M8-1 (143.60 g, 52% yield). ESI-MS m/z:136.05[M+H]+.

Step 2: Synthesis of compound M8-2

Under nitrogen protection, a solution of M8-1 (41.0 g) in acetic anhydride (200 mL) was heated at 110-120°C for 1.5 hours. The solvent was partially removed by concentration under reduced pressure, water was added, and the mixture was extracted with EA. The organic layers were combined, washed with saturated brine, dried over anhydrous sodium sulfate, concentrated under reduced pressure, and purified by column chromatography (PE/EA=100 to 3/1) to obtain compound M8-2 (37.7 g, 71% yield). ESI-MS m/z:178.12[M+H]+.

Step 3: Synthesis of compound M8-3

Under ice bath, to a solution of M8-2 (89.2) in EtOH (400 mL) was added dropwise a solution of NaOH (40.3 g) in water (400 mL), the ice bath was removed, the temperature was raised to room temperature, and the reaction was carried out for 1 hour. The mixture was concentrated under reduced pressure, water was added, and the mixture was extracted with DCM. The organic layers were combined, washed with saturated brine, dried over anhydrous sodium sulfate, and concentrated under reduced pressure to obtain compound M8-3 (59.8 g, 88% yield). ESI-MS m/z:136.08[M+H]+.

Step 4: Synthesis of Compound M8-4

The temperature was controlled at 15-30°C in an ice-water bath. Dess-Martin periodinane (206.4 g) was added in portions to M8-3 (59.8 g) in DCM (400 mL). After the addition was complete, the mixture was stirred at room temperature for 2 hours. The reaction solution was added to 10% aqueous sodium carbonate solution to quench, filtered, the filtrate was extracted with DCM, the organic layer was washed with 5% aqueous sodium sulfite solution, washed with saturated sodium chloride, dried over anhydrous sodium sulfate, concentrated under reduced pressure, and purified by column chromatography (PE:EA=1:1-1:4) to give compound M8-4 (47.8 g, 81% yield). ESI-MS m/z:134.11[M+H]+.

Step 5: Synthesis of Compound M8-5

At room temperature, add DBU (132.4 mL) to THF (700 mL) of M8-4 (59.0 g), control the temperature below 25°C in an ice-water bath, add tert-butyldimethylsilyl chloride (80.1 g) dropwise, and stir the reaction at room temperature for 2 hours. The reaction solution was added to saturated aqueous ammonium chloride solution to quench, extracted with EA, washed with saturated brine, dried over anhydrous sodium sulfate, concentrated under reduced pressure, and purified by column chromatography (PE:EA=95:5) to obtain compound M8-5 (103.5 g, 94% yield).

Step 6: Synthesis of Compound M8-6

Under nitrogen protection, M8-5 (56.0 g) was dissolved in THF (700 mL), cooled to -70°C with a dry ice ethanol bath, and LiHMDS (679 mL, 1.0 mol/L) was added dropwise. After the addition was completed, the temperature was slowly raised to -30 to -20°C and kept for reaction for 2 hours. Then 2,2'-dibromodiethyl ether (57.7) was added dropwise. After the addition was completed, the temperature was naturally raised to room temperature and stirred for reaction for 3 hours. The reaction solution was added to saturated aqueous ammonium chloride solution to quench, extracted with EA, and the organic layers were combined, washed with saturated brine, dried over anhydrous sodium sulfate, and concentrated under reduced pressure to obtain a crude product of compound M8-6 (88.2 g), which was directly used in the next reaction. ESI-MS m/z:318.33[M+H]+.

Step 7: Synthesis of Compound M8-7

M8-6 (88.0 g, crude product) was dissolved in THF (400 mL), cooled in an ice-water bath, and then a solution of hydrochloric acid (92 mL) in water (90 mL) was added dropwise. After the addition was completed, the mixture was stirred at room temperature for 4 hours. Concentrate to remove THF, add water to dissolve, extract with EA to remove impurities, then neutralize the acidic aqueous layer with sodium carbonate to pH about 9, extract with DCM, combine the organic phases, dry over anhydrous sodium sulfate, and concentrate under reduced pressure to obtain compound M8-7 (40.5 g, 72% yield). ESI-MS m/z:204.16[M+H]+.

Step 8: Synthesis of Compound M8-8

Compound M8-7 (24.3 g) was dissolved in THF (100 mL) and MeOH (300 mL), cooled in an ice-water bath, and sodium borohydride (6.8 g) was added in portions. After the addition, the mixture was stirred at room temperature for 1.5 hours. The reaction solution was concentrated, and the concentrate was added to saturated ammonium chloride, extracted with DCM, and the organic phases were combined, dried over anhydrous sodium sulfate, concentrated under reduced pressure, and separated and purified by column chromatography (DCM/MeOH=100-100/5) to obtain compound M8-8 (17.0 g, 69% yield). ESI-MS m/z:206.25[M+H]+.

Step 9: Synthesis of Compound M8-9

Compound M8-8 (17.0 g) was dissolved in DCM (350 mL), cooled in an ice-water bath, and thionyl chloride (29.6 g) was added dropwise. After the addition was completed, the mixture was stirred at room temperature for 3 hours. The reaction solution was added to a cold aqueous sodium bicarbonate solution to quench, and sodium carbonate was added to a pH of about 7-8. The mixture was extracted with DCM, and the organic phases were combined, dried over anhydrous sodium sulfate, and concentrated under reduced pressure to obtain compound M8-9 (18.5 g, 99% yield). ESI-MS m/z:224.20[M+H]+.

Step 10: Synthesis of Compound M8-10

Compound M8-9 (18.1 g) was dissolved in DCM (250 mL), cooled in an ice-water bath, and the temperature was controlled at 15-30°C. mCPBA (36 g) was added in batches, and the mixture was stirred at room temperature for 4 hours. The reaction solution was cooled to below 5°C and the temperature was controlled below 10°C. Aqueous sodium carbonate solution was added dropwise to a pH of about 8-9 to quench the solution. The mixture was extracted with DCM, and the organic phases were combined, washed with 5% aqueous sodium sulfite solution, dried over anhydrous sodium sulfate, concentrated under reduced pressure, and purified by column chromatography to obtain compound M8-10 (12.6 g, 65% yield). ESI-MS m/z:240.08[M+H]+.

Step 11: Synthesis of Compound M8

Compound M8-10 (6.8 g) was dissolved in DMF (100 mL), cooled in an ice-water bath, and the temperature was controlled at 0-10°C. Phosphorus oxychloride (21.8 g) was added dropwise, and the mixture was stirred at room temperature for 9 hours after the addition was completed. The reaction solution was added to an ice-water mixture, filtered, and the filter cake was collected, dissolved in DCM, dried over anhydrous sodium sulfate, and concentrated under reduced pressure to obtain compound M8 (5.4 g, 71% yield). ESI-MS m/z:258.14[M+H]+. Synthesis of intermediate M9:

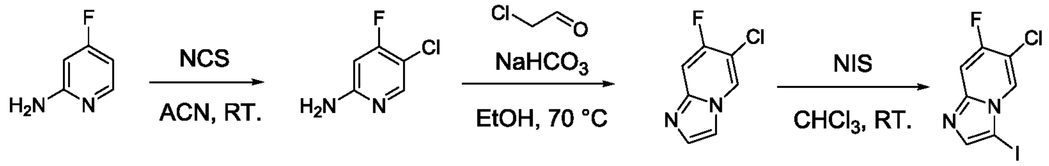


M9-1 M9-2 M9

Step 1: Synthesis of compound M9-1

At room temperature, 2-amino-4-fluoropyridine (3.00 g) was dissolved in acetonitrile (30.00 mL), and then NCS (3.93 g) was added in batches. The mixture was reacted at room temperature for 3 h, and then the reaction was stopped. The reaction was quenched with saturated sodium thiosulfate solution, extracted three times with EA, and the organic phases were combined, washed with saturated brine, dried over anhydrous sodium sulfate, and concentrated. The concentrate was separated by silica gel column chromatography (PE:EA=3:1) to obtain the target product M9-1 (1.00 g, yield 25.50%). ESI-MS m/z:113.0[M+H]+.

Step 2: Synthesis of compound M9-2

At room temperature, compound M9-1 (986.00 mg), chloroacetaldehyde (4.00 g, 40% in water), and $NaHCO_3$ (1.13 g) were dissolved in ethanol (20.00 mL), reacted at 80°C for 13 h, and then the reaction was stopped. The reaction solution was directly concentrated, mixed, and separated by column chromatography (PE:EA=3:1) to obtain the target product M9-2 (259 mg, yield 22.57%). ESI-MS m/z:171.0[M+H]+.

Step 3: Synthesis of Compound M9

At room temperature, compound M9-2 (259.00 mg) was dissolved in chloroform (5.00 mL), and then NCS (3.93 g) was added in portions. The mixture was reacted at room temperature for 7 h, and then the reaction was stopped. The reaction solution was directly concentrated, mixed, and separated by column chromatography (PE:EA=4:1) to obtain the target product M9 (218 mg, yield 48.43%). ESI-MS m/z:29.0[M+H]+.

Synthesis of intermediate M10:

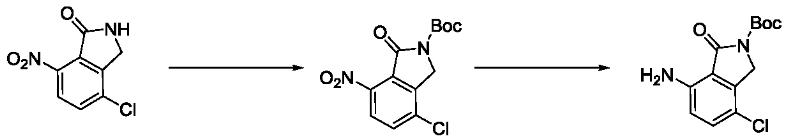

M10-1 M10

Step 1: Synthesis of compound M10-1

At room temperature, 4-chloro-7-nitroisoindolin-1-one (3.00 g) and DMAP (3.00 g) were added to THF (30.00 mL), and $Boc_2O$ (3.89 mL) was added dropwise under ice-water bath, and then reacted at room temperature for 1 h. The reaction solution was added dropwise into water (200.00 mL), filtered, and the filter cake was washed three times with water and dried to obtain the target product M10-1 (3.50 g, yield 79.31%). ESI-MS m/z:313.1[M+H]+.

Step 2: M10 synthesis of compound

Compound M10-1 (4.20 g), ammonium chloride (1.44 g) and water (8.00 mL) were added to ethanol (40.00 mL) at room temperature, and then iron powder (3.00 g) was added in portions at 70°C, and the mixture was reacted at 70°C for 1 h. The reaction solution was filtered, and the filter cake was washed twice with DCM. The filtrate was concentrated, diluted with water, and filtered. The filter cake was separated by column chromatography (PE:EA=65:35) to obtain the target product M10 (3.10 g, purity 81.64%). ESI-MS m/z:283.1[M+H]+.

Synthesis of intermediate M11:

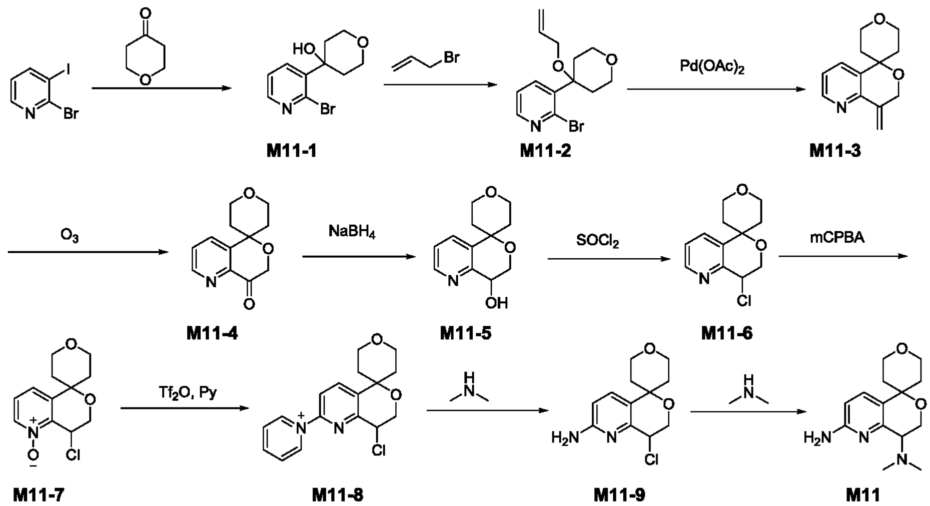


M11-1 M11-2 M11-3 M11-4 M11-5 M11-6 M11-7 M11-8 M11-9 M11

Step 1: Synthesis of compound M11-1

Dissolve 2-bromo-3-iodopyridine (10 g) in THF (150 mL), then place the reaction system at -20°C, slowly add isopropylmagnesium chloride (4.4 g) dropwise under nitrogen protection, react at this temperature for 2 h, add tetrahydropyrone (5.3 g), and then stir at room temperature overnight. The reaction solution was quenched with saturated ammonium chloride solution, concentrated under reduced pressure to remove part of the solvent, extracted with EA, and the organic layers were combined, washed with 1M HCl, water and saturated brine in sequence, dried over anhydrous sodium sulfate, concentrated under reduced pressure, and purified by column chromatography (PE:EA=70:30-65:35) to give compound M11-1 (3.2 g, 35% yield). ESI-MS m/z:258.0[M+H]+.

Step 2: Synthesis of compound M11-2

Compound M11-1 (3.0 g) was dissolved in DMF (25 mL), and NaH (0.74 g) was added under ice bath. After the addition was complete, the mixture was stirred at room temperature for 30 min. Subsequently, a DMF solution of 3-bromopropylene (1.6 g) and TBAI (0.34 g) was added dropwise, and the reaction was stirred at room temperature overnight. The mixture was quenched with water, concentrated under reduced pressure to remove part of the solvent, extracted with EA, and the organic layers were combined, washed with saturated brine, dried over anhydrous sodium sulfate, concentrated under reduced pressure, and purified by column chromatography (PE:EA=70:30-65:35) to give compound M11-2 (2.8 g, 80% yield). ESI-MS m/z:298.1[M+H]+.

Step 3: Synthesis of compound M11-3

Compound M11-2 (1.5 g), Pd(OAc)$_2$ (0.17 g), XantPhox (0.44 g), and TEA (2.1 ml) were dissolved in ACN (15 mL) and reacted at 90° C. for 6 h under nitrogen protection.After the reaction was completed, the residue was concentrated under reduced pressure and purified by column chromatography (PE:EA=70:30-65:35) to obtain compound M11-3 (0.84 g, 77% yield). ESI-MS m/z:218.1[M+H]+.

Step 4: Synthesis of compound M11-4

Compound M11-3 (0.34 g) was dissolved in MeOH (5 mL) and DCM (10 mL), and ozone was introduced at -50°C. After reacting for 30 min, dimethyl sulfide (0.34 g) was added.After the reaction was completed, the residue was concentrated under reduced pressure and purified by column chromatography (PE:EA=70:40-50:50) to obtain compound M11-4 (0.24 g, 83% yield).

ESI-MS m/z:220.1[M+H]+.

Step 5: Synthesis of Compound M11-5

Under ice bath, sodium borohydride (38.8 mg) was added portionwise to a solution of M11-4 (0.15 g) in MeOH (2.5 mL) and THF (2.5 mL), the ice bath was removed, and the mixture was reacted at room temperature for 1 hour. The reaction mixture was quenched with water, concentrated under reduced pressure to remove part of the solvent, extracted with EA, and the organic layers were combined, washed with saturated brine, dried over anhydrous sodium sulfate, and concentrated under reduced pressure to give compound M11-5 (112 mg, 74% yield).
ESI-MS m/z:222.1[M+H]+.

Step 6: Synthesis of Compound M11-6

Under ice bath, thionyl chloride (0.18 mL) was added dropwise to a solution of M11-5 (0.11 g) in DCM (5 mL), the ice bath was removed, the temperature was raised to 40°C, and the reaction was carried out for 1 hour. Concentrate under reduced pressure, add a small amount of methanol to dissolve, adjust the pH to alkaline with saturated sodium bicarbonate solution, extract with EA, combine the organic layers, wash with saturated brine, dry over anhydrous sodium sulfate, concentrate under reduced pressure, and purify by column chromatography (PE:EA=20:80) to obtain compound M11-6 (115 mg, 96% yield). ESI-MS m/z:239.0[M+H]+.

Step 7: Synthesis of Compound M11-7

At room temperature, M11-6 (410 mg) was dissolved in DCM (20 mL), and mCPBA (590 mg) was added at room temperature. After reacting at room temperature for 3 h, LC-MS detected that the reaction of the raw material was complete. Saturated sodium bicarbonate solution (10 mL) was added, and the mixture was stirred at room temperature for 30 min. The mixture was extracted with DCM, and the organic layers were combined, washed with saturated brine, dried over anhydrous sodium sulfate, concentrated under reduced pressure, and purified by column chromatography (DCM:MeOH=20:1) to give compound M11-7 (428 mg, 98% yield).
ESI-MS m/z:256.1[M+H]+.

Step 8: Synthesis of Compound M11-8

M11-7 (400 mg) and pyridine (1.2 g) were dissolved in DCM (10 mL), and $Tf_2O$ (1.3 g) was added under ice bath. After reacting at room temperature for 0.5 h, LC-MS detected that the reaction of the raw material was complete. The residue was concentrated under reduced pressure and purified by column chromatography (DCM:MeOH=20:1) to obtain compound M11-8 (472 mg, 96% yield). ESI-MS m/z:317.1[M+H]+.

Step 9: Synthesis of Compound M11-9

M11-8 (250 mg) was dissolved in dimethylamine tetrahydrofuran solution (15 mL) at room temperature and stirred for 0.5 h, then concentrated under reduced pressure and purified by column chromatography (DCM:MeOH=20:1) to obtain compound M11-9 (175 mg, 87% yield). ESI-MS m/z:255.1[M+H]+.

Step 10: Synthesis of Compound M11

M11-9 (150 mg), KI (12 mg), $Cs_2CO_3$ (575 mg), and methylamine tetrahydrofuran solution (2.4 mL) were dissolved in DMA (5.0 mL), stirred at 100°C overnight, concentrated under reduced pressure, and purified by column chromatography (DCM:MeOH=20:1) to give compound M11 (150 mg, 97% yield). ESI-MS m/z:264.2[M+H]+.

Synthesis of intermediate M12

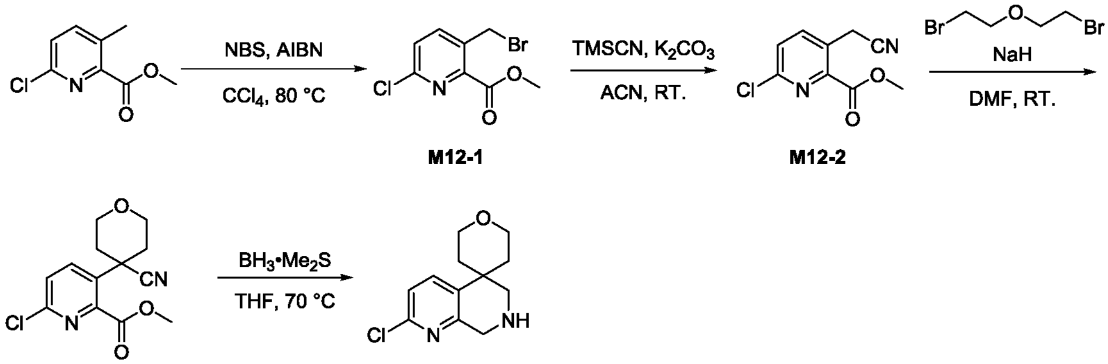


M12-1 M12-2 M12-3 M12

Step 1: Synthesis of compound M12-1

At room temperature, methyl 6-chloro-3-methyl-2-pyridinecarboxylate (33.00 g) was dissolved in carbon tetrachloride (400.00 mL), and then NBS (33.12 g) and AIBN (2.91 g) were added. Under nitrogen protection, the reaction was carried out at 80° C. for 3 h, and the reaction was stopped. A certain amount of water was added to the reaction solution, and the mixture was extracted three times with DCM. The organic phases were combined, washed with saturated brine, dried over anhydrous sodium sulfate, and concentrated. The concentrate was separated by silica gel column chromatography (PE:EA=2:1) to obtain the target product M12-1 (22.00 g, yield 46.81%). ESI-MS m/z:186.0$[M+H]^+$.

Step 2: Synthesis of compound M12-2

At room temperature, compound M12-1 (22.00 g) and $K_2CO_3$ (19.50 g) were dissolved in ACN (300.00 mL), and then TMSCN (12.38 g) was slowly added dropwise under ice bath conditions. The reaction was allowed to react at room temperature for 13 h, and then the reaction was stopped. A certain amount of water was added to the reaction solution, extracted three times with EA, the organic phases were combined, washed with saturated brine, dried over anhydrous sodium sulfate, and concentrated. The concentrate was separated by silica gel column chromatography (PE:EA=2:1) to obtain the target product M12-2 (10.00 g, yield 57.00%).

ESI-MS m/z:211.3[M+H]+.

Step 3: Synthesis of compound M12-3

At room temperature, compound NaH (2.29 g) was dissolved in DMF (65.00 mL), and then M12-2 (5.23 g) was slowly added under nitrogen protection in an ice bath, and the reaction was kept warm for 0.5 h. Then 2,2'-dibromodiethyl ether (9.20 g) was slowly added dropwise, and the reaction was carried out at room temperature for 3 h to stop the reaction. The reaction was quenched with saturated ammonium chloride solution under ice bath condition, extracted with EA three times, and the organic phases were combined, washed with saturated brine, dried over anhydrous sodium sulfate, and concentrated. The concentrate was separated by silica gel column chromatography (PE:EA=3:1) to obtain the target product M12-3 (4.00 g, yield 57.38%). ESI-MS m/z:281.2[M+H]+.

Step 4: Synthesis of Compound M12

At room temperature, compound M12-3 (3.00 g) was dissolved in THF (45.00 mL), and then borane dimethyl sulfide solution (4.3 mL, 10 M) was slowly added dropwise under ice bath conditions. Under nitrogen protection, the reaction was carried out at 70°C. for 16 h, and the reaction was stopped. The pH was adjusted to 1-2 with 1M HCl solution, and then heated to 70°C for 1 h to stop the reaction. The pH value was adjusted to 8-9 with 2M NaOH solution, extracted three times with EA, the organic phases were combined, washed with saturated brine, dried over anhydrous sodium sulfate, and concentrated. The concentrate was separated by silica gel column chromatography (DCM:MeOH=19:1) to give the target product M12 (750.00 mg, yield 29.41%).

ESI-MS m/z:239.4[M+H]+.

Synthesis of intermediate M13

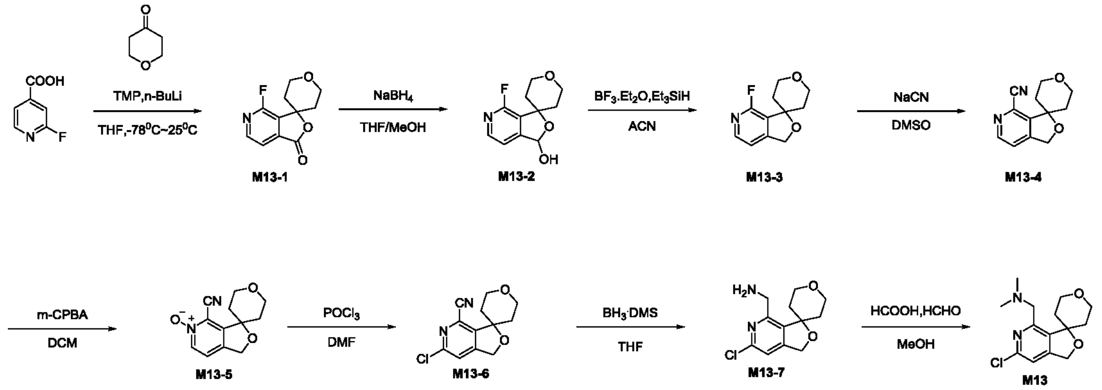


Step 1: Synthesis of intermediate M13-1

At room temperature, compound 2,2,6,6-tetramethylpiperidine (10.01 g) was added to THF (120.00 mL), and then under N 2 protection conditions, the temperature was controlled at -78°C, and n-butyl lithium (9.33 mL, 2.5 M/Hexane) was slowly added dropwise in batches. After the addition was completed, the temperature was controlled at -78°C to react for 40 min, and then a solution of 2-fluoro-4-pyridinecarboxylic acid (5.00 g) in THF (30.00 mL) was slowly added dropwise to the reaction system, the temperature was controlled at -78°C to react for 2 h, and tetrahydropyrone (7.10 g) was slowly added at -78°C. The temperature was returned to room temperature and the reaction was carried out overnight to stop the reaction. The reaction solution was quenched by adding 250 mL of ice water, and then extracted with 100 mL of EA three times. The organic phase was washed twice with 100 mL of saturated sodium chloride and dried over anhydrous sodium sulfate. The organic phase was filtered and concentrated directly to obtain a crude product. The crude product was separated by column chromatography (DCM: MeOH = 96:4 ~ 85:15) to obtain a white solid, i.e., the target product M13-1 (5.20 g, yield 65.75%). ESI-MS m/z:224.23[M+H]+.

Step 2: Synthesis of intermediate M13-2

At room temperature, compound M13-1 (4.50 g) was added to THF (15.00 mL) and MeOH (45.00 mL), and then under the protection of $N_2$, the temperature was controlled at 0-5°C and $NaBH_4$ (2.29 g) was slowly added in batches. After the addition was completed, the ice water bath was removed, and the reaction was carried out at room temperature at 25°C for 2 h, and the reaction was stopped. The reaction solution was added into 50 mL of methanol to quench, and then directly concentrated to obtain a crude product. The crude product was separated by column chromatography (DCM: MeOH = 96:4-90:10) to obtain a white solid, namely the target product M13-2 (3.50 g, yield 77.08%). ESI-MS m/z:226.23[M+H]+.

Step 3: Synthesis of intermediate M13-3

At room temperature, compound M13-2 (3.50 g) was added to ACN (60.00 mL), and then triethylsilane (9.90 mL) was slowly added under $N_2$ protection conditions, and boron trifluoride ether (3.92 mL) was slowly added. The reaction was carried out at room temperature at 25°C for 2 h, and the reaction was stopped. The reaction solution was added to 100 mL of saturated sodium bicarbonate to quench, and then extracted with 60 mL of EA three times. The organic phase was washed twice with 50 mL of saturated sodium chloride, dried over anhydrous sodium sulfate, filtered, and the organic phase was directly concentrated and dried to obtain a crude product. The crude product was separated by column chromatography (PE: EA = 10:1 to 1:1) to obtain a white solid, the target product M13-3 (3.10 g, yield 95.34%). ESI-MS m/z:210.21,[M+H]+.

Step 4: Synthesis of intermediate M13-4

At room temperature, compound M13-3 (1.50 g) and NaCN (0.88 g) were added to DMSO (6.00 mL), and then the reaction was controlled at 150° C. under $N_2$ protection conditions for 2.5 h, and the reaction was stopped. The reaction solution was added to 100 mL of saturated sodium bicarbonate to quench, and then extracted with 50 mL of EA for 3 times. The organic phase was washed twice with 50 mL of saturated sodium chloride, dried over anhydrous sodium sulfate, filtered, and the organic phase was directly concentrated and dried to obtain a crude product. The aqueous phases were combined and sodium hypochlorite solution was added to quench the sodium cyanide waste liquid. The crude product was separated by column chromatography (PE: EA = 10: 1 to 1: 1) to obtain a white powdery solid, i.e., the target product M13-4 (1.41 g, yield 87.72%).

ESI-MS m/z:217.21,[M+H]+.

Step 5: Synthesis of intermediate M13-5

At room temperature, compound M13-4 (1.40 g) was added to DCM (20.00 mL), and then m-chloroperbenzoic acid (2.23 g, 85%) was slowly added in batches under $N_2$ protection conditions. The reaction was carried out at room temperature of 25°C for 24 hours, and the reaction was stopped. The reaction solution was added to 100 mL of saturated sodium bicarbonate to quench, and then extracted with 40 mL of DCM three times. The organic phase was washed twice with 100 mL of saturated sodium chloride, dried over anhydrous sodium sulfate, filtered, and the organic phase was directly concentrated and dried to obtain a crude product. The crude product was separated by column chromatography (DCM: MeOH = 95:5 ~ 93:7) to obtain a white solid, i.e., the target product M13-5 (1.17 g, yield 77.81%). ESI-MS m/z:233.32[M+H]+.

Step 6: Synthesis of intermediate M13-6

At room temperature, compound M13-5 (1.10 g) was added to DMF (5.00 mL) for $N_2$ protection. Phosphorus oxychloride (5.00 mL) was slowly added in an ice-water bath, and the reaction was allowed to react for 1 h at room temperature, then kept at 50°C for 2.5 h. The reaction was stopped, and the reaction solution was added to 50 mL of ice water for quenching. Saturated sodium bicarbonate was added to adjust the pH to 8-9, and then extracted with EA50 mL 4 times, washed with saturated sodium chloride 100 mL twice, dried over anhydrous sodium sulfate, filtered, and the organic phase was directly concentrated and dried to obtain a crude product. The crude product was separated by column chromatography (PE: EA = 95:5-50:50) to obtain a white solid, i.e., the target product M13-6 (0.38 g, yield 32.00%). ESI-MS m/z:233.32[M+H]+.

Step 7: Synthesis of intermediate M13-7

At room temperature, compound M13-6 (0.36 g) was added to THF (15.00 mL), and then borane dimethyl sulfide (0.43 mL, 10 M) was slowly added under nitrogen protection and in an ice-water bath. The reaction was kept at 80° C. for 1.5 h and the reaction was stopped. The mixture was cooled to room temperature, 2 mL of ice water was added to the reaction solution, 10 mL of hydrochloric acid (6M-HCl) was continued to be added to the reaction solution, and the mixture was kept at 80°C for 1 h. Saturated sodium bicarbonate was added to adjust the pH to 8-9, and then extracted 4 times with 50 mL of EA, washed twice with 100 mL of saturated sodium chloride, dried over anhydrous sodium sulfate, filtered, and the organic phase was directly concentrated and dried to obtain a crude product. The crude product was separated by column chromatography (DCM: MeOH = 95:5-90:10) to obtain a colorless oil, i.e., the target product M13-7 (0.27 g, yield 73.81%). ESI-MS m/z:251.73[M+H]+.

Step 8: Synthesis of intermediate M13

At room temperature, compound M13-7 (0.24 g), formic acid (0.22 g), and formaldehyde aqueous solution (0.23 g, 37%) were added to methanol (3.00 mL), and then the reaction was carried out at 85°C. under $N_2$ protection conditions for 5 h, and the reaction was stopped. The reaction solution was added to 50 mL of ice water to quench, and saturated sodium bicarbonate was added to adjust the pH to 8-9, then extracted with 25 mL of EA for 5 times, washed with 30 mL of saturated sodium chloride for 2 times, dried over anhydrous sodium sulfate, filtered, and the organic phase was directly concentrated and dried to give a crude product. The crude product was separated by column chromatography (DCM: MeOH = 95:5-90:10) to give the target product M13 (0.20 g, yield 75.07%) as a colorless oil. ESI-MS m/z:283.72[M+H]+.

Synthesis of intermediate M14:

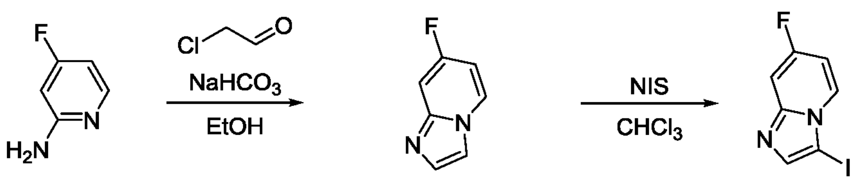


M14-1 M14

Step 1: Synthesis of compound M14-1

At room temperature, the compound 2-amino-4-fluoropyridine (20.00 g), 2-chloroacetaldehyde (50 ml, 40%/$H_2O$), and $NaHCO_3$ (37.47 g) were dissolved in ethanol (200.00 mL), reacted at 60°C for 4 hours, and then the reaction was stopped. The reaction solution was directly concentrated, mixed, and separated by column chromatography (DCM:MeOH=19:1) to obtain the target product M14-1 (22.00 g, yield 72.47%). ESI-MS m/z:136.95[M+H]+.

Step 2: Synthesis of compound M14

At room temperature, compound M14-1 (22.00 g) was dissolved in chloroform (200.00 mL), and $N_2$ was replaced. NIS (40.00 g) was added under ice bath, and the reaction was allowed to react at room temperature for 3 h, and then the reaction was stopped. The sample was stirred and separated by column chromatography (DCM:MeOH=40:1) to obtain the target product M14 (35.88 g, yield 84.73%). ESI-MS m/z:263.02[M+H]+.

Synthesis of intermediate M15:

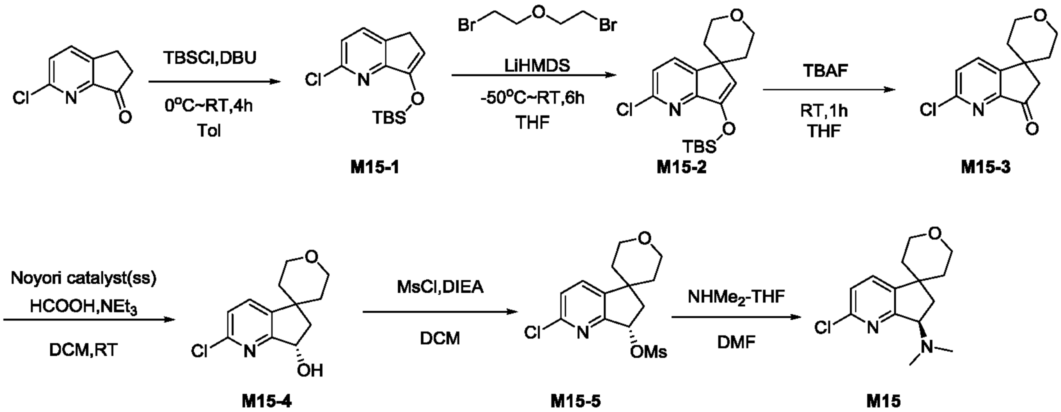


M15-1 M15-2 M15-3

M15-4 M15-5 M15

Step 1: Synthesis of compound M15-1

The raw materials 2-chloro-5,6-dihydrocyclopenta[B]pyridin-7-one (27.4 g) and TBSCl (36.96 g) were added to toluene (200 ml), and the temperature was controlled at 0-5°C under ice bath conditions, and a toluene solution of DBU (48.85 ml) was added dropwise. Then the temperature was raised to room temperature and the reaction was carried out for 1 hour. After the reaction was completed, the reaction solution was quenched with saturated ammonium chloride, and then extracted with EA three times (300 ml each time). The organic phase was washed with saturated brine, dried over anhydrous sodium sulfate, spin-dried, mixed, and separated by silica gel column chromatography (PE) to obtain the target product M15-1 (41.0 g, yield 88.97%).

ESI-MS m/z:282.10[M+H]+.

Step 2: Synthesis of compound M15-2

The raw material M15-1 (41.0 g) was added to THF (200 ml) to replace the $N_2$, and LiHMDS (363.67 ml) was added dropwise at -50°C. The temperature was raised to -20°C for reaction for 1 hour, 2,2'-dibromodiethyl ether (18.47 ml) was added dropwise, and then the temperature was raised to room temperature for reaction for 16 hours. The reaction solution was quenched with saturated ammonium chloride, and then extracted twice with EA (200 ml each time). The organic phase was washed with saturated brine, dried over anhydrous sodium sulfate, and spin-dried to obtain a crude product of compound M15-2, which was directly used for the next step reaction.

Step 3: Synthesis of compound M15-3

Compound M15-2 (51.0 g) was dissolved in THF (300 mL) at room temperature, and TBAF (144.91 ml) was added dropwise under ice-cooling conditions, followed by reaction at room temperature for 0.5 hours. The reaction solution was added to water, and saturated aqueous sodium bicarbonate solution was added to precipitate a solid. The

filter cake was washed with EA to obtain a white solid, namely the target product M15-3 (9.62 g, yield 27.93%).

ESI-MS m/z:238.06[M+H]+.

Step 4: Synthesis of compound M15-4

Compound M15-3 (4.3 g) was dissolved in DCM (60 mL), and triethylamine (12.57 ml), Noyori catalyst (ss) (0.46 g, 0.72 mmol), and $N_2$ were added. After substitution, formic acid (3.41 ml) was slowly added dropwise under ice bath conditions, and the temperature was controlled at 5-10°C. After completion of the addition, the mixture was reacted at room temperature for 4 hours. Water was added to the reaction solution, and then extracted with DCM (80 ml, 30 ml). The organic phases were combined, washed with saturated brine, dried over anhydrous sodium sulfate, spin-dried, mixed, and separated by silica gel column chromatography (PE:EA=2:1-1:1) to obtain a white solid, i.e., the target product M15-4 (3.67 g, yield 84.63%). ESI-MS m/z:240.07[M+H]+.

Step 4: Synthesis of compound M15-5

Compound M15-4 (1.0 g) was dissolved in DCM (25 mL), DIEA (2.07 ml) was added, and MsCl (0.48 ml) was added dropwise under ice bath conditions. After the addition was complete, the mixture was reacted at 10° C for 2 hours. The reaction solution was quenched with ice water and then extracted with DCM three times (25 ml each time). The organic phase was washed with saturated brine, dried over anhydrous sodium sulfate, chromatographed on a silica gel column, and then washed with DCM. The solvent was removed by spinning to obtain a concentrate, which was then slurried with PE:MTBE=2:1 and filtered to obtain a white solid, i.e., the target product M15-5 (1.16 g, yield 87.49%).

ESI-MS m/z:318.05[M+H]+.

Step 4: Synthesis of Compound M15

Compound M15-5 (1.16 g) was dissolved in DMF (10 mL), and a solution of dimethylamine in THF (9.13 ml, 2 M) was added, followed by stirring at room temperature for reaction overnight. The reaction solution was quenched with ice water, extracted with EA 4 times (15 ml each time), washed with saturated brine 4 times (10 ml each time), dried over anhydrous sodium sulfate, spin-dried, mixed, and separated by silica gel column chromatography (DCM:CH 3 OH=97:3-95:5) to give a white solid, i.e., the target product M15 (810 mg, yield 82.9%). ESI-MS m/z:267.12[M+H]+.

Synthesis of intermediate M16:

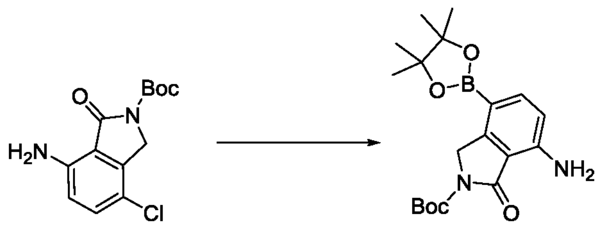

M10 M16

Step 1: Synthesis of compound M16

Compound M10 (40.00 g), diboronic acid pinacol ester (71.86 g), potassium acetate (41.65 g), $Pd_2(dba)_3$ (6.48 g) and Sphos (5.81 g) were dissolved in 1,4-Dioxane (500 mL), replaced with nitrogen, heated to 100°C, and reacted for 17 hours. The mixture was cooled, filtered, washed with DCM, and the filtrate was concentrated under reduced pressure. EA (40 ml) was added to the concentrate and dissolved by ultrasonic. PE (200 ml) was then slowly added dropwise at RT to precipitate a solid. The solid was filtered and further slurried with PE:MTBE=1:1. The solid was filtered and washed with PE to obtain compound M16 (42.42 g, 80% yield). ESI-MS m/z:375.2[M+H]+.

Example 1: Synthesis of Compound 7-(4-(dimethylamino)-2',3,3',4,5',6'-hexahydro-2H-spiro[naphthalene-1,4'-pyran]-6-yl)amino)-4 -(7-fluoroimidazo[1,2-a]pyridin-3-yl)isoindol-1-one

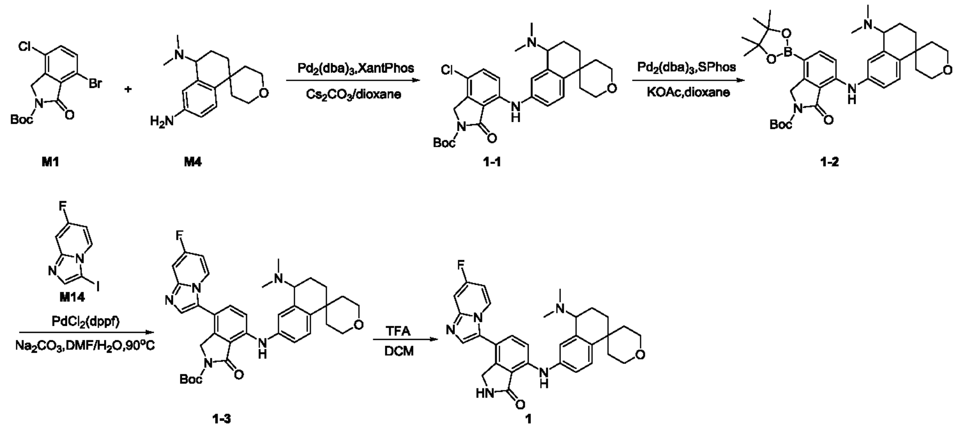


Step 1: Synthesis of compound 1-1

Under the protection of $N_2$, raw materials M4 (118 mg), M1 (191.71 mg), $Pd_2(dba)_3$ (21.06 mg), XantPhos (26.67 mg), and $Cs_2CO_3$ (300.34 mg) were added to 1,4-dioxane (5.00 mL) and heated at 90 degrees for 2 hours. The reaction solution was filtered, the filter cake was washed with EA, the filtrate was spin-dried, and the concentrate was separated by column chromatography (DCM:$CH_3OH$=30:1) to obtain the target product 1-1 (197 mg, yield 81.3%). ESI-MS m/z:526.1 [M+H]+.

Step 2: Synthesis of compound 1-2

At room temperature, compound 1-1 (165 mg), diboronic acid pinacol ester (159 mg), $Pd_2(dba)_3$ (29 mg), Sphos (26 mg), and potassium acetate (93 mg) were added to 1,4-dioxane (7.5 mL), and then reacted at 90°C for 18 hours under $N_2$ protection conditions, and the reaction was stopped. The reaction solution was filtered while hot, the filtrate was spin-dried, and the concentrate was separated by column chromatography (DCM:$CH_3OH$=20:1) to obtain the target product 1-2 (175 mg, yield 90.0%). ESI-MS m/z:618.6[M+H]+.

Step 3: Synthesis of Compound 1-3

Compound 1-2 (194 mg), M14, $[PdCl_2(dppf)]CH_2Cl_2$ (26 mg), and $Na_2CO_3$ (100 mg) were added to DMF (5 mL) and water (1 ml), and then reacted at 90°C for 2 h under $N_2$ protection conditions, and the reaction was stopped. The reaction solution was added with water and extracted with EA three times, 5 ml each time. The organic phases were combined, washed with saturated brine, dried over anhydrous sodium sulfate, and spin-dried. The concentrate was separated by column chromatography (DCM:$CH_3OH$=20:1) to obtain the target product 1-3 (102 mg, yield 51.9%). ESI-MS m/z:626.7[M+H]+.

Step 4: Synthesis of compound 1

At room temperature, compound 1-3 (102 mg) was added to dichloromethane (5.00 mL), and then trifluoroacetic acid (0.3 mL) was added in an ice-water bath under $N_2$ protection conditions. The reaction was allowed to react at room temperature for 1 h, and the reaction was stopped. The reaction solvent was removed by concentration, 10 mL of $H_2O$ and 10 mL of DCM were added to the reaction solution, and then NaOH was added to adjust the pH to 9, and then extracted with DCM for 3 times, 15 mL each time, the organic phases were combined, washed with saturated sodium chloride, and the organic phase was concentrated. The concentrate was separated by column chromatography (DCM:$CH_3OH$=10:1) to obtain the target product 1 (67.2 mg, yield 78.4%). ESI-MS m/z:526.6 [M+H]+. $^1$H NMR(500MHz,DMSO-d 6 ) δ9.08(s,1H), 8.68(s,1H), 8.37(t,J = 6.7Hz,1H), 7.7 7(s,1H), 7.59(d,J = 8.4Hz,1H),7.5507.37(m,3H),7.27(d,J = 8.5Hz,1H),7.21(d,J = 8.7Hz,1H), 6.97-6.93(m,1H), 4.36-4.28(m,2H), 3.78-3.59(m,5H), 2.47 (s,1H), 2.2 3(s,6H), 1.83-1.76(m,2H), 1.60-1.54(m,2H), 1.48-1.42(m,2H), 1.31-1.26 (m,2H).

Example 2: Synthesis and chiral resolution of compound 7-(7-(dimethylamino)-2',3',5',6,6',7-hexahydrospiro[cyclopentyl]pyridine-5,4'-pyran]-2-yl)amin o)-4-(7-fluoroimidazole[1,2-a]pyridin-3-yl)isoindol-1-one

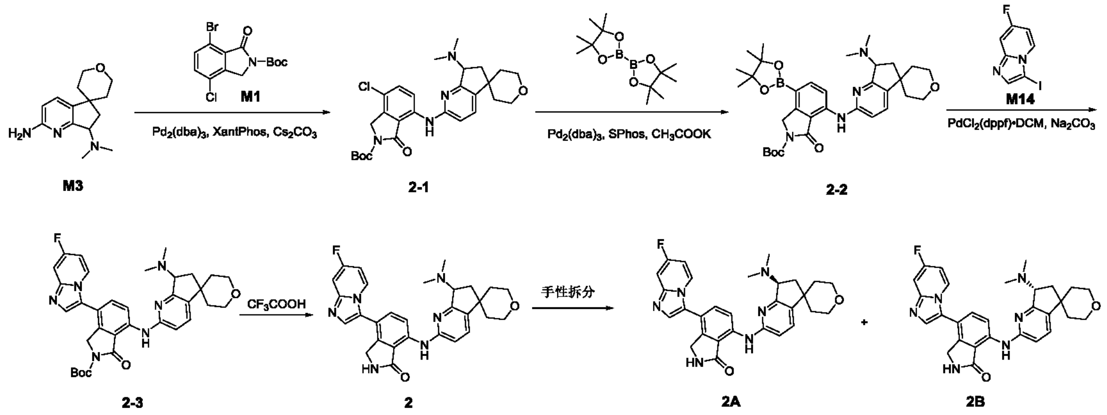


Step 1: Synthesis of compound 2-1

M3 (45 mg), M1 (76 mg), $Pd_2(dba)_3$ (17 mg), XantPhos (21 mg) and cesium carbonate (178 mg) were dissolved in 1,4-Dioxane (5 mL), replaced with nitrogen, heated to 90°C, and reacted for 3 hours. The mixture was cooled, filtered and washed with EA. The filtrate was concentrated under reduced pressure and purified by column chromatography (DCM:MeOH=92:8) to obtain compound 2-1 (73 mg, 78% yield). ESI-MS m/z:513.2[M+H]+.

Step 2: Synthesis of compound 2-2

Compound 2-1 (73 mg), diboronic acid pinacol ester (72 mg), $Pd_2(dba)_3$ (13 mg), SPhos (12 mg) and potassium acetate (42 mg) were dissolved in 1,4-Dioxane (5 mL), replaced with nitrogen, heated to 90°C, and reacted for 8 hours. The mixture was cooled, filtered, washed with EA, and the filtrate was concentrated under reduced pressure and purified by column chromatography (DCM:MeOH=93:7) to obtain compound 2-2 (82 mg, 95% yield). ESI-MS m/z:605.3[M+H]+.

Step 3: Synthesis of compound 2-3

Compound 2-2 (82 mg), M14 (53 mg), $[PdCl_2(dppf)]CH_2Cl_2$ (11 mg) and sodium carbonate (43 mg) were dissolved in DMF (4 mL) and water (0.5 mL), the nitrogen atmosphere was replaced, the temperature was raised to 90°C, and the reaction was carried out for 2 hours. The mixture was cooled, diluted with water, extracted with EA, the organic layers were combined, washed with saturated brine, dried over anhydrous sodium sulfate, concentrated under reduced pressure, and purified by column chromatography (DCM:MeOH=92:8) to obtain compound 2-3 (60 mg, 72% yield). ESI-MS m/z:613.2[M+H]+.

Step 4: Synthesis of compound 2

Compound 2-3 (60 mg) was dissolved in DCM (1 mL) and MeOH (1 mL), and hydrochloric acid (2 mL, 4M/1,4-Dioxane) was added. The temperature was raised to 50°C. and the reaction was carried out for 1 hour. The mixture was cooled, concentrated under reduced pressure, and purified by Pre-HPLC to obtain compound 2 (10 mg, 19.9% yield). ESI-MS m/z:513.2[M+H]+. $^1H$ NMR(500MHz,DMSO-$d_6$ )$\delta$10.14(s,1H),8.84(s,1H),8.73(d,J = 8.5Hz,1H),8.44(dd,J=7.6 ,5.7Hz,1H),7.82(s,1H),7.72(d,J = 8.6Hz,1H), 7.64(d,J = 8.4Hz,1H), 7.52(dd,J = 10.0, 2.7Hz, 1H), 6.97(td,J = 7.5,2.6Hz,1H), 6.87(d,J = 8.3Hz,1H),4.39(s,2H),4.26(s,1H),3.90-3.78(m,2H) , 3.62-3.50(m,2H), 2.39(m,7H), 2.02(m,1H), 1.90(m,1H), 1.68(m,1H), 1.45(m,1H), 1.34(m,1H ).

Step 5: Chiral separation of compound 2

A racemic mixture of compound 2 (120 mg) was dissolved in a mixed solution of DCM (8 ml), methanol (2 ml) and ethanol (45 ml), and separated by a preparative chiral column [CHIRALPAK IE (DAICEL) 20*250mm 5um, model: GX-271 (GILSON); mobile phase A: n-hexane (AR, Shuanglin Chemical, plus 0.1% diethylamine), mobile phase B: ethanol (AR,

Shuanglin Chemical, plus 0.1% diethylamine); isocratic (95% mobile phase B); flow rate: 10 ml/min, room temperature]. Compound 2A (peak 1): retention time = 38.58 min, recovered amount 37.4 mg; Compound 2B (peak 2): retention time = 46.88 min, recovered amount 38.6 mg.

Example 2B: Synthesis of Compound (R)-7-((7-(dimethylamino)-2',3',5',6,6',7-hexahydrospiro[cyclopentadienyl[b]pyridine-54'-pyran ]-2-yl)amino)-4-(7-fluoroimidazo[1,2-a]pyridin-3-yl)isoindolin-1-one

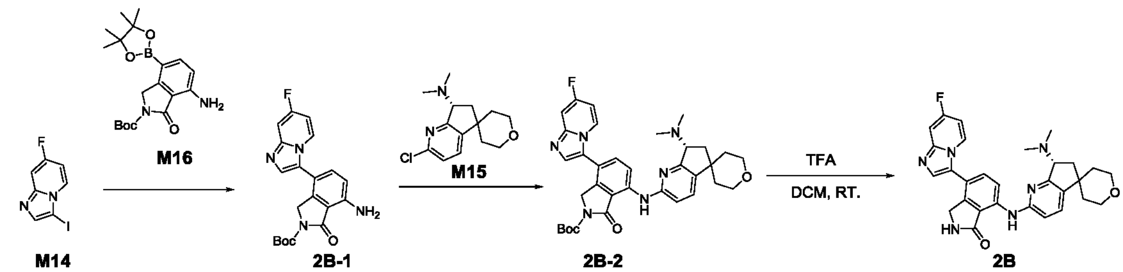


M14 2B-1 2B-2 2B

Step 1: Synthesis of compound 2B-1

Compound M14 (125.00 g), M16 (178.53 g), $[PdCl_2(dppf)]CH_2Cl_2$ (17.45 g), potassium phosphate (303.79 g), 1,4-dioxane (3000 mL) and water (500 mL) were added to a 5000 ml four-necked flask, replaced with nitrogen, and stirred at an internal temperature of 90°C for 2 h. The mixture was cooled to room temperature, filtered through celite pad, rinsed with dioxane, and water was added to precipitate solid, which was filtered. The filter cake was slurried with ethanol (300 ml) to obtain a solid, which was then slurried with THF (200 ml)/PE (200 ml) to obtain a gray solid, namely compound 2B-1 (130.0 g, 70.8% yield). ESI-MS m/z:383.2[M+H]+.

Step 2: Synthesis of compound 2B-2

Compound 2B-1 (121.84 g), M15 (85.00 g), palladium acetate (3.58 g), Xphos/2-dicyclohexylphosphino-2,4,6-triisopropylbiphenyl (15.19 g) and cesium carbonate (259.54 g), 1,4-dioxane (2500 ml) were added to a 5000 four-necked flask, replaced with nitrogen three times, pre-stirred at room temperature for 5 minutes, heated to an internal temperature of 90°C, and stirred for 16 hours. LCMS detected that the reaction was complete. The reaction solution was cooled to room temperature, then filtered with diatomaceous earth and rinsed with dioxane (300 ml). Water (5000 ml) was added to precipitate the solid, stirred evenly, and filtered. The solid was slurried with ethanol (500 ml) and EA (500 ml) in turn to give compound 2B-2 (141.00 g, 71.4% yield). ESI-MS m/z:613.3[M+H]+.

Step 3: Synthesis of compound 2B

Compound 2B-2 (54.00 g) was dissolved in DCM (500 mL), and trifluoroacetic acid (100.00 ml) was added dropwise at room temperature under $N_2$ protection, and the reaction was continued at room temperature for 1 hour. The reaction solution was concentrated to dryness, and the crude product was dissolved in DCM (80 ml). Saturated sodium bicarbonate was then slowly added to adjust the pH to 8-9, and a light yellow solid was precipitated. Ammonia water was added to adjust the pH to 11-12, stirred evenly, filtered off with suction, washed with water, methanol (600 ml) was added to the filter cake, stirred evenly, refluxed for 30 minutes, cooled naturally, stirred overnight, filtered off with suction, the solid was rinsed with methanol, and dried to obtain the target product 2B (43.20 g, purity 99.20%, yield 94.5%, retention time the same as 2B in Example 2). ESI-MS m/z:513.3[M+H]+. $^1$H NMR(500MHz,DMSO-d 6 )δ10.13(s,1H),8.82(s,1H),8.74(d,J = 8.5Hz,1H),8.45-8.42(m,1H),7.82( s,1H),7.72(d,J = 8.5Hz,1H),7.62(d,J = 8.2Hz,1H),7.52(dd,J = 10.1,2.7Hz,1H),6.98-6.94(m,1H ),6.85(d ,J = 8.3Hz,1H),4.39(s,2H),4.22(t,J = 7.7Hz,1H),3.88-3.78(m,2H),3.61-3.50(m,2H),2.35(s, 6H), 2.33-2.29(m,1H), 2.04-1.97(m,1H),1.89-1.84(m,1H), 1.71-1.65m,1H), 1.46-1.42(m,1H), 1.35-1.32(m,1H ).

Example 28: Synthesis of Compound (R)-7-((7-(dimethylamino)-4-methyl-2',3',5',6,6',7-hexahydrospiro[b]pyridin-5,4'-pyran]-2-yl)a mino)-4-(7-fluoroimidazo[1,2-a]pyridin-3-yl)isoindolin-1-one

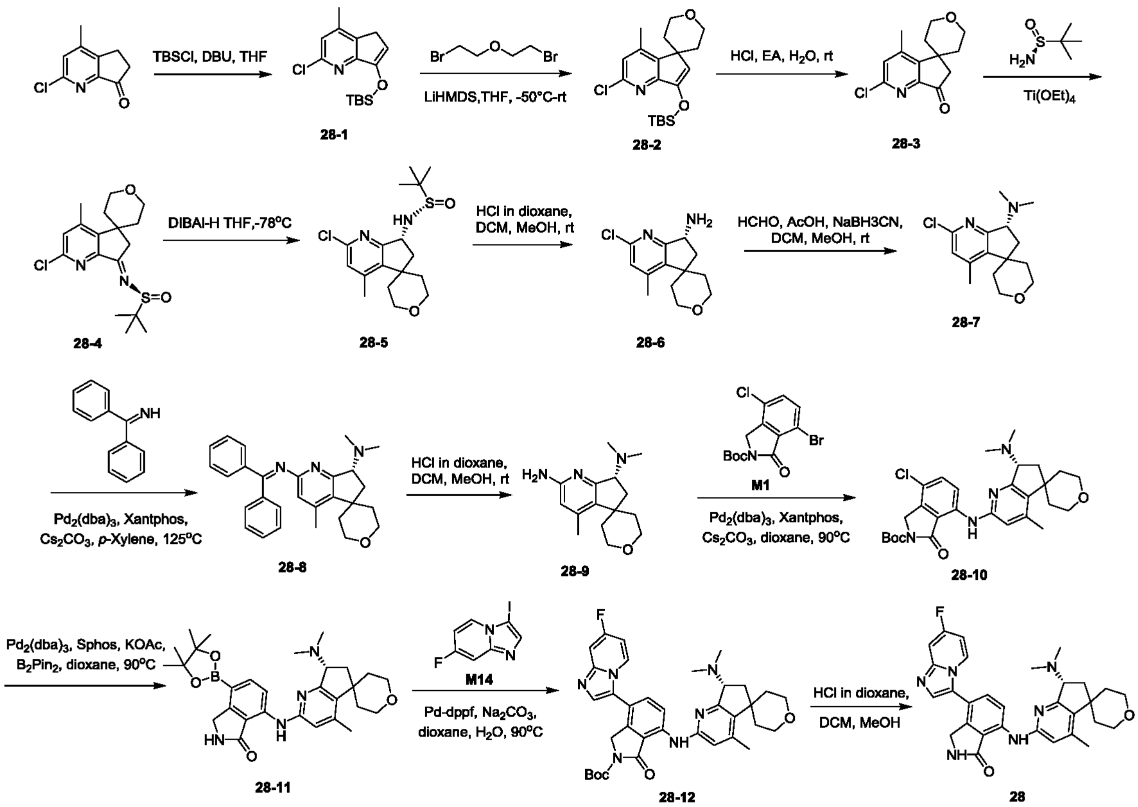

28-1 28-2 28-3 28-4 28-5 28-6 28-7 28-8 28-9 28-10 28-11 28-12 28

Step 1: Synthesis of compound 28-1

Under ice bath conditions, 2-chloro-4-methyl-5,6-dihydro-7H-cyclopenta[b]pyridin-7-one (6.45 g) and TBSCl (8.03 g) were dissolved in tetrahydrofuran (65 mL), DBU (10.61 mL) was slowly added dropwise, the temperature was slowly raised to room temperature and the reaction was carried out for 12 h, and the reaction was stopped. EA was added for extraction, and the organic phase was washed with saturated brine. The sample was mixed and separated by column chromatography (PE:EA=100:1) to obtain the target product 28-1 (8.30 g, yield 78.99%). ESI-MS m/z:296.12[M+H]+.

Step 2: Synthesis of compound 28-2

Weigh 28-1 (5.0 g), dissolve it in tetrahydrofuran (50 mL), slowly add LiHMDS (50.7 mL, 1M in THF) to the system at -50 °C under nitrogen protection, slowly raise the temperature to -30 °C, stir for 0.5 h, slowly add 2,2'-dibromodiethyl ether (4.31 g) to the system, slowly raise the temperature to room temperature for 3 h, and stop the reaction. The reaction was quenched with saturated ammonium chloride solution, the solution was spin-dried, extracted with EA, the organic phase was washed with saturated brine, the organic phase was collected and spin-dried to obtain the crude product of 28-2 (7.58 g). ESI-MS m/z:366.17[M+H]+.

Step 3: Synthesis of compound 28-3

At room temperature, weigh the crude product of 28-2 (8.34 g), dissolve it in EA (15 mL), add hydrochloric acid solution (60 mL, 1 M), react for 3 h, and stop the reaction. Sodium carbonate was added to adjust the system to neutrality, and EA was used for extraction. The organic phase was washed with saturated brine, mixed, and separated by column chromatography (PE:EA=1:1) to obtain the target product 28-3 (1.94 g, yield 33.79%). ESI-MS m/z: 252.08[M+H]+.

Step 4: Synthesis of compound 28-4

Weigh 28-3 (1.88 g), (R)-(+)-tert-butylsulfenamide (2.71 g), and tetraethyl titanate (30 mL), react at 50°C for 5 h, and then stop the reaction. The reaction solution was slowly added dropwise to ice water, EA was added, the mixture was stirred vigorously, diatomaceous earth was added for filtration, EA was extracted, the organic phase was washed with saturated brine, the organic phase was collected, dried, and separated by column chromatography (PE: EA = 3:1) to obtain the target product 28-4 (2.12 g, yield 80.19%). ESI-MS m/z:355.12[M+H]+.

Step 5: Synthesis of compound 28-5

Weigh 28-4 (2.58 g), dissolve it in tetrahydrofuran (30 mL), and slowly add DIBAL-H (25 mL, 1 M in THF) to the system under nitrogen protection at -78°C. React for 6 h and stop the reaction. Under nitrogen protection and -78°C, saturated potassium sodium tartrate solution was added to the system, slowly warmed to room temperature, vigorously stirred, filtered through diatomaceous earth, extracted with EA, and the organic phase was washed with saturated brine. The organic phase was collected, dried, and separated by column chromatography (PE: EA = 2: 1) to obtain the target product 28-5 (2.16 g, yield 83.25%). ESI-MS m/z:357.14[M+H]+.

Step 6: Synthesis of compound 28-6

Weigh 28-5 (2.16 g) and dissolve it in a mixed solution of DCM (10 mL) and MeOH (5 mL). Slowly add HCl solution (15.13 mL, 4 M/dioxane) under ice bath conditions, slowly warm to room temperature, react for 1 h, and stop the reaction. The solvent was dried by spin drying, PE and EA were added to precipitate the solid, which was filtered to obtain the hydrochloride salt of the target product 28-6 (1.23 g, yield 80.41%). ESI-MS m/z:253.11[M+H]+.

Step 7: Synthesis of compound 28-7

Weigh 28-6 (1.33 g), dissolve it in a mixed solution of DCE (20 mL) and MeOH (10 mL), add formaldehyde aqueous solution (1.40 mL, concentration 37%) and AcOH (15 drops), react at room temperature for 0.5 h, slowly add sodium cyanoborohydride (1.32 g) to the system, react for 12 h, and stop the reaction. The insoluble matter was removed by filtration through diatomaceous earth, and the organic phase was spin-dried and separated by column chromatography (DCM:MeOH=50:1) to obtain the target product 28-7 (1.18 g, yield 80.13%). ESI-MS m/z:281.14[M+H]+.

Step 8: Synthesis of compound 28-8

Weigh 28-7 (893 mg), benzophenone imine (865 mg), $Pd_2(dba)_3$ (291 mg), Xantphos (368 mg), and cesium carbonate (3.12 g), add o-xylene (10 mL), react under nitrogen protection at 130°C for 12 h, and stop the reaction. The mixture was filtered through celite, and the filter cake was washed with ethyl acetate. The organic phase was collected, dried by spin drying, and separated by column chromatography (DCM: MeOH = 25:1) to obtain the target product 28-8 (1.30 g, yield 96.05%). ESI-MS m/z:426.25[M+H]+.

Step 9: Synthesis of compound 28-9

Weigh compound 28-8 (1.30 g) and dissolve it in a mixed solution of DCM (20 mL) and MeOH (10 mL). Slowly add HCl solution (15.27 mL, 4 M/dioxnae) dropwise under ice bath conditions, slowly warm the temperature to room temperature and react for 3 h to stop the reaction. The solution was dried by spin drying, a small amount of methanol was added to dissolve, saturated sodium bicarbonate solution was added to adjust to weak alkalinity, EA was extracted, the organic phase was washed with saturated brine, the organic phase was collected, dried by spin drying, and separated by column chromatography (DCM: MeOH = 20:1) to obtain the target product 28-9 (530 mg, yield 66.38%). ESI-MS m/z:262.19[M+H]+.

Step 10: Synthesis of compound 28-10

Weigh 28-9 (500 mg), M1 (452 mg), $Pd_2(dba)_3$ (132 mg), XantPhos (167 mg), and cesium carbonate (1.41 g), add dioxane (10 mL), react at 90°C under nitrogen protection for 2.5 h, and stop the reaction. The organic phase was collected by filtration through celite, and dried by spin drying. The target product 28-10 (721 mg, yield 94.83%) was separated by column chromatography (DCM: MeOH = 33:1). ESI-MS m/z:527.24[M+H]+.

Step 11: Synthesis of compound 28-11

Weigh 28-10 (700 mg), diboronic acid pinacol ester (675 mg), $Pd_2(dba)_3$ (243 mg), SPhos (218 mg), and KOAC (391 mg), add dioxane (10 mL), react at 90°C under nitrogen protection for 12 h, and stop the reaction. The organic phase was collected by filtration through celite, and dried by spin drying. The target product 28-11 (422 mg, yield 51.37%) was separated by column chromatography (DCM: MeOH = 20:1). ESI-MS m/z:519.31[M+H]+.

Step 12: Synthesis of compound 28-12

Weigh 28-11 (100 mg), M14 (52 mg), $[PdCl_2(dppf)]CH_2Cl_2$ (13 mg), and sodium carbonate (51 mg), add to DMF (4 mL) and water (0.50 mL), then react at 90°C for 1 h under $N_2$ protection conditions, and stop the reaction. The reaction solution was concentrated, and the concentrate was separated by silica gel column chromatography (DCM:MeOH=12:1) to obtain the target product 28-12 (100 mg, yield 99%). ESI-MS m/z:627.31[M+H]+.

Step 13: Synthesis of compound 28

Compound 28-12 (100 mg) was weighed and dissolved in dichloromethane (1.5 mL) and MeOH (1.5 mL). HCl solution (3 mL, 4 M in dioxane) was slowly added dropwise under ice bath conditions. The temperature was slowly raised to room temperature and the reaction was carried out for 30 min to stop the reaction. The residue was concentrated and purified by preparative liquid separation to obtain the target product 28 (22.8 mg, purity 97.26%, yield 27.13%). ESI-MS m/z: 527.41 [M+H]+.

Example 39: Synthesis of Compound 7-(7-(azetidin-1-yl)-2',3',5',6,6',7-hexahydrospiro[b]pyridine-5,4'-pyran]-2-yl)amino)-4-(7-fluor oimidazo[1,2-a]pyridin-3-yl)isoindol-1-one

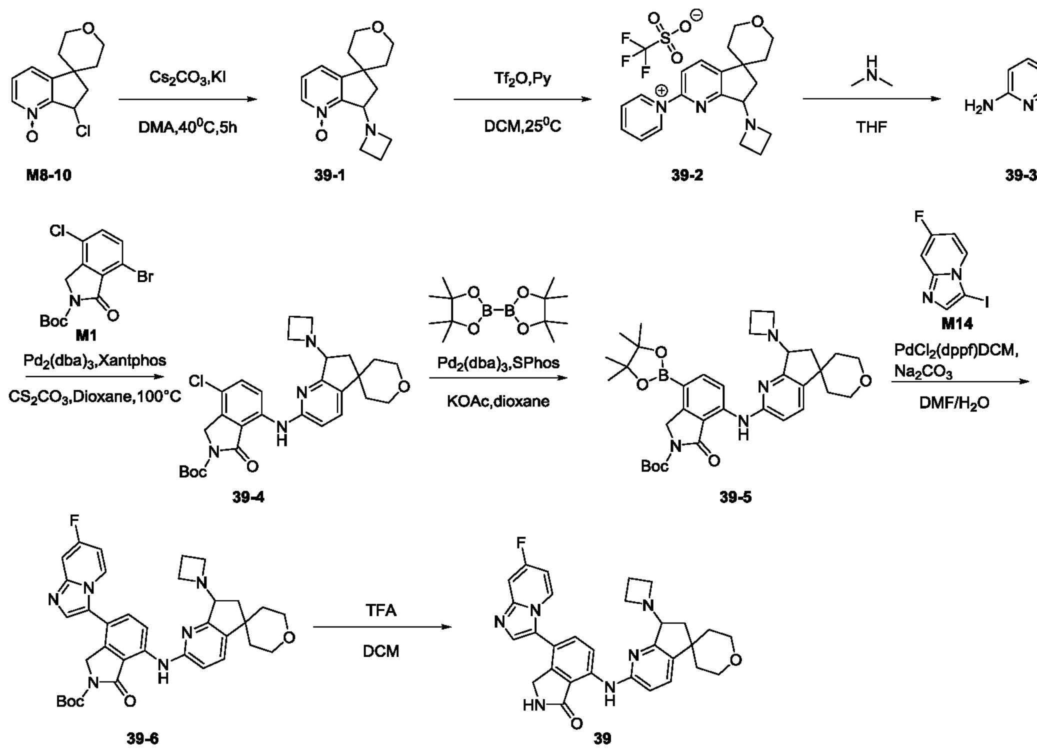


Step 1: Synthesis of Intermediate 39-1

At room temperature, compound M8-10 (1.00 g), KI (0.14 g), potassium carbonate (1.73 g), and azetidinamine (2.38 g) were added to DMF (12.00 mL), and then the reaction was carried out at 40°C. under $N_2$ protection conditions for 5 h, and the reaction was stopped. The reaction solution was directly concentrated to obtain a crude product, which was separated by column chromatography (DCM: MeOH = 95:5 to 90:10) to obtain the target product 39-1 (0.70 g, yield 64.45%). ESI-MS m/z:261.32[M+H]+. Step 2: Synthesis of Intermediate 39-2

At room temperature, compound 39-1 (0.60 g) and pyridine (0.93 mL) were added to DCM (15.00 mL), and then trifluoromethanesulfonic anhydride (0.78 mL) was slowly added in batches at -50 to -40°C under $N_2$ protection conditions, and the temperature was kept at -50 to -40 °C for 1 h to stop the reaction. The reaction solution was concentrated to dryness to obtain a crude product, which was separated by column chromatography (DCM: MeOH = 95:5 to 75:25) to obtain the target product 39-2 (1.0 g, yield 92.02%). ESI-MS m/z:322.43[M+H]+.

Step 3: Synthesis of Intermediate 39-3

At room temperature, compound 39-2 (1.00 g) was added to dimethylamine (15 mL, 2M in THF), and then reacted at room temperature at 25°C for 1 h under $N_2$ protection conditions, and then the reaction was stopped. The reaction solution was concentrated to dryness to obtain a crude product, which was separated by column chromatography (DCM: MeOH = 95:5-85:15) to obtain a total of 0.72 g of the target product. The target product 39-3 (0.20 g, yield 24.86%) was then separated by chromatography on a chromatographic plate (DCM: MeOH (7M-$NH_3$) = 12:1). ESI-MS m/z:260.36[M+H]+.

Step 4: Synthesis of Intermediate 39-4

At room temperature, compound 39-3 (200.00 mg), intermediate M1 (320.75 mg), $Pd_2(dba)_3$ (70.60 mg), XantPhos (89.22 mg), and cesium carbonate (753.82 mg) were added to 1,4-dioxane (6.00 mL), and then $N_2$ was protected and reacted at 100°C for 5 h to stop the reaction. The reaction solution was concentrated to obtain a crude product, which was separated by column chromatography (DCM: MeOH = 95:5 to 85:15) to obtain the target product 39-4 (240.00 mg, yield 59.27%). ESI-MS m/z:526.05[M+H]+.

Step 5: Synthesis of Intermediate 39-5

At room temperature, compound 39-4 (240.00 mg), diboronic acid pinacol ester (232.15 mg), $Pd_2(dba)_3$ (83.70 mg), SPhos (75.09 mg), and potassium acetate (134.58 mg) were added to 1,4-dioxane (8.00 mL), and then reacted at 100°C for 12 h under $N_2$ protection conditions to stop the reaction. The reaction solution was concentrated to obtain a crude product, which was separated by column chromatography (DCM: MeOH = 95:5 to 85:15) to obtain the target product 39-5 (220.00 mg, yield 78.06%). ESI-MS m/z:617.58[M+H]+.

Step 6: Synthesis of Intermediate 39-6

At room temperature, compound 39-5 (100.00 mg), M14 (51.02 mg), $[PdCl_2(dppf)]CH_2Cl_2$ (13.23 mg), and sodium carbonate (51.57 mg) were added to DMF (4.00 mL) and water (0.80 mL), and then reacted at 90°C for 1 h under $N_2$ protection conditions to stop the reaction. The reaction solution was concentrated to obtain a crude product, which was separated by column chromatography (DCM: MeOH = 95:5-85:15) to obtain the target product 39-6 (80.00 mg, yield 78.95%) as a red solid. ESI-MS m/z:625.73[M+H]+.

Step 7: Synthesis of compound 39

At room temperature, compound 39-6 (80.00 mg) was added to dichloromethane (4.00 mL), and then trifluoroacetic acid (0.80 mL) was added in an ice-water bath under $N_2$ protection conditions. The reaction was allowed to react at room temperature for 1 h, and the reaction was stopped. The reaction solvent was removed by rotary evaporation, 5 mL of $H_2O$ and 5 mL of DCM were added to the reaction solution, and then NaOH was added to adjust the pH to 12, and then extracted with DCM for 3 times, 15 mL each time, and the organic phases were combined and washed with saturated sodium chloride. The organic phases were concentrated to obtain a crude product, which was added to 10.00 mL of methanol for slurrying, and filtered and dried to obtain the target product 39 (34.60 mg, yield 51.49%). ESI-MS m/z:525.53 [M+H]+. $^1$H NMR(500MHz,DMSO-$d_6$) δ10.13(s,1H), 8.84(s,1H), 8.76(d,J = 8.7Hz,1H), 8.43(t,J = 6.7Hz,1H), 7 .82(s,1H),7.71(d,J = 8.6Hz,1H),7.62(d,J = 8.4Hz,1H),7.53-7.50(m,1H),6.99-6.95(m,1H), 6.84(d,J = 8.3Hz,1H), 4.38(d,J = 3.3Hz,2H), 3.83-3.79(m,2H), 3.73(s,1H), 3.57-3.49(m,2H), 3.44-3.35(m,4H), 2.49 (s, 6H), 2.18-2.13(m,1H), 2.02-1.97(m,2H), 1.90-1.75(m,3H), 1.56-1.53(m,1H), 1.33-1.29(m, 1H).

Example 40: Synthesis of Compound 4-(7-fluoroimidazo[1,2-a]pyridin-3-yl)-7-((7-(pyrrolidin-1-yl)-2',3',5',6,6',7-hexahydrospiro[b]p yridin-5,4'-pyran]-2-yl)amino)isoindolin-1-one

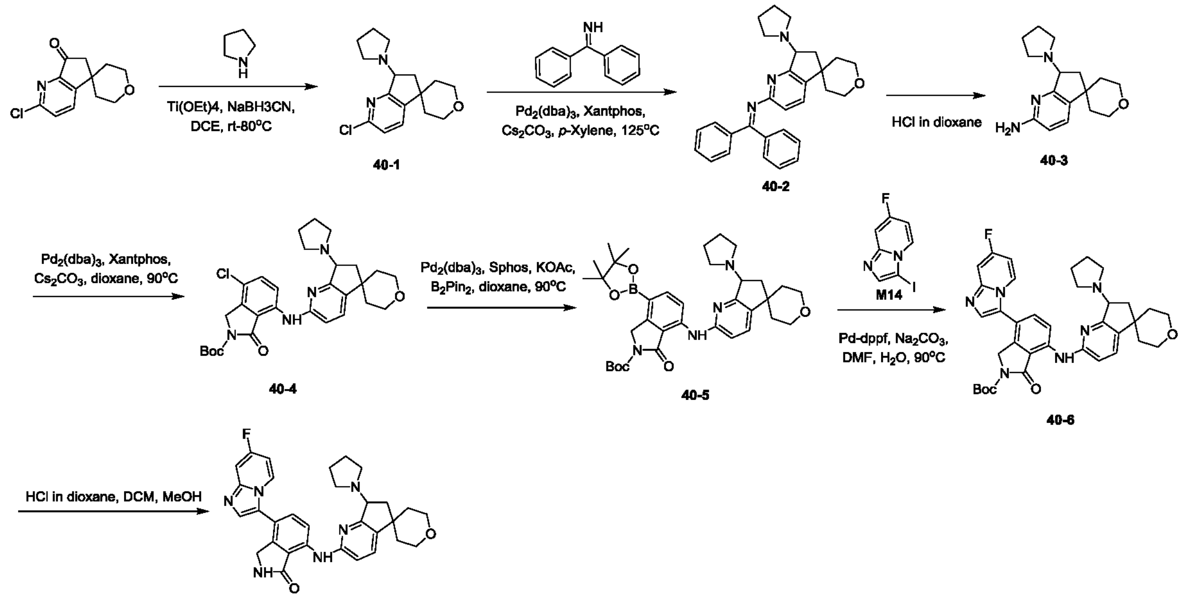

40-1
40-2
40-3
40-4
40-5
40-6
40

Step 1: Synthesis of compound 40-1

Weigh 2-chloro-2',3',5',6'-tetrahydrospiro[cyclopenta[b]pyridine-5,4'-pyran]-7(6H)-one (450 mg), pyrrolidine (404 mg), and tetraethyl titanate (216 mg), add DCE (10 mL), react at room temperature for 1 h, slowly add sodium cyanoborohydride (357 mg), raise the temperature to 80°C, react for 2 h, and stop the reaction. The mixture was extracted with DCM, and the organic phase was washed with saturated brine. The organic phase was collected, dried, and separated by column chromatography (DCM: MeOH = 33:1) to obtain the target product 40-1 (540 mg, yield 97.41%). ESI-MS m/z:293.14[M+H]+.

Step 2: Synthesis of compound 40-2

Weigh 40-1 (600 mg), benzophenone imine (557 mg), $Pd_2(dba)_3$ (375 mg), Xantphos (474 mg), and cesium carbonate (2.00 g), add o-xylene (10 mL), react under nitrogen protection at 130°C for 12 h, and stop the reaction. The mixture was filtered through celite, and the filter cake was washed with ethyl acetate. The organic phase was collected, dried by spin drying, and separated by column chromatography (DCM: MeOH = 20:1) to obtain the target product 40-2 (850 mg, yield 94.85%). ESI-MS m/z:438.25[M+H]+.

Step 3: Synthesis of compound 40-3

40-2 (1.20 g) was weighed and dissolved in a mixed solution of DCM (20 mL) and MeOH (10 mL). HCl solution (13.71 mL, 4 M in dioxane) was slowly added dropwise under ice bath conditions. The temperature was slowly raised to room temperature for 3 h, and the reaction was stopped. The solution was dried by spin drying, a small amount of methanol was added to dissolve, saturated sodium bicarbonate solution was added to adjust to weak alkalinity, EA was extracted, the organic phase was washed with saturated brine, the organic phase was collected, dried by spin drying, and separated by column chromatography (DCM: MeOH = 15:1) to obtain the target product 40-3 (750 mg, yield 99.10%). ESI-MS m/z:274.19[M+H]+.

Step 4: Synthesis of compound 40-4

Weigh 40-3 (1.0 g), intermediate M1 (660 mg), $Pd_2(dba)_3$ (221 mg), XantPhos (279 mg), and cesium carbonate (2.36 g), add dioxane (10 mL), react under nitrogen protection at 90°C for 2.5 h, and stop the reaction. The organic phase was collected by filtration through celite, and dried by spin drying. The target product 40-4 (893 mg, yield 68.61%) was separated by column chromatography (DCM: MeOH = 33:1). ESI-MS m/z:539.24[M+H]+.

Step 5: Synthesis of compound 40-5

Weigh 40-4 (883 mg), diboronic acid pinacol ester (832 mg), $Pd_2(dba)_3$ (300 mg), SPhos (269 mg), and KOAc

(482 mg), add dioxane (10 mL), react under nitrogen protection at 90°C for 12 h, and stop the reaction. The organic phase was collected by filtration through celite, and dried by spin drying. The target product 40-5 (905 mg, yield 87.62%) was separated by column chromatography (DCM:MeOH=20:1). ESI-MS m/z:631.37[M+H]+.

Step 6: Synthesis of compound 40-6

Weigh 40-5 (100 mg), M14 (62 mg), $[PdCl_2(dppf)]CH_2Cl_2$ (13 mg), and sodium carbonate (51 mg), add to dioxane (4 mL) and water (0.50 mL), then react at 90 °C for 1 h under $N_2$ protection conditions, and stop the reaction. The reaction solution was concentrated, and the concentrate was separated by silica gel column chromatography (DCM:MeOH=12:1) to obtain the target product 40-6 (70 mg, yield 69.10%). ESI-MS m/z:639.31[M+H]+.

Step 7: Synthesis of compound 40

Compound 40-6 (100 mg) was weighed and dissolved in dichloromethane (1.5 mL). TFA (1.5 mL) was slowly added dropwise under ice bath conditions. The temperature was slowly raised to room temperature and the reaction was allowed to proceed for 30 min to stop the reaction. After concentration, separation and purification by preparative liquid phase were performed to obtain the target product 40 (16.7 mg, purity 99.60%, yield 28.29%). 1H NMR (500MHz, DMSO) δ10.39(s,1H), 10.24(s,1H), 8.92(s,1H), 8.52(t,J=7.5Hz,2H), 8.07(s,1H), 7.83( d,J=8.5Hz,1 H), 7.77(t,J=9.5Hz,2H), 7.23(s,1H), 7.13(d,J=8.5Hz,1H), 5.11(dd,J= 14.6,7.7Hz,1H), 4.41( q,J=18.3Hz,2H), 3.98(s,1H), 3.92-3.81(m,2H),3.69-3.57(m,1H),3.56-3.50(m,1H),3.49-3.33(m,2H), 2.91(dd,J = 13.4, 8.2Hz,1H), 2.21-1.92(m,6H), 1.86(d,J = 7.0Hz,1H), 1.68(dd,J=12.1,8.6Hz,1H), 1.51 (d, J=12.9Hz,1H), 1.41( d,J=12.5Hz,1H).

ESI-MS m/z:539.42[M+H]+.

Synthesis of compounds 40A and 40B:

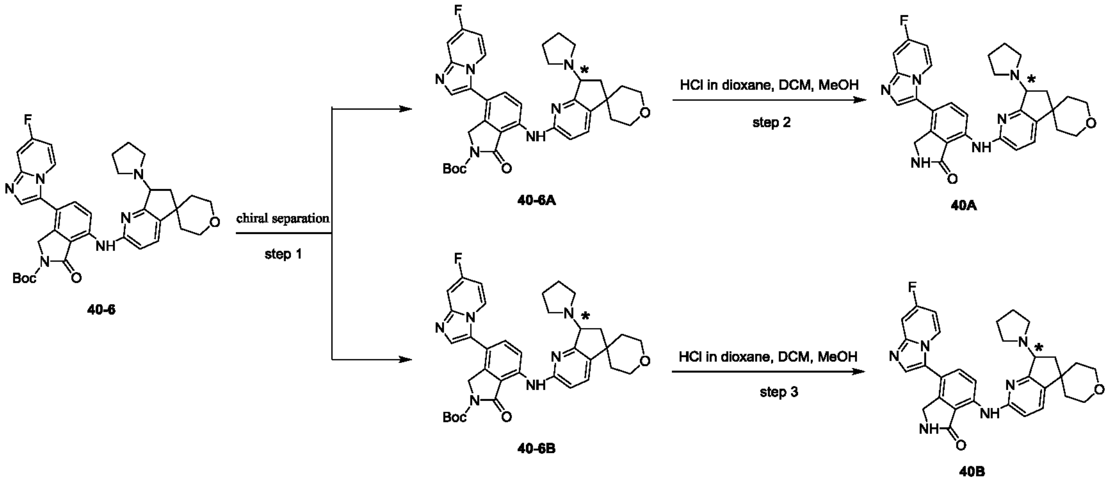


*Representing the R or S configuration, 40-6A or 40-6B are one of the R or S configurations, respectively

Step 1: Synthesis of compounds 40-6A and 40-6B

40-6 sample preparation: 70 mg of sample 40-6 was dissolved in 6 ml of ethanol:n-hexane (2:1) solution. Chromatographic conditions: 1. Chiral preparative column YMC CHIRAL ART Celluose-SB 250*20.0mm S-5um; 2. Instrument model GILSON GX-271; 3. Mobile phase: mobile phase A is n-hexane, mobile phase B is 0.1% diethylamine in ethanol, isocratic elution is with mobile phase A: mobile phase B in a ratio of 7:3, flow rate is 20 ml/min, wavelength is 254 nm, and separation is performed at room temperature. Compound 40-6A (peak 1): retention time = 13.035 min, recovered amount 35 mg; Compound 40-6B (peak 2): retention time = 17.645 min, recovered amount 36 mg.

Step 2: Synthesis of compound 40A

Weigh compound 40-6A (36 mg) and dissolve it in dichloromethane (1.5 mL) and methanol (1.5 mL). Slowly add HCl solution (3 mL, 4 M in dioxane) dropwise under ice bath conditions, slowly warm the temperature to room temperature

and react for 30 min to stop the reaction. The organic solvent was removed by concentration to obtain the target product 40A (21.1 mg, purity 99.74%, yield 69.46%). ESI-MS m/z:539.40[M+H]+. $^1$H NMR(500MHz,DMSO-d 6 )δ11.64(d,J = 5.4Hz,1H),10.25(s,1H),8.95(s,1H),8.73-8.65(m,2H),8.44(s ,1H),8.03(dd,J = 8.2,2.3Hz,1H), 7.82(dd,J = 8.5,3.8Hz,2H),7.54(td,J = 7.5,2.5Hz,1H),7.11(d, J = 8.5Hz,1 H),5.09(dd,J = 14.4,8.0Hz,1H),4.46-4.35(m,2H),3.95-3.81(m,3H),3.63-3.52(m,2H),3.42-3.33(m, 2H), 2.89 (dd, J = 13.4,8.2Hz,1H),2.21-1.84(m,7H),1.74-1.62(m,1H),1.59(d,J=12.4Hz,1H),1.45-1.37(m, 1H).

Step 3: Synthesis of compound 40B

Compound 40-6B (35 mg) was weighed and dissolved in dichloromethane (1.5 mL) and methanol (1.5 mL). HCl solution (3 mL, 4 M in dioxane) was slowly added dropwise under ice bath conditions. The temperature was slowly raised to room temperature and the reaction was carried out for 30 min to stop the reaction. The organic solvent was removed by concentration to obtain the target product 40B (22.2 mg, purity 99.10%, yield 75.21%). ESI-MS m/z:539.40[M+H]+. 1H NMR(500MHz,DMSO-d 6 )δ11.65(s,1H),10.26(s,1H),8.95(s,1H),8.75-8.62(m,2H),8.45(s,1H),8.04(d d,J = 8.2,2.3Hz,lH),7.82(dd,J = 8.5,5.0Hz,2H),7.55(td,J = 7.5,2.4Hz,1H),7.11(d,J = 8.5Hz,1H ),5.09(dd,J = 14.4,7.9Hz,1H),4.45-4.36(m,2H),3.96-3.80(m,3H),3.57-3.47(m,2H),3.37(dd,J = 13.0,6.5Hz,2H),2.93 -2. 84(m,1H),2.03(tdd,J = 23.7,15.1,9.6Hz,7H),1.70-1.63(m,1H),1.59(d,J=12.4Hz,1H),1.40(d,J= 12.5Hz,1H).

Example 41: Synthesis of Compound 7-((7-(dimethylamino)-2',3',5',6,6',7-hexahydrospiro[b]pyridin-5,4'-pyran]-2-yl) amino)-4-(7-(hy droxymethyl)imidazo[1,2-a]pyridin-3-yl)isoindol-1-one

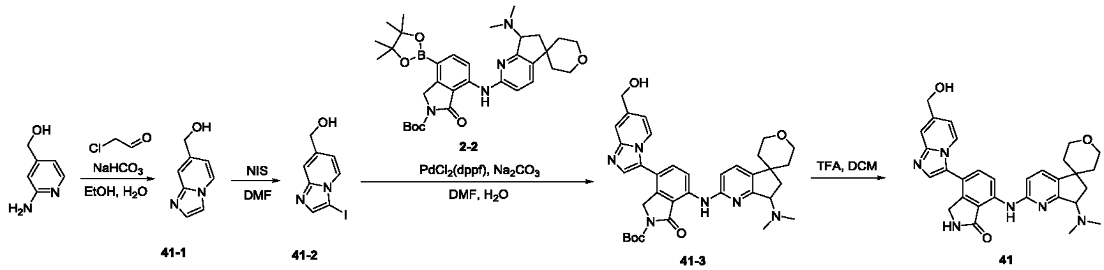


Step 1: Synthesis of compound 41-1

At room temperature, the compounds 2-aminopyridine-4-methanol (2.00 g), 2-chloroacetaldehyde (6.32 g, 40%/$H_2O$), and $NaHCO_3$ (2.71 g) were dissolved in ethanol (20.00 mL) and water (4.00 mL), reacted at 70°C for 2 h, and the reaction was stopped. The reaction solution was directly concentrated, mixed, and separated by column chromatography (DCM:MeOH=10:1) to obtain the target product 41-1 (2.34 g, yield 98.03%). ESI-MS m/z:149.12[M+H]+.

Step 2: Synthesis of compound 41-2

At room temperature, compound 41-1 (2.34 g) was dissolved in DMF (20.00 mL), and $N_2$ was replaced. NIS (3.91 g) was added under ice bath, and the reaction was allowed to react at room temperature for 2 h, and then the reaction was stopped. EA/THF and water were added to extract three times, the organic phase was washed with saturated brine, the sample was mixed, and the target product 41-2 (3.26 g, yield 75.32%) was separated by column chromatography (DCM:MeOH=13:1). ESI-MS m/z:275.10[M+H]+.

Step 3: Synthesis of compound 41-3

At room temperature, compound 2-2 (80.00 mg), 41-2 (44.00 mg), $[PdCl_2(dppf)]CH_2Cl_2$ (11.00 mg), and sodium carbonate (42.00 mg) were added to DMF (2.00 mL) and water (0.50 mL), and then reacted at 90°C for 1 h under $N_2$ protection conditions to stop the reaction. The reaction solution was concentrated, and the concentrate was separated by silica gel column chromatography (DCM:MeOH=7:1) to obtain the target product 41-3 (44.00 mg, yield 53.06%). ESI-MS m/z:625.50[M+H]+.

Step 4: Synthesis of compound 41

At room temperature, compound 41-3 (44.00 mg) was dissolved in dichloromethane (1.50 mL), TFA (0.40 mL) was added, and the mixture was reacted at room temperature for 10 min to stop the reaction. The reaction mixture was concentrated, the pH was adjusted to alkaline with saturated sodium bicarbonate solution, and the mixture was extracted three times with DCM. The organic phases were combined, washed with saturated brine, dried over anhydrous sodium sulfate, concentrated, and purified by preparative plate to obtain the target product 41 (15.70 mg, purity 97.16%, yield 41.26%). ESI-MS m/z: 525.51 [M+H]+. 1H NMR(500MHz,DMSO-d 6 ) δ10.13(s,1H),8.82(s,1H),8.73(d,J=8.5Hz,1H),8.34(d,J=7.1Hz,1H),7. 78(s,1H),7.70(d,J = 8.5Hz,1H),7.63(d,J=8.3Hz,1H),7.51(s,1H),6.91-6.83(m,2H),5.42(t ,J=5.7Hz,1H ),4.57(d,J= 5.7Hz,2H), 4.39(s,2H), 4.24(s,1H), 3.86-3.79(m,2H),3.63-3.50(m,2H),2.37(s,6H),2.34-2.29 (m, 1H), 2.03-1.97(m,2H),1.89-1.84m,1H),1.71-1.64(m,1H),1.48-1.41(m,1H),1.34-1.31(m,1H ).

Example 43: Synthesis of Compound 4-(7,8-difluoroimidazo[1,2-a]pyridin-3-yl)-7-((7-(dimethylamino)-2',3',5',6,6',7-hexahydrospiro [b]pyridin-5,4'-pyran]-2-yl)amino)isoindol-1-one

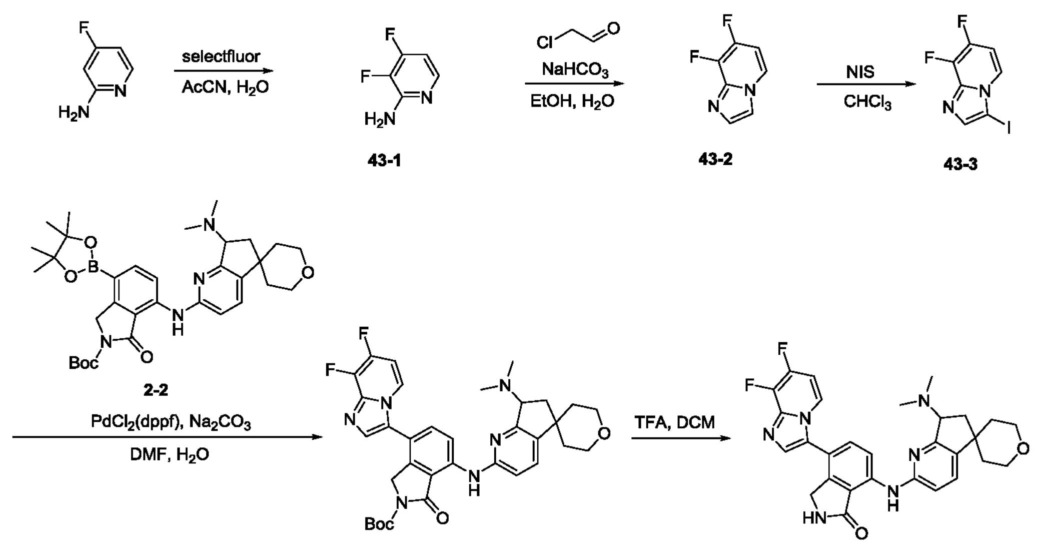


43-1 43-2 43-3 2-2 43-4 43

Step 1: Synthesis of compound 43-1

2-Amino-4-fluoropyridine (2.00 g) and selectfluor (7.58 g) were dissolved in acetonitrile (20.00 mL) and water (10.00 mL), $N_2$ was replaced, and the reaction was carried out at 60°C for 12 h, and the reaction was stopped. The residue was concentrated, dissolved in DCM, mixed, and separated by column chromatography (DCM:MeOH=16:1) to obtain the target product 43-1 (56.00 mg, yield 2.41%). ESI-MS m/z:131.06[M+H]+.

Step 2: Synthesis of compound 43-2

At room temperature, compound 43-1 (56.00 mg), 2-chloroacetaldehyde (169.00 mg, 40%/H 2 O), and $NaHCO_3$ (72.00 mg) were dissolved in ethanol (3.00 mL) and water (0.60 mL), reacted at 70°C for 12 h, and the reaction was stopped. The reaction solution was directly concentrated, mixed, and separated by column chromatography (DCM:MeOH=19:1) to obtain the target product 43-2 (53.00 mg, yield 78.85%). ESI-MS m/z:155.03[M+H]+.

Step 3: Synthesis of compound 43-3

At room temperature, compound 43-2 (53.00 mg) was dissolved in chloroform (2.00 mL), and $N_2$ was used for substitution. NIS (84.03 mg) was added under ice bath, and the reaction was allowed to react at room temperature for 2 h, and then the reaction was stopped. The sample was stirred and separated by column chromatography (DCM:MeOH=40:1) to obtain the target product 43-3 (75.00 mg, yield 78.33%). ESI-MS m/z:280.92[M+H]+.

Step 4: Synthesis of compound 43-4

At room temperature, compound 2-2 (70.00 mg), 43-3 (65.00 mg), $[PdCl_2(dppf)]CH_2Cl_2$ (9.00 mg), and sodium carbonate (37.00 mg) were added to DMF (2.00 mL) and water (0.50 mL), and then reacted at 90°C for 1 h under $N_2$ protection conditions to stop the reaction. The reaction solution was concentrated, and the concentrate was separated by silica gel column chromatography (DCM:MeOH=10:1) to obtain the target product 43-4 (17.00 mg, yield 23.27%). ESI-MS m/z:630.45[M+H]+.

Step 5: Synthesis of compound 43

At room temperature, compound 43-4 (17.00 mg) was dissolved in dichloromethane (2 mL), TFA (0.50 mL) was added, and the mixture was reacted at room temperature for 10 min to stop the reaction. The reaction mixture was concentrated, the pH was adjusted to alkaline with saturated sodium bicarbonate solution, and the mixture was extracted three times with DCM. The organic phases were combined, washed with saturated brine, dried over anhydrous sodium sulfate, concentrated, and purified by preparative plate to obtain the target product 43 (6.84 mg, purity 97.40%, yield 46.51%). ESI-MS m/z:531.21[M+H]+. 1H NMR(500MHz,DMSO-$d_6$) δ10.96(s,1H),10.28(s,1H),8.92(s,1H),8.76(d,J = 8.5Hz,1H),8.32-8.2 9(m,1H),8.05(s,1H),7.81(d,J = 8.5Hz,1H),7.73(d,J = 8.5Hz,1H),7.33-7.28(m,1H),7.09(d J = 8.5Hz,1H ),5.11-5.07(m,1H),4.39(s,2H),3.62-3.50(m,3H),2.93(d,J = 4.8Hz,3H),2.82(d,J = 4.9Hz,3H) ,2.77-2.72 (m,1H), 2.16-2.08(m,2H),2.02-1.95(m,1H),1.71-1.65(m,1H),1.55-1.51(m,1H),1.45-1.37(m, 1H).

Example 46: Synthesis of Compound 4-(6-chloro-7-fluoroimidazo[1,2-a]pyridin-3-yl)-7-((7-(dimethylamino)-2',3',5',6,6',7-hexahydro spiro[b]pyridin-5,4'-pyran]-2-yl)amino)isoindolin-1-one

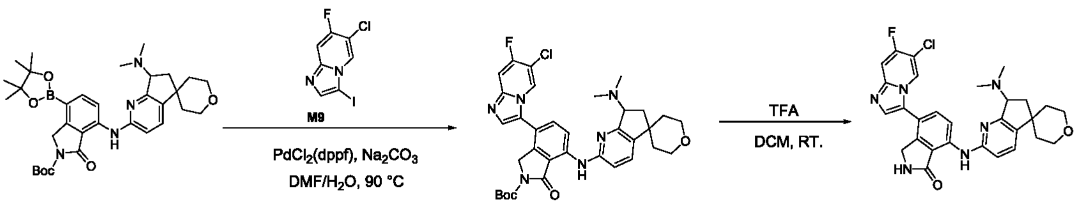


2-2 46-1 46

Step 1: Synthesis of compound 46-1

At room temperature, compound 2-2 (70.00 mg), M9 (40.00 mg), $[PdCl_2(dppf)]CH_2Cl_2$ (11.00 mg), and sodium carbonate (37.00 mg) were added to DMF (4.00 mL) and water (0.40 mL), and then reacted at 85°C for 40 min under $N_2$ protection conditions, and the reaction was stopped. A certain amount of water was added to the reaction solution, and the mixture was extracted three times with EA. The organic phases were combined, washed with saturated brine, dried over anhydrous sodium sulfate, and concentrated. The concentrate was separated by silica gel column chromatography (DCM:MeOH=13:1) to obtain the target product 46-1 (30.00 mg, yield 60.05%).

ESI-MS m/z:647.2[M+H]+.

Step 2: Synthesis of compound 46

At room temperature, compound 46-1 (30.00 mg) was dissolved in dichloromethane (5.00 mL), TFA (396.22 mg) was added, and the mixture was reacted at room temperature for 0.5 h, and the reaction was stopped. The pH was adjusted to alkaline with saturated sodium bicarbonate solution, extracted three times with DCM, the organic phases were combined, washed with saturated brine, dried over anhydrous sodium sulfate, concentrated, and slurried to obtain the target product 46 (27.30 mg, purity 98.45%, yield 70.69%). ESI-MS m/z:547.1[M+H]+. 1H NMR (500MHz, DMSO-$d_6$) δ10.15(s,1H),8.83(s,1H),8.73(d,J = 8.5Hz,1H),8.66(d,J = 6. 9Hz,1H), 7.87(s,1H),7.81(d,J = 9.9Hz,1H),7.75(d,J = 8.5Hz,1H),7.65(d,J = 8.3Hz,1H),6.89 (d,J = 8.4Hz,1H),4.37(s,2H),3.87-3.80(m,2H), 3.63-3.51(m,2H), 2.40(s,3H), 2.36(s,3H), 2.06-1.97(m,2H), 1.91-1.85(m,1H),1.69-1.64(m,2H),1.46-1.44(m,1H),1.35-1.32(m,1H).

Example 49: Compound 7-((8'-(dimethylamino)-2,3,5,6,7',8'-hexahydrospiro[pyran-4,5'-pyran[4,3-b]pyridine]-2'-yl)amino)-4-(7-fluoroimidazole[1,2-a]pyridin-3-yl)isoindol-1-one

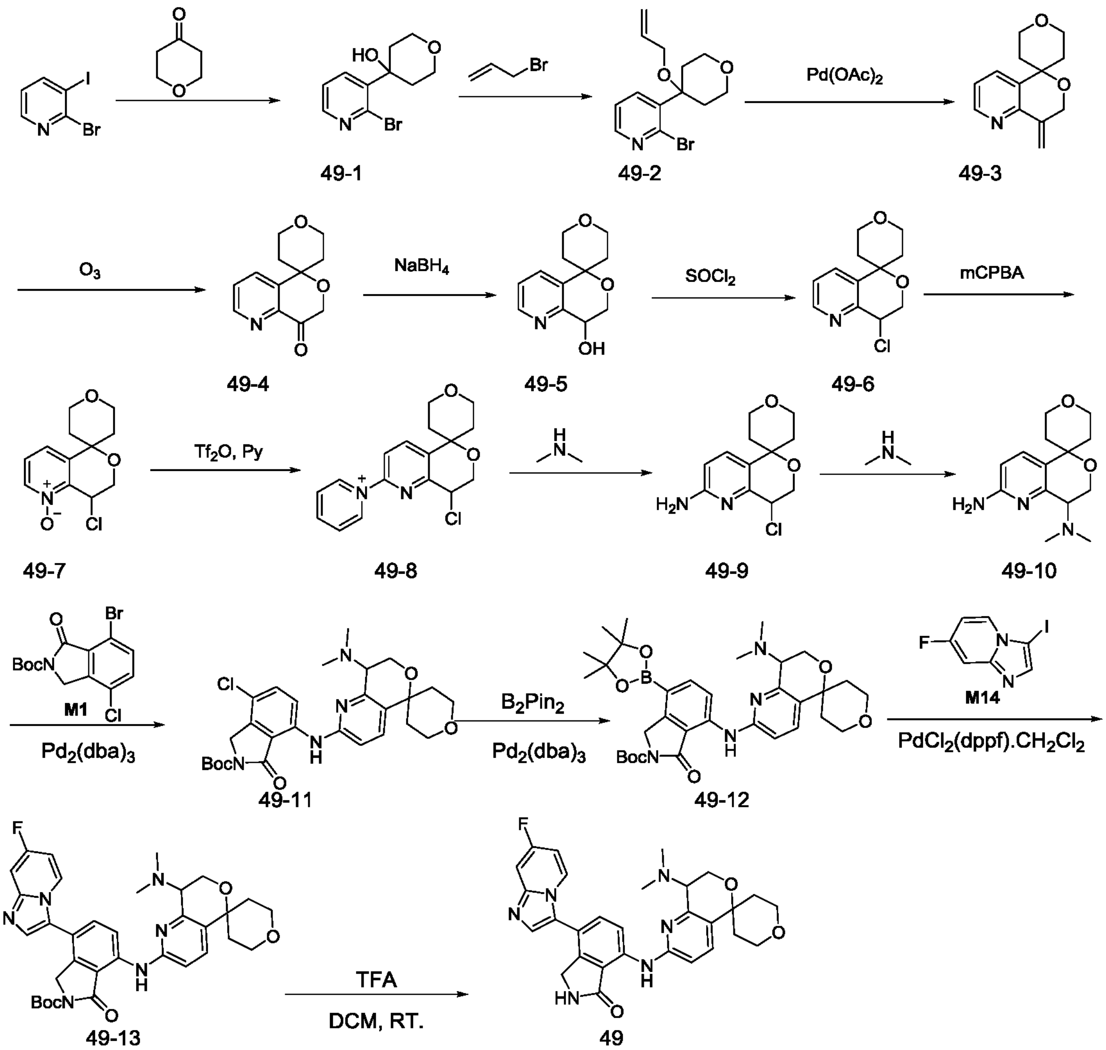


Step 1: Synthesis of compound 49-1

Dissolve 2-bromo-3-iodopyridine (10 g, 35.2 mmol) in THF (150 mL), then place the reaction system at -20°C, slowly add isopropylmagnesium chloride (4.4 g, 42.3 mmol) under nitrogen protection, react at this temperature for 2 h, add tetrahydropyrone (5.3 g, 52.8 mmol), and then stir at room temperature overnight. The reaction solution was quenched with saturated ammonium chloride solution, concentrated under reduced pressure to remove part of the solvent, extracted with EA, combined the organic layers, washed with 1M HCl, water and saturated brine in sequence, dried over anhydrous sodium sulfate, concentrated under reduced pressure, and purified by column chromatography (PE:EA=70:30-65:35) to give compound 49-1 (3.2 g, 35% yield). ESI-MS m/z:258.0[M+H]+.

Step 2: Synthesis of compound 49-2

Compound 49-1 (3.0 g, 9.3 mmol) was dissolved in DMF (25 mL), and NaH (0.74 g, 18.6 mmol) was added under ice bath. After the addition was complete, the mixture was stirred at room temperature for 30 min. Subsequently, a DMF solution of allyl bromide (1.6 g, 13.0 mmol) and TBAI (0.34 g, 0.93 mmol) was added dropwise, and the reaction was stirred at room temperature overnight. The mixture was quenched with water, concentrated under reduced pressure to remove part of the solvent, extracted with EA, the organic layers were combined, washed with saturated brine, dried over anhydrous sodium sulfate, concentrated under reduced pressure, and purified by column chromatography

(PE:EA=70:30-65:35) to give compound 49-2 (2.8 g, 80% yield). ESI-MS m/z:298.1[M+H]+.

Step 3: Synthesis of compound 49-3

Compound 49-2 (1.5 g, 5.0 mmol), $Pd(OAc)_2$ (0.17 g, 0.75 mmol), XantPhox (0.44 g, 0.75 mmol), and TEA (2.1 ml, 15 mmol) were dissolved in ACN (15 mL) and reacted at 90° C. for 6 h under nitrogen protection. After the reaction was completed, the reaction mixture was concentrated under reduced pressure and purified by column chromatography (PE:EA=70:30-65:35) to obtain compound 49-3 (0.84 g, 77% yield). ESI-MS m/z:218.1[M+H]+.

Step 4: Synthesis of compound 49-4

Compound 49-3 (0.34 g, 5.5 mmol) was dissolved in MeOH (5 mL) and DCM (10 mL), and ozone was introduced at -50°C. After reacting for 30 min, dimethyl sulfide (0.34 g, 5.5 mmol) was added. After the reaction was completed, the reaction mixture was concentrated under reduced pressure and purified by column chromatography (PE:EA=70:40-50:50) to obtain compound 49-4 (0.24 g, 83% yield). ESI-MS m/z:220.1[M+H]+.

Step 5: Synthesis of compound 49-5

Under ice bath, sodium borohydride (38.8 mg, 1.0 mmol) was added in portions to a solution of 49-4 (0.15 g, 0.68 mmol) in MeOH (2.5 mL) and THF (2.5 mL) and the ice bath was removed. The reaction was carried out at room temperature for 1 hour. The mixture was quenched with water, concentrated under reduced pressure to remove part of the solvent, extracted with EA, and the organic layers were combined, washed with saturated brine, dried over anhydrous sodium sulfate, and concentrated under reduced pressure to obtain compound 49-5 (112 mg, 74% yield). ESI-MS m/z:222.1[M+H]+.

Step 6: Synthesis of compound 49-6

To a solution of 49-5 (0.11 g, 0.50 mmol) in DCM (5 mL) was added thionyl chloride (0.18 mL) dropwise under ice bath. The ice bath was removed and the temperature was raised to 40° C. and the reaction was carried out for 1 hour. Concentrate under reduced pressure, add a small amount of methanol to dissolve, adjust the pH to alkaline with saturated sodium bicarbonate solution, extract with EA, combine the organic layers, wash with saturated brine, dry over anhydrous sodium sulfate, concentrate under reduced pressure, and purify by column chromatography (PE:EA=20:80) to obtain compound 49-6 (115 mg, 96% yield). ESI-MS m/z:239.0[M+H]+.

Step 7: Synthesis of compound 49-7

At room temperature, 49-6 (410 mg, 1.71 mmol) was dissolved in DCM (20 mL), and mCPBA (590 mg, 3.43 mmol) was added at room temperature. After reacting at room temperature for 3 h, LC-MS detected that the reaction of the raw material was complete. Saturated sodium bicarbonate solution (10 mL) was added, and the mixture was stirred at room temperature for 30 min. The mixture was extracted with DCM. The organic layers were combined, washed with saturated brine, dried over anhydrous sodium sulfate, concentrated under reduced pressure, and purified by column chromatography (DCM: MeOH = 20: 1) to give compound 49-7 (428 mg, 98% yield). ESI-MS m/z:256.1[M+H]+.

Step 8: Synthesis of compound 49-8

49-7 (400 mg, 1.56 mmol) and pyridine (1.2 g, 15.6 mmol) were dissolved in DCM (10 mL), and $Tf_2O$ (1.3 g, 4.68 mmol) was added under ice bath, and then reacted at room temperature for 0.5 h. LC-MS detected that the reaction of the raw material was complete. The residue was concentrated under reduced pressure and purified by column chromatography (DCM:MeOH=20:1) to obtain compound 49-8 (472 mg, 96% yield). ESI-MS m/z:317.1[M]+. Step 9: Synthesis of compound 49-9

49-8 (250 mg, 0.79 mmol) was dissolved in dimethylamine tetrahydrofuran solution (15 mL) at room temperature and stirred for 0.5 h, then concentrated under reduced pressure and purified by column chromatography (DCM:MeOH=20:1) to obtain compound 49-9 (175 mg, 87% yield). ESI-MS m/z:255.1[M+H]+.

Step 10: Synthesis of compound 49-10

49-9 (150 mg, 0.59 mmol), KI (12 mg, 0.12 mmol), $Cs_2CO_3$ (575 mg, 1.78 mmol), and dimethylamine tetrahydrofuran solution (2.4 mL) were dissolved in DMA (5.0 mL) and stirred at 100°C overnight, then concentrated

under reduced pressure and purified by column chromatography (DCM:MeOH=20:1) to give compound 49-10 (150 mg, 97% yield). ESI-MS m/z:264.2[M+H]+.

Step 11: Synthesis of compound 49-11

49-10 (300 mg, 1.14 mmol), intermediate M1 (434 mg, 1.25 mmol), $Pd_2(dba)_3$ (104 mg, 0.11 mmol), XantPhos (132 mg, 0.23 mmol), $Cs_2CO_3$ (1.11 g, 3.42 mmol) were dissolved in 1,4-dioxane (8.0 mL), and then sealed under nitrogen protection. After stirring at 90°C for 4 h, the reaction was concentrated under reduced pressure and purified by column chromatography (DCM:MeOH=20:1) to obtain compound 49-11 (480 mg, 80% yield). ESI-MS m/z:529.3[M+H]+.

Step 12: Synthesis of compound 49-12

49-11 (250 mg, 0.47 mmol), $Pd_2(dba)_3$ (86.5 mg, 0.09 mmol), bipyraclostrobin (240 mg, 0.95 mmol), SPhos (77.6 mg, 0.19 mmol), AcOK (139 mg, 1.42 mmol) were dissolved in 1,4-dioxane (4.0 mL), and then sealed under nitrogen protection. After stirring at 90°C for 16 h, the reaction was concentrated under reduced pressure and purified by column chromatography (DCM:MeOH=20:1) to obtain compound 49-12 (293 mg, 72% yield). ESI-MS m/z:621.5[M+H]+.

Step 13: Synthesis of compound 49-13

49-12 (100 mg, 0.47 mmol), M14 (54.9 mg, 0.21 mmol), $PdCl_2(dppf)\cdot CH_2Cl_2$ (86.5 mg, 0.09 mmol), $Na_2CO_3$ (51.2 mg, 0.48 mmol) were dissolved in DMF (5.0 mL) and $H_2O$ (0.5 mL), and then the tube was sealed under nitrogen protection. After stirring the reaction at 90°C for 0.5 h, it was concentrated under reduced pressure and purified by column chromatography (DCM:MeOH=10:1) to obtain compound 49-13 (60 mg, 59% yield). ESI-MS m/z:629.5[M+H]+.

Step 14: Synthesis of compound 49

49-13 (50 mg, 0.47 mmol) was dissolved in $CH_2Cl_2$ (5.0 mL), and then TFA (0.5 mL) was added at room temperature and stirred for 15 minutes, and the reaction was completed. The reaction mixture was washed with saturated sodium bicarbonate and then extracted with dichloromethane. The crude product was slurried with ethyl acetate and n-hexane to obtain compound 49 (25 mg, 60% yield). ESI-MS m/z:529.5[M+H]+. 1H NMR (500MHz, DMSO) δ10.11(s,1H),8.85(s,1H),8.79(d,J=8.6Hz,1H),8.45-8.40(m,1H),7.71(d,J=8.6 Hz,1H),7 .66(d,J = 8.6Hz,1H),7.53-7.47(m,1H),6.97(td,J = 7.5,2.6Hz,1H),6.94(d,J = 8.6Hz,1H),4.45-4.34(m,2H),4.0 9(dd,J = 12.3,2.3Hz,1H),3.95(dd,J = 12.3,3.8Hz,1H),3.77-3.58(m,4H),3.54-3.42(m,1H),2.41(s, 6H),2.37(d ,J=6.7Hz,1H),2.17-2.06(m,1H),2.04-1.95(m,1H),1.78(ddd,J= 19.4,18.8,8.8Hz,2H),1.58(d,J=12.6 Hz,1H).

Example 50: Synthesis of Compound 4-(7-fluoroimidazo[1,2-a]pyridin-3-yl)-7-((7'-methyl-2,3,5,6,7',8'-hexahydro-6'-spiro[pyran-4,5 '-[1,7]naphthyridine]-2'-yl)amino)isoindol-1-one

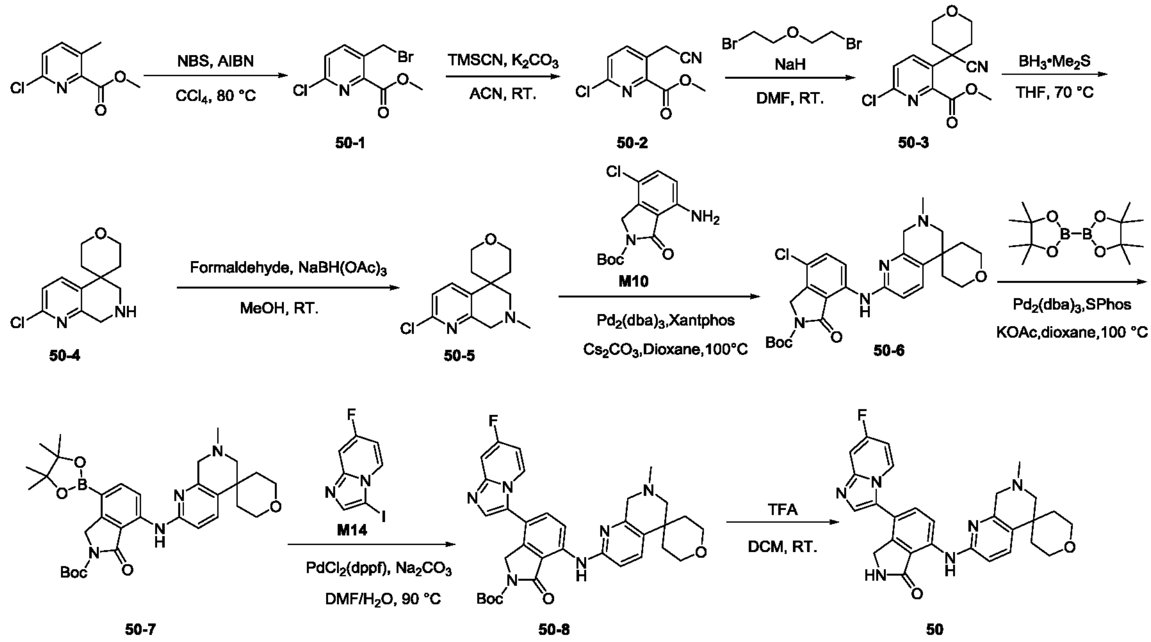


Step 1: Synthesis of compound 50-1

At room temperature, methyl 6-chloro-3-methyl-2-pyridinecarboxylate (5.00 g) was dissolved in carbon tetrachloride (60.00 mL), and then NBS (5.00 g) and AIBN (440.00 mg) were added. Under nitrogen protection, the mixture was reacted at 80° C. for 3 h, and the reaction was stopped. A certain amount of water was added to the reaction solution, and the mixture was extracted three times with DCM. The organic phases were combined, washed with saturated brine, dried over anhydrous sodium sulfate, and concentrated. The concentrate was separated by silica gel column chromatography (PE:EA=2:1) to obtain the target product 50-1 (5.43 g, yield 80.00%).

ESI-MS m/z:186.0[M+H]+.

Step 2: Synthesis of compound 50-2

At room temperature, compound 50-1 (5.00 g) and $K_2CO_3$ (5.23 g) were dissolved in ACN (70.00 mL), and then TMSCN (3.00 g) was slowly added dropwise under ice bath conditions. The reaction was allowed to react at room temperature for 13 h, and then the reaction was stopped. A certain amount of water was added to the reaction solution, and the mixture was extracted three times with EA. The organic phases were combined, washed with saturated brine, dried over anhydrous sodium sulfate, and concentrated. The concentrate was separated by silica gel column chromatography (PE:EA=2:1) to obtain the target product 50-2 (2.15 g, yield 54.00%). ESI-MS m/z:211.3[M+H]+.

Step 3: Synthesis of compound 50-3

At room temperature, compound NaH (520.00 mg) was dissolved in DMF (30.00 mL), and then 50-2 (2.00 g) was slowly added under ice bath and nitrogen protection. The reaction was kept warm for 0.5 h, and then 2,2'-dibromodiethyl ether (3.52 g) was slowly added dropwise. The reaction was carried out at room temperature for 3 h, and the reaction was stopped. The reaction was quenched with saturated ammonium chloride solution under ice bath condition, extracted with EA three times, and the organic phases were combined, washed with saturated brine, dried over anhydrous sodium sulfate, and concentrated. The concentrate was separated by silica gel column chromatography (PE:EA=3:1) to obtain the target product 50-3 (1.20 g, yield 45.02%). ESI-MS m/z:281.2[M+H]+.

Step 4: Synthesis of compound 50-4

At room temperature, compound 50-3 (1.20 g) was dissolved in THF (30.00 mL), and then borane dimethyl sulfide solution (1.6 mL, 10 M) was slowly added dropwise under ice bath conditions. Under nitrogen protection, the reaction was carried out at 70°C for 16 h, and the reaction was stopped. The pH was adjusted to 1-2 with 1M HCl solution, reacted at 70°C for 1 h, and the reaction was stopped. The pH value was adjusted to 8-9 with 2M NaOH solution, extracted three times with EA, the organic phases were combined, washed with saturated brine, dried over anhydrous sodium sulfate, and

concentrated. The concentrate was separated by silica gel column chromatography (DCM:MeOH=19:1) to give the target product 50-4 (250.00 mg, yield 30.00%). ESI-MS m/z:239.4[M+H]+.

Step 5: Synthesis of compound 50-5

At room temperature, compound 50-4 (250.00 mg) was dissolved in MeOH (10.00 mL), glacial acetic acid (1.00 mL) was added, and then formaldehyde aqueous solution (127.00 mg, 37% in water) was slowly added under ice bath conditions and nitrogen protection. The reaction was kept warm for 1 hour, and then $NaBH(OAc)_3$ (665.00 mg) was slowly added dropwise. The reaction was carried out at room temperature for 1 hour, and the reaction was stopped. The pH value was adjusted to 8-9 with saturated sodium bicarbonate solution, extracted with EA for 3 times, the organic phases were combined, washed with saturated brine, dried over anhydrous sodium sulfate, and concentrated. The concentrate was separated by silica gel column chromatography (DCM:MeOH=19:1) to give the target product 50-5 (208.00 mg, yield 79.00%).

ESI-MS m/z:253.4[M+H]+.

Step 6: Synthesis of compound 50-6

At room temperature, compound 50-5 (208.00 mg), M10 (267.00 mg), $Pd_2(dba)_3$ (76.00 mg), XantPhos (94.00 mg), and cesium carbonate (800.00 mg) were added to 1,4-dioxane (6.00 mL).

Then, under the protection of $N_2$, the reaction was carried out at 100°C for 3 hours and then the reaction was stopped. The reaction solution was concentrated, and the concentrate was separated by silica gel column chromatography (DCM:MeOH=13:1) to obtain the target product 50-6 (200.00 mg, yield 49.00%). ESI-MS m/z:499.3[M+H]+.

The subsequent synthesis steps were carried out by referring to the synthesis of Example 2 to prepare compound 50. $^1$H NMR(500MHz,DMSO-d 6 )δ10.06(s,1H),8.84(s,1H),8.72(d,J= 8.6Hz,1H), 8.45(dd ,J=7.6,5.7Hz,1H), 7.83(s,1H), 7.78(d,J=8.6Hz,1H),7.72(d,J=8.5Hz,1H), 7.53(dd,J=10.0,2.7Hz,1H), 6.99-6.95(m,1H),6.87(d,J=8.6Hz,1H),4.40(s,2H),3.76-3.71(m,2H), 3.64-3.58(m,2H),3.53(s,2H),2.74(s,2H),2.43(s,3H),1.96-1.89(m,2H),1.59-1.54(m,2H).

Example 58: Synthesis of Compound 7-((7-(dimethylamino)-4',5',6,7-tetrahydro-2'H-spiro[cyclopentane[b]pyridine-5,3'-furan]-2-yl)a mino)-4-(7-fluoroimidazo[1,2-a]pyridin-3-yl)isoindol-1-one

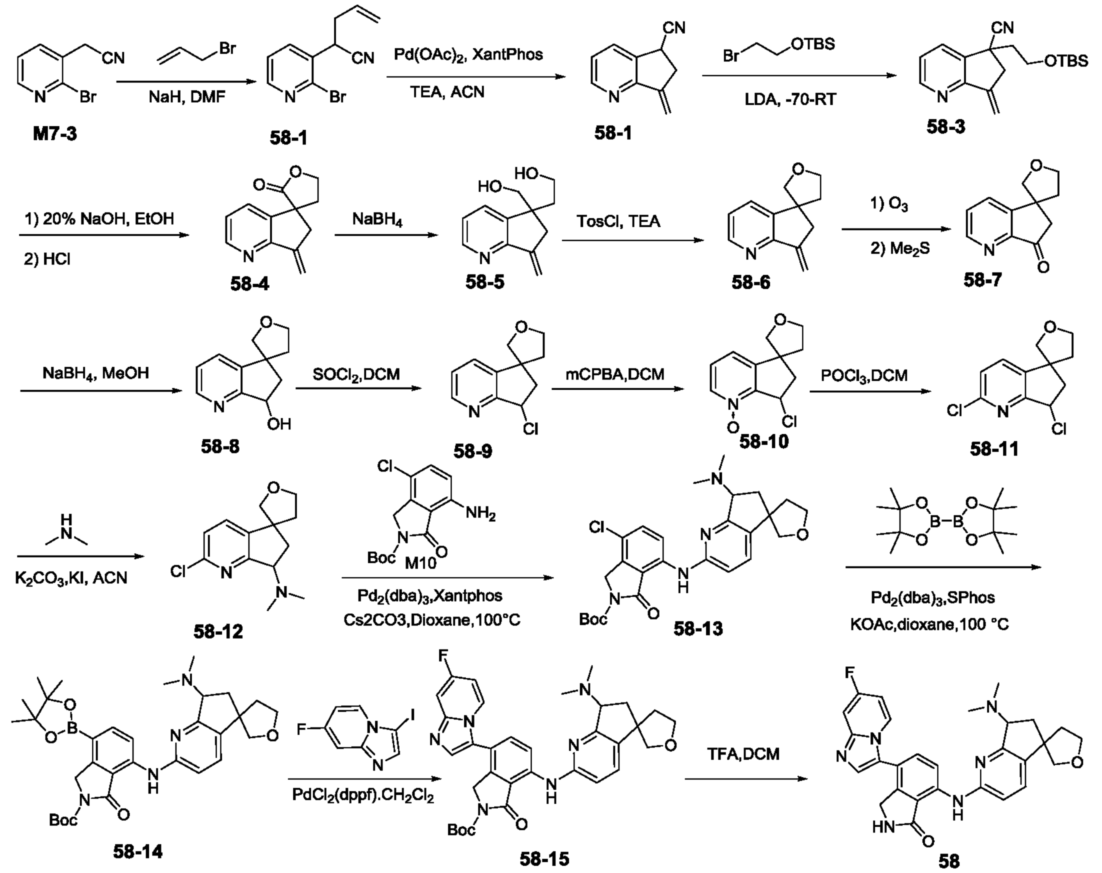


Step 1: Synthesis of compound 58-1

Dissolve NaH (2.03 g, 60%, 50.75 mmol) in DMF (80 mL), add 10 mL of DMF solution of compound 2-(2-bromopyridin-3-yl)acetonitrile (10.00 g, 50.75 mmol) (3.0 g, 9.3 mmol) under ice bath, and stir at 0°C for 30 min after addition. Subsequently, 20 mL of a DMF solution of allyl bromide (6.14 g, 50.75 mmol) was added dropwise, and the mixture was stirred at room temperature to react overnight. The mixture was quenched with water, concentrated under reduced pressure to remove part of the solvent, extracted with EA, and the organic layers were combined, washed with saturated brine, dried over anhydrous sodium sulfate, concentrated under reduced pressure, and purified by column chromatography (PE:EA=8:1-3:1) to obtain compound 58-1 (9.6 g, 80% yield). ESI-MS m/z:236.9[M+H]+.

Step 2: Synthesis of compound 58-2

Compound 58-1 (9.46 g, 39.90 mmol), palladium acetate (0.18 g, 0.80 mmol), XantPhox (0.46 g, 0.80 mmol), and TEA (16.64 mL, 119.70 mmol) were dissolved in ACN (150 mL) and reacted at 90°C. for 16 h under nitrogen protection. After the reaction was completed, the residue was concentrated under reduced pressure and purified by column chromatography (PE:EA=70:30-65:35) to obtain compound 58-2 (4.1 g, 66% yield). ESI-MS m/z:157.1[M+H]+.

Step 3: Synthesis of compound 58-3

Compound 58-2 (4.12 g, 26.38 mmol) was dissolved in THF (200 mL), and LDA (19.78 mL, 2.00 mol/L, 39.57 mmol) was added at -78 °C. After the addition was completed, the mixture was stirred at 0°C for 15 min. Subsequently, 10 mL of a THF solution of (2-bromoethoxy)-tert-butyldimethylsilane (9.47 g, 39.57 mmol) was added at -78°C, and the mixture was stirred at room temperature for 8 hours. The mixture was quenched with saturated ammonium chloride and extracted with EA. The organic layers were combined, washed with saturated brine, dried over anhydrous sodium sulfate, concentrated under reduced pressure, and purified by column chromatography (PE:EA=10:1-5:1) to give compound 58-3 (9.6 g, 80% yield). ESI-MS m/z: 315.4 [M+H]+.

Step 4: Synthesis of compound 58-4

Compound 58-3 (4.0 g, 12.7 mmol) was dissolved in EtOH (50 mL), 50 mL of 20% aqueous sodium hydroxide solution was added, and then heated at 100°C for 1 hour. After the reaction of the raw materials was complete, 4N hydrochloric acid was added to adjust the pH to 2-3, and then the reaction was stirred at room temperature overnight. Saturated sodium bicarbonate was added to adjust the pH to 7-8, and the mixture was extracted with EA. The organic layers were combined, washed with saturated brine, dried over anhydrous sodium sulfate, concentrated under reduced pressure, and purified by column chromatography (PE:EA=10:1-3:1) to obtain compound 58-4 (2.0 g, 78% yield). ESI-MS m/z: 202.2 [M+H]+.

Step 5: Synthesis of compound 58-5

Under ice bath, sodium borohydride (1.13 g, 29.8 mmol) was added in portions to a solution of 58-4 (2.0 g, 9.94 mmol) in MeOH (90 mL) and THF (30 mL), the ice bath was removed, and the reaction was carried out at room temperature for 1 hour. The reaction mixture was quenched by adding saturated ammonium chloride in water, concentrated under reduced pressure, and purified by column chromatography (DCM:MeOH=10:1) to obtain compound 58-5 (2.0 g, 98% yield). ESI-MS m/z:206.1[M+H]+.

Step 6: Synthesis of compound 58-6

Compound 58-5 (2.00 g, 9.74 mmol) and TEA (1.63 mL, 11.69 mmol) were dissolved in 50 mL of DCM, and p-toluenesulfonyl chloride (1.86 g, 9.74 mmol) was added at 0°C. The mixture was reacted overnight at room temperature, concentrated under reduced pressure, and purified by column chromatography (PE:EA=10:1-3:1) to obtain compound 58-6 (0.70 g, 37% yield). ESI-MS m/z: 188.1 [M+H]+.

Step 7: Synthesis of compound 58-7

Compound 58-6 (0.9 g, 4.81 mmol) was dissolved in a mixed solvent of MeOH (20 mL) and DCM (40 mL), and then ozone was introduced at -40°C to -20°C. After the reaction was complete, $Me_2S$ (1.49 g, 24.03 mmol) was added, and the mixture was concentrated under reduced pressure and purified by column chromatography (PE:EA=4:1-1:1) to obtain compound 58-7 (0.72 g, 79% yield). ESI-MS m/z: 190.2 [M+H]+.

Step 8: Synthesis of compound 58-8

Under ice bath, sodium borohydride (0.21 g, 5.63 mmol) was added in portions to a solution of 58-7 (0.71 g, 3.75 mmol) in MeOH (15 mL) and THF (15 mL) and the ice bath was removed. The reaction was allowed to proceed at room temperature for 1 hour. The reaction mixture was quenched with water, concentrated under reduced pressure to remove part of the solvent, extracted with EA, and the organic layers were combined, washed with saturated brine, dried over anhydrous sodium sulfate, and concentrated under reduced pressure to obtain compound 58-8 (0.69 g, 96% yield). ESI-MS m/z: 192.2 [M+H]+.

Step 9: Synthesis of compound 58-9

To a solution of 58-8 (0.69 g, 3.61 mmol) in DCM (25 mL) was added thionyl chloride (1.3 mL) dropwise under ice bath. The ice bath was removed and the temperature was raised to 40° C. and the reaction was carried out for 1 hour. Concentrate under reduced pressure, add a small amount of methanol to dissolve, adjust the pH to alkaline with saturated sodium bicarbonate solution, extract with EA, combine the organic layers, wash with saturated brine, dry over anhydrous sodium sulfate, concentrate under reduced pressure, and purify by column chromatography (PE:EA=20:80) to obtain compound 58-9 (0.69 g, 91% yield). ESI-MS m/z:209.1[M+H]+.

Step 10: Synthesis of compound 58-10

At room temperature, 58-9 (0.60 g, 2.85 mmol) was dissolved in DCM (20 mL), and mCPBA (0.98 g, 5.70 mmol) was added at room temperature. After reacting at room temperature for 3 h, LC-MS detected that the reaction of the raw material was complete. Saturated sodium bicarbonate solution (30 mL) was added, and the mixture was stirred at room temperature for 30 min. The mixture was extracted with DCM. The organic layers were combined, washed with saturated brine, dried over anhydrous sodium sulfate, concentrated under reduced pressure, and purified by column chromatography (DCM:MeOH=20:1) to give compound 58-10 (450 mg, 69% yield). ESI-MS m/z:225.1[M]+.

Step 11: Synthesis of compound 58-11

58-10 (450 mg, 2.0 mmol) was dissolved in DCM (15 mL), $POCl_3$ (1.5 g, 10.0 mmol) was added under ice bath, and then reacted at room temperature for 16 h. LC-MS detected that the reaction of the raw material was complete. Saturated sodium bicarbonate was added to adjust pH=7-8, extracted with EA, the organic layers were combined, washed with saturated brine, dried over anhydrous sodium sulfate, concentrated under reduced pressure, and purified by column chromatography (DCM:MeOH=20:1) to give compound 58-11 (300 mg, 61% yield). ESI-MS m/z:244.1[M]+.

Step 12: Synthesis of compound 58-12

58-11 (300 mg, 1.25 mmol), KI (20 mg, 0.10 mmol), $K_2CO_3$ (509 mg, 3.70 mmol), and methylamine tetrahydrofuran solution (5 mL) were dissolved in ACN (5.0 mL), stirred at 80°C overnight, concentrated under reduced pressure, and purified by column chromatography (DCM:MeOH=20:1) to give compound 58-12 (200 mg, 64% yield). ESI-MS m/z:253.1[M+H]+.

Step 13: Synthesis of compound 58-13

58-12 (200 mg, 0.80 mmol), compound M10 (257 mg, 0.90 mmol), $Pd_2(dba)_3$ (72.4 mg, 0.10 mmol), XantPhos (94.5 mg, 0.15 mmol), and $Cs_2CO_3$ (0.70 g, 2.35 mmol) were dissolved in 1,4-dioxane (8.0 mL), and then the tube was sealed under nitrogen protection. After stirring the reaction at 90°C for 4 h, the mixture was concentrated under reduced pressure and purified by column chromatography (DCM:MeOH=20:1) to obtain compound 58-13 (300 mg, 79% yield).

ESI-MS m/z:499.3[M+H]+.

Step 14: Synthesis of compound 58-14

58-13 (300 mg, 0.60 mmol), $Pd_2(dba)_3$ (110 mg, 0.10 mmol), bipyraclostrobin (305 mg, 1.2 mmol), SPhos (98.7 mg, 0.25 mmol), AcOK (177 mg, 1.8 mmol) were dissolved in 1,4-dioxane (8.0 mL), and then sealed under nitrogen protection. After stirring the reaction at 90°C for 16 h, the mixture was concentrated under reduced pressure and purified by column chromatography (DCM:MeOH=20:1) to give compound 58-14 (300 mg, 84% yield). ESI-MS m/z:591.5[M+H]+.

Step 15: Synthesis of Compound 58-15

58-14 (100 mg, 0.17 mmol), 7-fluoro-3-iodoimidazo[1,2-a]pyridine (48.6 mg, 0.18 mmol), $[PdCl_2(dppf)]CH_2Cl_2$ (13.8 mg, 0.02 mmol), $Na_2CO_3$ (54 mg, 0.5 mmol) were dissolved in DMF (4 mL) and $H_2O$ (0.4 mL), and then sealed under nitrogen protection. After stirring the reaction at 90°C for 0.5 h, the mixture was concentrated under reduced pressure and purified by column chromatography (DCM:MeOH=10:1) to give compound 58-15 (58 mg, 57% yield). ESI-MS m/z:599.4 [M+H]+.

Step 16: Synthesis of compound 58

58-15 (35 mg, 0.47 mmol) was dissolved in $CH_2Cl_2$ (5.0 mL), and then TFA (0.5 mL) was added at room temperature and stirred for 15 minutes, and the reaction was completed. The mixture was spin dried and the reaction mixture was prepared by reverse phase to give compound 58 (16.2 mg, 55% yield). ESI-MS m/z:499.3[M+H]+. 1H NMR(500MHz,DMSO) δ 10.32(s,2H), 8.94(s,1H), 8.64(d,J = 8.5Hz,1H),8.55(s,1H),8.14(s,1H),7.89-7.65(m,3H),7.29(s,1H),7.14(d,J = 8.6Hz ,1H), 5.20-5.01(m,1H),4.41(s,2H),3.97(dd,J = 30.7,22.7Hz,4H),2.9 6(s,3H), 2.84(s,3H), 2.35(d,J = 18.2Hz,2H),2.14(s,1H),1.99(s,1H).

Example 59: Synthesis of Compound 4-(7-fluoroimidazo[1,2-a]pyridin-3-yl)-7-(7-morpholin-2',3',5',6,6',7-hexahydrospiro[b]pyridin-5,4'-pyran]-2-yl)amino)isoindol-1-one

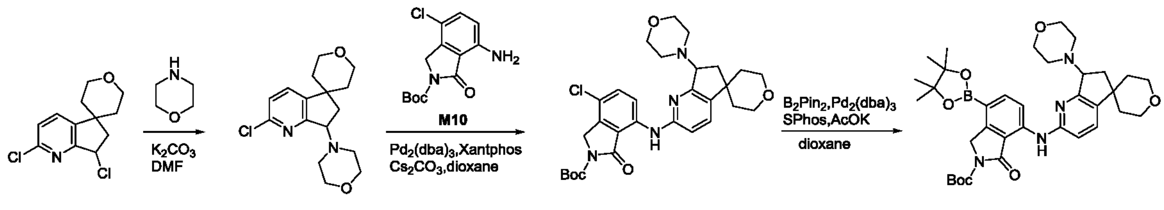


M8 59-1 59-2 59-3

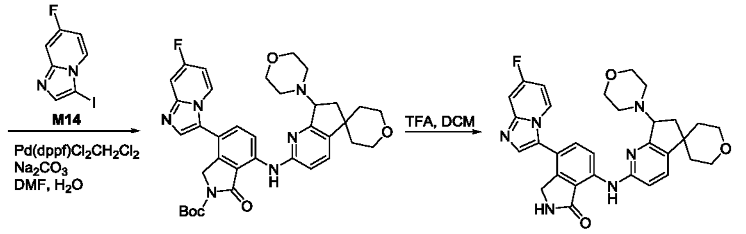


59-4 59

Step 1: Synthesis of compound 59-1

Intermediate M8 (200.00 mg), morpholine (202.00 mg) and potassium carbonate (321.00 mg) were dissolved in DMF (3.00 mL) and reacted at 80° C. for 4 h. After the reaction, ethyl acetate and water were added for extraction, and the organic phase was washed with saturated brine, separated, dried over anhydrous sodium sulfate, filtered, mixed, and separated by silica gel column chromatography (PE:EA=1:4) to obtain the target product 59-1 (173.00 mg, yield 72.31%). ESI-MS m/z:309.18[M+H]+.

Step 2: Synthesis of compound 59-2

59-1 (173.00 mg), M10 (190.00 mg), $Pd_2(dba)_3$ (51.00 mg), Xantphos (65.00 mg), and $Cs_2CO_3$ (547 mg) were dissolved in 1,4-dioxane (5.00 mL) and reacted at 100°C for 3 h. After the reaction was completed, the mixture was filtered, the filter cake was washed with dichloromethane, concentrated, mixed, and separated by silica gel column chromatography (PE:EA=1:4) to obtain the target product 59-2 (164.00 mg, yield 52.74%). ESI-MS m/z:555.35[M+H]+.

Step 3: Synthesis of compound 59-3

59-2 (164.00 mg), $B_2Pin_2$ (150.00 mg), $Pd_2(dba)_3$ (54.00 mg), Sphos (49.00 mg), and AcOK (87.00 mg) were dissolved in 1,4-dioxane (5.0 0 mL) and reacted at 100° C for 14 h.After the reaction was completed, the mixture was filtered, the filter cake was washed with dichloromethane, concentrated, mixed, and separated by silica gel column chromatography (PE:EA=1:5) to obtain the target product 59-3 (147.00 mg, yield 76.94%). ESI-MS m/z:647.53[M+H]+.

Step 4: Synthesis of compound 59-4

At room temperature, compound 59-3 (147.00 mg), M14 (77.00 mg), $[PdCl_2(dppf)]CH_2Cl_2$ (19.00 mg), and sodium carbonate (72.00 mg) were added to DMF (5.00 mL) and water (1.00 mL), and then reacted at 85°C for 1 h under $N_2$ protection conditions to stop the reaction. A certain amount of water was added to the reaction solution, and the mixture was extracted three times with EA. The organic phases were combined, washed with saturated brine, dried over anhydrous sodium sulfate, and concentrated. The concentrate was separated by silica gel column chromatography (DCM:MeOH=13:1) to give the target product 59-4 (77.00 mg, yield 51.74%). ESI-MS m/z:655.31[M+H]+.

Step 5: Synthesis of compound 59

At room temperature, compound 59-4 (77.00 mg) was dissolved in dichloromethane (5.00 mL), TFA (1.00 mL) was added, and the mixture was reacted at room temperature for 0.5 h, and the reaction was stopped. The pH was adjusted to alkaline with saturated sodium bicarbonate solution, extracted three times with DCM, the organic phases were combined, washed with saturated brine, dried over anhydrous sodium sulfate, concentrated, and purified by preparative plate to obtain the target product 59 (20.40 mg, purity 97.29%, yield 30.43%). $^1$H NMR(500MHz, DMSO-d 6 ) δ10.11(s,1H),8.83(s,1H),8.76(d,J = 8.6Hz,1H),8.42(t,J = 6.7Hz,1H),7.82( s,1H) , 7.72(d,J = 8.5Hz,1H),7.63(d,J = 8.3Hz,1H),7.52(d,J = 8.6Hz,1H),7.01-6.95(m,1H),6.86(d, J = 8.3Hz,1H),4.44-4 .32(m,2H),4.22(t,J = 7.8Hz,1H),3.83(t,J

= 11.3Hz,2H),3.61(s,4H),3.58-3.50(m,2H),2.88( d,J = 9.7Hz,2H),2.57 (s,2H),2.41-2.36(m,1H),2.01(d,J = 7.7Hz,1H),1.93-1.86(m,1H),1.68(s,1H),1.46(s,1H), 1.35(d,J=13.1Hz,1H). ESI-MS m/z:555.43[M+H]$^+$.

Example 60: Synthesis of Compound 4-(7-fluoroimidazo[1,2-a]pyridin-3-yl)-7-((7-(methylamino)-2',3',5',6,6',7-hexahydrospiro[b]pyr idin-5,4'-pyran]-2-yl)amino)isoindol-1-one

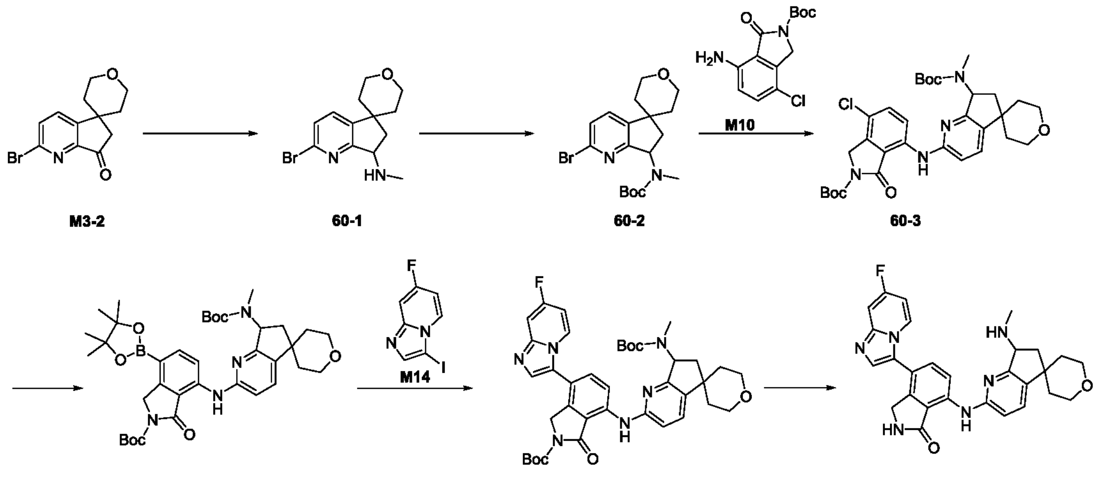


M3-2 60-1 60-2 60-3

60-4 60-5 60

Step 1: Synthesis of compound 60-1

At room temperature, intermediate M3-2 (300.00 mg), methylamine tetrahydrofuran solution (1.59 mL, 2.0 mol/L), and tetraethyl titanate (242.55 mg) were added to DCE (10.00 mL). After reacting at room temperature for 1 hour, sodium cyanoborohydride (200.45 mg) was added, and then the temperature was raised to 60°C. to react for 1 hour. 50 mL of $H_2O$ was added to the reaction solution, filtered, the filter cake was washed three times with DCM, and then extracted three times with 30 mL of DCM. The organic phases were combined, washed with saturated sodium chloride, and separated by column chromatography (DCM:MeOH=95:5) to obtain the target product 60-1 (160.00 mg, yield 50.63%). ESI-MS m/z:297.2[M+H]+.

Step 2: Synthesis of compound 60-2

At room temperature, 60-1 (160.00 mg) and DMAP (6.57 mg) were added to DCM (5.00 mL), and $Boc_2O$ (0.19 mL) was added dropwise under ice-water bath, and then reacted at room temperature for 1 h. The reaction solution was added dropwise into water (30.00 mL), and then extracted three times with 30 mL of DCM. The organic phases were combined, washed with saturated sodium chloride, and separated by column chromatography (PE:EA=60:40) to obtain the target product 60-2 (155.00 mg, yield 72.46%). ESI-MS m/z:397.1[M+H]+.

Step 3: Synthesis of compound 60-3

At room temperature, compound 60-2 (120.00 mg), M10 (85.38 mg), $Pd_2(dba)_3$ (27.65 mg), XantPhos (34.95 mg), and cesium carbonate (295.19 mg) were added to 1,4-dioxane (5.00 mL). Then, under $N_2$ protection conditions, the reaction was carried out at 100°C for 1 h. The reaction solution was concentrated, and the concentrate was separated by silica gel column chromatography (PE:EA=70:30) to obtain the target product 60-3 (150.00 mg, yield 82.90%). ESI-MS m/z:599.3[M+H]+.

Step 4: Synthesis of compound 60-4

At room temperature, compound 60-3 (180.00 mg), $B_2Pin_2$ (152.57 mg), $Pd_2(dba)_3$ (55.03 mg), SPhos (49.35 mg), and potassium acetate (88.44 mg) were added to 1,4-dioxane (5.00 mL). Then, under $N_2$ protection conditions, the reaction was carried out at 100°C for 6 hours. The reaction solution was concentrated, and the concentrate was separated by silica gel column chromatography (PE:EA=67:33) to obtain the target product 60-4 (190.00 mg, yield 91.58%). ESI-MS m/z:691.4[M+H]+.

Step 5: Synthesis of compound 60-5

At room temperature, compound 60-4 (180.00 mg), M14 (86.49 mg), $[PdCl_2(dppf)]CH_2Cl_2$ (22.46 mg), and sodium carbonate (87.47 mg) were added to DMF (4.00 mL) and water (0.40 mL), and then reacted at 100°C for 2 h under $N_2$ protection conditions. The reaction solution was concentrated, and the concentrate was separated by silica gel column chromatography (DCM:MeOH=97:3) to obtain the target product 60-5 (150.00 mg, yield 78.03%). ESI-MS m/z:699.5 [M+H]+.

Step 6: Synthesis of compound 60

At room temperature, compound 60-5 (150.00 mg) and TFA (1.00 mL) were added to DCM (2.00 mL), and then reacted at room temperature for 30 min. The reaction solution was concentrated, adjusted to pH 9 with saturated aqueous sodium bicarbonate solution, and extracted three times with 30 mL of DCM. The organic phases were combined, washed with saturated sodium chloride, and separated by column chromatography (DCM:MeOH=90:10) to obtain the target product 60 (19.50 mg, yield 17.37%). ESI-MS m/z:499.3[M+H]+. $^1$H NMR (500MHz, DMSO) δ10.16(s,1H),8.85(s,1H),8.76(d,J=8.5Hz,1H),8.44(t,J=6.6Hz,1H),7.83(s, 1 H),7.72(d,J = 8.6Hz,1H),7.66(d,J = 8.3Hz,1H),7.53(d,J = 9.9Hz,1H),6.98(t,J = 6.3Hz,1H) ,6.89(d,J = 8.3Hz,1H),4.40(s,2H),4.17(d,J = 6.6Hz,1H), 3.84(t,J = 10.5Hz,2H),3.64-3.48(m,2H),3.17(s ,1H ), 2.65-2.58(m,1H),2.56(s,3H),2.06-1.96(m,1H),1.81-1.66(m,2H),1.42(dd,J=42.1,12.9Hz,2H).

Example 61: Compound 7-((7-(cyclopropyl(methyl)amino)-2',3',5',6,6',7-hexahydrospiro[cyclopentyl[b]pyridine-5,4'-pyr an]-2-yl)amino)-4-(7-fluoroimidazole[1,2-a]pyridin-3-yl)isoindol-1-one

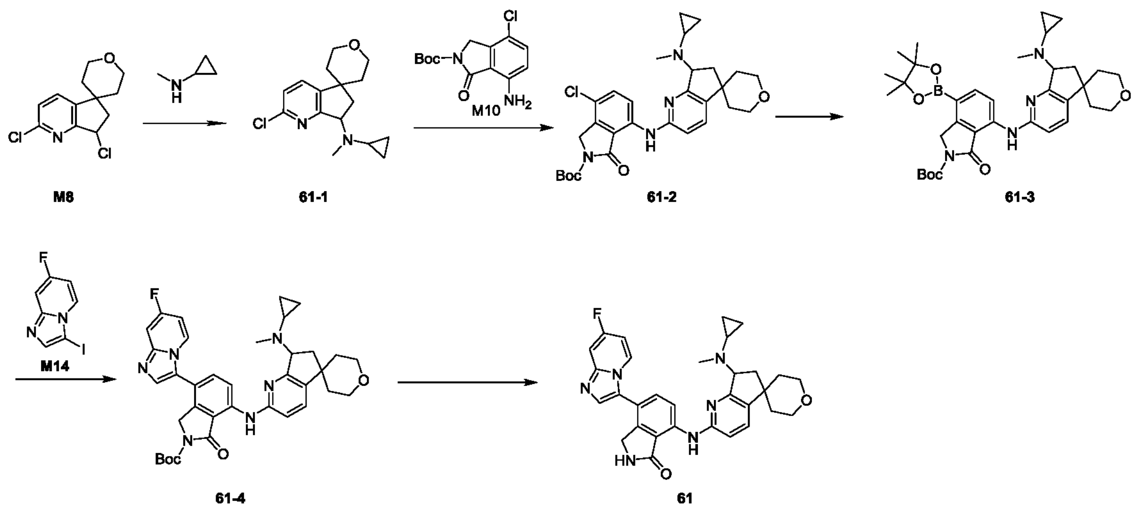

M8 61-1 61-2 61-3

61-4 61

Step 1: Synthesis of compound 61-1

M8 (300 mg), N-methylcyclopropylamine (248 mg), potassium carbonate (482 mg), and potassium iodide (39 mg) were weighed, ACN (10 mL) was added, and the mixture was reacted at 80° C. for 12 h, and the reaction was stopped. The insoluble matter was filtered off, the filter residue was washed with DCM, the filtrate was concentrated, and the target product 61-1 (230 mg, yield 67.59%) was separated by column chromatography (PE:EA=2:1). ESI-MS m/z:293.14 $[M+H]^+$.

Step 2: Synthesis of compound 61-2

Weigh 61-1 (265 mg), M10 (307 mg), $Pd_2(dba)_3$ (83 mg), XantPhos (105 mg), and cesium carbonate (885 mg), add dioxane (10 mL), react at 120°C under nitrogen protection for 4 h, and stop the reaction. The organic phase was collected by filtration through celite, and dried by spin drying. The target product 61-2 (679 mg, yield 139.18%) was separated by column chromatography (DCM:MeOH=33:1). ESI-MS m/z:539.41[M+H]+.

Step 3: Synthesis of compound 61-3

Weigh 61-2 (670 mg), diboronic acid pinacol ester (631 mg), $Pd_2(dba)_3$ (341 mg), SPhos (306 mg), and KOAc (366 mg), add dioxane (10 mL), react under nitrogen protection at 120°C for 8 h, and stop the reaction. The organic phase was collected by filtration through celite, and dried by spin drying. The target product 61-3 (290 mg, 37.00%) was separated by column chromatography (DCM: MeOH = 20:1), ESI-MS m/z: 631.39 [M+H]+.

Step 4: Synthesis of compound 61-4

Weigh 61-3 (290 mg), M14 (145 mg), $[PdCl_2(dppf)]CH_2Cl_2$ (38 mg), and sodium carbonate (146 mg), add to DMF (8 mL) and water (2 mL), then react at 90 °C for 2 h under $N_2$ protection conditions, and stop the reaction. The insoluble material was filtered off, the filter residue was washed with DCM, the filtrate was concentrated, and the concentrate was separated by silica gel column chromatography (DCM: MeOH = 8:1) to obtain the target product 61-4 (120 mg, yield 40.85%). ESI-MS m/z:639.39[M+H]+.

Step 5: Synthesis of compound 61

Weigh compound 61-4 (120 mg) and dissolve it in dichloromethane (1.5 mL) and methanol (1.5 mL). Slowly add a solution of hydrogen chloride in dioxane (3 mL, 4 M in dioxane) dropwise under ice bath conditions, slowly warm the temperature to room temperature and react for 30 min, then stop the reaction. After concentration, separation and purification by preparative liquid phase were performed to obtain the target product 61 (41.9 mg, yield 41.40%). ESI-MS m/z:539.41[M+H]+. $^1H$ NMR(500MHz,DMSO-$d_6$)δ10.24(s,2H),8.93(s,1H),8.63-8.53(m,1H), 8.50(s,1H),8.18(s,1H), 7.86(t,J = 9.0Hz,2H),7.76(d,J = 8.5Hz,1H),7.34(s,1H), 7.16(d,J = 7.7Hz,1H),5.28(s,1H), 4.40(s,2H) ,3.93-3.84(m,3H),3.63-3.49(m,2H),2.98(s,3H), 2.82-2.73(m,1H), 2.28(s,1H),2.17-2.07(m,1H) ,1. 72(t,J = 10.6Hz,1H),1.57(d,J = 13.0Hz,1H), 1.45(d,J=11.7Hz,1H),1.05(d,J= 39.3Hz,2H),0.87( s,2H).

Example 62: Compound 7-((7-(cyclopropylamino)-2',3',5',6,6',7-hexahydrospiro[cyclopentyl[b]pyridine-5,4'-pyran]-2-yl )amino)-4-(7-fluoroimidazole[1,2-a]pyridin-3-yl)isoindol-1-one

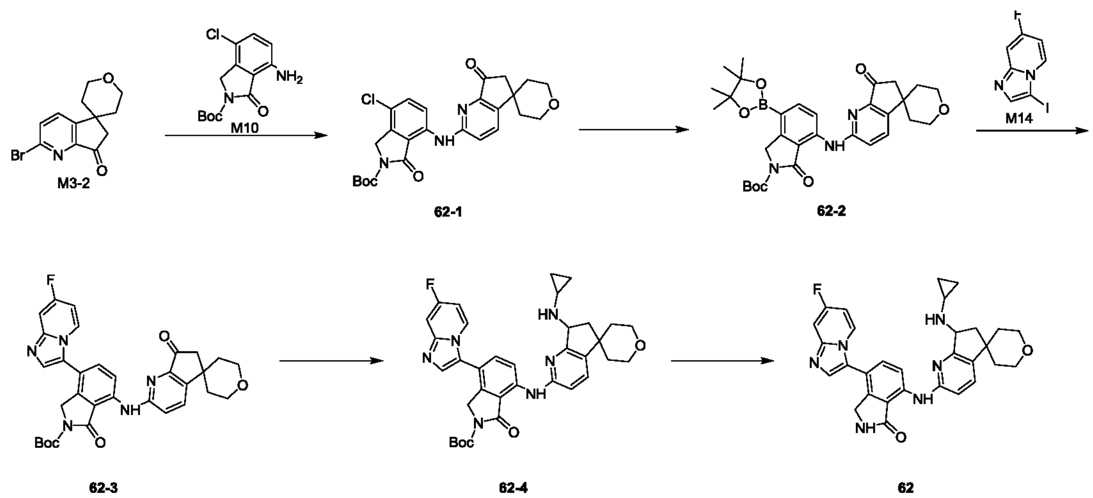

M3-2
62-1
62-2
62-3
62-4
62

Step 1: Synthesis of compound 62-1

Weigh M3-2 (700 mg), M10 (842 mg), $Pd_2(dba)_3$ (227 mg), XantPhos (287 mg), and cesium carbonate (2.43 g), add dioxane (15 mL), react under nitrogen protection at 120°C for 4 h, and stop the reaction. The organic phase was collected by filtration through diatomaceous earth, and then spin-dried and separated by column chromatography (DCM:MeOH=40:1) to obtain the target product 62-1 (1.20 g, yield 84.12%). ESI-MS m/z:484.16[M+H]+.

Step 2: Synthesis of compound 62-2

Weigh 62-1 (1.01 g), diboronic acid pinacol ester (1.06 g), $Pd_2(dba)_3$ (573 mg), SPhos (514 mg), and KOAC (615

mg), add dioxane (25 mL), react under nitrogen protection at 120°C for 8 h, and stop the reaction. The organic phase was collected by filtration through celite, and dried by spin drying. The target product 62-2 (1.10 g, 91.59%) was separated by column chromatography (DCM: MeOH = 20:1), ESI-MS m/z: 576.29 [M+H]+.

Step 3: Synthesis of compound 62-3

Weigh 62-2 (2.20 g), M14 (1.20 g), $[PdCl_2(dppf)]CH_2Cl_2$ (312 mg), and sodium carbonate (1.22 g), add to DMF (40 mL) and water (10 mL), then react at 90 °C for 2 h under $N_2$ protection conditions, and stop the reaction. The insoluble matter was filtered off, the filter residue was washed with DCM, the filtrate was concentrated, and the concentrate was separated by silica gel column chromatography (DCM: MeOH = 8:1) to obtain the target product 62-3 (1.63 g, yield 73.06%). ESI-MS m/z:584.26[M+H]+.

Step 4: Synthesis of compound 62-4

Weigh 62-3 (150 mg), cyclopropylamine (73 mg), and tetraethyl titanate (176 mg), add DCE (5 mL) and MeOH (2 mL), react at room temperature for 1 h, slowly add sodium cyanoborohydride (48 mg), raise the temperature to 80°C for 2 h, and stop the reaction. The mixture was extracted with DCM, and the organic phase was washed with saturated brine. The organic phase was collected, dried by spin drying, and separated by column chromatography (DCM: MeOH = 33:1) to obtain the target product 62-4 (175 mg, yield 109.00%). ESI-MS m/z:625.34[M+H]+.

Step 5: Synthesis of compound 62

Weigh compound 62-4 (350 mg) and dissolve it in dichloromethane (3 mL) and methanol (3 mL). Slowly add a solution of hydrogen chloride in dioxane (6 mL, 4 M in dioxane) dropwise under ice bath conditions, slowly warm the temperature to room temperature and react for 30 min, then stop the reaction. After concentration, separation and purification by preparative liquid phase were performed to obtain the target product 62 (51.40 mg, yield 17.49%). ESI-MS m/z:525.48[M+H]+. 1H NMR(500MHz,DMSO-d 6 )δ10.28(s,1H),9.78(s,1H), 9.34(s,1H), 8.93(s,1H), 8.72(d,J = 8.5Hz,1H), 8.54(dd,J=7. 3,5.6Hz,1H),8.15(s,1H),7.83(dd,J = 14.7,5.3Hz,2H), 7.74(d,J = 8.5Hz,1H),7.31-7.27(m,1H), 7.11(d,J=12.2Hz,1H),5. 01(t,J = 7.5Hz,1H),4.50-4.33(m,2H),3.93-3.83(m,2H),3.55(dt,J] = 38.8,11.2Hz,2H),3.10(s,1H) ,2.88(dd,J = 13.4,8.2 Hz,1H),2.16-2.03(m,2H),1.71(td,J = 12.9,4.4Hz,1H),1.52-1.44(m,2H),1.02(dt,J=15.9,7.3Hz,1H) ,0.96-0.80(m,3H).

Example 67: Synthesis of Compound 7-(7-(3-fluoroazetidine-1-yl)-2',3',5',6,6',7-hexahydrospiro[b]pyridine-5,4'-pyran]-2-yl)amino)-4 -(7-fluoroimidazo[1,2-a]pyridin-3-yl)isoindolin-1-one

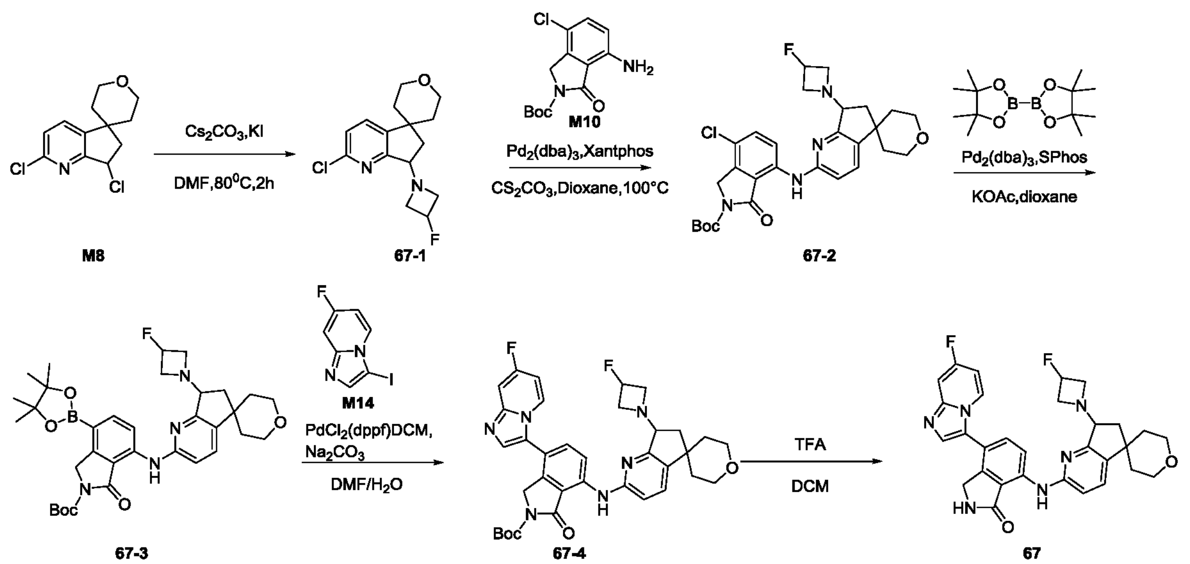

Step 1: Synthesis of Intermediate 67-1

At room temperature, compound M8 (300.00 mg), KI (39.60 mg), potassium carbonate (963.70 mg), and 3-fluoroazetidine hydrochloride (648.10 mg) were added to DMF (6.00 mL), and then the mixture was reacted at 80°C. for 2 h under $N_2$ protection conditions, and the reaction was stopped. The reaction solution was directly concentrated to obtain a crude product, which was separated by column chromatography (PE:EA=95:5-65:35) to obtain the target product 67-1 (250.00 mg, yield 72.49%). ESI-MS m/z:297.78[M+H]+.

Step 2: Synthesis of Intermediate 67-2

At room temperature, compound 67-1 (250.00 mg), intermediate M10 (274.35 mg), $Pd_2(dba)_3$ (74.08 mg), XantPhos (93.56 mg), and cesium carbonate (790.47 mg) were added to 1,4-dioxane (6.00 mL), and then reacted at 90°C for 2 h under $N_2$ protection conditions to stop the reaction.

The reaction solution was concentrated to obtain a crude product, which was separated by column chromatography (DCM: MeOH = 95:5 to 85:15) to obtain the target product 67-2 (250.00 mg, yield 56.93%). ESI-MS m/z:544.05 [M+H]+.

Step 3: Synthesis of Intermediate 67-3

At room temperature, compound 67-2 (250.00 mg), diboronic acid pinacol ester (233.83 mg), $Pd_2(dba)_3$ (84.34 mg), SPhos (75.62 mg), and potassium acetate (135.55 mg) were added to 1,4-dioxane (8.00 mL), and then reacted at 100°C for 12 h under $N_2$ protection conditions to stop the reaction. The reaction solution was concentrated to obtain a crude product, which was separated by column chromatography (DCM: MeOH = 95:5 to 85:15) to obtain the target product 67-3 (160.00 mg, yield 54.77%). ESI-MS m/z:634.55[M+H]+.

Step 4: Synthesis of Intermediate 67-4

At room temperature, compound 67-3 (100.00 mg), M14 (49.57 mg), $[PdCl_2(dppf)]CH_2Cl_2$ (12.82 mg), and sodium carbonate (50.11 mg) were added to DMF (4.00 mL) and water (0.80 mL), and then reacted at 90°C for 1 h under $N_2$ protection conditions, and the reaction was stopped. The reaction solution was concentrated to obtain a crude product, which was separated by column chromatography (DCM: MeOH = 95:5 to 85:15) to obtain the target product 67-4 (80.00 mg, yield 78.98%). ESI-MS m/z:643.71[M+H]+.

Step 5: Synthesis of compound 67

At room temperature, compound 67-4 (80.00 mg) was added to dichloromethane (4.00 mL), and then trifluoroacetic acid (0.80 mL) was added in an ice-water bath under $N_2$ protection conditions. The reaction was allowed to react at room temperature for 1 h, and the reaction was stopped. The reaction solvent was removed by rotary evaporation, 5 mL of $H_2O$ and 5 mL of DCM were added to the reaction solution, and then NaOH was added to adjust the pH to 12, and then extracted three times with 15 mL of DCM. The organic phases were combined, washed with saturated sodium chloride, and concentrated to obtain a crude product. The crude product was separated by column chromatography (DCM: MeOH = 95:5 ~ 85:15) to obtain a crude product, added to 5.00 mL of methanol for slurry, filtered and dried to obtain the target product 67 (37.50 mg, yield 55.51%). ESI-MS m/z:543.53[M+H]+. $^1$H NMR(500MHz,DMSO-d 6 )δ10.14(s,1H), 8.84(s,1H),8.71(d,J = 8.6Hz,1H),8.43(dd,J = 7.5,5.8Hz,1 H),7.82(s,1H),7.74(d,J = 8.5Hz,1H), 7.64(d,J = 8.4Hz,1H),7.52(dd,J = 10.1,2.7Hz,1H),699- 6.95(m,1H) ,6.86(d,J = 8.3Hz,1H),5.28-5.23(m,1H),5.17-5.11(m,1H),4.39(d,J = 5.3Hz,2H),3.89-3.75(m,4H ),3.55 -3.50(m,2H),3.43-3.35(m,2H), 2.29-2.24(m,1H),1.93-1.77(m,3H),1.53-1.49(m,1H),1.35-1.31(m, 1H).

Example 71: 4-(7-Fluoroimidazolo[1,2-a]pyridin-3-yl)-7-((7-((R)-3-fluoropyrrolidin-1-yl)-2',3',5',6,6',7-hexa hydrospiro [cyclopentyl[b]pyridin-5,4'-pyran]-2-yl)amino)isoindol-1-one

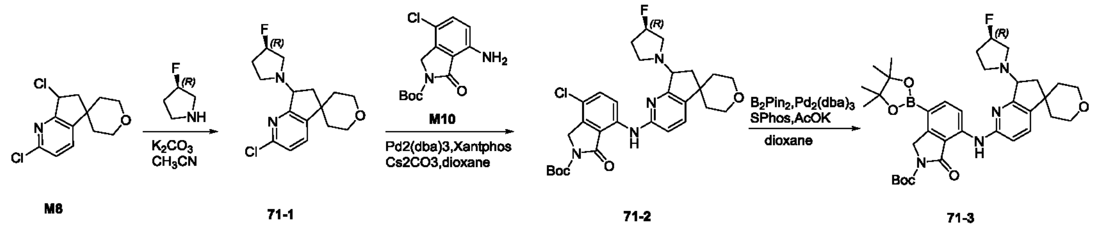


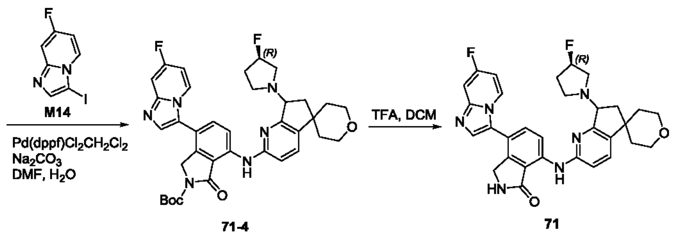


Step 1: Synthesis of compound 71-1

M8 (300.00 mg), 3(R)-fluoropyrrolidine hydrochloride (310 mg) and potassium carbonate (481 mg) were dissolved in acetonitrile (10 mL) and reacted at 80° C. for 12 h. After the reaction, ethyl acetate and water were added for extraction, and the organic phase was washed with saturated brine, separated, dried over anhydrous sodium sulfate, filtered, mixed, and separated by silica gel column chromatography (PE:EA=1:1) to obtain the target product 71-1 (280 mg, yield 77.53%).

ESI-MS m/z:311.14[M+H]+.

Step 2: Synthesis of compound 71-2

71-1 (280 mg), M10 (306 mg), $Pd_2(dba)_3$ (82 mg), Xantphos (104 mg), and $Cs_2CO_3$ (880 mg) were dissolved in 1,4-dioxane (10 mL) and reacted at 110°C for 5 h. After the reaction was completed, the mixture was filtered, the filter cake was washed with dichloromethane, concentrated, mixed, and separated by silica gel column chromatography (DCM:MeOH=50:1) to obtain the target product 71-2 (200 mg, yield 39.85%). ESI-MS m/z:557.37[M+H]+.

Step 3: Synthesis of compound 71-3

71-2 (200 mg), $B_2Pin_2$ (182 mg), $Pd_2(dba)_3$ (65 mg), Sphos (59 mg), and AcOK (105 mg) were dissolved in 1,4-dioxane (10 mL) and reacted at 100°C for 6 h. After the reaction was completed, the mixture was filtered, the filter cake was washed with dichloromethane, concentrated, mixed, and separated by silica gel column chromatography (DCM:MeOH=30:1) to obtain the target product 71-3 (171 mg, yield 73.44%). ESI-MS m/z:649.31[M+H]+.

Step 4: Synthesis of compound 71-4

At room temperature, compound 71-3 (171 mg), M14 (104 mg), $[PdCl_2(dppf)]CH_2Cl_2$ (21 mg), and sodium carbonate (83 mg) were added to DMF (4.5 mL) and water (0.5 mL), and then reacted at 90°C for 2 h under $N_2$ protection conditions, and the reaction was stopped. A certain amount of water was added to the reaction solution, and the mixture was extracted three times with EA. The organic phases were combined, washed with saturated brine, dried over anhydrous sodium sulfate, and concentrated. The concentrate was separated by silica gel column chromatography (DCM:MeOH=19:1) to give the target product 71-4 (138 mg, yield 79.69%). ESI-MS m/z:657.30[M+H]+.

Step 5: Synthesis of compound 71

At room temperature, compound 71-4 (138 mg) was dissolved in dichloromethane (5 mL), TFA (1 mL) was added, and the mixture was reacted at room temperature for 0.5 h, and the reaction was stopped. The pH was adjusted to alkaline with saturated sodium bicarbonate solution, extracted three times with DCM, the organic phases were combined, washed with saturated brine, dried over anhydrous sodium sulfate, concentrated, and the product was separated by silica gel column chromatography (DCM: MeOH = 17: 1). The product was dissolved in 2M HCl water to obtain the hydrochloride salt of the target product 71 (47.60 mg, yield 39.84%). ESI-MS m/z:557.19[M+H]+. 1H NMR(500MHz, DMSO-d 6 )δ12.27(s,1H),10.31-10.15(m,1H),8.95(d,J = 6.3Hz,1H),8.84-8. 47(m,2H),8.44(s,1H),8.04(dd,J = 8.2,2.6Hz,1H),7.84-7.80(m,2H),7.57-7.53(m,1H),7.20-7.06(m,1H),5.70-5.35(m,1H),5.18-5.13(m,1H),4.48-4.12(m,3-H),4.00-3.79 (m, 7H), 3.02-2.90(m, 1H),2.42-2.26(m,1H),2.25-1.98(m,3H),1.67-1.58(m,1H),1.66-1.55(m,1H), 1.43-1.40(m,1H).

Synthesis of compounds 71A and 71B:

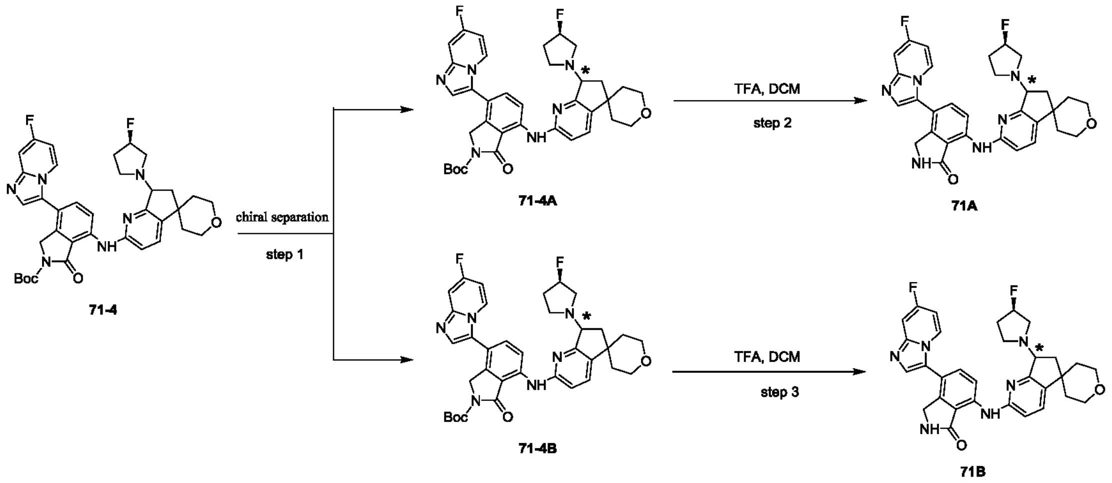


71-4, 71-4A, 71A, 71-4B, 71B

*Representing the R or S configuration, 71-4A or 71-4B are one of the R or S configurations, respectively

Step 1: Synthesis of compounds 71-4A and 71-4B

71-4 sample preparation: 200 mg of sample 71-4 was dissolved in 6 ml of dichloromethane:methanol (3:2) solution. Chromatographic conditions: 1. Chiral preparative column YMC CHIRAL ART Celluose-SB 250*20.0mm S-5um; 2. Instrument model GILSON GX-271; 3. Mobile phase, mobile phase A is n-hexane, mobile phase B is 0.1% diethylamine in ethanol, isocratic elution is with mobile phase A: mobile phase B in a ratio of 6:4, flow rate is 20 ml/min, wavelength is 254 nm, and separation is performed at room temperature. Compound 71-4A (peak 1): retention time = 13.566 min, recovered amount 70 mg; Compound 71-4B (peak 2): retention time = 20.645 min, recovered amount 55 mg.

Step 2: Synthesis of compound 71A

At room temperature, compound 71-4A (70 mg) was dissolved in dichloromethane (2 mL), TFA (0.5 mL) was added, and the mixture was reacted at room temperature for 0.5 h, and the reaction was stopped. The pH was adjusted to alkaline with saturated sodium bicarbonate solution, extracted three times with DCM, the organic phases were combined, washed with saturated brine, dried over anhydrous sodium sulfate, concentrated, and purified on a preparative plate (DCM: MeOH = 15:1) to isolate the product 71A (25.90 mg, purity 98.09%, yield 42.82%). ESI-MS m/z:557.39[M+H]+.

Step 3: Synthesis of compound 71B

At room temperature, compound 71-4B (55 mg) was dissolved in dichloromethane (2 mL), TFA (0.5 mL) was added, and the mixture was reacted at room temperature for 0.5 h, and then the reaction was stopped. The pH was adjusted to alkaline with saturated sodium bicarbonate solution, extracted three times with DCM, the organic phases were combined, washed with saturated brine, dried over anhydrous sodium sulfate, concentrated, and purified on a preparative plate (DCM: MeOH = 15:1) to isolate the product 71B (21.80 mg, purity 99.44%, yield 46.53%). ESI-MS m/z:557.39[M+H]+.

Example 72: Compound 7-((7-(3,3-difluoropyrrolidin-1-yl)-2',3',5',6,6',7-hexahydrospiro[b]pyridine-5,4'-pyran]-2-yl)amino)-4-(7-fluoroimidazole[1,2-a]pyridin-3-yl)isoindol-1-one

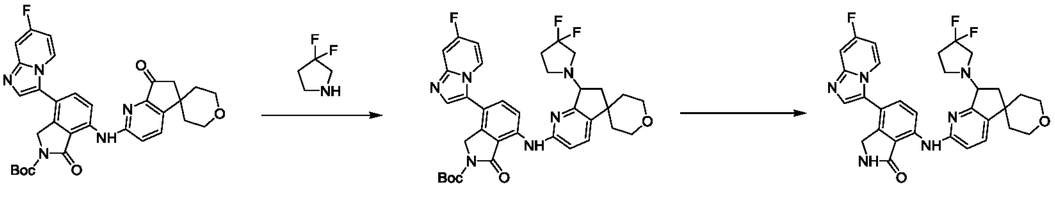

62-3 72-1 72

Step 1: Synthesis of compound 72-1

Weigh compound 62-3 (300 mg), 3,3-difluoropyrrolidine (165 mg), and tetraethyl titanate (117 mg), add DCE (5 mL) and MeOH (2 mL), react at room temperature for 1 h, slowly add sodium cyanoborohydride (97 mg), raise the temperature to 80°C, react for 2 h, and stop the reaction. The mixture was extracted with DCM, and the organic phase was washed with saturated brine. The organic phase was collected, dried, and separated by column chromatography (DCM: MeOH = 33:1) to obtain the target product 72-1 (290 mg, yield 83.62%). ESI-MS m/z:675.39 [M+H]+.

Step 2: Synthesis of compound 72

Weigh compound 72-1 (100 mg) and dissolve it in dichloromethane (3 mL) and methanol (3 mL). Slowly add a solution of hydrogen chloride in dioxane (6 mL, 4 M in dioxane) dropwise under ice bath conditions, slowly warm the temperature to room temperature and react for 30 min, then stop the reaction. After concentration, separation and purification by preparative liquid phase were performed to obtain the target product 72 (26.20 mg, yield 30.77%). ESI-MS m/z:575.44[M+H]+. $^1$H NMR(500MHz,DMSO-d 6 )δ10.19(s,1H),8.91(s,1H),8.66-8.58(m,1H), 8.56(d,J = 8.5Hz ,1H),8.24(s,1H),7.91(d,J = 7.3Hz,1H),7.76(dd,J = 14.8,8.5Hz,2H),7.38(t,J = 6.3Hz,1H),7.06(d,J = 8.4Hz,1H),4.71(s,1H),4.40(dd,J = 33.7,18.3Hz,2H),4.25-3.47(m,11H), 2.66(d,J = 2 3.5Hz,1H),2.15-1.96(m,2H),1.77-1.65(m,1H),1.51(d,J=12.5Hz,1H),1.38(d,J=12.6Hz,1H).

Example 85: 4-(7-fluoroimidazo[1,2-a]pyridin-3-yl)-7-((7-(isopropyl(methyl)amino)-2',3',5',6,6',7-hexahydro spiro[cyclopentyl[b]pyridin-5,4'-pyran]-2-yl)amino)isoindol-1-one

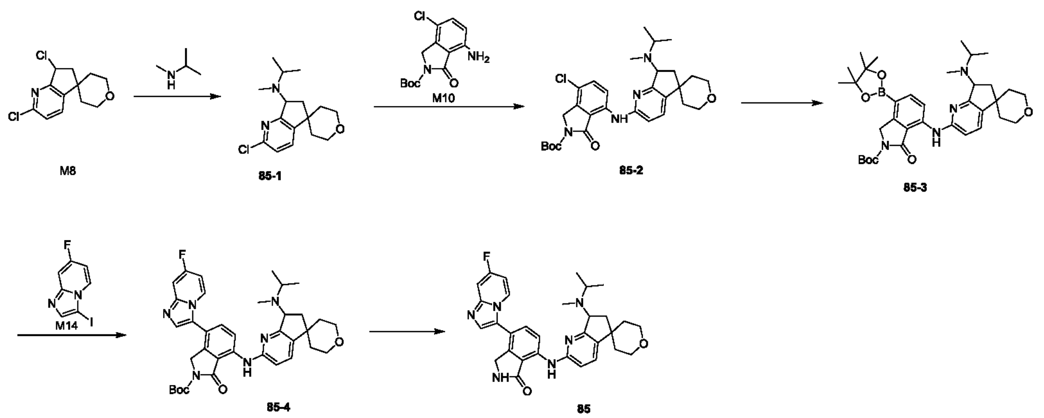


M8 85-1 85-2 85-3 85-4 85

Step 1: Synthesis of compound 85-1

M8 (300 mg), N-methyl isopropylamine (255 mg), potassium carbonate (482 mg), and potassium iodide (39 mg) were weighed, ACN (10 mL) was added, and the mixture was reacted at 80°C. for 12 h, and the reaction was stopped. The insoluble matter was filtered off, the filter residue was washed with DCM, the filtrate was concentrated, and the target product 85-1 (400 mg, yield 116.75%) was separated by column chromatography (PE:EA=2:1). ESI-MS m/z:295.17[M+H] +.

Step 2: Synthesis of compound 85-2

Weigh 85-1 (380 mg), M10 (437 mg), $Pd_2(dba)_3$ (118 mg), XantPhos (149 mg), and cesium carbonate (1.26 g),

add dioxane (15 mL), react at 120°C under nitrogen protection for 4 h, and stop the reaction. The organic phase was collected by filtration through celite, and dried by spin drying. The target product 85-2 (729 mg, yield 104.53%) was separated by column chromatography (DCM: MeOH = 33:1). ESI-MS m/z:541.28[M+H]+.

Step 3: Synthesis of compound 85-3

Weigh 85-2 (719 mg), diboronic acid pinacol ester (675 mg), $Pd_2(dba)_3$ (365 mg), SPhos (327 mg), and KOAC (391 mg), add dioxane (15 mL), react under nitrogen protection at 120°C for 8 h, and stop the reaction. The organic phase was collected by filtration through celite, and dried by spin drying. The target product 85-3 (324 mg, 38.54%) was separated by column chromatography (DCM: MeOH = 20:1), ESI-MS m/z: 633.41 [M+H]+.

Step 4: Synthesis of compound 85-4

Weigh 85-3 (324 mg), M14 (161 mg), $[PdCl_2(dppf)]CH_2Cl_2$ (42 mg), and sodium carbonate (163 mg), add to DMF (8 mL) and water (2 mL), then react at 90°C for 2 h under $N_2$ protection conditions, and stop the reaction. The insoluble matter was filtered off, the filter residue was washed with DCM, the filtrate was concentrated, and the concentrate was separated by silica gel column chromatography (DCM: MeOH = 8:1) to obtain the target product 85-4 (300 mg, yield 91.41%). ESI-MS m/z:641.36[M+H]+.

Step 5: Synthesis of compound 85

Weigh compound 85-4 (300 mg) and dissolve it in dichloromethane (3 mL) and methanol (3 mL). Slowly add a solution of hydrogen chloride in dioxane (6 mL, 4 M in dioxane) dropwise under ice bath conditions, slowly warm the temperature to room temperature and react for 30 min, then stop the reaction. The residue was concentrated and purified by preparative liquid separation to obtain the target product 85 (133.60 mg, yield 52.78%). ESI-MS m/z:541.50[M+H]+. $^1H$ NMR(500MHz,DMSO-d 6 )δ10.23(d,J = 5.9Hz,1H),9.97(d,J = 39.3Hz,1H),8.95(s,1H),8.69-8.56(m,1H), 8. 44(dd,J = 14.5,8.5Hz,1H),8.33(s,1H),8.01(dd,J= 8.4,1.9Hz,1H),7.88-7.77(m,2H),7.47(dd,J = 6.8,4.3Hz,1H) ,7.16(t,J = 8.0Hz,1H),5.24-5.13(m,1H),4.81-4.01(m,3H),3.94-3.83(m,2H),3.67-3.46(m,2H),3.02 (d,J=4.4Hz,1H),2.93-2.82(m,1H),2.74(d,J = 4.3Hz,2H),2.24-2.03(m,2H),1.64(dd,J = 12.1,8.4Hz,1H),1.58 - 1.33(m,8H).

Example 87: Compound 4-(7-fluoroimidazo[1,2-a]pyridin-3-yl)-7-((2,3,5,6,7',8'-hexahydro-6'H-spiro[pyran-4,5'-[1,7]naphthyridine]-2'-yl)amino)isoindol-1-one

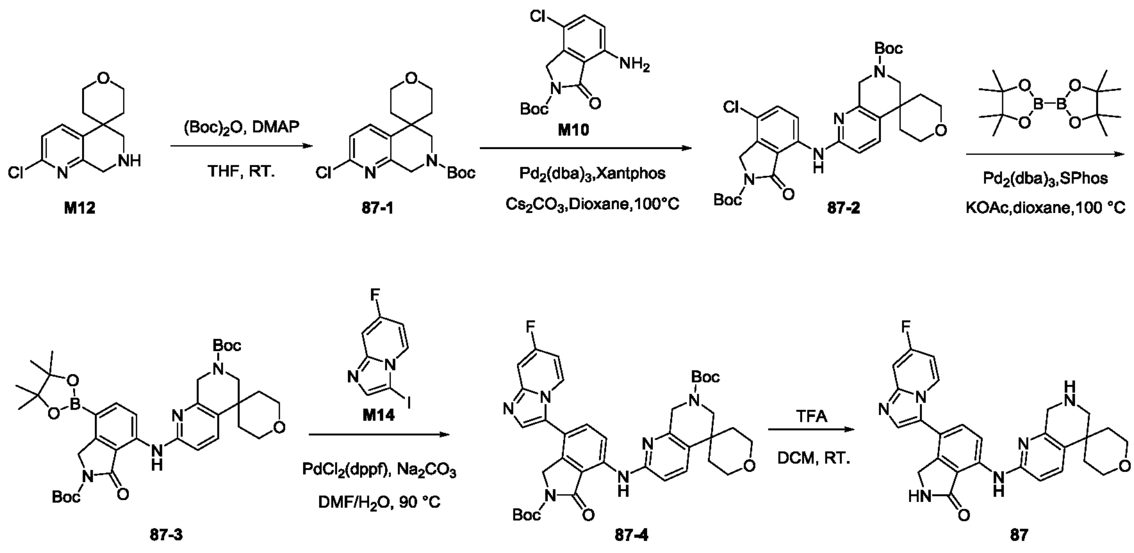


Step 1: Synthesis of compound 87-1

Compound M12 (250.00 mg) and DMAP (64.00 mg) were added to THF (6.00 mL) at room temperature, and di-tert-butyl dicarbonate (457.00 mg) was slowly added dropwise. The reaction was allowed to react at room temperature for

3 h and then stopped. A certain amount of water was added, extracted with EA for 3 times, the organic phases were combined, washed with saturated brine, dried over anhydrous sodium sulfate, and concentrated. The concentrate was separated by silica gel column chromatography (PE:EA=3:1) to obtain the target product 87-1 (136.00 mg, yield 38.41%). ESI-MS m/z:339.2[M+H]+.

Step 2: Synthesis of compound 87-2

Compound 87-1 (130.00 mg), M10 (130.17 mg), $Pd_2(dba)_3$ (35.16 mg), XantPhos (44.38 mg), and cesium carbonate (375.05 mg) were added to 1,4-dioxane (6.00 mL) at room temperature. Then, under the protection of $N_2$, the reaction was carried out at 100°C for 3 hours and then the reaction was stopped. The reaction solution was concentrated, and the concentrate was separated by silica gel column chromatography (PE:EA=1.5:1) to obtain the target product 87-2 (85.00 mg, yield 37.86%). ESI-MS m/z:585.3[M+H]+.

Step 3: Synthesis of compound 87-3

At room temperature, compound 87-2 (85.00 mg), $B_2Pin_2$ (73.79 mg), $Pd_2(dba)_3$ (26.65 mg), SPhos (23.85 mg), and potassium acetate (42.78 mg) were added to 1,4-dioxane (5.00 mL). Then, under $N_2$ protection conditions, the reaction was carried out at 100°C for 6 hours. The reaction solution was concentrated, and the concentrate was separated by silica gel column chromatography (DCM:MeOH=24:1) to obtain the target product 87-3 (80.00 mg, yield 81.37%). ESI-MS m/z: 677.4 [M+H]+.

Step 4: Synthesis of compound 87-4

At room temperature, compound 87-3 (80.00 mg), M14 (37.18 mg), $[PdCl_2(dppf)]CH_2Cl_2$ (10.00 mg), and sodium carbonate (37.58 mg) were added to DMF (4.00 mL) and water (0.40 mL), and then reacted at 85°C for 40 min under $N_2$ protection conditions, and the reaction was stopped. A certain amount of water was added to the reaction solution, and the mixture was extracted three times with EA. The organic phases were combined, washed with saturated brine, dried over anhydrous sodium sulfate, and concentrated. The concentrate was separated by silica gel column chromatography (DCM:MeOH=24:1) to give the target product 87-4 (50.00 mg, yield 61.78%).

ESI-MS m/z: 685.2 [M+H]+.

Step 5: Synthesis of compound 87

At room temperature, compound 87-4 (50.00 mg) was dissolved in dichloromethane (5.00 mL), TFA (396.22 mg) was added, and the mixture was reacted at room temperature for 0.5 h, and the reaction was stopped. The pH was adjusted to alkaline with saturated sodium bicarbonate solution, extracted three times with DCM, the organic phases were combined, washed with saturated brine, dried over anhydrous sodium sulfate, concentrated, and slurried to obtain the target product 87 (30.40 mg, purity 99.40%, yield 85.94%). ESI-MS m/z:585.1[M+H]+. $^1$H NMR(500MHz,DMSO-$d_6$) $\delta$10.03(s,1H),8.83(s,1H),8.69(d,J = 8.5Hz,1H),8.45( dd,J=7.6,5.7Hz,1H), 7.82(s,1H), 7.76(d,J=8.5Hz,1H), 7.72(d,J=8.5Hz,1H), 7.52(dd ,J=10.1,2.7Hz,1H), 6.98-6.94(m,1H), 6.85(d,J=8.5Hz,1H), 4.39(s,2H), 3.86(s,2H), 3.75-3.70(m,2H), 3.59-3.54(m,2H),3.08(s,2H),1.95-3.86(m,2H),1.60-1.55(m,2H).

Example 89: 7-((7'-cyclopropyl-2,3,5,6,7',8'-hexahydro-6'H-spiro[pyran-4,5'-[1,7]naphthyridine]-2'-yl)amino )-4-(7-fluoroimidazole[1,2-a]pyridin-3-yl)isoindol-1-one

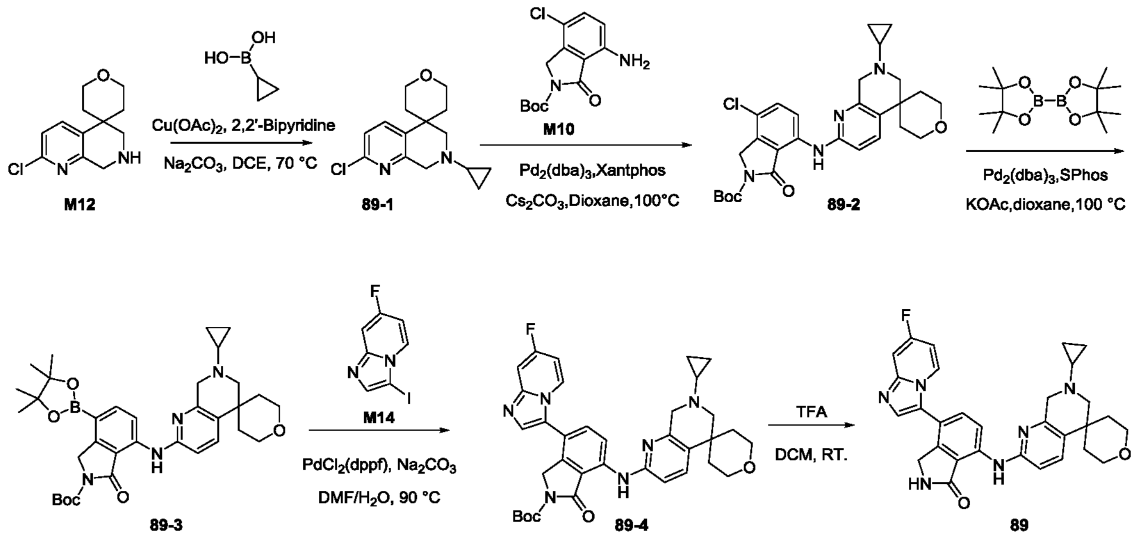


Step 1: Synthesis of compound 89-1

At room temperature, compound M12 (250.00 mg), cyclopropylboronic acid (180.00 mg), copper acetate (190.00 mg), 2,2'-bipyridine (163.00 mg) and sodium carbonate (222.00 mg) were added to DCE (6.00 mL), reacted at 70° C for 4 h, and the reaction was stopped. A certain amount of aqueous ammonia was added, extracted with DCM for three times, the organic phases were combined, washed with saturated brine, dried over anhydrous sodium sulfate, and concentrated. The concentrate was separated by silica gel column chromatography (PE:EA=5:1) to obtain the target product 89-1 (130.00 mg, yield 45.44%). ESI-MS m/z:279.2[M+H]+.

Step 2: Synthesis of compound 89-2

At room temperature, compound 89-1 (130.00 mg), M10 (145.17 mg), $Pd_2(dba)_3$ (41.02 mg), XantPhos/4,5-bis(diphenylphosphino)-9,9-dimethylxanthene (51.90 mg), and cesium carbonate (438.29 mg) were added to 1,4-dioxane (6.00 mL). Then, under the protection of $N_2$, the reaction was carried out at 100°C for 3 hours and then the reaction was stopped. The reaction solution was concentrated, and the concentrate was separated by silica gel column chromatography (PE:EA=4:1) to obtain the target product 89-2 (67.00 mg, yield 28.46%). ESI-MS m/z: 525.3 [M+H]+.

Step 3: Synthesis of compound 89-3

At room temperature, compound 89-2 (67.00 mg), $B_2Pin_2$/biboronic acid pinacol ester (62.88 mg), $Pd_2(dba)_3$ (22.71 mg), SPhos/2-dicyclohexylphosphino-2',6'-dimethoxybiphenyl (20.32 mg), and potassium acetate (36.45 mg) were added to 1,4-dioxane (5.00 mL). Then, under $N_2$ protection conditions, the reaction was carried out at 100°C for 6 hours. The reaction solution was concentrated, and the concentrate was separated by silica gel column chromatography (PE:EA=3:1) to obtain the target product 89-3 (70.00 mg, yield 91.71%). ESI-MS m/z: 617.4 [M+H]+.

Step 4: Synthesis of compound 89-4

At room temperature, compound 89-3 (70.00 mg), M14 (35.69 mg), $[PdCl_2(dppf)]CH_2Cl_2$ (10.00 mg), and sodium carbonate (37.58 mg) were added to DMF (4.00 mL) and water (0.40 mL), and then reacted at 85°C for 40 min under $N_2$ protection conditions, and the reaction was stopped. A certain amount of water was added to the reaction solution, and the mixture was extracted three times with EA. The organic phases were combined, washed with saturated brine, dried over anhydrous sodium sulfate, and concentrated. The concentrate was separated by silica gel column chromatography (DCM:MeOH=24:1) to give the target product 89-4 (50.00 mg, yield 70.52%).

ESI-MS m/z: 625.2 [M+H]+.

Step 5: Synthesis of compound 89

At room temperature, compound 89-4 (50.00 mg) was dissolved in dichloromethane (5.00 mL), TFA (396.22 mg) was added, and the mixture was reacted at room temperature for 0.5 h, and the reaction was stopped. The pH was adjusted to alkaline with saturated sodium bicarbonate solution, extracted three times with DCM, the organic phases were

combined, washed with saturated brine, dried over anhydrous sodium sulfate, concentrated, and slurried to obtain the target product 89 (28.00 mg, purity 99.22%, yield 55.17%). ESI-MS m/z:525.1 [M+H]+.

Example 100: Compound 4-(7-fluoroimidazo[1,2-a]pyridin-3-yl)-7-((7'-(methyl-d3)-2,3,5,6,7',8'-hexahydro-6'H-spiro [pyr an-4,5'-[1,7]naphthyridine]-2'-yl)amino)isoindol-1-one

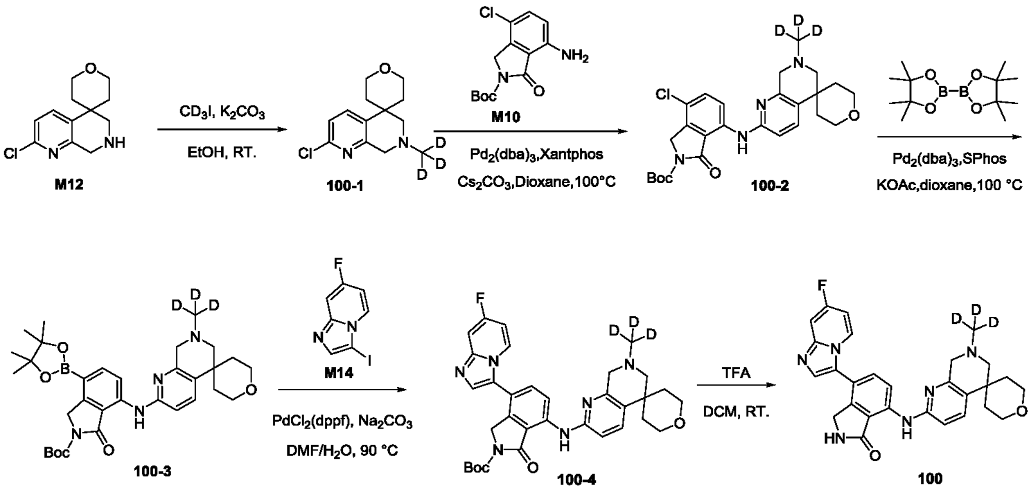


Step 1: Synthesis of compound 100-1

Compound M12 (250.00 mg) and potassium carbonate (283.69 mg) were added to ethanol (10.00 mL) at room temperature, and deuterated iodomethane (223.17 mg) was slowly added dropwise. The reaction was allowed to react at room temperature for 16 h and then stopped. A certain amount of water was added, extracted with EA for 3 times, the organic phases were combined, washed with saturated brine, dried over anhydrous sodium sulfate, and concentrated. The concentrate was separated by silica gel column chromatography (DCM:MeOH=19:1) to obtain the target product 100-1 (207.00 mg, yield 78.86%). ESI-MS m/z:256.2[M+H]+.

Step 2: Synthesis of compound 100-2

At room temperature, compound 100-1 (207.00 mg), M10 (256.17 mg), $Pd_2(dba)_3$ (72.02 mg), XantPhos/4,5-bis(diphenylphosphino)-9,9-dimethylxanthene (91.90 mg), and cesium carbonate (772.29 mg) were added to 1,4-dioxane (6.00 mL). Then, under the protection of $N_2$, the reaction was carried out at 100°C for 3 hours and then the reaction was stopped. The reaction solution was concentrated, and the concentrate was separated by silica gel column chromatography (DCM:MeOH=19:1) to obtain the target product 100-2 (149.00 mg, yield 36.69%). ESI-MS m/z: 502.3 [M+H]+.

Step 3: Synthesis of compound 100-3

At room temperature, compound 100-2 (149.00 mg), $B_2Pin_2$/biboronic acid pinacol ester (151.88 mg), $Pd_2(dba)_3$ (55.71 mg), SPhos/2-dicyclohexylphosphino-2',6'-dimethoxybiphenyl (49.32 mg), and potassium acetate (87.45 mg) were added to 1,4-dioxane (5.00 mL). Then, under $N_2$ protection conditions, the reaction was carried out at 100°C for 6 hours. The reaction solution was concentrated, and the concentrate was separated by silica gel column chromatography (DCM:MeOH=19:1) to obtain the target product 100-3 (76.00 mg, yield 43.18%). ESI-MS m/z:594.4[M+H]+.

Step 4: Synthesis of compound 100-4

At room temperature, compound 100-3 (76.00 mg), M14 (40.69 mg), $[PdCl_2(dppf)]CH_2Cl_2$ (11.00 mg), and sodium carbonate (41.58 mg) were added to DMF (4.00 mL) and water (0.40 mL), and then reacted at 85°C for 40 min under $N_2$ protection conditions, and the reaction was stopped. A certain amount of water was added to the reaction solution, extracted with EA three times, the organic phases were combined, washed with saturated brine, dried over anhydrous sodium sulfate, and concentrated. The concentrate was separated by silica gel column chromatography (DCM:MeOH=19:1) to obtain the target product 100-4 (50.00 mg, yield 64.93%). ESI-MS m/z: 602.2 [M+H]+.

Step 5: Synthesis of compound 100

At room temperature, compound 100-4 (50.00 mg) was dissolved in dichloromethane (5.00 mL), TFA (396.22 mg) was added, and the mixture was reacted at room temperature for 0.5 h, and the reaction was stopped. The pH was adjusted to alkaline with saturated sodium bicarbonate solution, extracted three times with DCM, the organic phases were combined, washed with saturated brine, dried over anhydrous sodium sulfate, concentrated, and slurried to obtain the target product 100 (30.7.00 mg, purity 96.46%, yield 59.21%). ESI-MS m/z:502.1[M+H]+. $^1$H NMR(500MHz,DMSO-d 6 ) δ10.06(s,1H),8.84(s,1H),8.71(d,J = 8.5Hz,1H),8.44 (dd,J = 7.5,5.8Hz,1H),7.83(s,1H),7.77(d,J = 8.6Hz,1H),7.72(d,J = 8.6Hz,1H),7.53(d d,J = 10.0,2.6Hz,1H),6.98-6.94(m,1H),6.86(d,J = 8.6Hz,1H),4.39(s,2H),3.75-3.71( m,2H),363-3.57(m,2H), 3.52(s,2H),2.73(s,2H),1.95-1.88(m,2H),1.58-1.54(m,2H).

Example 123: Synthesis of Compound 4-(7-(difluoromethoxy)imidazo[1,2-a]pyridin-3-yl)-7-((2,3,5,6,7',8'-hexahydro-6'H-spiro[pyran-4,5'-[1,7]naphthyridine]-2'-yl)amino)isoindol-1-one

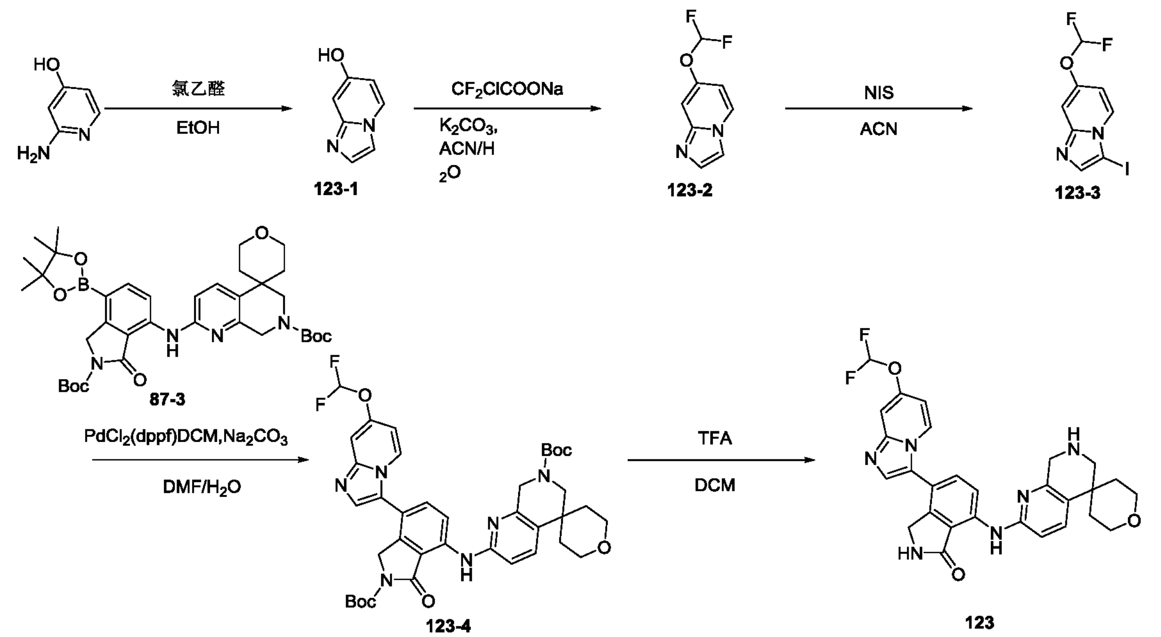


Step 1: Synthesis of compound 123-1

At room temperature, the compound 2-amino-4-hydroxypyridine (2.00 g) and chloroacetaldehyde (10.69 g, 40% aqueous solution) were added to ethanol (15.00 mL), and then under $N_2$ protection conditions, the reaction was kept at 80°C for 18 hours and the reaction was stopped. The reaction solution was directly concentrated to obtain a crude product, which was separated by column chromatography (DCM: MeOH = 95:5 to 85:15) to obtain the target product 123-1 (2.2 g, yield 90.30%). ESI-MS m/z:135.15[M+H]+.

Step 2: Synthesis of compound 123-2

At room temperature, compound 123-1 (1.00 g), sodium difluorochloroacetate (5.68 g), and potassium carbonate (2.06 g) were added to water (7.5 mL) and acetonitrile (37.50 mL), and then the reaction was carried out at 110° C. under $N_2$ protection conditions for 24 h, and the reaction was stopped. The solid was directly filtered from the reaction solution, and then washed with 10 mL (DCM:MeOH=10:1) for 3 times. The mother liquors were combined and concentrated to obtain a crude product. The crude product was separated by column chromatography (DCM:MeOH=97:3-90:10) to obtain the target product crude 123-2 (1.4 g, yield 101.98%). ESI-MS m/z:185.20[M+H]+.

Step 3: Synthesis of compound 123-3

At room temperature, the crude compound 123-2 (500.00 mg) was added to acetonitrile (10.00 mL), and then NIS

(733.10 mg) was slowly added in batches under ice-water bath conditions. The reaction was allowed to react at room temperature for 0.5 h, and then the reaction was stopped. The reaction solution was directly added with 50 mL of water to quench the reaction, and then extracted with 10 mL of DCM three times, washed with saturated sodium chloride, and the organic phase was concentrated and dried to obtain a crude product. The crude product was separated by column chromatography (DCM: MeOH = 97:3 to 90:10) to obtain the target product 123-3 (75 mg, yield 8.91%). ESI-MS m/z:311.10[M+H]+.

Step 4: Synthesis of compound 123-4

At room temperature, compound 123-3 (25.20 mg), compound 87-3 (50.00 mg), $[PdCl_2(dppf)]CH_2Cl_2$ (6.04 mg), and sodium carbonate (23.50 mg) were added to DMF (2.00 mL) and water (0.40 mL), and then reacted at 90°C for 4 h under $N_2$ protection conditions, and the reaction was stopped. The reaction solution was concentrated to obtain a crude product, which was separated by column chromatography (DCM:MeOH=95:5-85:15) to obtain the target product 123-4 (30.00 mg, yield 55.40%). ESI-MS m/z:733.71[M+H]+.

Step 5: Synthesis of compound 123

At room temperature, compound 123-4 (30.00 mg) was added to dichloromethane (4.00 mL), and then trifluoroacetic acid (1.00 mL) was added in an ice-water bath under $N_2$ protection conditions. The reaction was allowed to react at room temperature for 1 h, and the reaction was stopped. The reaction solvent was removed by rotary evaporation, and the target product 123 (2.20 mg, purity 99.42%, yield 10.04%) was obtained by preparation and isolation. ESI-MS m/z:533.53[M+H]+. 1H NMR (500MHz, DMSO) δ10.27(s,1H),9.31(s,2H),8.98(s,1H),8.77(d,J = 8.6Hz,1H),8.67(d,J = 7.6Hz, 1 H),8.32(s,1H),7.96(d,J = 8.8Hz,1H),7.82(s,1H),7.80(d,J = 8.6Hz,1H),7.75(d,J = 1.9Hz, 1H),7.68 (s,1H),7.53(s,1H),7.29(dd,J = 7.4,2.2Hz,1H),7.08(d,J = 8.7Hz,1H),4.42(s,2H),4.29(s, 2H),3.8 0(dd,J=11.4,4.5Hz,2H),3.65(d,J=11.7Hz,4H),2.02(td,J = 13.6,4.9Hz,2H),1.69(d,J=13.2Hz,2H ).

Example 133: Synthesis of Compound 7-((7',8'-dihydro-6'H-spiro[cyclopropane-1,5'-[1,7]naphthyridine]-2'-yl)amino)-4-(7-fluoroimid azo[1,2-a]pyridin-3-yl)isoindol-1-one

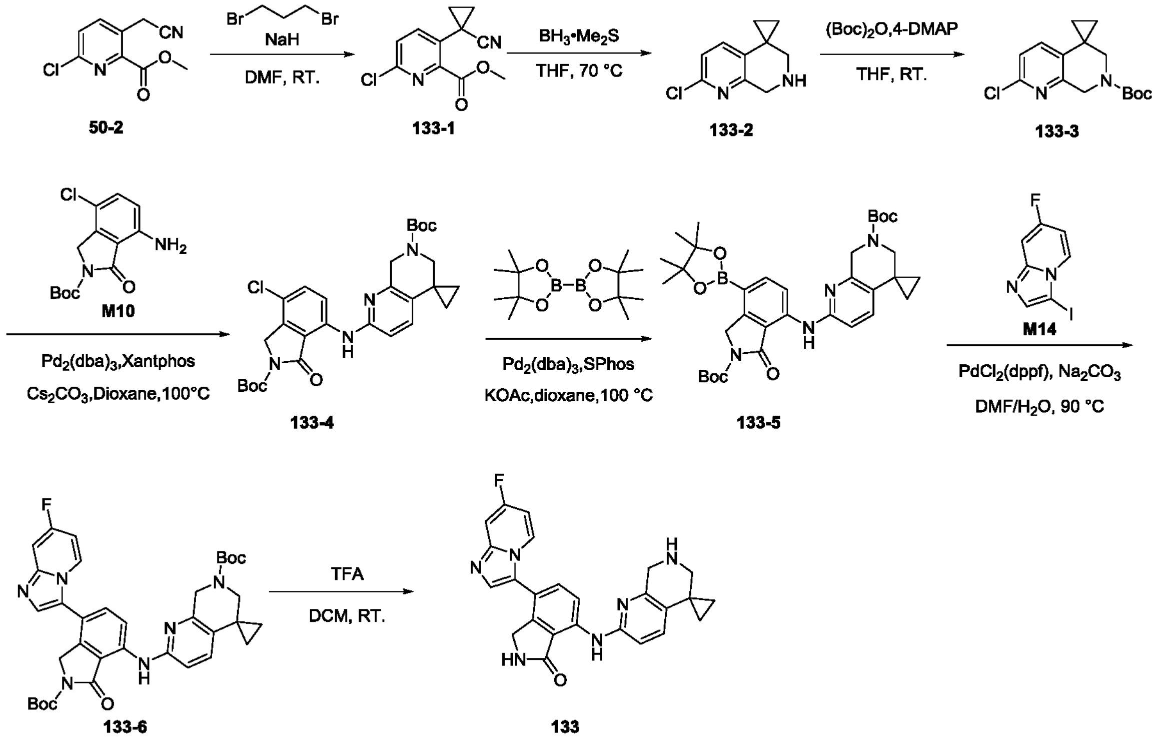


50-2 133-1 133-2 133-3

M10 133-4 133-5 M14

133-6 133

Step 1: Synthesis of compound 133-1

At room temperature, compound NaH (2.18 g) was dissolved in DMF (40.00 mL), and then 50-2 (5.00 g) dissolved in DMF (10.00 mL) was slowly added under ice bath and nitrogen protection, and the reaction was kept warm for 0.5 h. Then 1,2-dibromoethane (3.52 g) was slowly added dropwise, and the reaction was carried out at room temperature for 3 h to stop the reaction. The reaction was quenched with saturated ammonium chloride solution under ice bath condition, extracted with EA three times, and the organic phases were combined, washed with saturated brine, dried over anhydrous sodium sulfate, and concentrated. The concentrate was separated by silica gel column chromatography (PE:EA=3:1) to obtain the target product 133-1 (1.28 g, yield 22.78%).

ESI-MS m/z:236.78[M+H]+.

Step 2: Synthesis of compound 133-2

At room temperature, compound 133-1 (1.28 g) was dissolved in THF (20.00 mL), and then borane dimethyl sulfide solution (2.16 mL, 10 M) was slowly added dropwise under ice bath conditions. Under nitrogen protection, the reaction was carried out at 70°C. for 20 h, and the reaction was stopped. The pH was adjusted to 1-2 with 1M HCl solution, reacted at 70°C for 1 h, and the reaction was stopped. The pH value was adjusted to 8-9 with 2M NaOH solution, extracted three times with EA, the organic phases were combined, washed with saturated brine, dried over anhydrous sodium sulfate, and concentrated. The concentrate was separated by silica gel column chromatography (DCM:MeOH=19:1) to obtain the target product 133-2 (240.00 mg, yield 22.79%). ESI-MS m/z:237.65[M+H]+.

Step 3: Synthesis of compound 133-3

Compound 133-2 (240.00 mg) and 4-DMAP (150.00 mg) were added to THF (8.00 mL) at room temperature, and di-tert-butyl dicarbonate (670.00 mg) was slowly added dropwise. The reaction was allowed to react at room temperature for 5 h and then stopped. 8 ml of water was added, and the mixture was extracted three times with EA. The organic phases were combined, washed with saturated brine, dried over anhydrous sodium sulfate, and concentrated. The concentrate was separated by silica gel column chromatography (PE:EA=3:1) to obtain the target product 133-3 (310.00 mg, yield 85.31%). ESI-MS m/z:295.80[M+H]+.

Step 4: Synthesis of compound 133-4

At room temperature, compound 133-3 (310.00 mg), M10 (327.05 mg), $Pd_2(dba)_3$ (96.33 mg), XantPhos/4,5-bis(diphenylphosphino)-9,9-dimethylxanthene (121.63 mg), and cesium carbonate (1027.90 mg) were added to 1,4-dioxane (8.00 mL). Then, under the protection of $N_2$, the reaction was carried out at 100°C for 8 hours and then the reaction was stopped. The reaction solution was concentrated, and the concentrate was separated by silica gel column chromatography (PE:EA=1.5:1) to obtain the target product 133-4 (330.00 mg, yield 37.86%). ESI-MS m/z:542.08 [M+H]+.

Step 5: Synthesis of Compound 133-5

At room temperature, compound 133-4 (330.00 mg), biboronic acid pinacol ester (309.80 mg), $Pd_2(dba)_3$ (111.70 mg), SPhos/2-dicyclohexylphosphino-2',6'-dimethoxybiphenyl (100.20 mg), and potassium acetate (179.60 mg) were added to 1,4-dioxane (8.00 mL).Then, under $N_2$ protection conditions, the reaction was carried out at 100°C for 8 hours.The reaction solution was concentrated, and the concentrate was separated by silica gel column chromatography (PE:EA:DCM=10:1:1-5:1:1-3:1:1) to obtain the target product 133-5 (150.00 mg, yield 38.88%). ESI-MS m/z: 633.62 [M+H]+.

Step 6: Synthesis of compound 133-6

At room temperature, compound 133-5 (80.00 mg), M14 (41.01 mg), $[PdCl_2(dppf)]CH_2Cl_2$ (11.60 mg), and sodium carbonate (45.25 mg) were added to DMF (4.00 mL) and water (0.40 mL), and then reacted at 85°C for 30 min under $N_2$ protection conditions, and the reaction was stopped. The reaction solution was directly spin-dried to obtain a crude product. The concentrate was separated by silica gel column chromatography (DCM:MeOH=97:3-88:12) to obtain the target product 133-6 (70.00 mg, yield 76.78%). ESI-MS m/z: 641.63 [M+H]+.

Step 7: Synthesis of compound 133

At room temperature, compound 133-6 (70.00 mg) was dissolved in dichloromethane (4.00 mL), TFA (1.00 mL) was added, and the mixture was reacted at room temperature for 1.5 h, and the reaction was stopped. The reaction solution was concentrated to obtain a crude product, which was then separated and prepared to give the target product 133 (34.10 mg, purity 99.80%, yield 70.69%). ESI-MS m/z:441.16[M+H]+. Trifluoroacetate $^1$H NMR (500 MHz, DMSO) $\delta$=10.19 (s, 1H), 9.53 (s, 2H), 8.93 (s, 1H), 8.74 (d, J=8.5, 1H), 8.68-8.60 (m, 1H), 8.22 (s, 1H), 7.89 (d, J=7.7, 1H), 7.78 (d, J=8.5, 1H), 7.33 (d, J=7.0, 1H), 7.30-7.25 (m, 1H), 7.10 (d, J=51.0, 1H), 6.98 (d, J=8.6, 1H), 4.41 (s, 4H), 1.19 (d, J=48.0, 2H), 1.10 (s, 4H).

Example 146: Synthesis of Compound 3-(7-((2,3,5,6,7',8'-hexahydro-6'-H-spiro[pyran-4,5'-[1,7]naphthyridine]-2'-yl) amino)-1-oxoisoi ndol-4-yl)imidazo[1,2-a]pyridine-7-carbonitrile

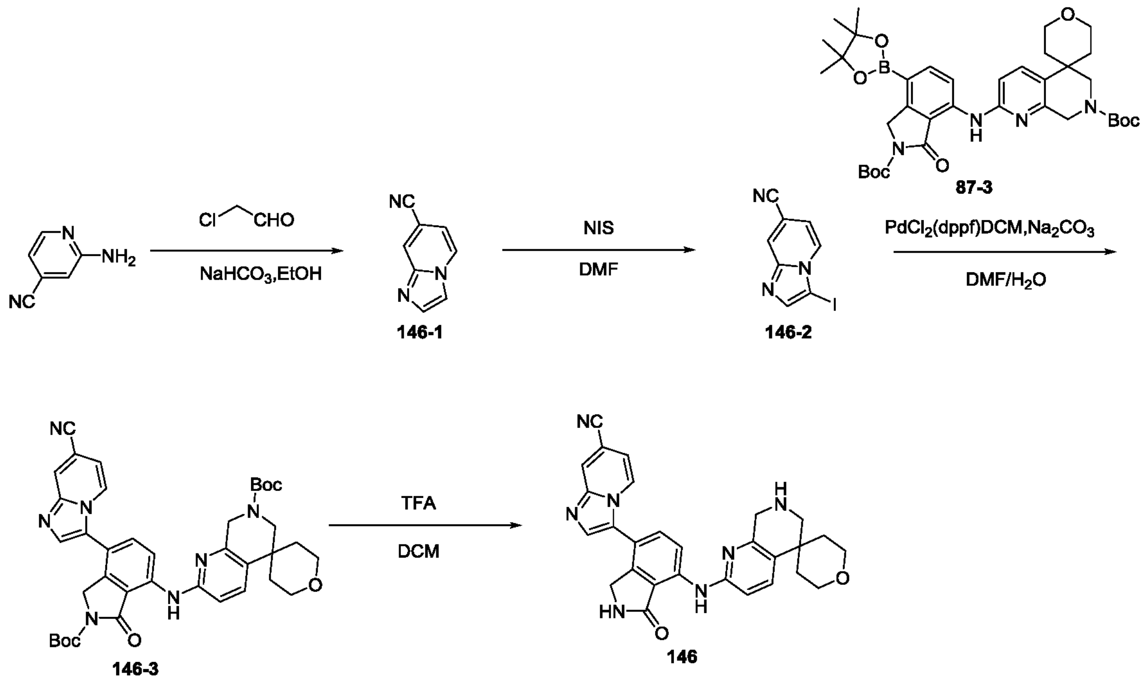


Step 1: Synthesis of Intermediate 146-1

At room temperature, the compound 2-amino-4-cyanopyridine (2.00 g), chloroacetaldehyde (6.59 g, 40% aqueous solution), and sodium bicarbonate (4.23 g) were added to ethanol (30.00 mL), and then under $N_2$ protection conditions, the reaction was kept at 80°C for 8 hours and the reaction was stopped. The reaction solution was directly concentrated to obtain a crude product, which was separated by column chromatography (DCM: MeOH = 95:5 to 90:10) to obtain the target product 146-1 (1.64 g, yield 68.24%). ESI-MS m/z:144.15[M+H]+.

Step 2: Synthesis of Intermediate 146-2

At room temperature, compound 146-1 (1.64 g) was added to DMF (40.00 mL), and then NIS (3.09 g) was slowly added in batches under ice-water bath conditions. The mixture was reacted at room temperature for 2 h and the reaction was stopped. The reaction solution was directly added with 50 mL of ice water to quench the reaction. A large amount of white solid precipitated and was filtered and dried to obtain the target product 146-2 (2.30 g, yield 74.62%). ESI-MS m/z:270.12[M+H]+.

Step 3: Synthesis of Intermediate 146-3

At room temperature, compound 146-2 (43.80 mg), intermediate 87-3 (100.00 mg), [$PdCl_2$(dppf)]$CH_2Cl_2$ (12.00 mg), and sodium carbonate (47.00 mg) were added to DMF (4.00 mL) and water (0.50 mL), and then reacted at 90°C for 1 h

under $N_2$ protection conditions to stop the reaction. The reaction solution was concentrated to obtain a crude product, which was separated by column chromatography (DCM: MeOH = 95:5 to 85:15) to obtain the target product 146-3 (80.00 mg, yield 78.24%). ESI-MS m/z:692.73[M+H]+.

Step 4: Synthesis of compound 146

At room temperature, compound 146-3 (80.00 mg) was added to dichloromethane (4.00 mL), and then trifluoroacetic acid (1.00 mL) was added in an ice-water bath under $N_2$ protection conditions. The reaction was allowed to react at room temperature for 1.5 h, and the reaction was stopped. The reaction solvent was removed by rotary evaporation, and the target product 146 (55.50 mg, purity 99.67%, yield 97.67%) was obtained by preparation and isolation. ESI-MS m/z:491.53[M+H]+. Trifluoroacetate $^1$H NMR (500MHz, DMSO) δ10.27 (s, 1H), 9.27 (s, 2H), 8.95 (s, 1H), 8.75 (d, J = 8.6 Hz, 1H), 8.57 (d, J = 7.2 Hz, 1H), 8.50 (s, 1H), 8.21 (s, 1H), 7.94 (d, J = 8.8 Hz, 1H), 7.80 (d,J= 8.6Hz,1H),7.27(dd,J=7.2,1.3Hz,1H),7.06(d,J= 8.7Hz,1H), 4.44(s,2H), 4.30( s,2H),3.83-3.78(m,2H),3.71-3.58(m,4H),2.05-1.98(m,2H),1.71-1.68(m,2H).

Example 147: Synthesis of Compound 7-((2,3,5,6,7',8'-hexahydro-6'-H-pyrano[4,5'-[1,7]naphthyridine]-2'-yl)amino)-4-(7-(trifluorome thyl)imidazo[1,2-a]pyridin-3-yl)isoindol-1-one

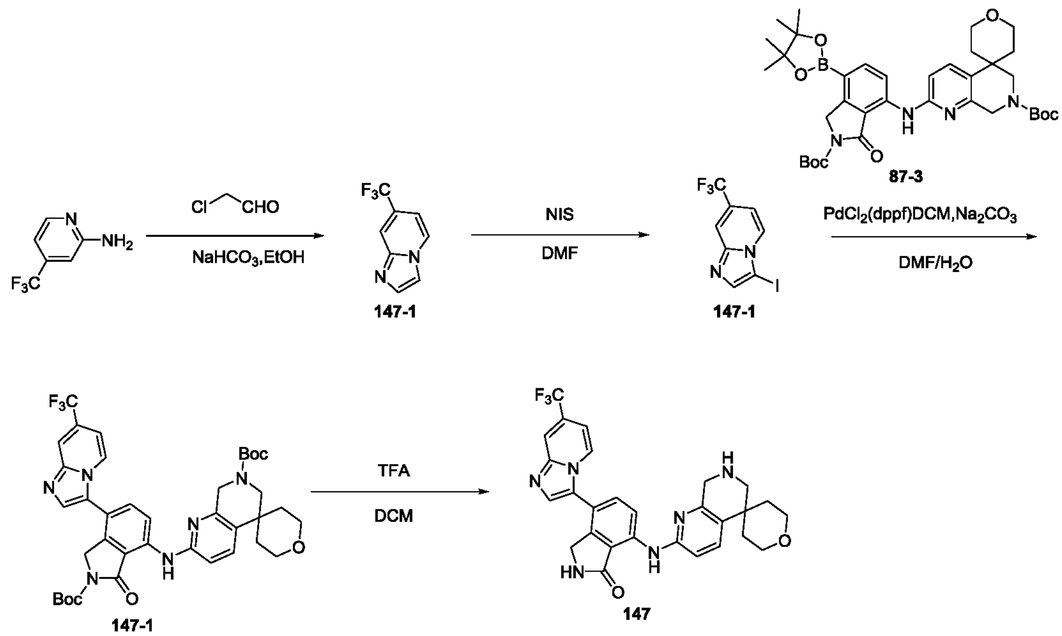


Step 1: Synthesis of Intermediate 147-1

At room temperature, compound 2-amino-4-trifluoromethylpyridine (1.00 g), chloroacetaldehyde (2.42 g, 40% aqueous solution), and sodium bicarbonate (1.55 g) were added to ethanol (15.00 mL), and then under $N_2$ protection conditions, the reaction was kept at 80°C for 4 hours and the reaction was stopped. The reaction solution was directly concentrated to obtain a crude product, which was separated by column chromatography (DCM: MeOH = 95:5 to 90:10) to obtain the target product 147-1 (1.00 g, yield 87.09%). ESI-MS m/z:187.15[M+H]+.

Step 2: Synthesis of Intermediate 147-2

At room temperature, compound 147-1 (1.00 g) was added to DMF (15.00 mL), and then NIS (1.05 g) was slowly added in batches under ice-water bath conditions. The reaction was allowed to react at room temperature for 1 h, and then the reaction was stopped. The reaction solution was directly added with 50 mL of ice water to quench the reaction. A large amount of light yellow solid precipitated and was filtered and dried to obtain the target product 147-2 (0.70 g, yield 41.76%). ESI-MS m/z:313.12[M+H]+.

Step 3: Synthesis of Intermediate 147-3

At room temperature, compound 147-2 (40.56 mg), intermediate 87-3 (80.00 mg), [$PdCl_2$(dppf)]$CH_2Cl_2$ (9.60 mg), and sodium carbonate (37.60 mg) were added to DMF (4.00 mL) and water (0.50 mL), and then reacted at 90°C for 1 h under $N_2$ protection conditions, and the reaction was stopped. The reaction solution was concentrated to obtain a crude product, which was separated by column chromatography (PE:EA=95:5-50:50) to obtain the target product 147-3 (70.00 mg, yield 80.60%). ESI-MS m/z:735.73[M+H]+.

Step 4: Synthesis of compound 147

At room temperature, compound 147-3 (70.00 mg) was added to dichloromethane (4.00 mL), and then trifluoroacetic acid (1.00 mL) was added in an ice-water bath under $N_2$ protection conditions. The reaction was allowed to react at room temperature for 1.5 h, and the reaction was stopped. The reaction solvent was removed by rotary evaporation, and the target product 147 (50.80 mg, purity 99.77%, yield 99.49%) was obtained by preparation and isolation. ESI-MS m/z:534.53[M+H]+. Trifluoroacetate $^1$H NMR (500MHz, DMSO) δ10.26 (s, 1H), 9.20 (s, 2H), 8.94 (s, 1H), 8.75 (d, J = 8.5 Hz, 1H), 8.61 (d, J = 7.3 Hz, 1H), 8.24 (s, 1H), 8.16 (s, 1H), 7.94 (d, J = 8.8 Hz, 1H), 7.81 (d, J = 8.6Hz,1H), 7.23(dd,J=7.3,1.8Hz,1H), 7.06(d,J= 8.7Hz,1H), 4.44(s,2H),4.29( s,2H),3.82-3.78(m,2H),3.66-3.61(m,4H),2.04-1.98(m,2H),1.70-1.65(m,2H).

Example 149: Synthesis of Compound 4-(8-fluoro-7-methyfimidazo[1,2-a]pyridin-3-yl)-7-((2,3,5,6,7',8'-hexahydro-6'H-spiro[pyran-4, 5'-[1,7]naphthyridine]-2'-yl)amino)isoindol-1-one

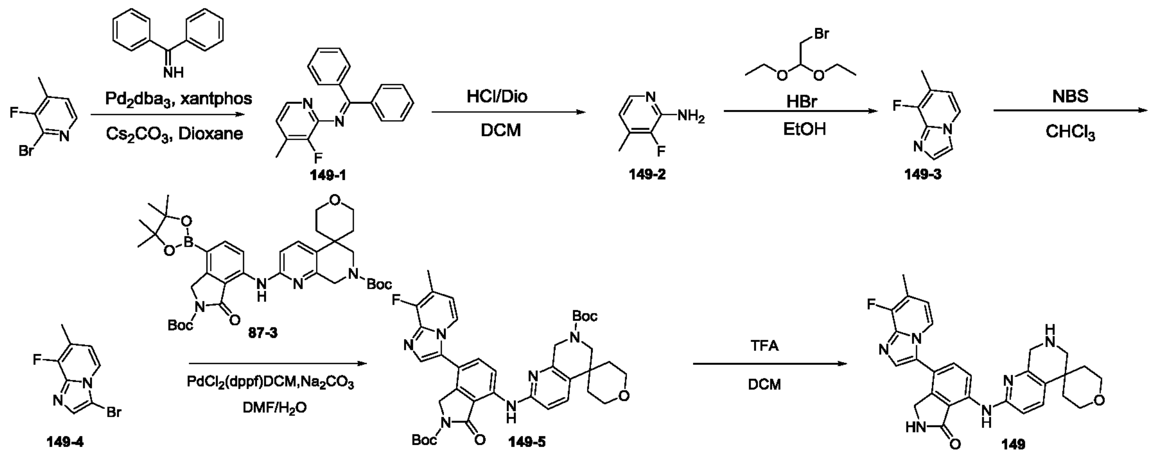


Step 1: Synthesis of compound 149-1

Compound 2-bromo-3-fluoro-4-methylpyridine (3.00 g), benzophenone imine (4.29 g), $Pd_2(dba)_3$ (1.45 g), XantPhos (1.83 g), cesium carbonate (15.43 g) were added to 1,4-dioxane (40.00 mL) at room temperature. Then, under the protection of $N_2$, the reaction was carried out at 110°C for 20 hours and the reaction was stopped. The reaction solution was filtered to remove salt, and the filter cake was washed with EA for 3 times, 30 mL each time. The filtrates were combined and concentrated. The concentrate was separated by silica gel column chromatography (PE:EA=90:10-75:25) to obtain the target product 149-1 (4.20 g, yield 91.63%). ESI-MS m/z:291.41[M+H]+.

Step 2: Synthesis of compound 149-2

At room temperature, compound 149-1 (4.20 mg) was added to dichloromethane (15.00 mL), and then HCl (10.00 mL, 4 M in Dioxane) was added in an ice-water bath. The reaction was allowed to react at room temperature for 1 h, and then the reaction was stopped. The reaction solvent was removed by rotary evaporation to obtain a crude product, and then 30 mL of $H_2O$ and 30 mL of EA were added. EA was used for extraction to remove impurities, and saturated sodium bicarbonate was added to the remaining aqueous phase to adjust the alkali to pH = 8-9, and then DCM was extracted 3 times, 50 mL each time, and washed with saturated sodium chloride. The target product 149-2 (1.20 g, yield 65.77%) was separated by column chromatography (PE: EA = 90:10-50:50) as a light yellow solid was obtained. ESI-MS m/z:127.13[M+H]+.

Step 3: Synthesis of Intermediate 149-3

At room temperature, compound 149-2 (1.24 g) and 2-bromo-1,1-diethoxyethane (3.75 g) were added to ethanol (8.00 mL), and then HBr (1.5 mL, 40% $H_2O$) was slowly added under $N_2$ protection conditions and an ice-water bath. The reaction was kept at 80°C for 36 hours and the reaction was stopped. Aqueous NaOH solution was added to the reaction solution to adjust the alkali to pH>10, then extracted with DCM 4 times, 50 mL each time, washed with saturated sodium chloride, and separated by column chromatography (PE:EA=90:10-50:50) to obtain the target product 149-3 (0.62 g, yield 43.40%) as a light yellow solid. ESI-MS m/z:151.13[M+H]+.

Step 4: Synthesis of Intermediate 149-4

At room temperature, compound 149-3 (0.62 g) was added to DMF (10.00 mL), and then NIS (1.11 g) was slowly added in batches under ice-water bath conditions. The mixture was reacted at room temperature for 2 h and the reaction was stopped. The reaction solution was directly added with 50 mL of ice water to quench the reaction. A large amount of light yellow solid precipitated, which was filtered and dried to obtain the target product 149-4 (0.90 g, yield 78.96%) as a light yellow solid. ESI-MS m/z:277.10[M+H]+.

Step 5: Synthesis of Intermediate 149-5

At room temperature, compound 149-4 (35.90 mg), intermediate 87-3 (80.00 mg), $[PdCl_2(dppf)]CH_2Cl_2$ (9.60 mg), and sodium carbonate (37.60 mg) were added to DMF (4.00 mL) and water (0.50 mL), and then reacted at 90°C for 1 h under $N_2$ protection conditions to stop the reaction. The reaction solution was concentrated to obtain a crude product, which was separated by column chromatography (DCM:EA=95:5-50:50) to obtain the desired product 149-5 (70.00 mg, yield 84.75%) as a light yellow solid. ESI-MS m/z:699.80[M+H]+.

Step 6: Synthesis of compound 149

At room temperature, compound 149-5 (70.00 mg) was added to dichloromethane (4.00 mL), and then trifluoroacetic acid (1.00 mL) was added in an ice-water bath under $N_2$ protection conditions. The reaction was allowed to react at room temperature for 1.5 h, and then the reaction was stopped. The reaction solvent was removed by rotary evaporation, and the target product 149 (4.50 mg, purity 99.86%, yield 9.00%) was obtained as a white solid. ESI-MS m/z:499.81[M+H]+. Trifluoroacetate $^1H$ NMR (500 MHz, DMSO) δ 10.24 (s, 1H), 9.22 (s, 2H), 8.93 (s, 1H), 8.73 (d, J = 8.6 Hz, 1H), 8.21 (d, J = 6.9 Hz, 1H), 7.93 (d, J = 8.8 Hz, 2H), 7.74 (d, J = 8.5 Hz, 1H), 7.05 (d, J = 8.7 Hz, 1H), 6.95 (s, 1H), 4.41 (s, 2H), 4.28 (s, 2H), 3.84-3.77 (m, 2H), 3.64 (d, J = 11.5 Hz, 4H), 2.37 (s, 3H), 2.04-1.98 (m, 2H), 1.70-1.65 (m, 2H).

Example 152: Synthesis of Compound 4-(7-chloroimidazo[1,2-a]pyridin-3-yl)-7-((7',8'-dihydro-6'H-spiro[cyclopropane-1,5'-[1,7]naph thyridine]-2'-yl)amino)isoindol-1-one

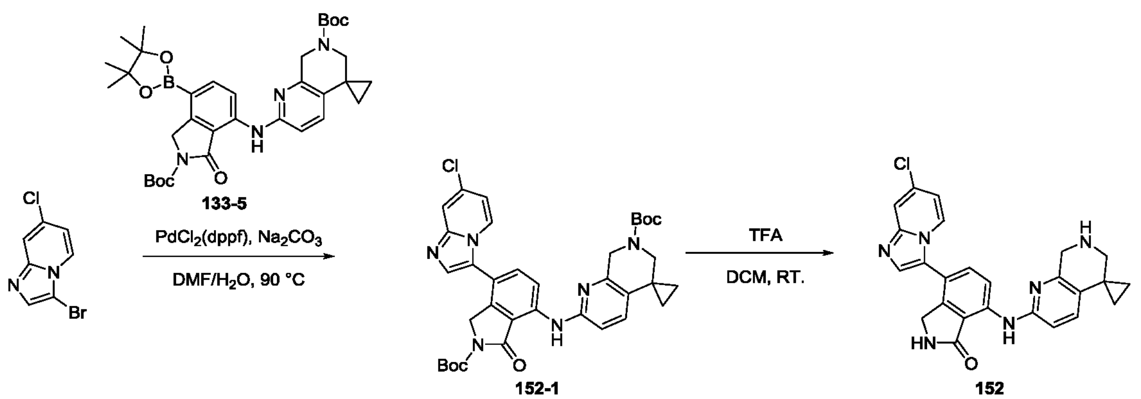

Step 1: Synthesis of Intermediate 152-1

At room temperature, compound 3-bromo-7-chloroimidazo[1,2-A]pyridine (18.10 mg), intermediate 133-5 (45.00 mg), $[PdCl_2(dppf)]CH_2Cl_2$ (5.80 mg), and sodium carbonate (22.61 mg) were added to DMF (2.00 mL) and water (0.40 mL), and then reacted at 90°C for 0.5 h under $N_2$ protection conditions to stop the reaction. The reaction solution was concentrated to obtain a crude product, which was separated by column chromatography (DCM:MeOH=95:5-85:15) to obtain the target product 152-1 (40.00 mg, yield 85.60%) as a white solid. ESI-MS m/z:657.22[M+H]+.

Step 2: Synthesis of compound 152

At room temperature, compound 152-1 (40.00 mg) was added to dichloromethane (4.00 mL), and then trifluoroacetic acid (1.00 mL) was added in an ice-water bath under $N_2$ protection conditions. The reaction was allowed to react at room temperature for 1.5 h, and the reaction was stopped. The reaction solvent was removed by rotary evaporation, and the target product 152 (31.60 mg, purity 99.57%, yield 90.49%) was obtained as a white solid. ESI-MS m/z:457.23[M+H]+. Trifluoroacetate 1H NMR (500 MHz, DMSO) δ 10.18 (s, 1H), 9.45 (s, 2H), 8.91 (s, 1H), 8.72 (d, J = 8.5 Hz, 1H), 8.48 (d, J = 7.4 Hz, 1H), 8.05 (s, 1H), 7.99 (s, 1H), 7.76 (d, J = 8.5 Hz, 1H), 7.27 (d, J = 8.7 Hz, 1H), 7.15 (d, J = 7.3 Hz, 1H), 6.97 (d, J = 8.6 Hz, 1H), 4.41 (s, 4H), 1.10 (s, 4H).

Example 157: Synthesis of Compound 3-(7-((7-(dimethylamino)-2',3',5',6,6',7-hexahydrospiro[cyclopentane[b]pyridine-5,4'-pyran]-2-yl)amino)-1-oxoisoindol-4-yl)imidazo[1,2-a]pyridine-7-carbonitrile

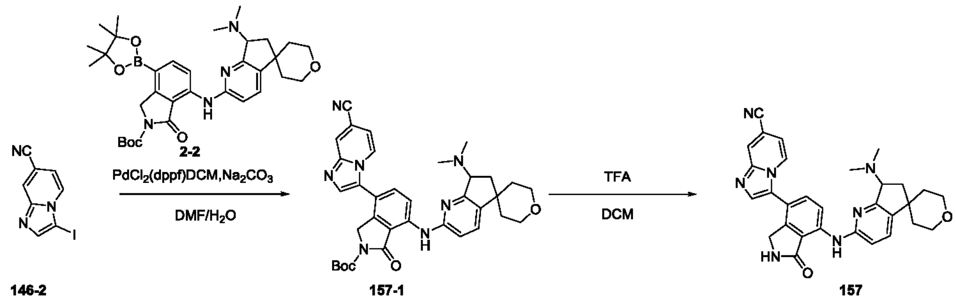


146-2 157-1 157

Step 1: Synthesis of Intermediate 157-1

At room temperature, compound 146-2 (48.94 mg), intermediate 2-2 (100.00 mg), $[PdCl_2(dppf)]CH_2Cl_2$ (13.50 mg), and sodium carbonate (52.60 mg) were added to DMF (4.00 mL) and water (0.50 mL), and then reacted at 90°C for 0.5 h under $N_2$ protection conditions to stop the reaction. The reaction solution was concentrated to obtain a crude product, which was separated by column chromatography (DCM:MeOH=95:5-85:15) to obtain the target product 157-1 (90.00 mg, yield 87.80%). ESI-MS m/z:620.71[M+H]+.

Step 2: Synthesis of compound 157

At room temperature, compound 157-1 (90.00 mg) was added to dichloromethane (4.00 mL), and then trifluoroacetic acid (1.00 mL) was added in an ice-water bath under $N_2$ protection conditions. The reaction was allowed to react at room temperature for 1.5 h, and the reaction was stopped. The reaction solvent was removed by rotary evaporation, 10 mL of $H_2O$ was added to the crude product, and saturated sodium bicarbonate was added to adjust the pH to 8-9. A light yellow solid was precipitated, which was then filtered. 10 mL of methanol was added to the filter cake for pulping, and the target product 157 (46.80 mg, purity 99.28%, yield 61.58%) was obtained by filtration and drying. ESI-MS m/z:520.23[M+H]+. $^1H$ NMR(500MHz,DMSO-d 6 )δ10.17(s,1H),8.87(s,1H),8.77(d,J = 8.5Hz,1H),8.56(d,J = 7.1Hz,1H), 8.45(s,1H), 8.16(s,1H), 7.77(d,J = 8.6Hz,1H),7.64(d,J = 8.4Hz,1H),7.20(dd,J = 7.2,1.8Hz, 1H),6.86(d, J = 8.3Hz,1H), 4.42(s,2H), 4.22(t,J = 7.8Hz,1H),3.87-3.80(m,2H),3.61-3.52(m,2H),2.35(s,6H),2.34- 2.27(m,1H), 2.01-1.96(m,2H),1.89-1.84(m,1H),1.73-1.64(m,1H),1.46-1.42(m,1H),1.35-1.32m,1H) .

Example 158: Synthesis of Compound 4-(7-(difluoromethoxy)imidazo[1,2-a]pyridin-3-yl)-7-((7-(methylamino)-2',3',5',6,6',7-hexahydr ospiro[cyclopenta[b]pyridine-5,4'-pyran]-2-yl)amino)isoindol-1-one

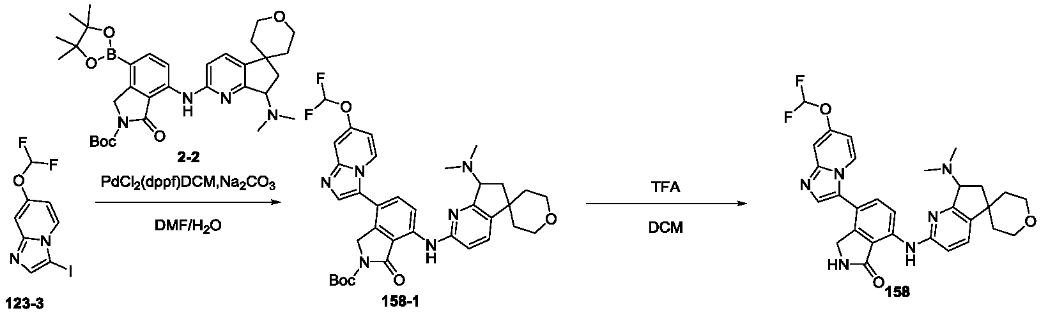


Step 1: Synthesis of Intermediate 158-1

At room temperature, compound 123-3 (56.40 mg), intermediate 2-2 (100.00 mg), $[PdCl_2(dppf)]CH_2Cl_2$ (13.47 mg), and sodium carbonate (52.59 mg) were added to DMF (4.00 mL) and water (0.50 mL), and then reacted at 80°C for 3 h under $N_2$ protection conditions to stop the reaction. The reaction solution was concentrated to obtain a crude product, which was separated by column chromatography (DCM:MeOH=95:5-85:15) to obtain the target product 158-1 (80.00 mg, yield 73.21%). ESI-MS m/z:661.75 [M+H]+.

Step 2: Synthesis of compound 158

At room temperature, compound 158-1 (80.00 mg) was added to dichloromethane (4.00 mL), and then trifluoroacetic acid (1.00 mL) was added in an ice-water bath under $N_2$ protection conditions. The reaction was allowed to react at room temperature for 1 h, and the reaction was stopped. The reaction solvent was removed by rotary evaporation, and the target product 158 (43.70 mg, purity 99.59%, yield 64.11%) was obtained by preparation and isolation. ESI-MS m/z:561.56 [M+H]+. Trifluoroacetate 1H NMR (500MHz, DMSO) δ10.34 (d, J=19.9Hz, 2H), 8.98 (s, 1H), 8.68 (d, J=8.6Hz, 1H), 8.60 (d, J=7.6Hz, 1H), 8.28 (s, 1H), 7.84 (d, J=8.5Hz, 1H), 7.81 (s, 1H), 7.79-7.72 (m, 2H), 7.66 (s, 1H), 7.52 (s, 1H), 7.27 (d, J=5.6Hz, 1H), 7.13 (d, J= 8.5Hz,1H),5.16-5.11(m,1H),4.42(s,2H),3.93-3.85(m,2H),3.63-3.51(m,2H),2.99(d,J = 4.7Hz,3H), 2.81(d,J = 4.8Hz,3H), 2.77-2.71(m,1H), 2.17-2.06(m,2H), 1.73-1.64(m,1H), 1.52(d,J=12.1Hz,1H), 1.41(d,J=11.9Hz,1H).

Chiral separation of Example 158:

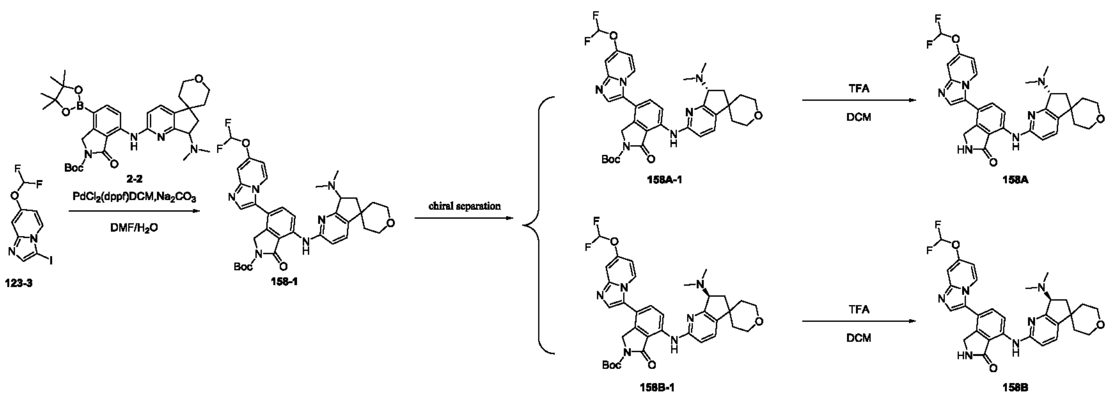


The racemic mixture of compound 158-1 (2800 mg) was dissolved in a mixed solution of DCM (22 ml) and ethanol (10 ml), and passed through a preparative chiral preparative column [CHIRAL ART Cellulose IC (YMC) 30*250mm 5um, model: GX-271 (GILSON); mobile phase A: n-hexane (AR, Shuanglin Chemical, plus 0.1% diethylamine), mobile phase B: ethanol (AR, Shuanglin Chemical, plus 0.1% diethylamine); isocratic (65% mobile phase B); flow rate: 40 ml/min,

separation at room temperature. Compound 158A-1 (peak 1): retention time = 21.00 min, recovered amount 1200 mg; Compound 158B-1 (peak 2): retention time = 28.70 min, recovered amount 1020 mg.

Step 2: Synthesis of compound 158A

At room temperature, compound 158A-1 (1200.00 mg) was added to dichloromethane (40.00 mL), and then trifluoroacetic acid (5.00 mL) was added in an ice-water bath under $N_2$ protection conditions. The reaction was allowed to react at room temperature for 0.5 h, and then the reaction was stopped. The reaction solvent was removed by rotary evaporation, and the concentrate was dissolved with 5 ml of DCM. Saturated sodium bicarbonate was then slowly added to adjust the pH to 8-9, and a light yellow solid was precipitated. Ammonia water was added to adjust the pH to 11-12, the aqueous phase was decanted, and 40 ml of water was added to slurry, and the mixture was allowed to stand and the aqueous phase was decanted. 20 ml of methanol was added to the remaining solid to slurry, and the solid was filtered. The solid was washed with methanol and dried to obtain the target product 158A (872.2 mg, purity 99.24%, yield 85.01%). ESI-MS m/z:561.56[M+H]+.

Step 3: Synthesis of compound 158B

At room temperature, compound 158B-1 (1040.00 mg) was added to dichloromethane (40.00 mL), and then trifluoroacetic acid (5.00 mL) was added in an ice-water bath under $N_2$ protection conditions. The reaction was allowed to react at room temperature for 0.5 h, and then the reaction was stopped. The reaction solvent was removed by rotary evaporation, and the concentrate was dissolved with 5 ml of DCM. Saturated sodium bicarbonate was then slowly added to adjust the pH to 8-9, and a light yellow solid was precipitated. Ammonia water was added to adjust the pH to 11-12, the aqueous phase was poured out, and 40 ml of water was added to slurry, and the mixture was allowed to stand and the aqueous phase was poured out. 20 ml of methanol was added to the remaining solid to slurry, and the solid was filtered, washed with methanol, and dried to obtain the target product 158B (797.50 mg, purity 99.12%, yield 89.69%). ESI-MS m/z: 561.56 [M+H]+.

Example 158A: Synthesis of Compound (R)-4-(7-(difluoromethoxy)imidazo[1,2-a]pyridin-3-yl)-7-((7-dimethylamino)-2',3',5',6,6',7-hex ahydrospiro[cyclopentadienyl[b]pyridine-54'-pyran]-2-yl)amino)isoindolin-1-one

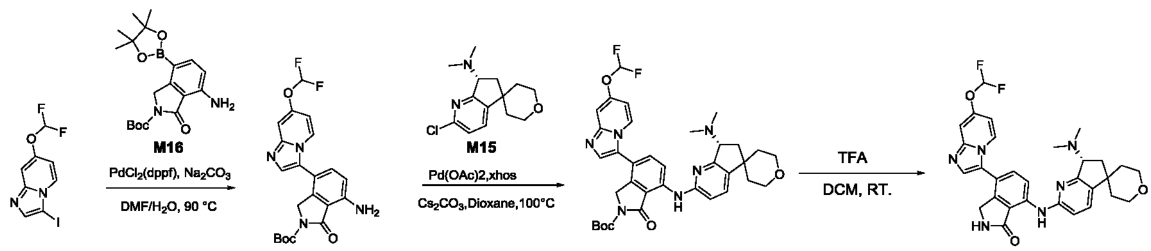


123-3 158A-1 158A-2 158A

Step 1: Synthesis of compound 158A-1

Compound 123-3 (6.74 g), 158A-1 (7.40 g), $[PdCl_2(dppf)]CH_2Cl_2$ (1.61 g), and potassium carbonate (8.2 g) were dissolved in 1,4-Dioxane (80 mL) and water (20 mL), nitrogen was replaced, the temperature was raised to 100°C, and the reaction was carried out for 4 hours. The mixture was cooled, filtered, washed with DCM, and the filtrate was concentrated under reduced pressure. The mixture was purified by column chromatography (DCM:EA=95:5-50:50) to obtain compound 158A-1 (5.75 g, 67% yield). ESI-MS m/z:431.2[M+H]+.

Step 2: Synthesis of compound 158A-2

Compound 158A-1 (3.70 g), M15 (2.41 g), palladium acetate (97.00 mg), XantPhos (497 mg) and cesium carbonate (7.00 g) were dissolved in 1,4-Dioxane (60 mL), replaced with nitrogen, heated to 90° C, and reacted for 4 hours. The mixture was cooled, filtered and washed with EA. The filtrate was concentrated under reduced pressure and purified by column chromatography (DCM:MeOH=93:7-90:10) to obtain compound 158A-2 (5.05 g, 88% yield). ESI-MS m/z:661.3 [M+H]+.

Step 3: Synthesis of Compound 158A

Compound 158A-2 (4.30 g) was dissolved in DCM (60 mL), and trifluoroacetic acid (10.00 ml) was added dropwise at room temperature under $N_2$ protection, and the reaction was continued at room temperature for 1 hour. The reaction solution was concentrated to dryness, and the crude product was dissolved in DCM (8 ml). Saturated sodium bicarbonate was then slowly added to adjust the pH to 8-9, and a light yellow solid was precipitated. Ammonia water was added to adjust the pH to 11-12, the aqueous phase was decanted, and 50 ml of water was added to slurry, allowed to stand, and the aqueous phase was decanted. To the remaining solid, 100 ml of methanol was added for slurrying, and the solid was filtered. The solid was washed with methanol (5 ml/time) 3 times and dried to obtain the target product 158A (3.158 g, purity 99.20%, yield 85.88%, retention time the same as 158A in Example 158). ESI-MS m/z:561.56[M+H]+. $^{1}$H NMR(500MHz,DMSO-d 6 )δ10.13(s,1H),8.83(s,1H),8.74(d,J = 8.5Hz,1H),8.43(d,J=7.5Hz,1H),7 .83(s,1H),7.71(d,J= 8.SHz,1H),7.63(d,J= 8.3Hz,1H),7.43(t,J =73.5Hz,1H),7.41(d,J= 2.5Hz,1H),6.88 -6.81(m,2H), 4.39(s,2H),4.22(t,J=7.8Hz,1H),3.92-3.77(m,2H),3.61-3.51(m,2H), 2.35(s,6H ), 2.33-2.29 (m,1H), 2.03-1.98(m,1H),1.89-1.84(m,1H),1.70-1.65(m,1H), 1.46-1.42(m,1H),1.36-1.31(m,1H ).

Example 160: Synthesis of Compound 4-(7-(difluoromethyl)imidazo[1,2-a]pyridin-3-yl)-7-((7-(methylamino)-2',3',5',6,6',7-hexahydro spiro[cyclopentane[b]pyridine-5,4'-pyran]-2-yl)amino)isoindol-1-one

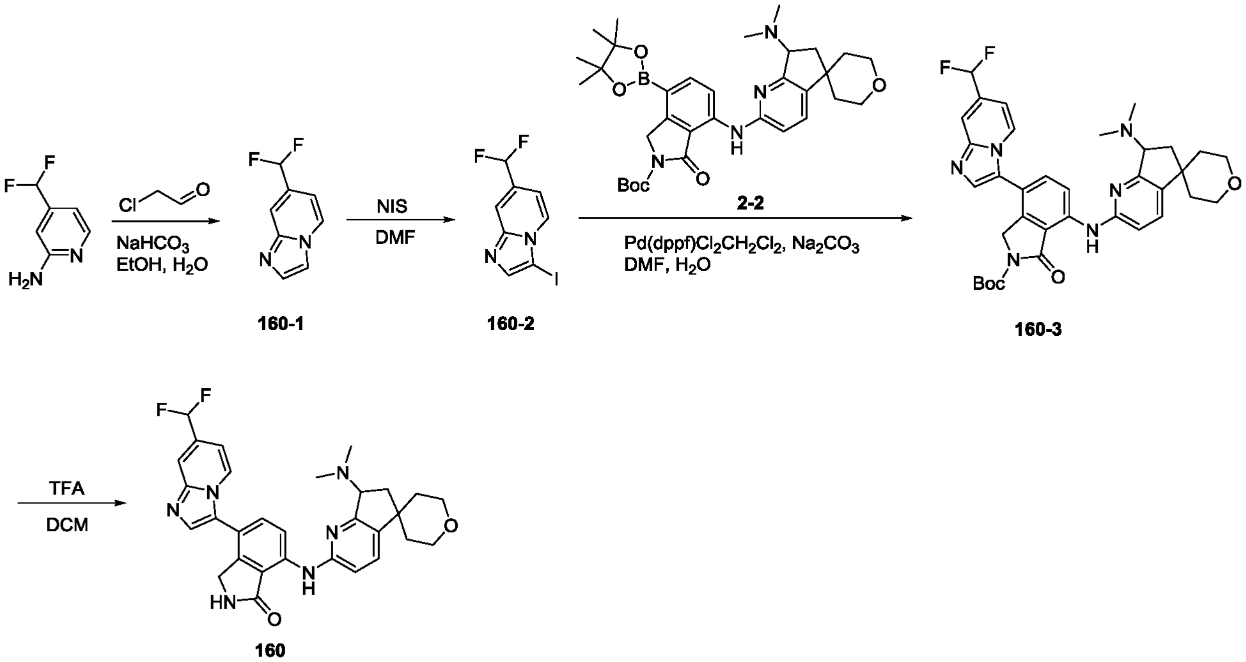


Step 1: Synthesis of compound 160-1

4-(Difluoromethyl)pyridin-2-amine (100.00 mg), 40% aqueous chloroacetaldehyde solution (272.00 mg), and sodium bicarbonate (116.00 mg) were dissolved in ethanol (5.00 mL) and water (1.00 mL), and the mixture was reacted at 70°C. for 2 h. After the reaction was completed, the mixture was concentrated, mixed, and separated by silica gel column chromatography (DCM:MeOH=24:1) to obtain the target product 160-1 (110.00 mg, yield 94.28%). ESI-MS m/z:169.03 [M+H]+.

Step 2: Synthesis of compound 160-2

160-1 (110.00 mg) was dissolved in DMF (5.00 mL), and NIS (162.00 mg) was added under ice-cooling, and the mixture was reacted at room temperature for 2 h. After the reaction, EA/THF and water were added for extraction and separation. The organic phase was washed with saturated brine, concentrated, mixed, and separated by silica gel column chromatography (DCM:MeOH=25:1) to obtain the target product 160-2 (150.00 mg, yield 77.98%). ESI-MS m/z:295.05 [M+H]+.

Step 3: Synthesis of compound 160-3

160-2 (58.00 mg), 2-2 (100.00 mg), [$PdCl_2$(dppf)]$CH_2Cl_2$ (13.00 mg), and sodium carbonate (52.00 mg) were dissolved in DMF (2.00 mL) and water (0.30 mL) and reacted at 90°C for 1 h. After the reaction was completed, the mixture was concentrated, mixed, and separated by silica gel column chromatography (DCM:MeOH=19:1) to obtain the target product 160-3 (49.00 mg, yield 45.95%). ESI-MS m/z:645.45[M+H]+.

Step 4: Synthesis of compound 160

At room temperature, compound 160-3 (49.00 mg) was dissolved in dichloromethane (4.00 mL), TFA (1.00 mL) was added, and the mixture was reacted at room temperature for 0.5 h, and the reaction was stopped. The product was concentrated and purified by RP-HPLC to give the trifluoroacetate salt 160 (32.60 mg, purity 99.9%, yield 78.69%). ESI-MS m/z:545.20[M+H]+.

$^1$H NMR(500MHz,DMSO-$d_6$)δ10.36(s,1H),10.32(s,1H),8.96(s,1H),8.68(d,J = 8.4Hz,1H), 8.58(d,J=7.2Hz,1H), 8.22(s,1H),8.11(s,1H),7.84(d,J=8.5Hz,1H),7.77(d,J=8.5Hz,1H), 7.36-7.09 (m,3H), 5.16-5.12(m,1H),4.43(s,2H),3.92-3.86(m,2H),3.70-3.46(m,2H), 2.99(d,J = 4.6Hz,3H), 2.82(d, J=4 .7Hz,3H),2.76-2.72(m,1H),2.23-2.03(m,2H), 1.73-1.66(m,1H), 1.53-1.51(m,1H),1.43-1.40(m,1H) .

Example 163: Synthesis of Compound 7-((7-(dimethylamino)-2',3',5',6,6',7-hexahydrospiro[cyclopentane[b]pyridine-5,4'-pyran]-2-yl)a mino)-4-(7-isopropylimidazo[1,2-a]pyridin-3-yl)isoindol-1-one

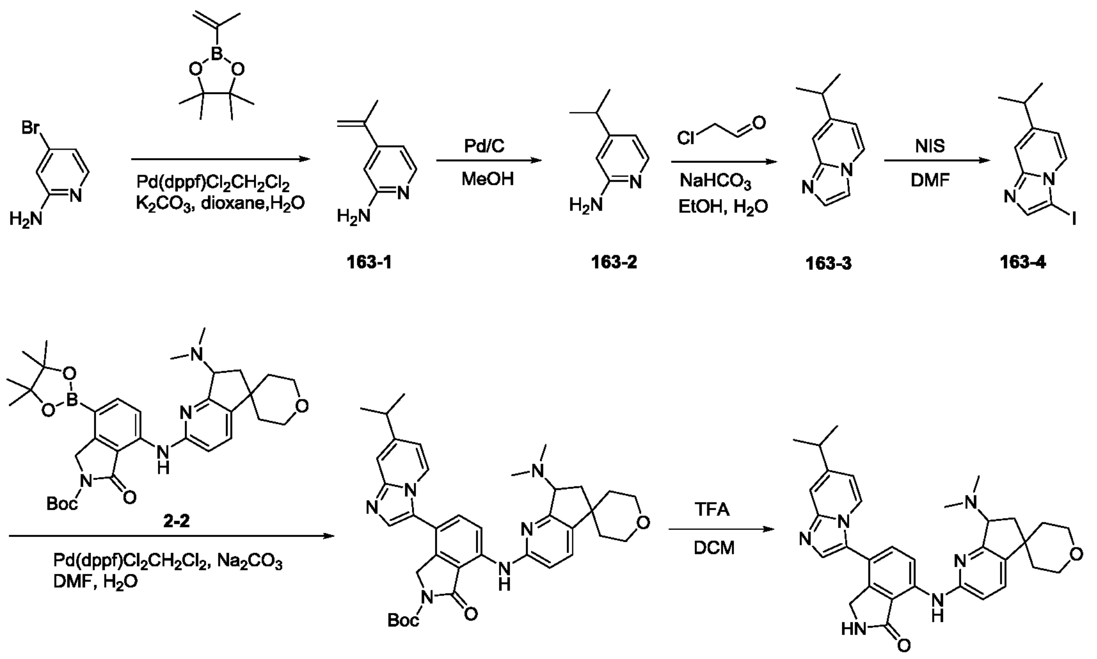


Step 1: Synthesis of compound 163-1

4-Bromopyridin-2-amine (1.00 g), 4,4,5,5-tetramethyl-2-(propyl-1-en-2-yl)-1,3,2-dioxaborolane (1.17 g), [$PdCl_2$(dppf)]$CH_2Cl_2$ (470.00 mg), and potassium carbonate (2.40 g) were dissolved in 1,4-dioxane (10.00 mL) and water (2.00 mL), and reacted at 90°C for 4 h. After the reaction was completed, the mixture was filtered, washed with dichloromethane, concentrated, mixed, and separated by silica gel column chromatography (DCM:MeOH=30:1) to obtain the target product 163-1 (510.00 mg, yield 65.76%). ESI-MS m/z:135.11[M+H]+.

Step 2: Synthesis of compound 163-2

163-1 (510.00 mg) and Pd/C (161.00 mg) were dissolved in methanol (10.00 mL), replaced with $H_2$, and reacted at room temperature overnight. After the reaction was completed, the reaction mixture was filtered, the filtrate was mixed,

and the target product 163-2 (252.00 mg, yield 48.68%) was separated by silica gel column chromatography (DCM:MeOH=15:1). ESI-MS m/z:137.10[M+H]+.

Step 3: Synthesis of compound 163-3

2-Chloroacetaldehyde (252.00 mg), 40% chloroacetaldehyde aqueous solution (726.00 mg), and sodium bicarbonate (310.00 mg) were dissolved in ethanol (5.00 mL) and water (1.00 mL), and reacted at 70° C for 2 h. After the reaction was completed, the mixture was concentrated, mixed, and separated by silica gel column chromatography (DCM:MeOH=19:1) to obtain the target product 163-3 (290.00 mg, yield 97.83%). ESI-MS m/z:161.19[M+H]+.

Step 4: Synthesis of compound 163-4

163-3 (290.00 mg) was dissolved in DMF (5.00 mL), and NIS (448.00 mg) was added under ice-cooling, and the mixture was reacted at room temperature for 2 h. After the reaction, EA/THF and water were added for extraction and separation. The organic phase was washed with saturated brine, concentrated, mixed, and separated by silica gel column chromatography (DCM:MeOH=15:1) to obtain the target product 163-4 (298.00 mg, yield 57.54%). ESI-MS m/z:287.07 [M+H]+.

Step 5: Synthesis of Compound 163-5

163-4 (56.00 mg), 2-2 (100.00 mg), [PdCl$_2$(dppf)]CH$_2$Cl$_2$ (13.00 mg), and sodium carbonate (52.00 mg) were dissolved in DMF (2.00 mL) and water (0.30 mL) and reacted at 90°C for 1 h. After the reaction was completed, the mixture was concentrated, mixed, and separated by silica gel column chromatography (DCM:MeOH=15:1) to obtain the target product 163-5 (65.00 mg, yield 61.71%). ESI-MS m/z:637.30[M+H]+.

Step 6: Synthesis of compound 163

At room temperature, compound 163-5 (65.00 mg) was dissolved in dichloromethane (4.00 mL), TFA (1.00 mL) was added, and the mixture was reacted at room temperature for 0.5 h, and the reaction was stopped. The reaction mixture was concentrated, the pH was adjusted to alkaline with saturated sodium bicarbonate solution, and the mixture was extracted three times with DCM. The organic phases were combined, washed with saturated brine, dried over anhydrous sodium sulfate, concentrated, and purified on a preparative plate (DCM: MeOH = 18:1) to obtain the product 163 (11.50 mg, purity 98.73%, yield 20.72%). ESI-MS m/z:537.20[M+H]+. 1H NMR(500MHz,DMSO-d 6 ) δ10.43(s,1H),10.32(s,1H),8.97(s,1H),8.68(d,J = 8.5Hz,1H),8.54(d,J = 7.1 Hz,1H),8 .38(s,1H),7.89-7.83(m,2H),7.79(d,J = 8.5Hz,1H),7.45(dd,J = 7.2,1.7Hz,1H),7.14(d,J = 8.5 Hz,1H),5.15-5.12(m,1H) ,4.42(s,2H),3.92-3.88(m,2H),3.66-3.49(m,2H),3.24-3.17(m,1H),2.98(d,J = 4.6Hz,3H),2.82(d ,J = 4.7Hz,3H),2.75( dd,J = 13.7,8.5Hz,1H),2.16-2.11(m,2H),1.72-1.67(m,1H),1.54-1.51(m,1H),1.42-1.40(m,1H),1.31(d,J=6.8Hz,6H).

Example 164: Synthesis of Compound 4-(7-cyclopropylimidazo[1,2-a]pyridin-3-yl)-7-((7-(dimethylamino)-2',3',5',6,6',7-hexahydrospi ro[cyclopentane[b]pyridine-5,4'-pyran]-2-yl)amino)isoindol-1-one

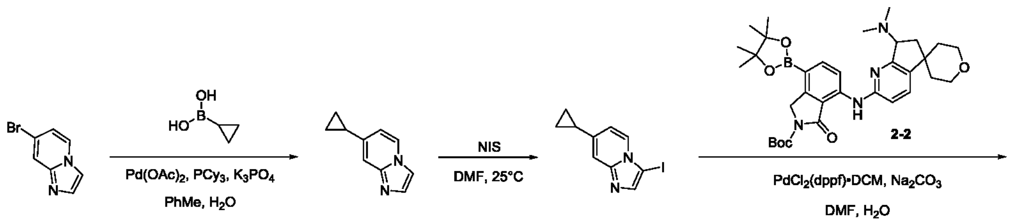


164-1 164-2

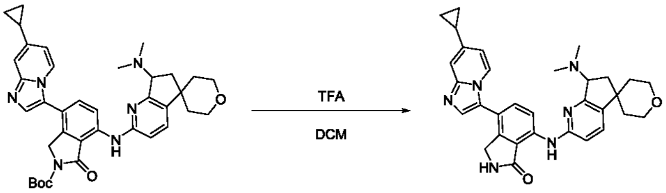


164-3 164

Step 1: Synthesis of compound 164-1

At room temperature, 7-bromoimidazo[1,2-a]pyridine (1.00 g), cyclopropylboronic acid (566.70 mg), palladium acetate (113.90 mg), tricyclohexylphosphine (284.70 mg) and potassium phosphate (3.23 g) were dissolved in toluene (10 mL) and water (1 mL), the temperature was raised to 100° C., and the reaction was carried out for 15 hours. The reaction solution was cooled, filtered through celite, and the filtrate was concentrated under reduced pressure and purified by column chromatography (DCM:MeOH=97:3) to obtain compound 164-1 (707.00 mg, 88% yield). ESI-MS m/z:159.2[M+H]+.

Step 2: Synthesis of compound 164-2

At room temperature, 164-1 (707.00 mg) was dissolved in DMF (8 mL), and NIS (1.21 g) was added. The mixture was reacted at room temperature for 3 hours, diluted with water, extracted with DCM, and the organic layers were combined, washed with saturated brine, dried over anhydrous sodium sulfate, concentrated under reduced pressure, and purified by column chromatography (DCM: MeOH = 97: 3) to give compound 164-2 (1.15 g, 91% yield). ESI-MS m/z:285.0 [M+H]+.

Step 3: Synthesis of compound 164-3

At room temperature, 2-2 (100.00 mg), 164-2 (56.39 mg), $[PdCl_2(dppf)]CH_2Cl_2$ (13.47 mg) and potassium carbonate (68.58 mg) were dissolved in DMF (4 mL) and water (0.5 mL), nitrogen was replaced, the temperature was raised to 90°C, and the reaction was carried out for 2 hours. The mixture was cooled and filtered through celite. The filtrate was diluted with water and extracted with EA. The organic layers were combined, washed with saturated brine, dried over anhydrous sodium sulfate, concentrated under reduced pressure, and purified by column chromatography (DCM:MeOH=95:5) to give compound 164-3 (86.00 mg, 82% yield). ESI-MS m/z:635.4[M+H]+.

Step 4: Synthesis of compound 164

At room temperature, 164-3 (86.00 mg) was dissolved in DCM (4 mL), trifluoroacetic acid (1 mL) was added, and the mixture was reacted at room temperature for 1 hour. The reaction solution was concentrated, dissolved in a small amount of methanol, adjusted to alkaline pH with saturated sodium bicarbonate, extracted with DCM, the organic layers were combined, washed with saturated brine, dried, concentrated under reduced pressure, and purified by column chromatography (DCM:MeOH=92:8) to give compound 164 (16.20 mg, 21% yield). ESI-MS m/z:535.3[M+H]+. 1H NMR(500MHz,DMSO-d 6 )δ10.12(s,1H),8.84(s,1H),8.73(d,J = 8.6Hz,1H),8.26(d,J = 7.2Hz,1H),7.7 4(s,1H),7.69(d,J = 8.6Hz,1H),7.62(d,J = 8.3Hz,1H), 7.37(s,1H), 6.85(d,J = 8.3Hz,1H), 6.65(dd,J=7.3,1. 9Hz,1H),4.39(s,2H),4.23(s,1H),3.83(t,J = 13.2Hz,2H),3.56(dt,J=24.2,12.0Hz,2H),2.36(s,6H ),2.32( m,1H),2.07-1.97(m,2H), 1.87(m,1H), 1.66(m,1H),1.44(m,1H),1.36-1.31(m,1H),1.02(m,2H) ,0.80(m,2H).

Example 165: Synthesis of Compound 4-(5,6-dihydro-8H-imidazo[2,1-c][1,4]oxazin-3-yl)-7-((7-(dimethylamino)-2',3',5',6,6',7-hexahy drospiro[cyclopenta[b]pyridine-5,4'-pyran]-2-yl)amino)isoindol-1-one

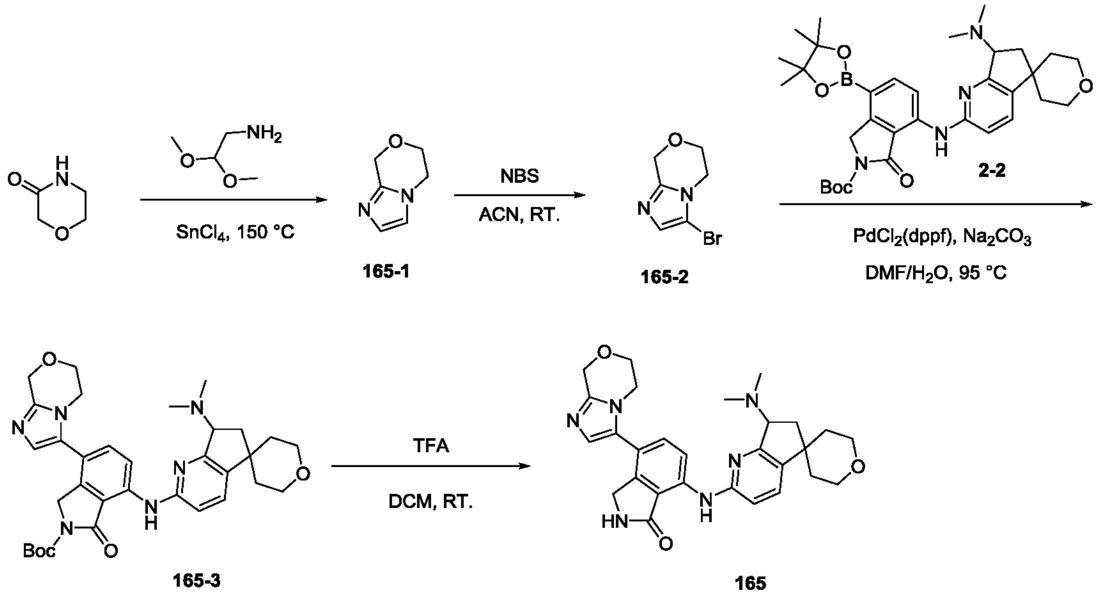


Step 1: Synthesis of compound 165-1

At room temperature, 3-morpholinone (1.00 g) and aminoacetaldehyde dimethyl acetal (2.08 g) were added to the reaction bottle, and then $SnCl_4$ (0.35 mL) was slowly added dropwise, and then under $N_2$ protection conditions, the reaction was carried out at 150°C for 3 hours to stop the reaction. 50 mL of water was added to the reaction solution to quench the reaction, and then filtered through celite. The filter cake was washed with water, and then the pH was adjusted to 8-9 with a saturated sodium bicarbonate solution. The mixture was extracted with EA, and the organic phases were combined, washed with saturated brine, dried over anhydrous sodium sulfate, and concentrated. The concentrate was separated by silica gel column chromatography (DCM: MeOH = 9:1) to obtain the target product 165-1 (840.00 mg, yield 68.41%). ESI-MS m/z:125.2[M+H]+.

Step 2: Synthesis of compound 165-2

At room temperature, 165-1 (840.00 mg) was dissolved in ACN (15.00 mL), and NBS (1.20 g) was added in portions under ice-water bath conditions. The mixture was reacted at room temperature for 1 h, and the reaction was stopped. The reaction solution was poured into 50 mL of water and extracted with EA. The organic phases were combined, washed with saturated brine, dried over anhydrous sodium sulfate, and concentrated. The concentrate was separated by silica gel column chromatography (PE:EA=20:80) to obtain the target product 165-2 (840.00 mg, yield 61.14%). ESI-MS m/z:203.2[M+H]+.

Step 3: Synthesis of compound 165-3

At room temperature, compound 2-2 (80.00 mg), 165-2 (31.00 mg), $[PdCl_2(dppf)]CH_2Cl_2$ (11.00 mg), and sodium carbonate (42.00 mg) were added to DMF (4.00 mL) and water (0.40 mL), and then reacted at 95°C for 6 hours under $N_2$ protection conditions, and the reaction was stopped.

The reaction solution was directly concentrated. The concentrate was separated by silica gel column chromatography (DCM:MeOH=10:1) to obtain the target product 165-3 (60.00 mg, yield 75.47%). ESI-MS m/z:601.2[M+H]+.

Step 4: Synthesis of compound 165

At room temperature, compound 165-3 (60.00 mg) was dissolved in dichloromethane (5.00 mL), TFA (396.22 mg) was added, and the mixture was reacted at room temperature for 0.5 h, and the reaction was stopped. The reaction solution was directly concentrated. The concentrate was separated by preparative liquid phase to obtain the target product

165 (31.50 mg, yield 63.00%). ESI-MS m/z:501.2[M+H]+. 1H NMR(500MHz,DMSO-d 6 ) δ10.28(s,1H),8.98(s,1H),8.60(d,J=8.5Hz,1H),7.91(s,1H),7.83(d, J=8.5Hz,1H),7.69(d,J= 8.5Hz,1H),7.10(d,J = 8.4Hz,1H),5.11(s,3H),4.46(s,2H),4.10(s, 4H),3.95-3 .82(m,2H),3.57(ddd,J = 32.3,12.8, 10.6Hz,2H), 2.98(d,J = 4.4Hz,3H),2.81(d,J = 4.5Hz,3H),2.74( dd,J =13.8,8.6Hz,1H), 2.22-2.05(m,2H), 1.69(td,J=12.8,4.7Hz,1H),1.56-1.47(m,1H),1.44-1.35(m,1H).

Example 166: Synthesis of Compound 7-((7-(dimethylamino)-2',3',5',6,6',7-hexahydrospiro[cyclopentane[b]pyridine-5,4'-pyran]-2-yl)a mino)-4-(5,6,7,8-tetrahydroimidazo[1,2-a]pyridin-3-yl)isoindol-1-one

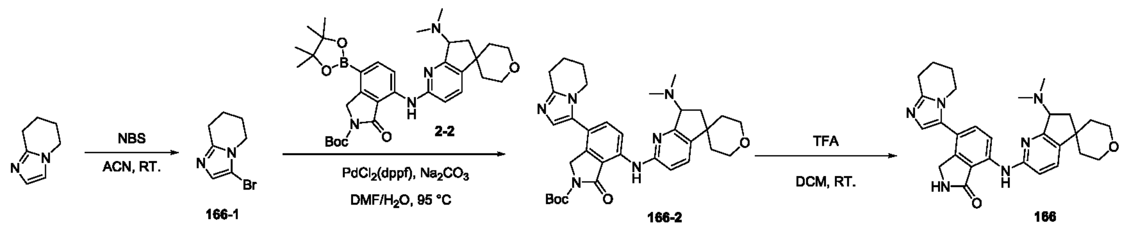


Step 1: Synthesis of compound 166-1

At room temperature, 5,6,7,8-tetrahydro-imidazole [1,2-A] pyridine (300.00 mg) was dissolved in ACN (8.00 mL), and then NBS (480.76 mg) was added in batches. The mixture was reacted at room temperature for 1 h, and the reaction was stopped. The reaction solution was directly concentrated. The concentrate was separated by silica gel column chromatography (DCM:MeOH=19:1) to obtain the target product 166-1 (200.00 mg, yield 40.51%). ESI-MS m/z:201.2 [M+H]+.

Step 2: Synthesis of compound 166-2

At room temperature, compound 2-2 (80.00 mg), 166-1 (32.18 mg), [PdCl$_2$(dppf)]CH$_2$Cl$_2$ (11.00 mg), and sodium carbonate (42.00 mg) were added to DMF (4.00 mL) and water (0.40 mL), and then reacted at 95°C for 6 hours under N$_2$ protection conditions to stop the reaction. The reaction solution was directly concentrated. The concentrate was separated by silica gel column chromatography (DCM:MeOH=10:1) to obtain the target product 166-2 (60.00 mg, yield 75.59%).

ESI-MS m/z:599.2[M+H]+.

Step 3: Synthesis of compound 166

At room temperature, compound 166-2 (60.00 mg) was dissolved in dichloromethane (5.00 mL), TFA (396.22 mg) was added, and the mixture was reacted at room temperature for 0.5 h, and the reaction was stopped. The reaction solution was directly concentrated. The concentrate was separated by preparative liquid phase to obtain the target product 166 (22.40 mg, yield 44.80%).

ESI-MS m/z:499.2[M+H]+. 1H NMR(500MHz,DMSO-d 6 )δ10.26(s,1H),8.96(s,1H),8.58(d,J= 8.5Hz,1H),7.87-7.80(m,2H),7.63(d,J = 8.6Hz,1H),7.11(d,J = 8.5Hz,1H),5.12(t,J = 7.4Hz,1H),4.42(s,2H),3.96(s,2H),3.89(t,J = 13.2Hz,2H),3.65- 3.50(m,2H),3.07(d,J = 5.4Hz,2H),2.98(d,J=4.5Hz,3H),2.81(d,J=4.7Hz,3H),2.74(dd,J=13.8 ,8.5Hz,1H),2. 12(dt,J= 13.0,6.8Hz,2H),1.95(s,4H),1.69(td,J = 12.9,4.7Hz,1H),1.51(d,J = 12.9Hz,1H),1.43-1.37 (m,1H).

Example 168: Synthesis of Compound 3-(1-oxo-7-((7-(pyrrolidin-1-yl)-2',3',5',6,6',7-hexahydrospiro[cyclopenta[b]pyridine-5,4'-pyran] -2-yl)amino)isoindol-4-yl)imidazo[1,2-a]pyridine-7-carbonitrile

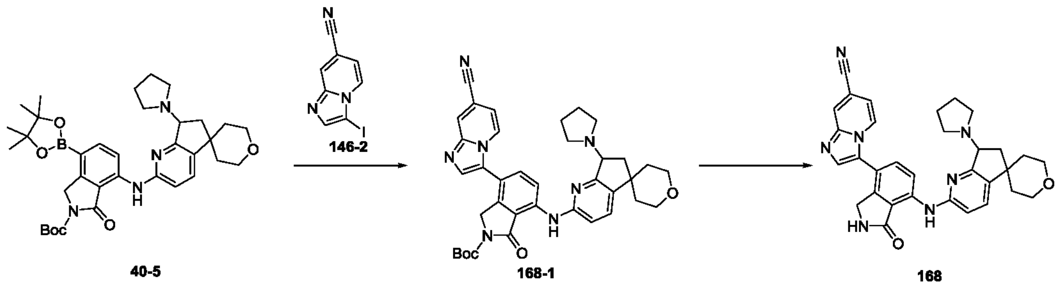


40-5 168-1 168

Step 1: Synthesis of compound 168-1

Compound 40-5 (300.00 mg), 146-2 (128.01 mg), [1,1'-bis(diphenylphosphino) ferrocene]dichloropalladium dichloromethane complex (38.87 mg), sodium carbonate (151.29 mg), and water (1.00 mL) were added to 1,4-dioxane (6.00 mL), nitrogen was replaced, and the reaction was carried out at 90°C for 2 h. The reaction solution was concentrated and separated by column chromatography (DCM:MeOH=90%:10%) to obtain the target product 168-1 (200.00 mg, yield 65.09%). ESI-MS m/z:646.5[M+H]+.

Step 2: Synthesis of compound 168

Compound 168-1 (200.00 mg) was added to DCM (5.00 mL), and TFA (1.00 mL) was added, and the mixture was reacted at room temperature for 0.5 h. The reaction solution was concentrated and the target product 168 (81.30 mg, yield 46.77%) was obtained by preparative isolation. ESI-MS m/z:546.3[M+H]+. Trifluoroacetate $^1$H NMR(500MHz,DMSO) δ10.50(d,J= 5.5Hz,1H),10.26(s,1H),8.94(s,1H),8.58-8 .46(m,3H),8.19(s,1H),7.82(t,J = 8.6Hz,2H),7.27(d,J = 7.1Hz,1H),7.12(d,J = 8.5Hz,1H),5.12(dd,J = 14.5,7.8Hz,1H),4.44(d,J = 12.2Hz,2H),3.92-3.84(m ,2H),3.70-3.32(m,6H),2.91(dd,J = 13.2,8.1Hz,1H),2.10(dtd,J = 21.8,13.2, 6.6Hz,4H), 1.99-1.80(m,2H),1.68(dd,J=12.3,8.6Hz,1H),1.52(d,J=12.7Hz,1H),1.41(d,J=12.1Hz,1H).

Example 169: Synthesis of Compound 4-(7-(difluoromethoxy)imidazo[1,2-a]pyridin-3-yl)-7-((7-(pyrrolidin-1-yl)-2',3',5',6,6',7-hexahy drospiro[cyclopenta[b]pyridine-5,4'-pyran]-2-yl)amino)isoindol-1-one

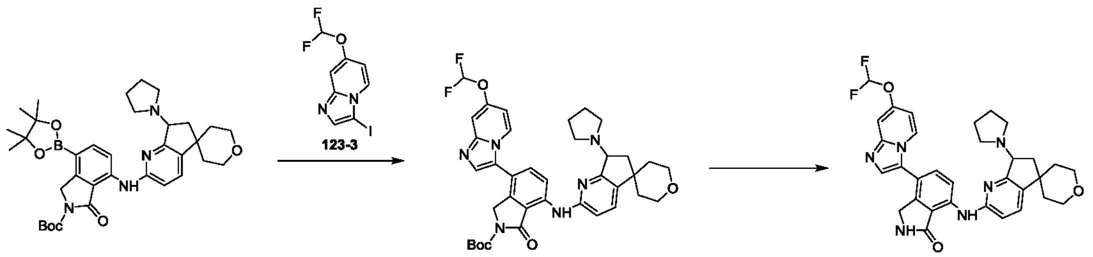


40-5 169-1 169

Step 1: Synthesis of compound 169-1

Compound 40-5 (150 mg), 123-3 (82 mg), [1,1'-bis(diphenylphosphino) ferrocene]dichloropalladium dichloromethane complex (17.4 mg), and aqueous sodium carbonate solution (0.357 mL, 2M) were added to DMF (10 mL), nitrogen was replaced, and the reaction was carried out at 95°C. for 2 h. The reaction solution was concentrated and separated by column chromatography (DCM/MeOH=90/10) to obtain the target product 169-1 (110 mg, yield 67.3%). ESI-MS m/z: 687.3 [M+H]+.

Step 2: Synthesis of compound 169

Compound 169-1 (110 mg) was added to DCM (3 mL) and MeOH (3 mL), and a solution of hydrogen chloride in dioxane (6 mL) was added, and the mixture was reacted at room temperature for 2 h. The reaction solution was concentrated and the target product 169 (17.8 mg, yield 20.8%) was obtained by preparative isolation. ESI-MS m/z:587.4[M+H]+. Hydrochloride $^1$H NMR (500MHz, DMSO) δ11.26 (s, 1H), 10.25 (s, 1H), 8.96 (s, 1H), 8.67-8.54 (m,

2H), 8.31 (s, 1H), 7.82 (dd, J=8.3, 4.4 Hz, 2H), 7.70 (d, J=17.2 Hz, 2H), 7.29 (s, 1H), 7.12 (d, J=8.5 Hz, 1H), 5.10 (d, J=6.6 Hz, 1H), 4.48-4.35 (m, 2H), 4.00-3.80 (m, 3H), 3.69-3.30 (m, 4H), 3.00-2.80 (m, 1H), 2.21-1.20 (m, 10H).

Example 172 Synthesis of Compound 4-(7-(difluoromethoxy)imidazo[1,2-a]pyridin-3-yl)-7-((7-(methylamino)-4',5',6,7-tetrahydro-2'-H-pyrido[cyclopentyl[b]pyridine-5,3'-furan]-2-yl)amino)isoindolin-1-one

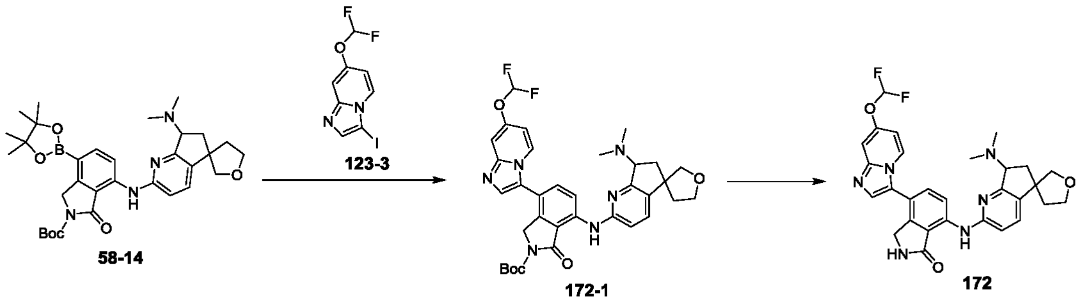


58-14 172-1 172

Step 1: Synthesis of compound 172-1

Compound 58-14 (260 mg), 123-3 (164 mg), [1,1'-bis(diphenylphosphino) ferrocene] dichloropalladium dichloromethane complex (36 mg), and sodium carbonate (140 mg) were added to DMF (10 mL) and water (2 ml), nitrogen was replaced, and the reaction was carried out at 95° C. for 2 h. The reaction solution was concentrated and separated by column chromatography (DCM/MeOH=92/8) to obtain the target product 172-1 (221 mg, yield 77.6%). ESI-MS m/z:647.30[M+H]+.

Step 2: Synthesis of the hydrochloride salt of compound 172

Weigh compound 172-1 (221 mg) and dissolve it in dichloromethane (3 mL) and methanol (3 mL). Slowly add a solution of hydrogen chloride in dioxane (6 mL, 4 M in dioxane) dropwise under ice bath conditions. Slowly warm the temperature to room temperature and react for 30 min to stop the reaction. The reaction solution was concentrated and purified by preparative liquid separation to obtain the hydrochloride salt of the target product 172 (87.50 mg, purity 99.27%, yield 46.85%). ESI-MS m/z:547.38[M+H]+. $^1$H NMR (500MHz, DMSO) δ11.58(s,1H), 10.32(s,1H),8.95(d,J = 41.9Hz,2H),8.68(s,1H),8.46(s,1H),7.94-7.58(m,4H), 7.39(s,1H), 7.11(s,1H),5.13(s,1H),4.42(s,2H),4.15-3.33(m,4H),2.85(d,J = 17.5Hz,6H), 2.45-2.25(m,2H) ,2.20-2.05(m,1H),2.05-1.85(m,1H).

Example 174: Compound 174 Synthesis of 4-(7-aminoimidazo[1,2-a]pyridin-3-yl)-7-((7-(dimethylamino)-2',3',5',6,6',7-hexahydrospiro[cy clopentadienyl[b]pyridine-54'-pyran]-2-yl)amino)isoindol-1-one

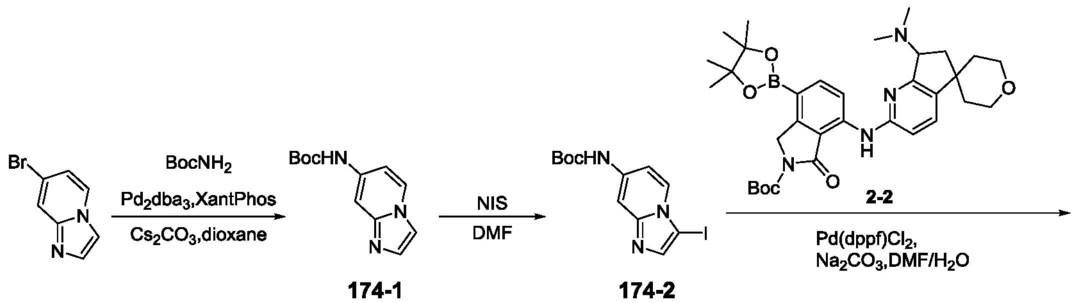


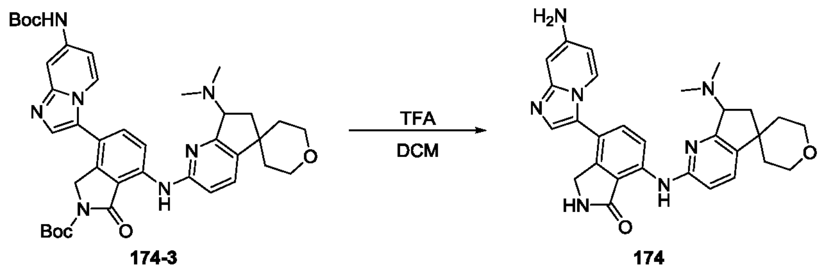


Step 1: Synthesis of compound 174-1

7-Bromoimidazo[1,2-A]pyridine (1.00 g), $BocNH_2$ (890.00 mg), $Pd_2(dba)_3$ (460.00 mg), XantPhos (590.00 mg), and cesium carbonate (4.96 g) were dissolved in 1,4-dioxane (10.00 mL), replaced by $N_2$, and reacted at 100°C for 8 h. After the reaction was completed, the reaction solution was filtered through celite, and the filtrate was concentrated and separated by silica gel column chromatography (DCM:MeOH=32:1) to obtain the target product 174-1 (980.00 mg, yield 82.78%).

Step 2: Synthesis of compound 174-2

174-1 (960.00 mg) was dissolved in DMF (10.00 mL), and NIS (1.11 g) was added under ice-cooling, and the mixture was reacted at room temperature for 5 h. After the reaction was completed, the reaction system was poured into ice water, and the product precipitated. It was filtered and dried to obtain the target product 174-2 (1.32 g, yield 89.30%).

Step 3: Synthesis of compound 174-3

174-2 (300.00 mg), 2-2 (214.00 mg), $[PdCl_2(dppf)]CH_2Cl_2$ (40.00 mg), and sodium carbonate (158.00 mg) were dissolved in DMF (3.00 mL) and water (0.40 mL) and reacted at 100°C for 2 h. After the reaction, water was added and extracted with EA/THF (1:1). The organic phase was washed with saturated brine, separated and concentrated, mixed, and separated by silica gel column chromatography (DCM:MeOH=13:1) to obtain the target product 174-3 (160.00 mg, yield 45.43%). ESI-MS m/z:710.49[M+H]+.

Step 4: Synthesis of compound 174

At room temperature, compound 174-3 (160.00 mg) was dissolved in dichloromethane (4.00 mL), TFA (1.00 mL) was added, and the reaction was carried out at room temperature for 0.5 h. The reaction was stopped, and the reaction solution was concentrated and purified by RP-HPLC to obtain the trifluoroacetate salt of compound 174 (86.90 mg, purity 99.32%, yield 75.14%). ESI-MS m/z:510.40[M+H]+. 1H NMR(500MHz,DMSO-$d_6$)$\delta$13.50(s,1H), 10.82(s,1H), 10.31(s,1H), 8.97(s,1H), 8.69(d,J = 8.5Hz,1 H),8.16(d,J = 7.5Hz,1H),7.95(s,1H),7.82(d,J = 8.5Hz,1H),7.69(d,J = 8.5Hz,1H), 6.81(dd, J = 7.5,2.2Hz,1 H),6.68(d,J=2.1Hz,1H),5.16-5.12(m,1H), 4.42(s,2H), 3.91-3.85(m,2H),3.63-3.52(m,2H),2.97 (s,3H) ,2.82(s,3H),2.77-2.72(m,1H),2.17-2.10(m,2H), 1.72-1.66(m,1H),1.54-1.51(m,1H),1.43-1.40(m, 1H).

Example 177: Synthesis of Compound 177 4-(7-(difluoromethoxy)imidazo[1,2-a]pyridin-3-yl)-7-((7-morpholine-2',3',5',6,6',7-hexahydrosp iro[b]pyridin-54'-pyran]-2-yl)amino)isoindol-1-one:

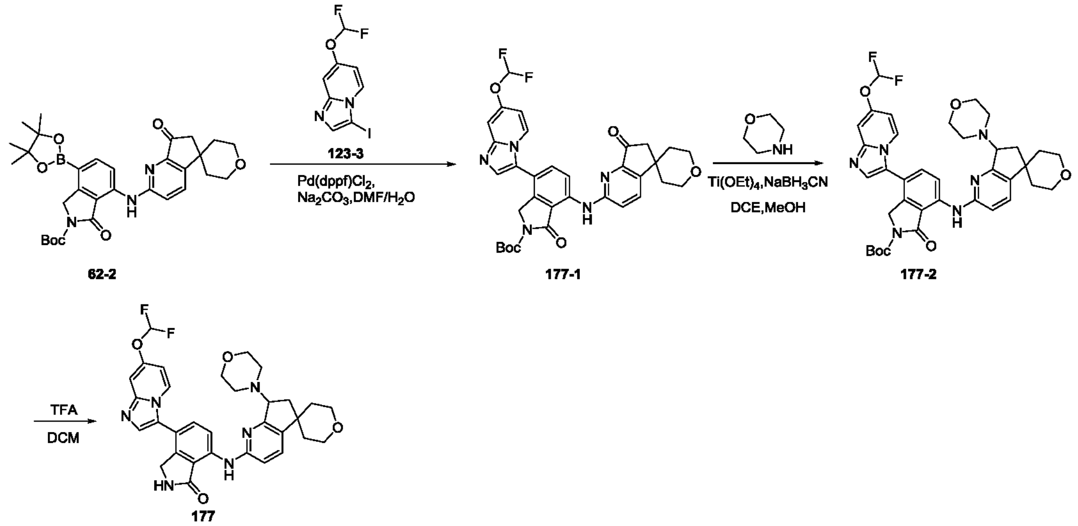


Step 1: Synthesis of compound 177-1

Compound 62-2 (835.00 mg), 123-3 (300.00 mg), $[PdCl_2(dppf)]CH_2Cl_2$ (79.00 mg), and sodium carbonate (307.00 mg) were dissolved in DMF (10.00 mL) and water (2.00 mL), and reacted at 95°C for 2 h. After the reaction, the insoluble matter was filtered out, water was added, and the mixture was extracted with EA/THF (1:1). The organic phase was washed with saturated brine, separated and concentrated, mixed, and separated by silica gel column chromatography (PE:EA=1:99) to obtain the target compound 177-1 (176.00 mg, yield 28.80%). ESI-MS m/z:632.23[M+H]+.

Step 2: Synthesis of compound 177-2

Compound 177-1 (176.00 mg), morpholine (121.00 mg), and $Ti(OEt)_4$ (63.00 mg) were dissolved in DCE (10.00 mL) and methanol (2.00 mL) and reacted at 40°C overnight. $NaBH_3CN$ (60.00 mg) was added and the mixture was reacted at 40°C for 2 h. After the reaction was completed, water was added to quench the reaction. The reaction solution was concentrated and separated by silica gel column chromatography (DCM:MeOH=13:1) to obtain the target compound 177-2 (35.00 mg, yield 17.88%). ESI-MS m/z:703.24[M+H]+.

Step 3: Synthesis of compound 177

At room temperature, compound 177-2 (35.00 mg) was dissolved in dichloromethane (4.00 mL), TFA (1.00 mL) was added, and the mixture was reacted at room temperature for 0.5 h, and the reaction was stopped. The reaction solution was concentrated, the pH was adjusted to alkaline with saturated sodium bicarbonate solution, extracted three times with DCM, the organic phases were combined, washed with saturated brine, dried over anhydrous sodium sulfate, concentrated, and purified by silica gel column chromatography (DCM: MeOH = 10:1) to separate and obtain compound 177 (24.10 mg, purity 95.23%, yield 76.47%). ESI-MS m/z:603.43[M+H]+. $^1$H NMR(500MHz,DMSO-d 6 ) δ10.19(s,1H),8.92(s,1H),8.50-8.45(m,2H),7.99(s,1H),7.82(d,J=8.4Hz,1H ),7.77(d,J=8.5Hz,1H),7.66-7.37(m 3H),7.01(d,J= 7.5Hz,1H),5.11(s,1H),4.42-4.41(m,2H),4.10-4.01(m,4H),3.91-3.86(m,4H),2.80 - 2.75(m,2H) ,2.65-2.63(m,1H),2.19-2.10(m,2H),2.06-1.94(m,2H),1.71-1.66(m,1H),1.53-1.50(m,1H),1.43-1.40( m,2H).

Example 178: Synthesis of Compound 178 7-((7-(cyclopropylamino)-2',3',5',6,6',7-hexahydrospiro[cyclopentadienyl[b] pyridine-54'-pyran] -2-yl)amino)-4-(7-(difluoromethoxy)imidazo[1,2-a]pyridin-3-yl)isoindolin-1-one

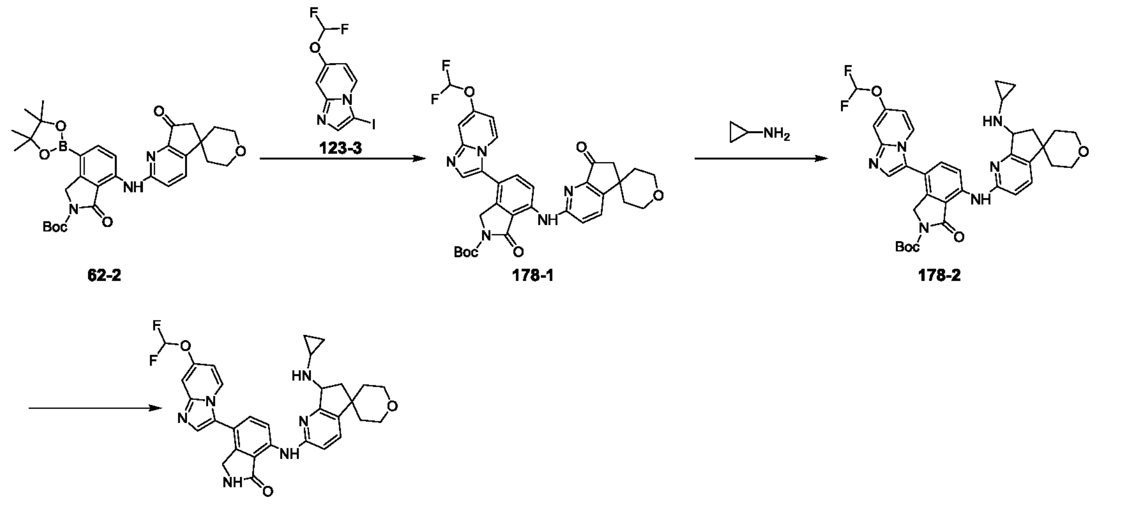


62-2 178-1 178-2

178

Step 1: Synthesis of compound 178-1

Compound 62-2 (700 mg), 123-3 (245 mg), [1,1'-bis(diphenylphosphino) ferrocene]dichloropalladium dichloromethane complex (99 mg), and sodium carbonate (387 mg) were added to DMF (20 mL) and water (4 ml), the atmosphere was replaced with nitrogen, and the mixture was reacted at 95° C for 2 h. The reaction solution was concentrated and separated by column chromatography (DCM/MeOH=95/5) to obtain the target product 178-1 (615 mg, yield 80.1%). ESI-MS m/z:632.29[M+H]+.

Step 2: Synthesis of compound 178-2

Weigh compound 178-1 (615 mg), cyclopropylamine (405 mL), and tetraethyl titanate (222 mg), add DCE (5 mL) and MeOH (2 mL), react at 40°C for 4 h, slowly add sodium cyanoborohydride (184 mg), raise the temperature to 80°C, react for 1 h, and stop the reaction. The reaction solution was extracted with DCM, and the organic phase was washed with saturated brine. The organic phase was collected, dried, and separated by column chromatography (DCM: MeOH = 90:10) to obtain the target compound 178-2 (655 mg, yield 99.8%). ESI-MS m/z:673.35[M+H]+.

Step 3: Synthesis of the hydrochloride salt of compound 178

Weigh compound 178-2 (655 mg) and dissolve it in dichloromethane (5 mL) and methanol (5 mL). Slowly add a solution of hydrogen chloride in dioxane (10 mL, 4 M in dioxane) dropwise under ice bath conditions. Slowly warm the temperature to room temperature and react for 30 min to stop the reaction. After concentration, separation and purification by preparative liquid phase were performed to obtain the hydrochloride salt of the target product 178 (83.40 mg, purity 96.37%, yield 14.96%). ESI-MS m/z:573.40[M+H]+. $^1$H NMR (500MHz, DMSO) δ10.29(s,1H), 9.69(s,1H), 9.21(s,1H),8.96(s,1H),8.71(t,J = 12.0Hz,1H),8.56( d,J = 7.5Hz,1H), 8.23(s,1H), 7.83(t,J = 11.9Hz,1H), 7.79-7.75(m,1H),7.72(s,1H),7.57(d,J = 72.4Hz,1H),7.23(d,J = 5.5Hz, 1H),7.14(d,J = 8.5Hz,1H),5.02(s,1H ),4.49-4.35(m,2H),3.95-3.81(m,2H),3.57(dt,J=39.2,11.5Hz,2H),3.09(s,1H),2.89(dd,J= 13.3,8.2 Hz, 1H), 2.15 (dd, J = 12.1, 8. 5Hz,1H),2.08-1.97(m,1H),1.80-1.63(m,1H),1.54-1.41(m,2H),1.10-0.96(m,1H),0.87(dd,J=23.2,7.1 Hz,2H),0.64-0.49(m,1H).

Example 180: Synthesis of Compound 7-((7-(dimethylamino)-4',5',6,7-tetrahydro-2'H-spiro[cyclopentadienyl[b]pyridine-5,3'-furan]-2-yl)amino)-4-(7-methoxyimidazo[1,2-a]pyridin-3-yl)isoindolin-1-one

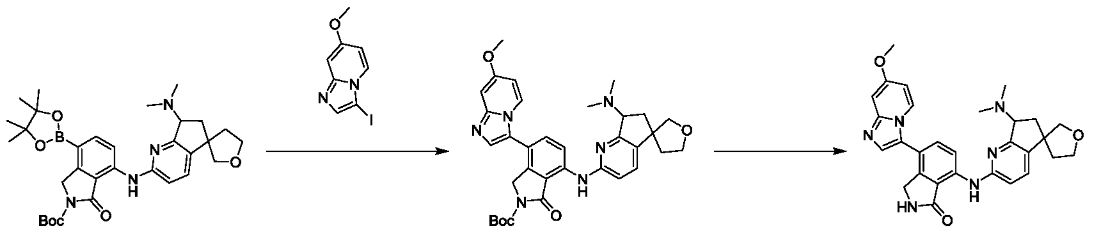

58-14 180-1 180

Step 1: Synthesis of compound 180-1

Compound 58-14 (347 mg), 7-methoxy H-imidazo[1,2-a]pyridine (193 mg), [1,1'-bis(diphenylphosphino)ferrocene]dichloropalladium dichloromethane complex (48 mg), and sodium carbonate (187 mg) were added to DMF (10 mL) and water (2 ml), nitrogen was replaced, and the reaction was carried out at 95°C for 2 h. The reaction solution was concentrated and separated by column chromatography (DCM/MeOH=92/8) to obtain the target compound 180-1 (85 mg, yield 23.7%). ESI-MS m/z:611.32[M+H]+.

Step 2: Synthesis of Compound 180 Hydrochloride

Weigh compound 180-1 (85 mg) and dissolve it in dichloromethane (3 mL) and methanol (3 mL). Slowly add a solution of hydrogen chloride in dioxane (6 mL, 4 M in dioxane) dropwise under ice bath conditions, slowly warm the temperature to room temperature and react for 30 min, then stop the reaction. The residue was concentrated and purified by preparative liquid separation to obtain the hydrochloride salt of the target product 180 (4.50 mg, purity 95.01%, yield 5.38%). ESI-MS m/z: 511.42[M+H]+.

The following compounds were synthesized by the above methods or by similar methods using corresponding intermediates.

| | | | |
|---|---|---|---|
| 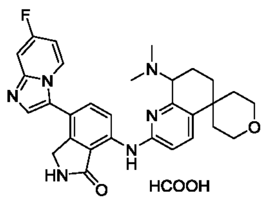 HCOOH<br>**3**<br>[M+H]+: 573.3 | 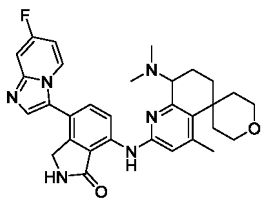<br>**4**<br>[M+H]+: 541.3 | 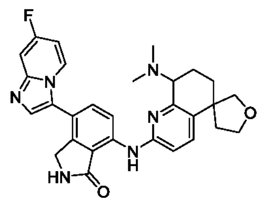<br>**5**<br>[M+H]+: 513.2 | 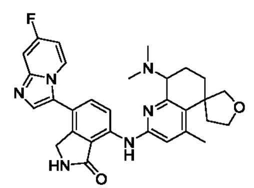<br>**6**<br>[M+H]+: 527.3 |
| 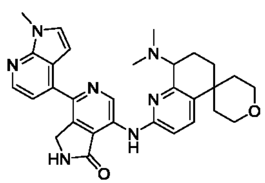<br>**7**<br>[M+H]+: 524.3 | 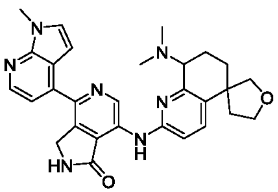<br>**8**<br>[M+H]+: 510.3 | 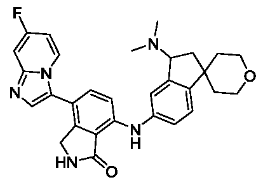<br>**9**<br>[M+H]+: 512.3 | 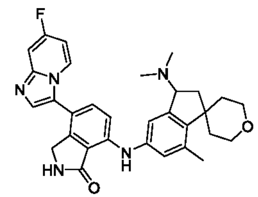<br>**10**<br>[M+H]+: 526.3 |
| 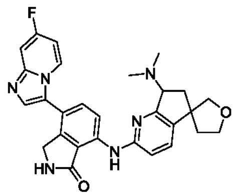<br>**11**<br>[M+H]+: 499.2 | 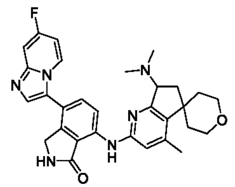<br>**12**<br>[M+H]+: 527.3 | 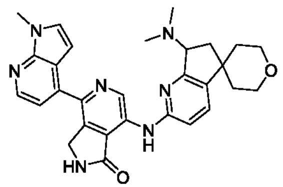<br>**13**<br>[M+H]+: 510.3 | 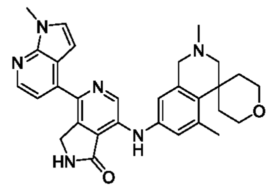<br>**14**<br>[M+H]+: 509.3 |
| 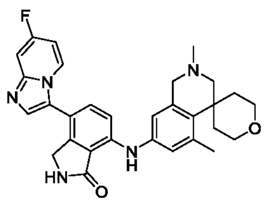<br>**15**<br>[M+H]+: 512.2 | 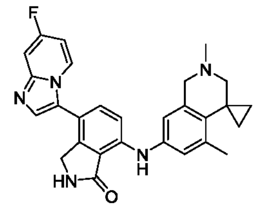<br>**16**<br>[M+H]+: 468.2 | 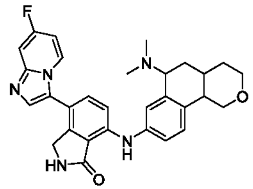<br>**17**<br>[M+H]+: 512.2 | 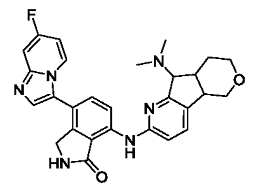<br>**18**<br>[M+H]+: 499.2 |

(continued)

| | | | |
|---|---|---|---|
| 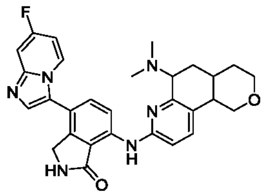 **19** [M+H]+: 513.2 | 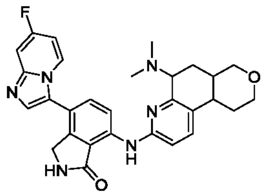 **20** [M+H]+: 513.2 | 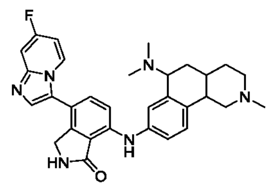 **21** [M+H]+: 525.3 | 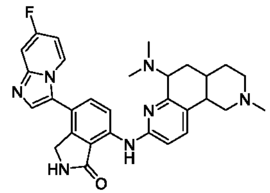 **22** [M+H]+: 526.3 |
| 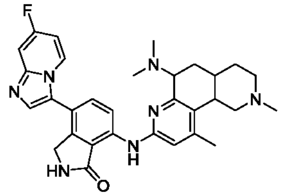 **23** [M+H]+: 540.3 | 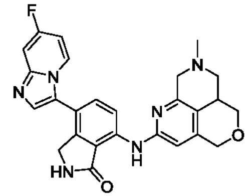 **24** [M+H]+: 471.2 | 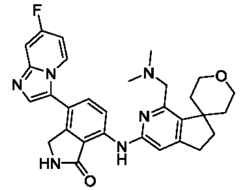 **25** [M+H]+: 527.3 | 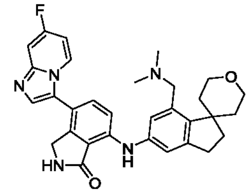 **26** [M+H]+: 526.3 |
| 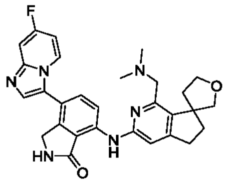 **27** [M+H]+: 513.2 | 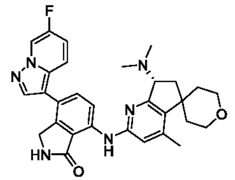 **29** [M+H]+: 527.3 | 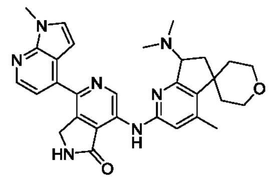 **30** [M+H]+: 524.3 | 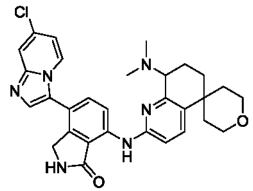 **31** [M+H]+: 543.2 |
| 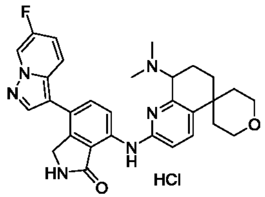 HCl **32** [M+H]+: 563.2 | 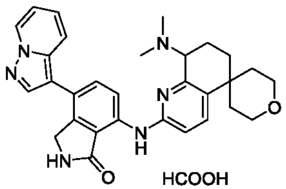 HCOOH **33** [M+H]+: 554.3 | 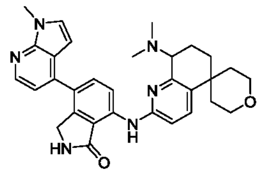 HCl **34** [M+H]+: 559.3 | 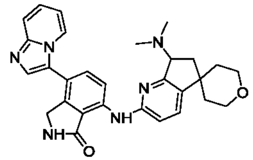 **35** [M+H]+: 595.2 |

(continued)

| | | | |
|---|---|---|---|
| 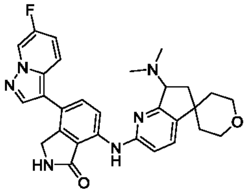 **36** [M+H]+: 513.2 | 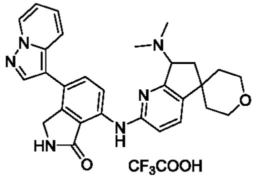 $CF_3COOH$ **37** [M+H]+: 609.2 | 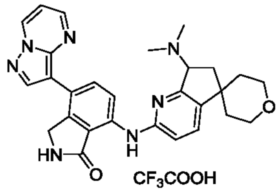 $CF_3COOH$ **38** [M+H]+: 609.2 | 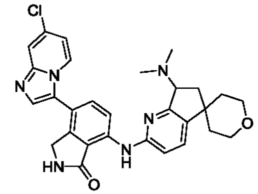 **42** [M+H]+: 529.2 |
| 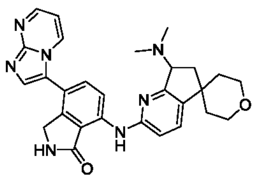 **44** [M+H]+: 496.2 | 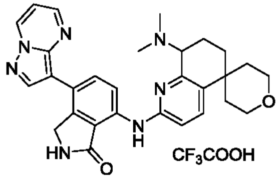 $CF_3COOH$ **45** [M+H]+: 624.3 | 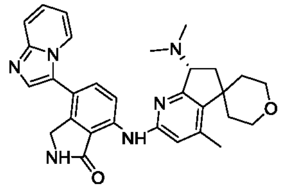 **47** [M+H]+: 509.3 | 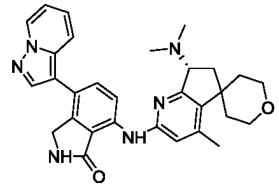 **48** [M+H]+: 509.3 |
| 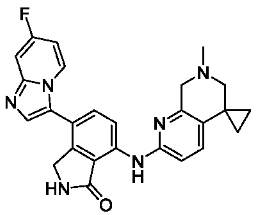 **51** [M+H]+: 455.2 | 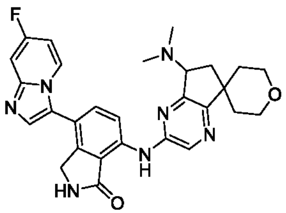 **52** [M+H]+: 514.2 | 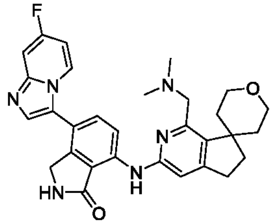 **53** [M+H]+: 527.3 | 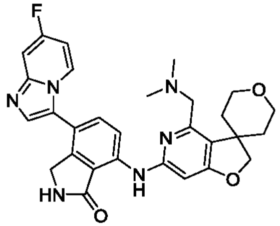 **54** [M+H]+: 529.3 |

(continued)

| | | | |
|---|---|---|---|
| 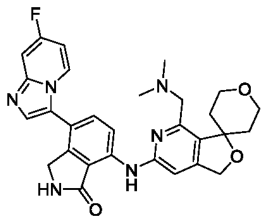 **55** [M+H]+: 529.2 | 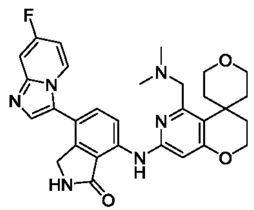 **56** [M+H]+: 543.2 | 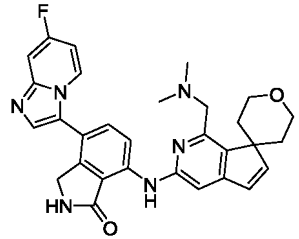 **57** [M+H]+: 525.2 | 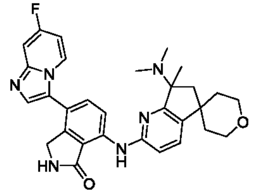 **63** [M+H]+: 527.3 |
| 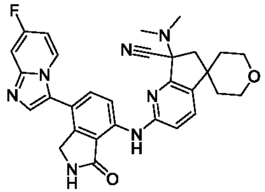 **64** [M+H]+: 538.2 | 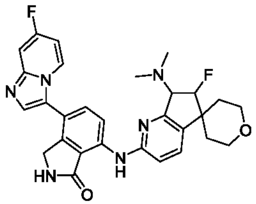 **65** [M+H]+: 531.2 | 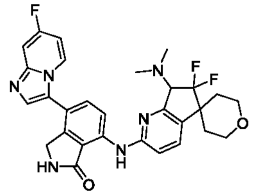 **66** [M+H]+: 549.2 | 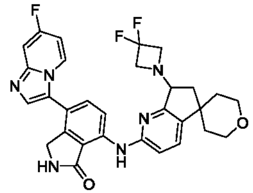 **68** [M+H]+: 561.2 |
| 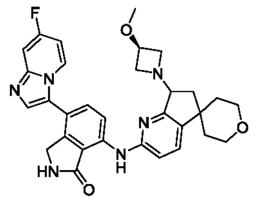 **69** [M+H]+: 555.2 | 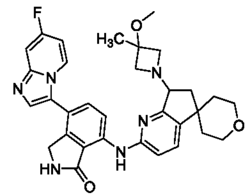 **70** [M+H]+: 569.3 | 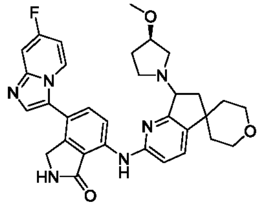 **73** [M+H]+: 569.3 | 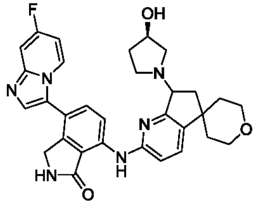 **74** [M+H]+: 555.2 |
| 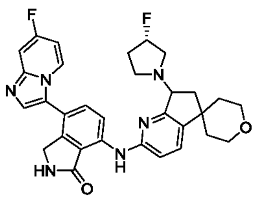 **75** | 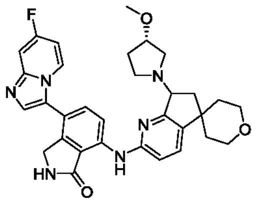 **76** | 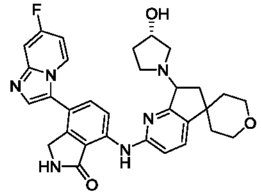 **77** | 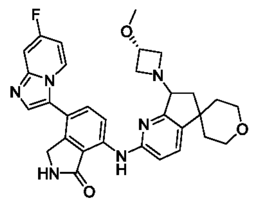 **78** |

(continued)

| | | | |
|---|---|---|---|
| [M+H]+: 557.2 | [M+H]+: 569.3 | [M+H]+: 555.2 | [M+H]+: 555.2 |
| 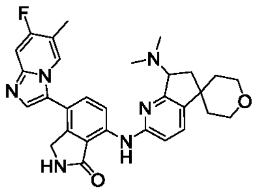 **79** [M+H]+: 527.3 | 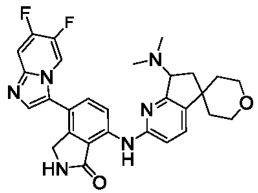 **80** [M+H]+: 531.2 | 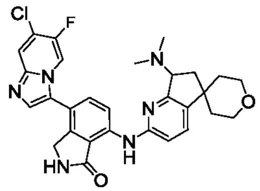 **81** [M+H]+: 547.2 | 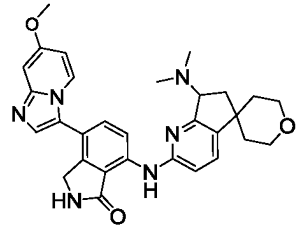 82 [M+H]+: 525.3 |
| 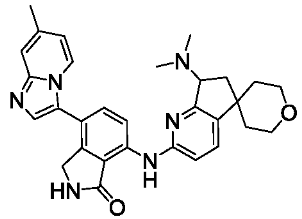 83 [M+H]+: 509.3 | 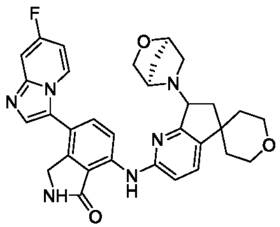 84 [M+H]+: 567.2 | 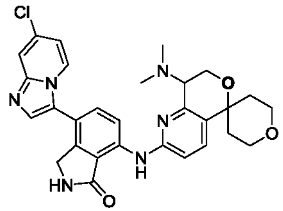 86 [M+H]+: 545.2 | 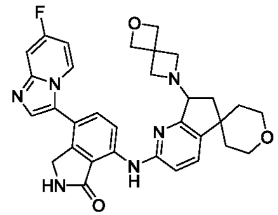 88 [M+H]+: 567.2 |
| 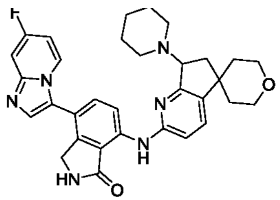 90 [M+H]+: 553.2 | 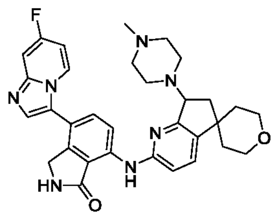 91 [M+H]+: 568.3 | 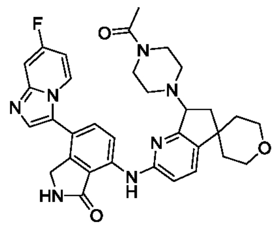 92 [M+H]+: 596.3 | 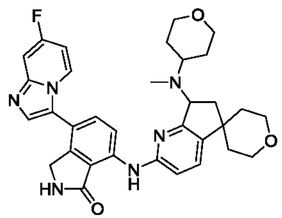 93 [M+H]+: 583.3 |

(continued)

| | | | |
|---|---|---|---|
| 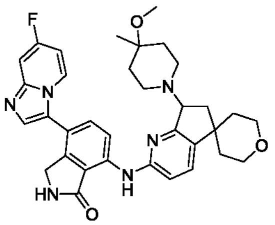<br>94<br>$[M+H]^+$: 597.3 | 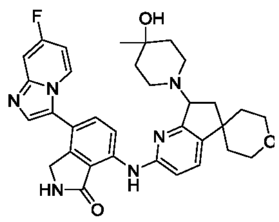<br>95<br>$[M+H]^+$: 583.2 | 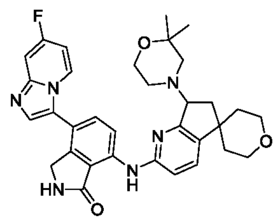<br>96<br>$[M+H]^+$: 583.2 | 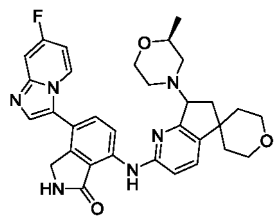<br>97<br>$[M+H]^+$: 569.2 |
| 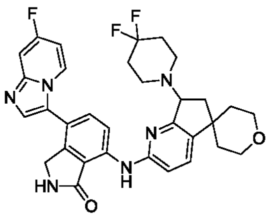<br>98<br>$[M+H]^+$: 589.2 | 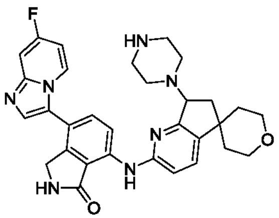<br>99<br>$[M+H]^+$: 554.2 | 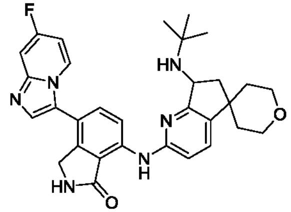<br>101<br>$[M+H]^+$: 541.2 | 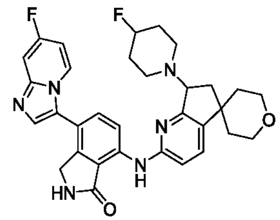<br>102<br>$[M+H]^+$: 571.2 |
| 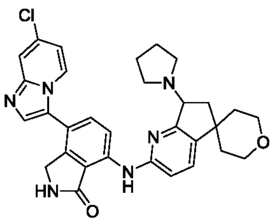<br>103<br>$[M+H]^+$: 555.2 | 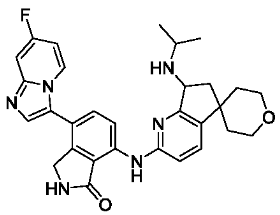<br>104<br>$[M+H]^+$: 527.2 | 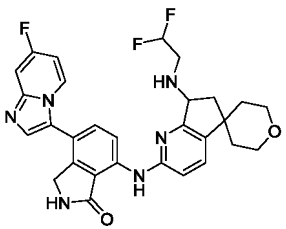<br>105<br>$[M+H]^+$: 549.2 | 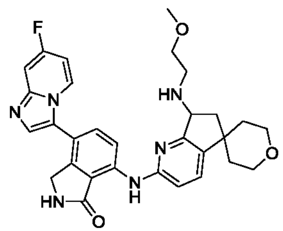<br>106<br>$[M+H]^+$: 543.2 |

(continued)

| | | | |
|---|---|---|---|
| 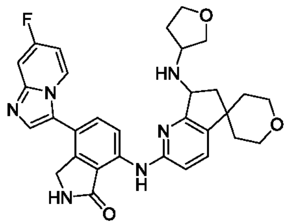 107 [M+H]$^+$: 555.2 | 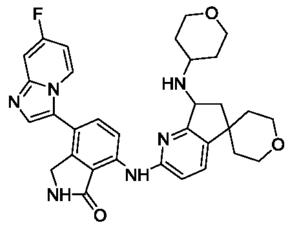 108 [M+H]$^+$: 569.2 | 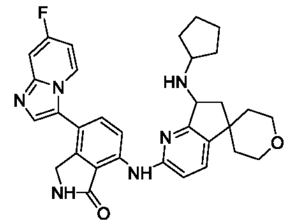 109 [M+H]$^+$: 553.2 | 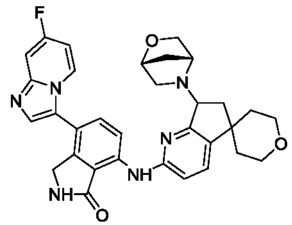 110 [M+H]$^+$: 567.2 |
| 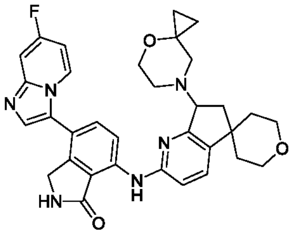 111 [M+H]$^+$: 581.2 | 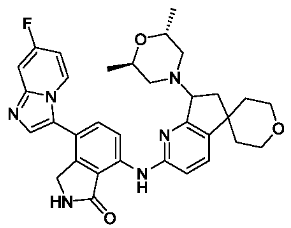 112 [M+H]$^+$: 583.2 | 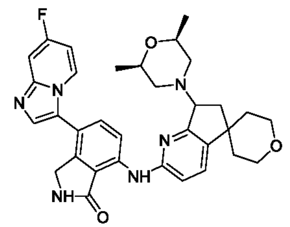 113 [M+H]$^+$: 583.2 | 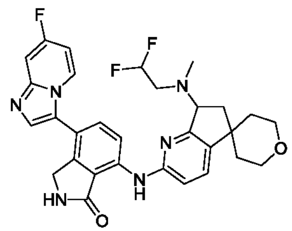 114 [M+H]$^+$: 563.2 |
| 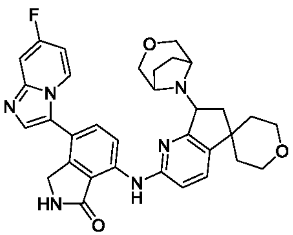 115 [M+H]$^+$: 581.2 | 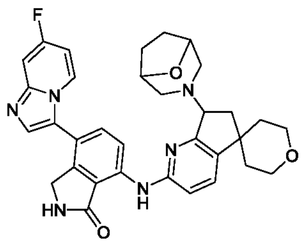 116 [M+H]$^+$: 581.2 | 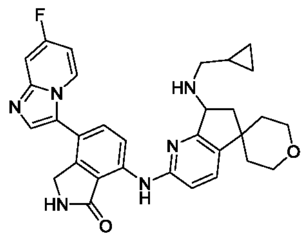 117 [M+H]$^+$: 539.2 | 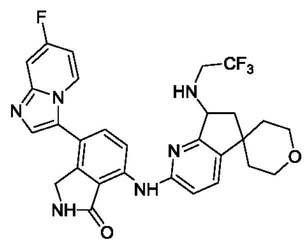 118 [M+H]$^+$: 567.2 |

(continued)

| | | | |
|---|---|---|---|
| 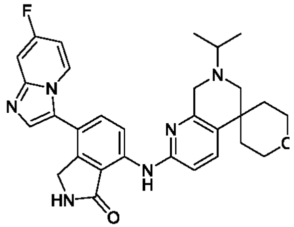 **119** [M+H]+: 527.2 | 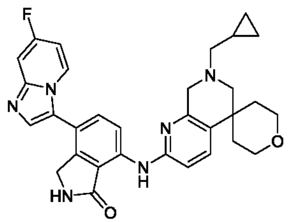 120 [M+H]+: 539.2 | 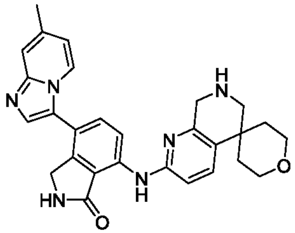 **121** [M+H]+: 481.2 | 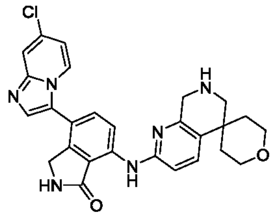 **122** [M+H]+: 501.2 |
| 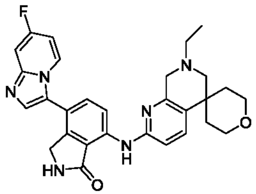 **124** [M+H]+: 513.2 | 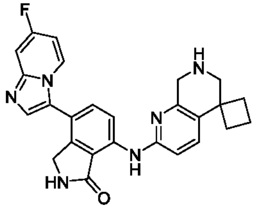 **125** [M+H]+: 455.2 | 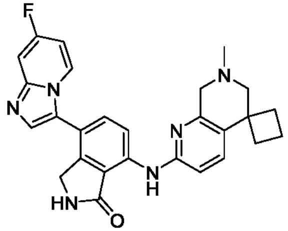 **126** [M+H]+: 469.2 | 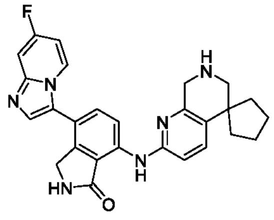 **127** [M+H]+: 469.2 |
| 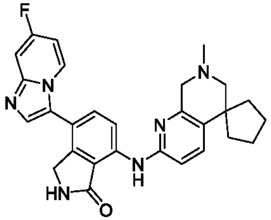 **128** [M+H]+: 483.2 | 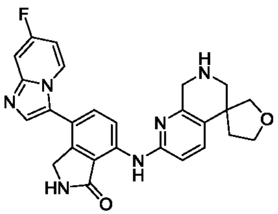 **129** [M+H]+: 471.2 | 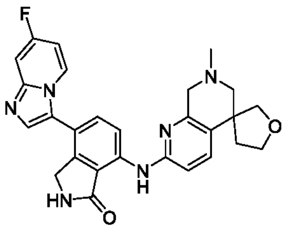 **130** [M+H]+: 485.2 | 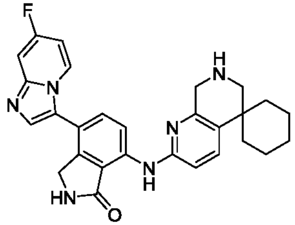 **131** [M+H]+: 483.2 |

(continued)

| | | | |
|---|---|---|---|
| 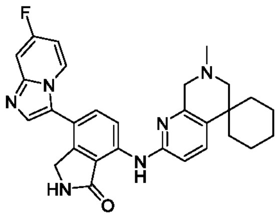 132 [M+H]+: 497.2 | 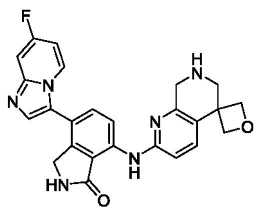 134 [M+H]+: 457.2 | 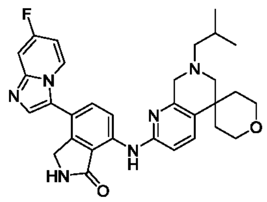 135 [M+H]+: 541.2 | 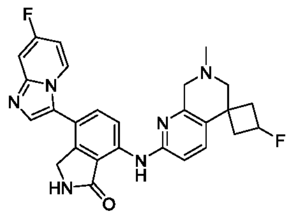 136 [M+H]+: 487.2 |
| 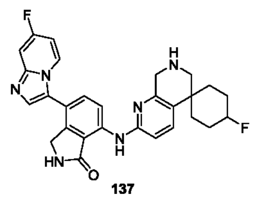 137 [M+H]+: 501.2 | 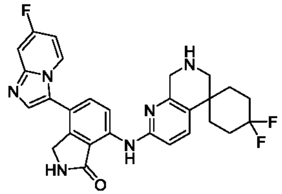 138 [M+H]+: 519.2 | 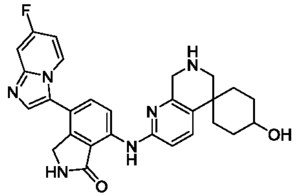 139 [M+H]+: 499.2 | 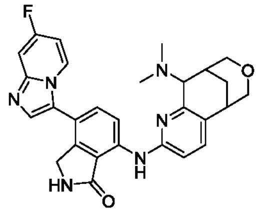 140 [M+H]+: 499.2 |
| 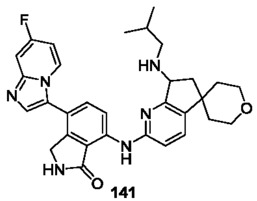 141 [M+H]+: 541.3 | 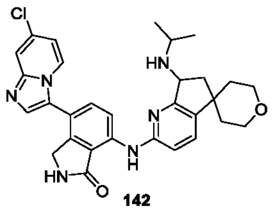 142 [M+H]+: 543.2 | 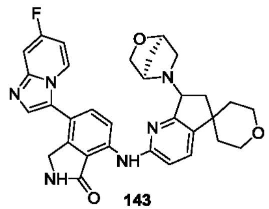 143 [M+H]+: 567.2 | 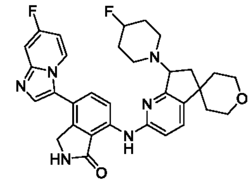 144 [M+H]+: 571.3 |
| 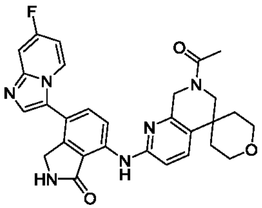 145 [M+H]+: 527.2 | 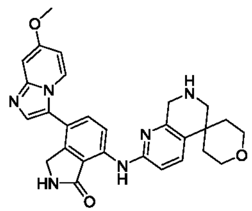 148 [M+H]+: 497.2 | 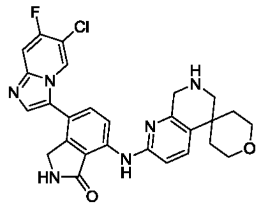 150 [M+H]+: 519.2 | 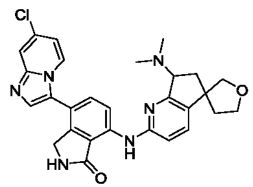 151 [M+H]+: 515.2 |

(continued)

| | | | |
|---|---|---|---|
| 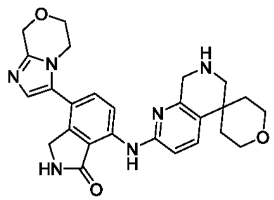 153 [M+H]$^+$: 473.2 | 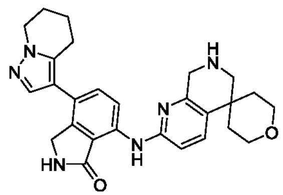 154 [M+H]$^+$: 571.2 | 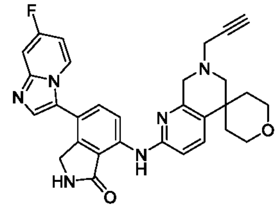 155 [M+H]$^+$: 523.2 | 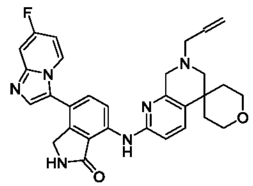 156 [M+H]$^+$: 525.2 |
| 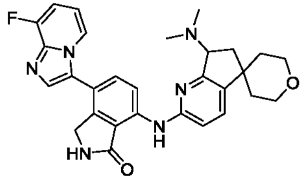 159 [M+H]$^+$: 513.2 | 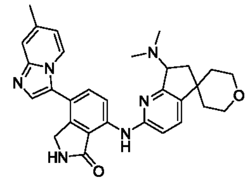 161 [M+H]$^+$: 509.3 | 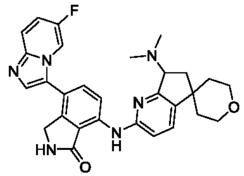 162 [M+H]$^+$: 513.3 | 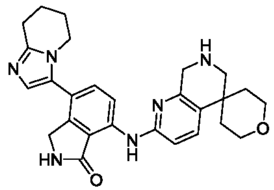 167 [M+H]$^+$: 471.2 |
| 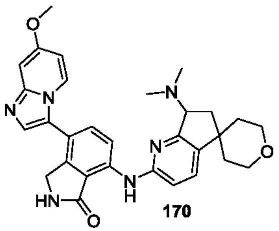 170 [M+H]$^+$: 525.2 | 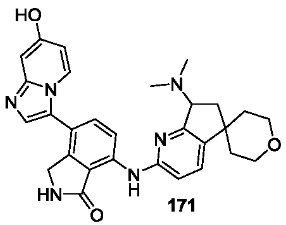 171 [M+H]$^+$: 511.2 | 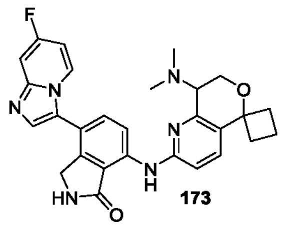 173 [M+H]$^+$: 499.2 | 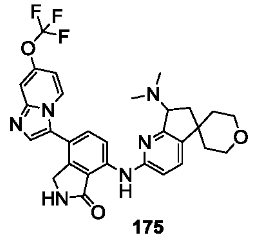 175 [M+H]$^+$: 579.2 |

(continued)

| | | | |
|---|---|---|---|
| 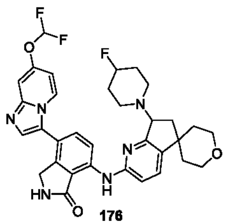 176 [M+H]+: 619.2 | 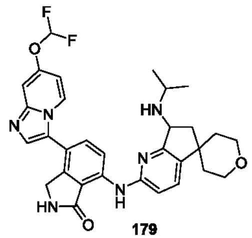 179 [M+H]+: 575.2 | 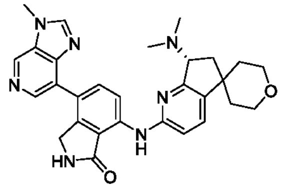 **181** [M+H]+: 510.3 | |

Compound 3: 1H NMR (500MHz, DMSO-d 6 ) δ10.00 (s, 1H), 9.94 (s, 1H), 8.92 (s, 1H), 8.60 (t, J = 6.5 Hz, 1H), 8.30 (d, J = 8.5 Hz, 1H), 8.21 (s, 1H), 8.00 (d, J = 8.8 Hz, 1H), 7.83 (d, J = 8.8 Hz, 1H), 7.79 (d, J = 8.5 Hz, 1H), 7.35-7.31 (m, 1H ),7.26(d,J=8.7Hz,1H),4.68-4.64(m,1H), 4.42(s,2H),3.79-3.74(m,2H),3.68-3.62(m,2H),2.94(s,3H), 2.73(s,3H), 2.54-2.53 (m, 1H), 2.21-2.02(m,3H),1.83-1.81(m,1H),1.75-1.65(m,2H),1.34-1.31(m,1H).

Compound 14: 1H NMR (500MHz, DMSO-d 6 ) δ9.06 (s, 1H), 8.79 (s, 1H), 8.71 (s, 1H), 8.33 (d, J = 5.0 Hz, 1H), 7.54 (d, J = 3.4 Hz, 1H), 7.35 (d, J = 5.0 Hz, 1H), 7.02 (d, J = 2.6 Hz, 1H), 6.96 (d, J = 2.4 Hz, 1H ),6.90(d,J = 3.4Hz,1H),4.69(s,2H),3.86(s,3H),3.79-3.75(m,2H),3.62-3.56 (m,2H), 3.50(s,2H),2.71(s,2H),2.59(s,3H),2.47-2.39(m,2H),2.35(s,3H),1.50(d,J= 13.8Hz, 2H). Compound 15: 1H NMR (500MHz, DMSO-d 6 ) δ9.21 (s, 1H), 8.77 (s, 1H), 8.51 (t, J = 6.6 Hz, 1H), 8.07 (s, 1H), 7.80-7.74 (m, 1H), 7.65 (d, J = 8.4 Hz, 1H), 7.43 (d, J = 8.4 Hz, 1H), 7.24 (d, J = 8.6 Hz, 1H) ,7.18(s,1H),7.06(s,1H),4.43-4.24(m,6H),3.84(s,2H),3.50-3.46(m,1H),3.20-3.16( m,1H),3.00(s,3H),2.78-3.71(m,1-H),2.63(s,3 H),2.31-2.24(m,2H),1.33-1.29(m,1H).

Compound 16: 1H NMR (500MHz, DMSO-d 6 ) δ8.96 (s, 1H), 8.67 (s, 1H), 8.37 (dd, J = 7.6, 5.7 Hz, 1H), 7.78 (s, 1H), 7.58 (d, J = 8.4 Hz, 1H), 7.51 (dd, J = 10.1, 2.7 Hz, 1H), 7.29 (d, J = 8.4 Hz, 1H), 6.96 (td,J=7.5,2.7Hz,1H),6.92(d,J=2.5Hz,1H),6.85(d,J=2.5Hz,1H), 4.34(s,2H), 3 .63 (s,2H), 2.34(s,1H),2.29(s,3H),2.22(s,3H),1.50-1.48(m,2H),0.68-0.64(m,2H).

Compound 29: 1H NMR (500MHz, DMSO) δ10.17 (d, J = 22.3 Hz, 2H), 9.09 (s, 1H), 8.89 (s, 1H), 8.58 (d, J = 8.3 Hz, 1H), 8.38 (s, 1H), 7.88-7.77 (m, 1H), 7.67 (d, J = 8.4 Hz, 1H), 7.45 (t, J = 8.7 Hz, 1H), 6.87 (s, 1H), 5.04 (s, 1 H),4.52(s,2H),3.89(s,2H),3.65(t,J= 11.4Hz,1H),3.54(t,J = 11.7Hz,1H),2.97(s,3H),2.81(s ,4H),2.46(s,3H),2.06(dd,J = 26.5,17.3Hz,3H),1.54(d,J = 12.8Hz, 1H), 1.34(d,J=12.9Hz,1H).

Compound 31: 1H NMR (500MHz, DMSO-d 6 ) δ10.04 (s, 1H), 8.88-8.80 (m, 2H), 8.39 (d, J = 7.5 Hz, 1H), 7.87 (s, 1H), 7.82 (s, 1H), 7.79 (d, J = 8.5 Hz, 1H), 7.70 (d, J = 8.5 Hz, 1H), 6.98 (d, J = 7.4 Hz, 1H), 6.91 (d, J = 8.6 Hz, 1H),4.38(s,1H),3.76-3.71(m,2H),3.66-3.58(m,3H),3.39-3.36(m,1H),2.29(s,6H),2.17-2.11(m,1H ),1.97-1.90(m,3-H),1.88-1.82(m,1H),1.75-1.70(m,1H), 1.46-1.42 (m,1H),1.36-1.32(m,1H).

Compound 32: 1H NMR (500 MHz, DMSO-d 6 ) δ 9.89 (s, 1H), 9.66 (s, 1H), 9.10 (dd, J = 4.8, 2.2 Hz, 1H), 8.88 (s, 1H), 8.39 (s, 1H), 8.13 (d, J = 8.4 Hz, 1H), 7.97 (d, J = 8.8 Hz, 1H), 7.86 (dd, J = 9.8, 5.6 Hz, 1H), 7.72 (d, J = 8.4 Hz, 1H), 7.48-7.43 (m, 1H), 7 .23(d,J = 8.7Hz,1H),4.68-4.60(m,1H),4.53(s,2H),3.78-3.74(m,2H),3.68-3.61(m,2H),2.95(s,3H),2.70(s,3H),2.58-2.52 (m, 1H), 2.23-2.13(m,2H),2.04-1.97(m,1H),1.81-17.5(m,1H),1.69-1.62(m,2H),1.33-1.30(m,1H).

Compound 33: 1H NMR (500MHz, DMSO-d 6 ) δ9.93 (s, 1H), 8.81 (s, 1H), 8.75 (d, J = 7.0 Hz, 1H), 8.58 (d, J = 8.4 Hz, 1H), 8.34 (s, 1H), 8.15 (s, 2H), 7.83-7.80 (m, 2H), 7.68 (d, J = 8.5 Hz, 1H), 7.35-7.28 ( m,1H),6.99-6.95(m,2H),4.51(s,2H),3.76-3.72(m,2H),3.67-3.59(m,2H), 2.45(s, 6H), 2. 28-2.22(m,1H),2.04-1.85(m,5H),1.82-1.72(m,1H),1.51-1.47(m,1H),1.35-1.32(m,1H).

Compound 34: 1H NMR (500MHz, DMSO-d 6 ) δ10.05 (s, 1H), 9.73 (s, 1H), 8.88 (s, 1H), 8.37 (d, J = 5.1 Hz, 1H), 8.25 (d, J = 8.5 Hz, 1H), 7.99 (d, J = 8.7 Hz, 1H), 7.81 (d, J = 8.5 Hz, 1H), 7.66 (d, J = 3. 5Hz,1H),7.36(d,J = 5.2Hz,1H),7.26(d,J = 8.7Hz,1H),6.54(d,J = 3.5Hz,1H),4.67-4.62( m,1H),4.49(s,2H),3.92(s,3H),3.79-3.74(m,2H),3.69-3.60(m,2H),2.95(d,J = 4.9Hz, 3H), 2.71 (d,J = 4.9Hz,3H),2.56-2.54(m,1H),2.24-2.11(m,2H),2.06 -1.99(m,1H),1.82-1.76(m,1H), 1.72-1.63(m,2H),1.34-1.31(m,1H).

Compound 35: 1H NMR (500MHz, DMSO-d 6 ) δ10.33 (s, 1H), 8.95 (s, 1H), 8.67 (d, J = 8.5 Hz, 1H), 8.56 (d, J = 7.0 Hz, 1H), 8.30 (s, 1H), 7.99 (d, J = 9.0 Hz, 1H), 7.84-7.81 (m, 2H), 7.78 (d, J = 8.5 Hz, 1H), 7.35 (t, J = 6.9 Hz, 1H), 7.13 (d, J = 8.5 Hz, 1H z,1H),5.13(t,J= 8.5Hz,1H), 4.42(s,2H),3.93-3.86(m,2H),3.65-3.51(m,2H),2.99(d,J = 3.8Hz,3H),2.82(d ,J = 4.0Hz,3H),2.78-2.71(m,1H),2.16-2.10(m,2H),1.77-1.65(m,1H),1.54-1.50(m,1H),1.44-1.38(m,1H).

Compound 36: 1H NMR (500 MHz, DMSO-d 6 ) δ 10.07 (s, 1H), 9.06 (dd, J = 4.8, 2.2 Hz, 1H), 8.82 (s, 1H), 8.68 (d, J = 8.5 Hz, 1H), 8.36 (s, 1H), 7.90 (dd, J = 9.9, 5.6 Hz, 1H), 7.67 (d, J = 8.5 Hz, 1H), 7.60 (d, J = 8.4 Hz, 1H), 7.41-7.36 (m, 1H), 6 .82(d,J = 8.3Hz,1H),4.50(s,2H),4.21-4.17(m,1H),3.88-3.78(m,2H),3.60-3.50(m,2H),2.37(s,6H),2.34 - 2.29(m,1H),2.00-1.97(m,2H),

1.87-1.84(m,1H),1.71-1.63(m,1H),1.46-1.41(m,1H),1.34-1.31(m,1H).

Compound 37: 1H NMR (500MHz, DMSO-d 6 ) δ10.21 (s, 1H), 8.89 (s, 1H), 8.77 (d, J = 7.0 Hz, 1H), 8.56 (d, J = 8.4 Hz, 1H), 8.37 (s, 1H), 7.79 (dd, J = 8.7, 2.2 Hz, 2H), 7.70 (d, J = 8.4 Hz, 1H), 7.35-7.31 (m, 1H), 7.06 (d, J = 8.4 Hz, 1H), 7.00-6. 97(m,1H),5.13-5.08(m,1H),4.54(s,2H),3.91-3.85(m,2H),3.65-3.52(m,2H),3.00(d,J = 4.6Hz,3H),2.85(d ,J = 4.7Hz,3H),2.75-2.71(m,1H),2.17-2.07(m,2H),1.77-1.64(m,1H),1.S3-1.49(m,1H),1.43-1.38(m,1H).

Compound 38: 1H NMR (500MHz, DMSO-d 6 ) δ10.29 (s, 1H), 9.21 (d, J = 7.0 Hz, 1H), 8.94 (s, 1H), 8.67-8.63 (m, 2H), 8.58 (s, 1H), 8.39 (d, J = 8.6 Hz, 1H), 7.78 (d, J = 8.5 Hz, 1H), 7.24-7.10 (m, 2H), 7.03 (d, J = 8.6 Hz, 1H), 5.17-5. 09(m,1H),4.69(d,J=5.2Hz,2H),3.94-3.84(m,2H),3.63-3.52(m,2H),3.01(d,J = 3.9Hz,3H),2.80(d,J = 4 .1Hz,3H),2.74-2.71(m,1H),2.14-2.09(m,2H),1.71-1.65(m,1H),1.52-1.48(m,1H),1.43-1.39(m,1 H).

Compound 42: 1H NMR (500MHz, DMSO-d 6 ) δ10.18 (s, 1H), 8.85 (s, 1H), 8.72 (d, J = 8.5 Hz, 1H), 8.40 (d, J = 7.4 Hz, 1H), 7.88 (s, 1H), 7.84 (s, 1H), 7.74-7.67 (m, 2H), 6.99 (d, J = 7.2 Hz, 1H), 6.93 (d, J = 8. 2Hz,1H),4.49-4.44(m,1H),4.40(s,2H),3.89-3.80(m,2H),3.62-3.51(m,2H), 2.48 (s,6H), 2.47-2.43(m,1H)2.08-1.99(m,2H),1.71-1.64(m,1H),1.48-1.44(m,1H), 1.37-1.33 (m,1H).

Compound 44: 1H NMR (500 MHz, DMSO-d 6 ) δ 10.16 (s, 1H), 8.89-8.84 (m, 2H), 8.75 (d, J = 8.5 Hz, 1H), 8.59 (dd, J = 4.1, 1.9 Hz, 1H), 8.02 (s, 1H), 7.76 (d, J = 8.6 Hz, 1H), 7.64 (d, J = 8.4 Hz,1H),7.08(dd,J = 6.9,4.0Hz,1H),6.87(d,J = 8.3Hz,1H),4.43(s,2H),4.30-4.21(m,1H),3.88-3.81 (m,2H), 3.56(dt,J = 24 .0,11.7Hz,2H),2.38(s,6H),2.05-1.98(m,1H),1.90-1.85(m,1H),1.71-1.65 (m,1H), 1.46-1.41(m,1H),1.35-1.32(m,1H).

Compound 45: 1H NMR (500 MHz, DMSO-d 6 ) δ9.96 (s, 1H), 9.36 (s, 1H), 9.21 (d, J = 7.0 Hz, 1H), 8.90 (s, 1H), 8.68 (d, J = 4.0 Hz, 1H), 8.58 (s, 1H), 8.34 (d, J = 8.5 Hz, 1H), 8.16 (d, J = 8.5 Hz, 1H), 7.96 (d, J = 8.8 Hz, 1H), 7.22 (d, J = 8.7 Hz, 1H), 7.1 7-7.14(m,1H),4.68(s,2H),4.66-4.64(m,1H),3.78-3.73(m,2H),3.68-3.60(m,2H),3.02(s,3H),2.64(s ,3H),2.59-2.52(m,1-H),2.22-2.15

(m,2H),2 .02-1.95(m,1H),1.80-1.75(m,1H),1.67-1.59(m,2H),1.32-1.28(m,1H).

Compound 47: 1H NMR (500MHz, DMSO) δ10.37 (s, 1H), 10.26 (s, 1H), 8.95 (s, 1H), 8.70 (d, J = 8.5 Hz, 1H), 8.57 (d, J = 6.8 Hz, 1H), 8.34 (s, 1H), 8.02 (d, J = 9.1 Hz, 1H), 7.92-7.83 (m, 1H), 7.76 (d, J = 8.5 Hz, 1H), 7.39 (t, J = 6.8 Hz, 1H), 6.94 (s, 1H), 5.07 (t ,J = 8.7Hz,1H),4.39(d,J = 18.8Hz,2H),3.90(dd,J = 13.6,6.8Hz,2H),3.66(t,J = 11.7Hz,1H),3.55(t,J = 11.6Hz,1H), 2.97(s,3H), 2.86-2.71(m,4H),2.48(s,3H),2.12-1.97(m,3H),1.55(d,J = 13.0Hz,1H),1.35(d,J= 13.1Hz,1H).

Compound 48: 1H NMR (500MHz, DMSO) δ10.14 (s, 1H), 10.08 (s, 1H), 8.88 (s, 1H), 8.77 (d, J = 7.0 Hz, 1H), 8.57 (d, J = 8.4 Hz, 1H), 8.36 (s, 1H), 7.79 (d, J = 8.9 Hz, 1H), 7.69 (d, J = 8.5 Hz, 1H), 7.36-7.29 (m, 1H), 6.99 (t, J = 6.8 Hz, 1H), 6.87 (s, 1H), 5.04 (t, J = 8.6 Hz, 1H), 4.51(d,J = 18.9Hz,2H),3.89(t,J = 8.2Hz,2H),3.65(t,J = 11.4Hz,1H),3.54(t,J = 11.6Hz,1H),2.98(d,J = 4.3Hz,3H),2.80 (dd,J = 14.4,6.8Hz,4H),2.46(s,3H),2.03(ddd,J = 23.6,14.8,7.3Hz,3H),1.54(d,J=12.8Hz,1H),1.34(d,J=13.1Hz,1H).

Trifluoroacetate of compound 54: δ10.12 (s, 1H), 9.52 (s, 1H), 8.93 (s, 1H), 8.58 (dd, J = 7.6, 5.3 Hz, 1H), 8.28-8.22 (m, 2H), 7.92 (d, J = 8.5 Hz, 1H), 7.76 (d, J = 8.5 Hz, 1H), 7.40 (t, J = 7.4 Hz,1H),7.26(s,1H),5.03(s,2H),4.60(d,J = 5.4Hz,2H),4.41(s,2H),3.87-3.84( m,2H),3.77-3.71(m,2H),2.99(s,4H),2.23-2.16(m,2H),1.62(d,J = 13.0Hz,2H).

Compound 67: 1H NMR (500MHz, DMSO-d 6 ) δ10.14 (s, 1H), 8.84 (s, 1H), 8.71 (d, J = 8.6 Hz, 1H), 8.44-8.40 (m, 1H), 7.82 (s, 1H), 7.74 (d, J = 8.5 Hz, 1H), 7.64 (d, J = 8.4 Hz, 1H), 7.52 (dd, J = 10.1, 2.7 Hz, 1H), 6.98 (m, 1H), 6.86 (d, J = 8.3 Hz ,1H),5.28-5.23(m,1H),5.17-5.11(m,1H),4.43-4.34(m,2H),3.89-3.75(m,5H),3.53(t,J = 11.9Hz,2H),3.4 4-3.36(m,1H),2.30-2.24(m,1H),1.91-1.85(m,1H),1.83-1.78(m,2H),1.52-1.49(m,1H),1.35-1.31(m, 1H).

Compound 70: 1H NMR (500 MHz, DMSO-d 6 ) δ 10.12 (s, 1H), 8.84 (s, 1H), 8.74 (d, J = 8.6 Hz, 1H), 8.41 (t, J = 6.7 Hz, 1H), 7.81 (s, 1H), 7.71 (d, J = 8.6 Hz, 1H), 7.63 (d, J = 8.3 Hz, 1H), 7.53-7.50 (m, 1H), 6.99-6.94 (m, 1H), 6.85 (d, J = 8.4 Hz, 1H), 4.38 (d ,J = 25.2Hz,3H),3.85-3.78(m,2H),3.56-3.49(m,2H),3.41-3.34(m,2H),3.29-3.26(m,2H),3.22-3.19(m,1H),3.12

(s,3H),2.26-2.21(m,1H),1.91-1.86(m,1H),1.85-1.78(m,2H),1.55-1.50(m,1H),1.36(s,3H),1.33-1.31(m,1H).

Compound 73: 1H NMR (500MHz, DMSO-d 6 ) δ10.11 (s, 1H), 8.84 (s, 1H), 8.70 (d, J = 8.1 Hz, 1H), 8.40 (t, J = 6.5 Hz, 1H), 7.82 (s, 1H), 7.72-7.60 (m, 2H), 7.53 (dd, J = 10.0, 2.1 Hz, 1H), 6.98 (t, J = 7.4 Hz, 1H), 6.87 (d, J = 7.2 Hz, 1H), 4.39 (dt, J = 31.6, 11.1 Hz, 2H) ,3.92(d,J = 22.1Hz,2H),3.83(t,J = 9.2Hz,2H),3.57(dd,J = 22.1,10.7Hz,2H),3.16(d,J = 8.7Hz,3H),2.88(dd,J = 98.3,40 .7Hz,4H),2.36(s,1H),2.05-1.91(m,4H),1.72(dd,J = 34.0,21.7Hz,2H),1.52(d, J=13.2Hz,1H),1.35(d, J=12.7Hz,1H).

Compound 74: 1H NMR (500 MHz, DMSO-d 6 ) δ 10.10 (s, 1H), 8.84 (s, 1H), 8.70 (t, J = 5.8 Hz, 1H), 8.42 (t, J = 6.7 Hz, 1H), 7.83 (s, 1H), 7.70 (t, J = 7.9 Hz, 1H), 7.65 (d, J = 8.4 Hz, 1H), 7.54-7.52 (m, 1H), 7.00-6.96 (m, 1H), 6.88 (d, J = 8.4 Hz ,1H),4.69-4.63(m,1H),4.39(d,J= 15.1Hz,2H),4.23-4.19(m,1H),4.00-3.95(m,1H),3.86-3.79(m,2H),3.6 1-3.52(m,2H),3.01-2.84(m,3H),2.08-1.92(m,4H),1.76-1.71(m,1H),1.57-1.52(m,1H),1.38-1.34( m,1H).

Compound 75: 1H NMR (500 MHz, DMSO-d 6 ) δ 10.10 (s, 1H), 8.84 (s, 1H), 8.66 (d, J = 8.6 Hz, 1H), 8.42-8.40 (m, 1H), 7.82 (s, 1H), 7.67 (dd, J = 19.5, 8.4 Hz, 2H), 7.52 (d, J = 9.9 Hz, 1H), 6.98-6.87 (m, 2H), 5.36-5.14 (m, 1H), 4.38 (q, J = 18 .2Hz,2H),4.08-3.97(m,1H),3.85-3.81(m,2H),3.65-3.53(m,2H),3.13-3.01(m,2H),2.66-2.62(m,1H),2.4 0-2.36(m,2H),2.02-1.96(m,3H),1.76-1.74(m,1H),1.55-1.51(m,1H),1.48-1.44(m,1H),1.37-1.35(m,1H). Compound 76: 1H NMR (500 MHz, DMSO-d 6 ) δ 10.11 (s, 1H), 8.84 (s, 1H), 8.70 (d, J = 8.1 Hz, 1H), 8.40 (t, J = 6.5 Hz, 1H), 7.82 (s, 1H), 7.72-7.59 (m, 2H), 7.53 (dd, J = 10.0, 2.1 Hz, 1H), 6.98 (t, J = 7.4 Hz, 1H), 6.87 (d, J = 7.2 Hz, 1H), 4.39 (dt, J = 31.6, 11.1 Hz, 2H), 3.92 (d, J = 22.1Hz,2H),3.83(t,J = 9.2Hz,2H),3.57(dd,J = 22.1,10.7Hz,2H),3.16(d,J = 8.7Hz,3H),2.98(d,J=15.9Hz,2H),2.85(s,1H),2.72(s,1H),2.36(s,1H),2.04-1.93(m,3H),1.75(t,J= 12.3Hz,1H), 1.66 (s, 1H), 1.52(d,J=13.2Hz,1H),1.35(d,J= 12.7Hz,1H).

Compound 84: 1H NMR (500 MHz, DMSO-d 6 ) δ 10.12 (s, 1H), 8.83 (s, 1H), 8.62 (d, J = 24.3 Hz, 1H), 8.41 (t, J = 6.5 Hz, 1H), 7.82 (s, 1H), 7.69 (d, J = 36.7 Hz, 2H), 7.52 (d, J = 9.6 Hz, 1H), 6.99 (s, 1H), 6.87 (s, 1H), 4.38 (q, J = 17.8 Hz, 3H), 4.13-3.70 (dt, J = 108.1, 2 2.1Hz,5H),3.65-3.51(m,2H),3.28-2.90(m,2H),2.39-2.06(m,2H),2.00(dd,J = 13.2,5.7Hz,1H),1.87(s,1H),1.75 (s, 1H), 1.70-1.52(m,2H),1.49-1.28(m,2H).

Compound 86: 1H NMR (500MHz, DMSO) δ10.12 (s, 1H), 8.86 (s, 1H), 8.80 (d, J = 8.6 Hz, 1H), 8.40 (d, J = 7.3 Hz, 1H), 7.86 (d, J = 25.9 Hz, 2H), 7.69 (dd, J = 25.9, 8.6 Hz, 2H), 7.06-6.89 (m, 2H), 4.46-4.35 (m, 2H), 4.07 (t, J = 16.2 Hz,1H),3.95(dd,J = 12.3,3.6Hz,1H),3.69(ddd,J = 33.2,20.1,8.7Hz,4H),3.49(s,1H),2.41(s,6H) ,2.16-2.08(m,1H),2.05-1.93(m,1H),1.78(ddd,J=19.6,18.6,8.9Hz,2H),1.58(-

d,J=12.6Hz,1H). Compound 88: 1H NMR (500 MHz, DMSO-d 6 ) δ 10.12 (s, 1H), 8.84 (s, 1H), 8.74 (d, J = 8.6 Hz, 1H), 8.43 (t, J = 6.8 Hz, 1H), 7.85-7.77 (m, 2H), 7.61 (d, J = 8.3 Hz, 1H), 7.53 (d, J = 10.2 Hz, 1H), 7.01-6.95 (m, 1H), 6.8 5(d,J = 8.5Hz,1H),4.64-4.60(m,4H),4.39(d,J = 26.7Hz,2H),3.85-3.79(m,2H),3.72-3.68(m,1H),3.60-3.48(m,6H),2.20-2.16(m,1H),1.89-1.73(m,3H),1.51-1.46 (m,1H), 1.32-1.27(m,1H).

Compound 90: 1H NMR (500 MHz, DMSO-d 6 ) δ 10.10 (s, 1H), 8.84-8.81 (m, 2H), 8.41 (dd, J = 7.6, 5.7 Hz, 1H), 7.81 (s, 1H), 7.68 (d, J = 8.6 Hz, 1H), 7.61 (d, J = 8.4 Hz, 1H), 7.52 (dd, J = 10.1, 2.7 Hz, 1H), 6.99-6.95 (m, 1H), 6.83 (d, J = 8.3 Hz, 1H), 4.37 (d, J = 22.1 Hz,2H),4.26(t,J = 8.1Hz,1H),3.87-3.78(m,2H),3.63-3.48(m,2H),2.84-2.78(m,2H),2.54-2.52(m,2H),2.39-2.35(m, 1H), 2.04-1.99(m,1H),1.83-1.81(m,1H),1.66-1.60(m,1H),1.54-1.50(m,4H),1.42-1.38(m,3H), 1.36-1.32 (m,1H).

Compound 91: 1H NMR (500 MHz, DMSO-d 6 ) δ 10.09 (s, 1H), 8.83 (s, 1H), 8.76 (d, J = 8.6 Hz, 1H), 8.41 (dd, J = 7.6, 5.8 Hz, 1H), 7.82 (s, 1H), 7.65 (dd, J = 23.9, 8.5 Hz, 2H), 7.53 (dd, J = 10.0, 2.7 Hz, 1H), 6.99-6.95 (m, 1H), 6.86 (d, J = 8.3 Hz, 1H), 4.37 (d, J = 27.2 Hz, 2H ),4.22(t,J = 7.7Hz,1H),3.87-3.79(m,2H),3.63-3.49(m,2H),2.90-2.84(m,1H),2.58-2.54(m,1H),2.39-2.32 (m,3H), 2.16-2.13(m,2H),2.05-1.95(m,2H),1.89-1.84(m,1H),1.70-1.64(m,1H),1.47-1.42(m,1H), 1.36-1.32(m,1H),1.24(s,3H). Trifluoroacetate salt of compound 94: 1H NMR (500 MHz, DMSO-d 6 ) δ 10.23 (s, 1H), 9.97 (s, 1H), 8.92 (s, 1H), 8.55-8.50 (m, 2H), 8.16 (s, 1H), 7.86-7.81 (m, 2H), 7.75 (d, J = 8.5 Hz, 1H), 7.34-7.30 (m, 1H), 7.13 ( d,J = 8.5Hz,1H),5.14-5.07(m,1H),4.39(d,J = 12.1Hz,2H),3.91-3.85(m,2H),3.76-3.72(m,2H),3.61-3.48(m,2H),3.40-3.35(m, 1H),3.09-3.07(m, 1H), 3.04(s,3H), 2.84-2.79 (m,1H), 2.17-2.07(m,2H),2.03-1.98(m,1H),1.81-1.73(m,2H), 1.70-1.62(m,1H),1.56-1.53(m,1H),1.43-1.40(m,1H),1.14(s,3H).

Trifluoroacetate salt of compound 95: 1H NMR (500 MHz, DMSO) δ10.22 (s, 1H), 9.80 (s, 1H), 8.91 (s, 1H), 8.56 (d, J = 8.6 Hz, 1H), 8.54-8.47 (m, 1H), 8.11 (s, 1H), 7.86-7.72 (m, 3H), 7.27 (s, 1H), 7.12 (d, J = 8.5 Hz, 1H), 5.11 (s, 1H), 4.38 (d, J = 11.9 Hz, 2H), 3.88 (d, J = 12.3 Hz, 3H), 3.74 (s, 2H), 3.60 (d, J = 11.9Hz,1H),3.51(d,J = 11.6Hz,1H),3.34(dd,J = 30.3,11.3Hz,2H),2.84(dd,J = 13.1,8.2Hz,1H),2.19-2.03(m,2H),2.02-1 .95(m,1H),1.80(dd,J = 32.8,15.9Hz,3H),1.66(t,J = 10.5Hz,1H),1.54(d,J = 12.2Hz,1H),1.43(d,J = 12.4Hz,1H),1.20(s,3H).

Trifluoroacetate salt of compound 96: 1H NMR (500 MHz, DMSO) δ10.23-9.99 (m, 1H), 8.93 (s, 1H), 8.71 (s, 1H), 8.47 (s, 1H), 8.18 (s, 1H), 7.84 (s, 2H), 7.69 (d, J = 7.8 Hz, 1H), 7.35 (s, 1H), 5.16 (s, 1H), 4.40 (d, J = 4.4 Hz, 2H), 3.91 (d, J = 11.4 Hz, 5H), 3 .79(s,1H),3.60(d,J= 11.1Hz,1H),3.53(d,J = 12.2Hz,1H),3.20(d,J = 62.4Hz,3H),2.78(s,1H),2.09 (d,J = 61.6Hz,2H),2.04-1.93(m,1H),1.68(s,1H),1.52(d,J=13.4Hz,1H),1.24(s,3H),1.19(s,3H). Compound 97: 1H NMR (500MHz, DMSO-d 6 ) δ10.11 (d, J = 1.8 Hz, 1H), 8.83 (s, 1H), 8.76 (d, J = 8.5 Hz, 1H), 8.41-8.38 (m, 1H), 7.81 (s, 1H), 7.72-7.65 (m, 1H), 7.65-7.60 (m, 1H), 7.54-7.50 (m, 1H), 6.99-6.95 (m, 1H), 6.86 (d, J = 8.3 Hz, 1H), 4.45-4.31 (m, 2H), 4.26-4.17 ( m,1H),3.85-3.72(m,2H),3.64-3.48(m,4H),3.05-3.02(m,1H),2.85-2.82(m,1H),2.80-2.71(m,1H),2.64-2.60 (m,1H), 2.44-2.36(m,2H),2.13-1.97(m,2H),1.89-1.84(m,1H),1.71-1.62(m,1H),1.46-1.42 (m,1H), 1.37-1.32(m,1H),1.06-1.02(m,3H).

Compound 98: 1H NMR (500 MHz, DMSO-d 6 ) δ 10.24 (s, 1H), 8.95 (s, 1H), 8.66 (dd, J = 7.6, 5.1 Hz, 1H), 8.47 (d, J = 8.5 Hz, 1H), 8.39 (s, 1H), 8.06 (dd, J = 8.4, 2.6 Hz, 1H), 7.86 (d, J = 8.5 Hz, 1H), 7.80 (d, J = 8.5 Hz, 1H), 7.55-7.51 (m, 1H), 7 .17(d,J=8.5Hz,1H),5.24(t,J= 8.6Hz,1H),4.40(d,J = 13.4Hz,2H),3.92-3.86(m,2H),3.63-3.50(m,2H),2.8 6-2.80(m,1H),2.44-2.32(m,5H),2.21-2.13(m,2H),1.69-1.63(m,1H),1.54-1.52(m,1H),1.45-1.40( m,1H).

Trifluoroacetate of compound 99: 1H NMR (500 MHz, DMSO) δ10.13 (s, 1H), 8.88 (s, 1H), 8.64 (s, 1H), 8.53 (s, 2H), 8.05 (s, 1H), 7.73 (dd, J = 20.7, 8.4 Hz, 3H), 7.21 (s, 2H), 6.98 (s, 1H), 6.66 (s, 1H), 5.32 (t, J = 5.0 Hz, 1H), 4.43 (d,J = 18.2Hz,1H),4.35(d,J = 18.4Hz,1H),3.84(d,J = 12.5Hz,2H),3.60(s,2H),2.64(s,1H),2.36(s,1H),2.03-1.98(m,3H),1.46(d,J =8.5Hz,3H), 1.35(s,1H), 0.85(d,J=7.1Hz,2H).

Trifluoroacetate salt of compound 101: 1H NMR (500 MHz, DMSO-d 6 ) δ10.22 (s, 1H), 8.91 (s, 1H), 8.66 (s, 1H), 8.62 (d, J = 8.6 Hz, 1H), 8.52-8.43 (m, 2H), 8.13 (s, 1H), 7.82 (t, J = 7.6 Hz, 2H), 7.73 (d, J = 8.5 Hz, 1H), 7.30 (s, 1H), 7.15 (d, J = 8.4 Hz, 1H), 4.99 (s, 1H), 4.39 (d,J = 11.9Hz,2H),3.92-3.84(m,2H),3.63-3.57(m,1H),3.60-3.50(m,1H),2.76-2.70(m,1H),2.33-2.28 (m,1H), 2.06-2.00(m,1H),1.83-1.77(m,1H),1.49(s,9H).

Trifluoroacetate salt of compound 102: 1H NMR (500MHz, DMSO) δ10.32 (s, 1H), 10.22 (s, 1H), 8.92 (s, 1H), 8.55 (s, 1H), 8.50 (d, J = 8.2 Hz, 1H), 8.20 (s, 1H), 7.94-7.81 (m, 2H), 7.74 (d, J = 8.5 Hz, 1H), 7.36 (s, 1H), 7.15 (d, J = 8.5 Hz, 1H), 5.15 (s, 1H), 5.00 (t, J = 43.7 Hz, 1H), 4.46-4.30(m,2H),3.95-3.81(m,3H),3.59(dd,J = 23.5,11.9Hz,1H),3.58-3.46(m,2H),3.27(s,1H),2.81(d,J= 7.9Hz, 1H), 2.12 (t, J = 39.7Hz, 5H), 1.68 (dd, J = 12.6, 8.4Hz, 1H), 1.54 (d, J = 12.2Hz, 1H), 1.43 (d, J = 12.8Hz, 1H).

Compound 103: 1H NMR (500 MHz, DMSO-d 6 ) δ 10.43 (d, J = 5.7 Hz, 1H), 10.24 (s, 1H), 8.93 (s, 1H), 8.53 (d, J = 8.5 Hz, 1H), 8.45 (d, J = 7.3 Hz, 1H), 8.08 (s, 1H), 8.02 (s, 1H), 7.83 (d, J = 8.5 Hz, 1H), 7.78 (d, J = 8.5 Hz, 1H), 7.21 (dd, J = 9.8, 4.2 Hz, 1H), 7.13 (d, J = 8.1 Hz, 1H ),5.11(dd,J = 14.6,8.0Hz,1H),4.41(q,J = 18.3Hz,2H),4.02-3.82(m,3H),3.66-3.31(m,4H), 2.91(dd,J = 13.4,8.2Hz,1H),2 .20-1.92(m,6H),1.84(dd,J = 12.8,6.7Hz,1H),1.68(td,J = 12.8,4.4Hz,1H), 1.51(d,J=12.4Hz,1H),1.41(d,J=12.6Hz,1H).

Compound 104: 1H NMR (500 MHz, DMSO-d6) δ 10.12 (s, 1H), 8.86 (s, 1H), 8.69 (s, 1H), 8.41 (t, J = 6.8 Hz, 1H),

7.82 (s, 1H), 7.70 (d, J = 8.4 Hz, 1H), 7.64 (s, 1H), 7.53 (d, J = 9.6 Hz, 1H), 6.98 (d, J = 7.4Hz,1H),6.87(s,1H),4.39(d,J=9.1Hz,2H),4.22(m,1H),3.86-3.81(m,2H),3.61 -3.51(m,2H),2.20-1.96(m,3H),1.70-1.66(m,2H),1.46-1.42(m,2H),1.24(s,6H).

Compound 105: 1H NMR (500 MHz, DMSO-d 6 ) δ 10.11 (s, 1H), 8.83 (s, 1H), 8.68 (d, J = 8.6 Hz, 1H), 8.40 (dd, J = 7.6, 5.8 Hz, 1H), 7.82 (s, 1H), 7.65 (dd, J = 8.4, 5.7 Hz, 2H), 7.53 (dd, J = 10.0, 2.6 Hz, 1H), 6.98 (td, J = 7.5, 2.7 Hz, 1H), 6.89 (d, J = 8.3 Hz ,1H),6.24-5.92(m,1H),4.38(d,J = 5.4Hz,2H),4.23-4.21(m,1H),3.86-3.81(m,2H),3.61-3.51(m,2H),3.20-3.17(m,1H),2.63-2.59(m,1H),2.07-1.93(m,2H),1.78-1.63(m,2H),1.46(d,J = 12.6Hz,1H),1.37(d,J=13.6Hz,1H).

Compound 106: 1H NMR (500MHz, DMSO-d 6 ) δ10.12 (s, 1H), 8.84 (s, 1H), 8.71 (d, J = 8.5 Hz, 1H), 8.44-8.39 (m, 1H), 7.82 (s, 1H), 7.65 (dd, J = 17.2, 8.4 Hz, 2H), 7.52 (dd, J = 10.0, 2.4 Hz, 1H) ,6.98(td,J = 7.5,2.5Hz,1H),6.87(d,J = 8.3Hz,1H),4.47-4.31(m,2H),4.15(t,J = 7.1H z,1H),3.83(t,J = 10.7Hz,2H),3.65-3.40(m,4H),3.28(s,3H),3.06(dt,J = 11.8,5.9Hz, 1H),2.92-2.84(m,1H),2.57(dd,J = 12.8,7.6Hz,1H),2.22(s,1H),2.01(td,J = 12.8,4. 4Hz,1H),1.71(dd,J = 12.6,7.0Hz,2H),1.45(d,J=12.5Hz,1H),1.37(d,J=12.7Hz,1H).

Compound 107: 1H NMR (500 MHz, DMSO-d 6 ) δ 10.10 (d, J = 5.6 Hz, 1H), 8.84 (s, 1H), 8.69 (d, J = 8.6 Hz, 1H), 8.41 (dd, J = 7.6, 5.7 Hz, 1H), 7.82 (s, 1H), 7.69 (dd, J = 8.6, 4.2 Hz, 1H), 7.64 (d, J = 8.3 Hz, 1H), 7.53 (dd, J = 10.0, 2.7 Hz, 1H), 7.04-6.94 (m, 1H) ,6.88(d,J= 8.3Hz,1H),4.46-4.31(m,2H),4.19-4.17(m,1H),3.85-3.80(m,4H),3.75-3.64(m,1H),3.64-3.44(m,3H ),2.68-2.55 (m,1H), 2.24-2.22(s,1H),2.13-2.09(m,1H),2.06-1.96(m,1H),1.86-1.63(m,3H),1.51-1.35(m,2H).

Compound 108: 1H NMR (500MHz, DMSO-d 6 ) δ10.27 (s, 1H), 9.77 (s, 1H), 9.50 (s, 1H), 8.96 (s, 1H), 8.72-8.54 (m, 2H), 8.43 (s, 1H), 8.05 (d, J = 6.9 Hz, 1H), 7.81 (dd, J = 15.5, 8.5 Hz, 2H), 7.54 (s, 1H), 7.14 (d,J = 7.7Hz,1H),5.04(s,1H),4.41(q,J = 18.9Hz,2H),4.08-3.82(m,6H),3.37(d,J = 22.4Hz,2H),2.89(s,1H),2.24-1.81(m,7H),1.68(s,1H),1.57(d,J = 12.4Hz,1H),1.49(d,J=12.4Hz,1H).

Compound 109: 1H NMR (500 MHz, DMSO-d 6 ) δ 10.24 (s, 1H), 9.23 (d, J = 5.2 Hz, 1H), 9.13 (s, 1H), 8.94 (s, 1H), 8.53 (dd, J = 10.6, 7.2 Hz, 2H), 8.17 (s, 1H), 7.85 (dd, J = 14.3, 8.8 Hz, 2H), 7.75 (t, J = 11.0 Hz, 1H), 7.33 (t, J = 6.3 Hz, 1H), 7.16 (d, J = 8.5 Hz, 1H) ,4.95-4.85(m,1H),4.47-4.33(m,2H),4.29-4.20(m,1H),3.94-3.83(m,2H),3.64-3.55(m,1H),3.53-3.44(m,1H),2.88(dd,J = 13.3,8.2Hz,1H),2.21-2.07(m,3H),1.93(dd,J = 13.2,8.0Hz,1H),1.84-1.56(m,7H),1.49(d,J=13.1Hz,2H).

Compound 110: 1H NMR (500 MHz, DMSO-d 6 ) δ 10.19 (dd, J = 28.7, 18.4 Hz, 1H), 8.92 (s, 1H), 8.59 (s, 1H), 8.38 (ddd, J = 23.8, 16.1, 8.4 Hz, 1H), 8.19 (s, 1H), 7.92-7.75 (m, 3H), 7.34 (s, 1H), 7.24-7.04 (m, 1H), 4.79-4.65(m,1H),4.48-4.31(m,2H),4.17(dd,J = 29.3,8.9Hz,1H),4.00-3.74(m,4H),3.58(d,J = 27. 0Hz,2H),3.30-3.09(m,2H),3.05-2.86(m,1H),2.25-1.93(m,5H),1.64(s,1H),1.54-1.35(m,2H).

Compound 112: 1H NMR (500 MHz, DMSO-d 6 ) δ10.13 (d, J = 6.7 Hz, 1H), 8.94 (d, J = 8.5 Hz, 1H), 8.85-8.83 (m, 1H), 8.44-8.34 (m, 1H), 7.82 (d, J = 4.6 Hz, 1H), 7.66-7.62 (m, 2H), 7.53 (dd, J = 10.1, 2.6 Hz, 1H), 7.07-6.96 (m, 1H), 6.85-6.83 (m, 1H), 4.48-4.29 (m, 2H), 4.24-4. .14(m,1H),3.95-3.91(m,2H),3.85-3.81(m,2H),3.61-3.51(m,2H),2.83-3.81(m,1H),2.63(d,J = 4.5Hz,1H),2.41-2.36(m,1 H),2.32-2.21(m,2H),2.02-1.92(m,2H),1.74-1.62(m,1H),1.45(d,J=12.7Hz,1H),1.34(d,J=13.1Hz,1H),1.17-1.14(m,6H).

Compound 113: 1H NMR (500MHz, DMSO-d 6 ) δ10.82 (s, 1H), 10.25 (s, 1H), 8.93 (s, 1H), 8.51 (d, J = 5.9 Hz, 1H), 8.46 (d, J = 8.4 Hz, 1H), 8.18 (s, 1H), 7.93-7.79 (m, 2H), 7.72 (d, J = 8.5 Hz, 1H), 7.35 (s, 1H), 7.16 (d, J = 8.5 Hz, 1H), 5.07 (s, 1H ),4.48-4.33(m,2H),4.11(d,J= 11.4Hz,1H),3.99-3.10(m,7H),2.86-2.73(m,2H),2.21-2.09(m,2H),2.03-1.93(m,1H),1.69(t,J = 10.6Hz,1H),1.53(d,J=12.5Hz,1H),1.40(d,J=12.7Hz,1H),1.16(dd,J=13.7,6.1Hz,6H).

Compound 114: 1H NMR (500 MHz, DMSO-d 6 ) δ 10.25 (s, 1H), 8.93 (s, 1H), 8.69 (d, J = 8.5 Hz, 1H), 8.64 (dd, J = 7.4, 5.3 Hz, 1H), 8.33 (s, 1H), 8.00 (dd, J = 8.5, 2.4 Hz, 1H), 7.76 (dd, J = 15.9, 8.5 Hz, 2H), 7.47 (td, J = 7.5, 2.5 Hz, 1H), 7.05 (d, J = 8.4 Hz, 1H), 6.42 (t, J = 54.8 Hz, 1 H),4.91(s,1H),4.46-4.33(m,2H),3.86(dd,J = 19.9,8.0Hz,2H),3.75-3.30(m,4H),2.79(s,3H),2.69(dt,J = 27.2,13.7Hz,1H) ,2.11(td,J = 12.9,4.6Hz,1H),2.02-1.93(m,1H),1.66(td,J=12.8,4.5Hz,1H),1.49(d,J=12.3Hz,1H),1.40(d,J=12.2Hz,1H).

Compound 115: 1H NMR (500 MHz, DMSO-d 6 ) δ 10.60 (d, J = 7.9 Hz, 1H), 10.13 (s, 1H), 8.92 (s, 1H), 8.64-8.57 (m, 1H), 8.27-8.20 (m, 2H), 7.91 (d, J = 8.8 Hz, 1H), 7.85 (d, J = 8.5 Hz, 1H), 7.79 (d, J = 8.5 Hz, 1H), 7.38 (dd, J = 7.3, 5.2 Hz, 1H), 7.18 (d, J = 8.5 Hz, 1H), 5.33 (d, J = 6.2 Hz, 1H), 4.84 (q, J = 8.4 Hz, 1H), 4.70 (d, J = 8.6 Hz, 1H), 4.98 (d, J = 8.8 Hz, 1H), 4.30 (d, J = 8.9 Hz, 1H), 4.69 (d, J = 8.8 Hz, 1H), 4.83 (d, J = 8.9 Hz, 1H), 4.91 (d, J = 8.8 Hz, 1H), 4.60 (d, J = 8.9 Hz, 1H), 4.55 (d, J = 8.8 .41(q,J=18.4Hz,2H),4.17(d,J=6.2Hz,1H),4.09-3.77(m,6H),3.66(dd,J = 18.0,6.6Hz,1H),3.53(t,J = 11.9Hz,1H),3.00(dd,J = 12.6,7.7Hz,1 H),2.49-2.45(m,1H),2.32(d,J = 9.7Hz,1H),2.27-2.15(m,3H),2.00-1 .92(m,1H),1.62(td,J = 12.6,4.1Hz,1H),1.49(dd,J=26.7,12.6Hz,2H).

Compound 116: 1H NMR (500 MHz, DMSO-d 6 ) δ 10.43 (s, 1H), 10.18 (s, 1H), 8.92 (s, 1H), 8.58 (dd, J = 17.0, 11.5 Hz, 2H), 8.24 (s, 1H), 7.91 (d, J = 9.1 Hz, 1H), 7.82 (d, J = 8.2 Hz, 1H), 7.77 (s, 1H), 7.40 (t, J = 6.3 Hz, 1H), 7.13 (d, J = 7.8 Hz, 1H), 5.06 (s, 1H), 4.56 (s,2H),4.40(q,J = 18.3Hz,2H),3.88(t,J = 11.3Hz,4H),3.54(dt,J = 34.8,11.5Hz,2H), 3.39(s,1H), 3.23(s,1H), 2.80(s,1H), 2.12(s,4H),1.98(d,J=16.2Hz,2H),1.66(td,J=12.8,4.5Hz,1H),1.51(d,J=13.0Hz,1H), 1.40(d,J=13.0Hz,1H).

Compound 118: 1H NMR (500MHz, DMSO-d 6 ) δ10.20 (s, 1H), 8.96-8.85 (m, 1H), 8.70 (d, J = 8.5 Hz, 1H),

8.66-8.56 (m, 1H), 8.26 (s, 1H), 7.93 (d, J = 7.2 Hz, 1H), 7.80-7.63 (m, 2H), 7.42 (s, 1H), 6.99 (d, J = 8.2 Hz, 1H ),4.55-4.32(m,3H),3.98-3.26(m,5H),2.70(dd,J = 13.1,7.9Hz,1H), 2.11-1.93(m,3H), 1.81(dd ,J = 12.9,7.3Hz,1H),1.71(td,J = 12.9,4.5Hz,1H),1.49(d,J=12.9Hz,1H), 1.40(d,J=11.9Hz,1H).

Compound 119: 1H NMR (500 MHz, DMSO-d 6 ) δ 10.04 (s, 1H), 8.83 (s, 1H), 8.71 (d, J = 8.5 Hz, 1H), 8.45 (dd, J = 7.6, 5.7 Hz, 1H), 7.83 (s, 1H), 7.73 (dd, J = 12.3, 8.6 Hz, 2H), 7.53 (dd, J = 10.0, 2.7 Hz, 1H), 6.97 (td, J = 7.5, 2.7 Hz, 1H z,1H),6.84(d,J = 8.5Hz,1H),4.39(s,2H),3.77-3.72(m,2H),3.69(s,2H),3.61(t,J = 11.8Hz,2H),2.99 -2.92(m,1H),2.79(s,2H),1.91(td,J = 13.1,5.0Hz,2H), 1.58(d,J = 13.2Hz,2H),1.10(d,J = 6.5Hz,6H).

Compound 121: 1H NMR (500MHz, DMSO-d 6 ) δ10.02 (s, 1H), 8.81 (s, 1H), 8.68 (d, J = 8.5 Hz, 1H), 8.30 (d, J = 7.1 Hz, 1H), 7.78-7.72 (m, 2H), 7.70 (d, J = 8.6 Hz, 1H), 7.42 (s, 1H), 6.84 (d, J = 8.5Hz,1H),6.78(d,J=7.2Hz,1H),4.38(s,2H),3.86(s,2H),3.76-3.71(m,2H),3.57( t,J= 12.0Hz,2H),3.08(s,2H),2.38(s,3H),1.95-1.87(m,2H),1.58(d,J=13.3Hz,2H).

Trifluoroacetate salt of compound 122: 1H NMR (500 MHz, DMSO-d 6 ) δ 10.25 (s, 1H), 9.26 (s, 2H), 8.92 (s, 1H), 8.73 (d, J = 8.5 Hz, 1H), 8.49 (d, J = 7.3 Hz, 1H), 8.08 (s, 1H), 8.01 (d, J = 2.2 Hz, 1H), 7.94 (d, J = 8.7 Hz, 1H), 7 .76(d,J=8.5Hz,1H),7.18(dd,J=7.3,2.1Hz,1H),7.06(d,J = 8.7Hz,1H),4.41(s,2H),4.2 9(s,2H),3.82-3.78(m,2H),3.66-3.59(m,4H),2.05-1.98(m,2H),1.69(d,J=13.6Hz,2H).

Trifluoroacetate salt of compound 124: 1H NMR (500MHz, Methanol-d 4 ) δ8.93 (d, J = 8.5 Hz, 1H), 8.58 (s, 1H), 8.14 (s, 1H), 7.98-7.91 (m, 1H), 7.77 (dd, J = 6.7, 3.9 Hz, 2H), 7.37 (dd, J = 8.4, 6.3 Hz, 1H), 7.06 (dd,J = 8.9,4.8Hz,1H),4.51(s,2H),4.42(s,2H),3.96-3.92(m,2H),3.77-3.71(m, 2H),3.60-3.49(m,2H),2.26-2.17(m,2H),1.53(t,J=7.2Hz,3H),1.35-1.27(m,2H).

Compound 125: 1H NMR (500 MHz, DMSO-d 6 ) δ 10.04 (s, 1H), 8.83 (s, 1H), 8.70 (d, J = 8.5 Hz, 1H), 8.45 (t, J = 6.7 Hz, 1H), 7.92 (d, J = 8.5 Hz, 1H), 7.82 (s, 1H), 7.72 (d, J = 8.5 Hz, 1H), 7.5 3(td,J = 10.1,3.2Hz,1H),7.48(s,1H),6.96(td,J = 7.5,2.7Hz,1H),6.89(d,J = 8.4H z,1H),4.39(s,2H),3.85(s,2H),3.03(s,2H),2.27-2.20(m,2H),2.02-1.90(m,4H).

Compound 127: 1H NMR(500MHz,DMSO-d 6 )δ10.01(s,1H),8.83(s,1H),8.71(d,J = 8.6Hz,1H) ,8.45(dd,J = 7.7,5.7Hz,1H),7.82(s,1H),7.72(d,J = 8.5Hz,1H),7.62(dJ = 8.5Hz ,1H),7.52(dd,J = 10.0,2.7Hz,1H),6.96(td,J = 7.5,2.7Hz,1H),6.84(d,J = 8.5Hz, 1H),4.39(s,2H),3.87(s,2H),2.72(s,2H),1.82-1.76(m,4H),1.71-1.67(m,4H).

Trifluoroacetate salt of compound 145: ESI-MS m/z: 527.3 [M+H] +; 1H NMR (500MHz, DMSO) δ10.18(s,1H),8.92(s,1H),8.77(dd,J = 36.3,28.4Hz,2H),8.28(s,1H),7.94(s,1H),7.83(dd,J = 31.6,8.5Hz,2H),7.41(s ,1H),6.94(s,1H),4.68(d,J = 36.2Hz,2H),4.41(s,2H),3.85(s,3H),3. 66(s,3H),2.16(d,J=9.3Hz,3H),1.99-1.90(m,2H),1.46-1.36(m,2H).

Trifluoroacetate salt of compound 148: 1H NMR (500 MHz, DMSO-d 6 ) δ 10.25 (s, 1H), 9.33 (s, 2H), 8.96 (s, 1H), 8.75 (d, J = 8.5 Hz, 1H), 8.47 (d, J = 7.6 Hz, 1H), 8.19 (s, 1H), 7.95 (d, J = 8.7 Hz, 1H), 7.79 (d, J = 8 .6Hz,1H),7.35(s,1H),7.07(d,J=8.4Hz,2H), 4.43(s,2H), 4.29(s,2H), 4.01(s,3H ),3.83-3.77(m,2H),3.67-3.61(m,4H),2.05-1.96(m,2H),1.69(d,J = 13.6Hz,2H).

Compound 150: 1H NMR (500 MHz, DMSO-d 6 ) δ 10.04 (s, 1H), 8.83 (s, 1H), 8.68 (dd, J = 11.4, 7.8 Hz, 2H), 7.87 (s, 1H), 7.82 (d, J = 9.9 Hz, 1H), 7.76 (t, J = 8.1 Hz, 2H), 6.85 (d, J = 8.5 Hz, 1H), 4.37 (s, 2H), 3.86 (s, 2H), 3.77-3.70 (m, 2H), 3.57 (t, J = 12.1 Hz, 2H), 3.08 (s, 2H), 1.94-1.86 (m, 2H), 1.58 (d, J = 13.3 Hz, 2H).

Compound 151: 1H NMR (500MHz, DMSO) δ10.31 (d, J = 19.7 Hz, 2H), 8.96 (s, 1H), 8.66 (d, J = 8.6 Hz, 1H), 8.48 (d, J = 7.4 Hz, 1H), 8.10 (d, J = 33.9 Hz, 2H), 7.84-7.72 (m, 2H), 7.25 (d, J = 7.1 Hz, 1H), 7.14 (dd, J = 8.6, 2.9 Hz, 1H), 5 .20-5.07(m,1H),4.42(s,2H),4.11-3.33(m,4H),2.96(d,J = 4.6Hz,3H),2.83(t,J = 4.1Hz,3H),2.58 -2.55(m,1H),2.35(ddd,J = 30.2,14.0,8.0Hz,2H),2.13(dd,J=12.0,6.7Hz,1H),2.06-1.91(m,1H).

Trifluoroacetate salt of compound 153: 1H NMR(500MHz,DMSO-d 6 )δ10.23(s,1H), 9.31(s,2H),8.98(s,1H),8.70( d,J=8.6Hz,1H),7.95-7.92(m,2H),7.74(d,J=8.6Hz,1H), 7.05(d,J= 8.7Hz,1H), 5.11(s,2H),4.46(s,2H),4.28(d,J = 4.9Hz,2H),4.15-4.09(m,4H),3.81-3.76(m ,2H),3.67-3.59(m,4H),2.04-1.97(m,2H),1.99(s,1H),1.69(d,J=13.6Hz,2H).

Compound 154: 1H NMR (500 MHz, DMSO-d 6 ) δ 9.95 (s, 1H), 8.79 (s, 1H), 8.54 (d, J = 8.5 Hz, 1H), 7.74 (d, J = 8.6 Hz, 1H), 7.70 (s, 1H), 7.43 (d, J = 8.5 Hz, 1H), 6.81 (d, J = 8.5 Hz, 1H), 4.44 (s, 2H), 4.11 (t, J =6.1Hz,2H),3.89(s,2H),3.76-3.71(m,2H),3.57(t,J=12.0Hz,2H), 3.14(s,2H),2.85(t,J = 6.3Hz,2H),2.02-1.98(m,2H),1.95-1.88(m,2H),1.83-1.77(m,2H),1.58(d,J =13.3Hz,2H).

Trifluoroacetate salt of compound 167: 1H NMR (500 MHz, DMSO-d 6 ) δ 10.21 (s, 1H), 9.39 (s, 2H), 8.96 (s, 1H), 8.69 (d, J = 8.6 Hz, 1H), 7.94 (d, J = 8.8 Hz, 1H), 7.85 (s, 1H), 7.67 (d, J = 8.6 Hz, 1H), 7.05 (d, J = 8.7 Hz, 1H ),4.43(s,2H),4.28(d,J = 4.9Hz,2H),3.97(d,J = 5.7Hz,2H),3.79(dd,J = 11.5,4.7Hz,2H ),3.67-3.61(m,3H),3.62(d,J = 11.2Hz,1H),2.06-1.91(m,6H),1.69(d,J=13.5Hz,2H).

Compound 173: 1H NMR (500MHz, DMSO-d 6 ) δ10.30 (s, 1H), 9.92 (s, 1H), 8.98 (s, 1H), 8.67 (d, J = 8.5 Hz, 1H), 8.61-8.51 (m, 1H), 8.21 (s, 1H), 8.05 (d, J = 8.6 Hz, 1H), 7.89 (d, J = 9.1 Hz, 1H), 7.78 (d, J = 8.5 Hz, 1H), 7.41-7.27 (m, 2H), 4.54-4.37 (m, 3H), 4.05 (dd, J = 14.0, 3.2 Hz, 1H),2.87(m,6H),2.65(d,J=10.6Hz,1H),2.39-2.30(m,2H),2.25-2.18(m,2H),2.13-1.93(m,2H).

Trifluoroacetate salt of compound 176: 1H NMR (500 MHz, DMSO) δ10.44 (s, 1H), 10.23 (s, 1H), 8.95 (s, 1H), 8.56 (d, J = 7.3 Hz, 1H), 8.52 (d, J = 8.3 Hz, 1H), 8.26 (s, 1H), 7.85 (d, J = 8.5 Hz, 1H), 7.80-7.49 (m, 3H), 7.27 (dd, J = 7.5, 2.1

Hz, 1H), 7.15 (d, J = 8.5 Hz, 1H), 5.16 (s,1H),5.05(d,J = 47.7Hz,1H),4.47-4.35(m,2H),4.09-3.66(m,4H),3.53(ddd,J = 153.4, 76.4, 65.3Hz, 4H), 2.87-2.77(m,1H),2.14(dt,J = 61.9,31.9Hz,6H),1.68(td,J = 12.9,4.3Hz,1H),1.49(dd,J= 53.7,12.6Hz,2H).

Trifluoroacetate salt of compound 179: 1H NMR (500 MHz, DMSO) δ10.24 (s, 1H), 9.04 (s, 2H), 8.96 (s, 1H), 8.58 (d, J = 7.5 Hz, 1H), 8.53 (d, J = 8.5 Hz, 1H), 8.27 (s, 1H), 7.84 (d, J = 8.5 Hz, 1H), 7.81-7.49 (m, 3H), 7.28 (dd, J = 7.5, 2.2 Hz, 1H), 7.16 (d, J = 8.5 Hz, 1H), 5.07-4.96 (m, 1H), 4.41 (q, J = 18.3 Hz, 2H), 4.02 (dt, J = 12.4, 6.2 Hz,1H),3.94-3.82(m,2H),3.61(t,J = 11.2Hz,1H),3.51(t,J = 11.4Hz,1H),2.92-2.83(m,1H),2.17(td,J = 13.0,4.6Hz,1H),1.90(dd,J = 13.0,8.4Hz,1H),1.68(td,J = 12.7,4.2Hz,1H),1.53(d,J = 13.1Hz,1H),1.48(d,J = 12.4Hz,1H), 1.43(d,J=6.4Hz,3H),1.39(d,J=6.5Hz,3H).

Comparative compound D1:

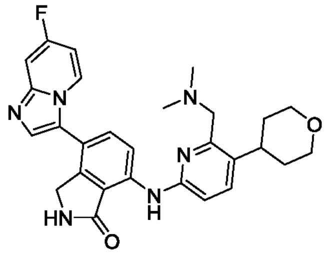

The synthesis of the comparative compound refers to the specific synthesis steps of compound I-792 in patent WO2021050964.

**Biological tests**

Pharmacological experiment 1: HPK1 enzyme activity detection (1mM ATP system)

(1) Take ADP-GloTMKinase Assay Kit (Promega, Cat.No. V9102) to prepare 4× kinase buffer.
(2) Compound gradient dilution: The compound to be tested is diluted 3-fold, and 11 gradient concentrations are set, and each concentration is set for duplicate well detection. Serial dilutions were made in a 384-well plate to the corresponding 100× final concentration solution, and then 0.1 μL was transferred to the 384-well reaction plate for testing using Echo. Transfer 0.1 μL of 100% DMSO to the smallest and largest wells.
(3) Prepare HPK1 enzyme working solution using 4× kinase buffer.
(4) Add 5 μL of HPK1 enzyme working solution to each well and 5 μL of 1× kinase buffer to the smallest well. Centrifuge at 1000 rpm for 1 min and incubate at 25°C for 15 min.
(5) During the incubation period, prepare the substrate working solution using 4× kinase buffer.
(6) Add 5 μL of substrate working solution containing 1 mM ATP to each well of the reaction plate, centrifuge at 1000 rpm for 1 min, and incubate at 25°C for 60 min.
(7) At the end of incubation, add 5 μL of ADP Glo reagent to each well. Centrifuge at 1000 rpm for 1 min and incubate at 25°C for 60 min.
(8) Add 10 μL of detection solution to each well, centrifuge at 1000 rpm for 1 min, and incubate at 25°C for 60 min.
(9) Read using EnVision.

Inhibition rate calculation formula:

$$\% \text{ Inhibition} = \frac{\text{Vehicle} - \text{Compound}}{\text{Vehicle} - \text{Blank}} \text{ X } 100\%$$

Vehicle: Reading value of DMSO control well
Blank: No enzyme well reading
Largest well: Reading value of DMSO control well
Minimum well: No enzyme well reading

The log value of the concentration was used as the X-axis and the percentage inhibition rate was used as the Y-axis. The "log (inhibitor) vs. response--variable slope" of the analysis software GraphPad Prism 5 was used to fit the dose-effect curve to obtain the IC 50 value of the compound's inhibition of kinase binding. See Table 1 for the enzymatic IC 50 data of the example compounds on HPK1.

Table 1

| Compound | $IC_{50}$ (nM) |
|---|---|
| 1 | 61.22 |
| 2 | 7.01 |
| 9 | 204.70 |
| 14 | 54.82 |
| 15 | 6.14 |
| 16 | 20.56 |

Pharmacological experiment 2: HPK1 enzyme activity detection (1mM ATP system)

(1) Prepare the detection buffer: add 50 mM 4-(2-hydroxyethyl)-1-piperazineethanesulfonic acid (HEPES), 10 mM magnesium chloride, 1 mM ethylene glycol bis(2-aminoethyl ether) tetraacetic acid (EGTA), 2 mM dithiothreitol (DTT) and 0.01% NER1570 to deionized water and mix well.
(2) Prepare 2×ATP-MBP and 2×HPK1 solutions: Use detection buffer to prepare a mixed solution of 2 mM ATP (V915B, Promega) and 0.1 mg/mL MBP (M42-51N, Signalchem) as the 2×ATP-MBP solution; use detection buffer to prepare a 20 nM HPK1 solution as the 2×HPK1 solution.
(3) Compound dilution and transfer: The test compound was serially diluted using DMSO to 200 times the detection concentration.
20 nL of compound was transferred to a 384-well plate using an Echo 665 (784075, Greiner).
(4) Incubation of compounds with HPK1: Transfer 2 μL of 2×HPK1 solution to a 384-well plate.
Centrifuge at 1000 × g for 30 seconds and incubate at 25°C for 15 minutes.
(5) Adding ATP and substrate: Add 2 μL of 2× ATP-MBP solution to the 384-well plate and centrifuge at 1000×g for 30 seconds.
Seal the plate with a sealing film and incubate at 25°C for 60 minutes.
(6) Add ADP-Glo reagent: Add 4 μL ADP-Glo reagent (Promega, V9103) to the 384-well plate, centrifuge at 1000 × g for 30 seconds, and incubate at 25°C for 60 minutes.
(7) Detection of luminescent signal: Add 8 μL of luminescent detection reagent (Promega, V9103) to a 384-well plate, centrifuge at 1000 × g for 30 seconds, and incubate at 25° C. for 60 minutes.
The chemiluminescent signal was read using a BMG instrument.
(8) Calculation of percentage inhibition rate: Percent inhibition rate (%) = 100% - (compound - positive control) / (negative control - positive control) * 100% Wherein, the positive control is 3 μM comparative compound D1, and the negative control is 0.5% DMSO.
(9) $IC_{50}$ calculation: With the log value of the test concentration as the X-axis and the percentage inhibition rate as the Y-axis, the "log (inhibitor) vs. response - variable slope" equation of the analysis software GraphPad Prism 7 was used to fit the dose-effect curve to obtain the $IC_{50}$ value of the compound's inhibition of kinase binding.

See Table 2 for the enzymatic IC 50 data of the example compounds on HPK1. Pharmacological experiment 3: GLK kinase assay (1mM ATP system)

(1) Prepare the detection buffer: add 50 mM 4-(2-hydroxyethyl)-1-piperazineethanesulfonic acid (HEPES), 10 mM magnesium chloride, 1 mM ethylene glycol bis(2-aminoethyl ether) tetraacetic acid (EGTA), 2 mM dithiothreitol (DTT) and 0.01% NER1574 to deionized water and mix well.
(2) Prepare 2×ATP-MBP and 2×GLK solutions: Use detection buffer to prepare a mixed solution of 2 mM ATP (V915B, Promega) and 0.2 mg/mL MBP (M42-51N, Signalchem) as the 2×ATP-MBP solution; use detection buffer to prepare a 100 nM GLK solution as the 2×GLK solution.
(3) Compound dilution and transfer: The test compound was serially diluted using DMSO to 200 times the detection concentration.
20 nL of compound was transferred to a 384-well plate using an Echo 665 (784075, Greiner).
(4) Incubation of compounds with GLK: Transfer 2 μL of 2×GLK solution to a 384-well plate. Centrifuge at 1000 × g for

30 seconds and incubate at 25°C for 15 minutes.
(5) Add ATP and substrate: Add 2 μL of 2× ATP-MBP solution to the 384-well plate and centrifuge at 1000 × g for 30 seconds.
Seal the plate with a sealing film and incubate at 25°C for 60 minutes.
(6) Add ADP-Glo reagent: Add 4 μL ADP-Glo reagent (Promega, V9103) to the 384-well plate, centrifuge at 1000 × g for 30 seconds, and incubate at 25°C for 60 minutes.
(7) Detection of luminescent signal: Add 8 μL of luminescent detection reagent (Promega, V9103) to a 384-well plate, centrifuge at 1000 × g for 30 seconds, and incubate at 25° C. for 60 minutes.
The chemiluminescent signal was read using a BMG microplate reader.
(8)

Calculation of percentage inhibition rate:

Percent inhibition rate (%) = 100% - (compound - positive control) / (negative control - positive control) * 100%

The positive control was 30 μM of the comparative compound D1, and the negative control was 0.5% DMSO.
(9) $IC_{50}$ calculation: With the log value of the test concentration as the X-axis and the percentage inhibition rate as the Y-axis, the "log (inhibitor) vs. response-variable slope" equation of the analysis software GraphPad Prism 7 was used to fit the dose-effect curve to obtain the $IC_{50}$ value of the compound's inhibition of kinase binding.

See Table 2 for the GLK kinase $IC_{50}$ data of the example compounds on HPK1.

Table 2

| Compound | HPK1 $IC_{50}$ (nM) | GLK $IC_{50}$ (nM) |
|---|---|---|
| 2 | 11.2 | 1169.5 |
| 2B | 1.022 | 464.3 |
| 3 | 4.16 | 2855 |
| 28 | 1.267 | / |
| 29 | 1.984 | 9.77 |
| 30 | 4.22 | / |
| 31 | 4.53 | / |
| 32 | 4.52 | 596 |
| 33 | 4.76 | 631 |
| 34 | 8.18 | 779 |
| 35 | 3.70 | / |
| 36 | 2.735 | 78 |
| 37 | 2.254 | 157.7 |
| 38 | 2.915 | 231.5 |
| 39 | 2.78 | 88.17 |
| 40 | 4.8 | / |
| 41 | 3.762 | 70.7 |
| 42 | 4.617 | 119 |
| 43 | 4.22 | 656.7 |
| 44 | 3.046 | 378.2 |
| 45 | 3.076 | / |
| 46 | 11.81 | 970.7 |
| 47 | 1.515 | 30.3 |
| 48 | 2.061 | 8.81 |

(continued)

| Compound | HPK1 $IC_{50}$ (nM) | GLK $IC_{50}$ (nM) |
|---|---|---|
| 49 | 4.44 | 337.5 |
| 50 | 3.39 | 1230 |
| 55 | 7.56 | 2150 |
| 58 | 5.25 | 1481.0 |
| 59 | 6.09 | >30000 |
| 60 | 2.14 | 122.8 |
| 61 | 7.7 | >30000 |
| 62 | 10.21 | >30000 |
| 67 | 2.49 | 135.9 |
| 70 | 2.99 | 188.2 |
| 71 | 3.27 | 293 |
| 72 | 142.7 | / |
| 73 | 3.48 | 205.5 |
| 74 | 3.02 | 299.1 |
| 75 | 2.78 | 272.6 |
| 76 | 3.55 | 286.3 |
| 85 | 5.19 | 830.6 |
| 86 | 2.61 | 158.1 |
| 87 | 5.12 | 724.1 |
| 88 | 5.29 | 207.9 |
| 90 | 5.58 | 597.6 |
| 91 | 86.21 | >30000 |
| 92 | 75.23 | / |
| 93 | 27.02 | >25000 |
| 94 | 9.27 | 4530 |
| 95 | 6.36 | / |
| 96 | 72.47 | / |
| 97 | 7.5 | / |
| 98 | 12.43 | >30000 |
| 99 | 171.2 | / |
| 100 | 3.10 | / |
| 101 | 5.22 | 857.9 |
| 102 | 7.42 | >30000 |
| 103 | 4.67 | 290.9 |
| 104 | 3.49 | 551.5 |
| 105 | 5.26 | / |
| 106 | 3.46 | 696.7 |
| 107 | 3.55 | 1203 |
| 108 | 3.29 | 1054 |

(continued)

| Compound | HPK1 $IC_{50}$ (nM) | GLK $IC_{50}$ (nM) |
|---|---|---|
| 109 | 5.97 | 790.7 |
| 110 | 2.67 | 317 |
| 111 | 30.17 | / |
| 112 | 48.72 | / |
| 113 | 6.21 | / |
| 114 | 92.46 | / |
| 115 | 7.15 | / |
| 116 | 79.79 | / |
| 117 | 5.04 | 561.6 |
| 118 | 71.06 | / |
| 119 | 29.76 | >3000 |
| 120 | 11.01 | >3000 |
| 121 | 3.35 | 679.30 |
| 122 | 3.21 | 455.40 |
| 123 | 12.13 | 896.1 |
| 124 | 8.47 | 2763.0 |
| 125 | 11.10 | 1196.0 |
| 127 | 5.23 | 767.10 |
| 141 | 3.07 | / |
| 142 | 4.52 | / |
| 143 | 3.51 | / |
| 144 | 7.42 | >3000 |
| 145 | 38.07 | / |
| 146 | 4.33 | 1226.00 |
| 147 | 21.98 | / |
| 148 | 21.76 | / |
| 149 | 4.42 | / |
| 150 | 30.42 | / |
| 151 | 6.34 | 970.10 |
| 152 | 14.37 | / |
| 153 | 34.55 | 2170.00 |
| 154 | 10.90 | 645.60 |
| 155 | 243.00 | / |
| 156 | 38.70 | / |
| 157 | 5.90 | 876.80 |
| 158 | 9.89 | 287.9 |
| 158A | 3.00 | 205.90 |
| 159 | 5.41 | / |
| 160 | 6.57 | 460.30 |

(continued)

| Compound | HPK1 $IC_{50}$ (nM) | GLK $IC_{50}$ (nM) |
|---|---|---|
| 161 | 3.57 | 310.90 |
| 162 | 18.62 | / |
| 163 | 25.28 | / |
| 164 | 10.62 | 104.8 |
| 165 | 24.87 | 1194 |
| 166 | 25.71 | / |
| 167 | 25.30 | 1373 |
| 168 | 5.27 | 288.6 |
| 169 | 9.19 | 222.8 |
| 170 | 6.88 | / |
| 171 | 34.98 | / |
| 172 | 13.81 | 1125.0 |
| 173 | 8.47 | 3196.0 |
| 174 | 19.22 | 566.4 |
| 175 | 16.84 | / |
| 176 | 12.06 | / |
| 177 | 101.6 | / |
| 178 | 13.68 | 1239 |
| 179 | 8.85 | 970.5 |
| 180 | 13.92 | 3910 |

Pharmacological experiment 4: Detection of IFN-γ secretion in human peripheral blood monocytes

(1) Take human peripheral blood mononuclear cells frozen in liquid nitrogen, thaw in a 37°C water bath, add 5 mL of preheated RPMI-1640 complete medium (containing 10% fetal bovine serum and penicillin/streptomycin), centrifuge at 500 g for 10 minutes, and discard the supernatant;
(2) Resuspend the cells in RPMI-1640 complete medium, adjust the density to 5 × 106 cells/mL, inoculate in a low-adhesion 6-well plate (Corning, 3471), and culture at 37°C for 2 days;
(3) Collect cultured human peripheral blood mononuclear cells, centrifuge at 500 g for 10 min, and discard the supernatant;
(4) Resuspend the cells in RPMI-1640 complete medium, adjust the density to 1×106cells/mL, and inoculate them in a round-bottom 96-well plate (Jet Biofil, TCP-002-096), with 100 μL per well;
(5) Add 50 μL of the test compound to each well and incubate at 37°C for 30 min;
(6) Take human anti-CD3/CD28 magnetic beads (Miltenyi, 130-091-441), wash once with RPMI-1640 complete medium, and adjust the density to 0.5×106beads/mL for later use;
(7) After the compound incubation, 50 μL of anti-human CD3/CD28 magnetic beads were added to each well and cultured at 37°C for 3 days;
(8) The cell culture supernatant was obtained and the IFN-γ secretion was detected using the Human IFN-gamma DuoSet ELISA kit (R&D, DY285B-05);
(9) The log value of concentration was used as the X-axis and the IFN-γ secretion amount was used as the Y-axis. The "log (agonist) vs. response - variable slope" equation of the analysis software GraphPad Prism 7 was used to fit the dose-effect curve, thereby obtaining the $EC_{3x}$ value and the maximum induction factor of the compound for the secretion of IFN-γ from human peripheral blood mononuclear cells to reach 3 times the baseline. The results are shown in Table 3.

Table 3

| Compound | $EC_{3x}$ (nM) | Maximum induction factor |
|---|---|---|
| 2 | 69 | 5.9 |
| 2B | 22 | 7.7 |
| 158A | 167 | 10.8 |
| 50 | 181 | 4.8 |
| 75 | 119 | 10.5 |
| 103 | 123 | 5.3 |
| 115 | 225 | 9.6 |
| 87 | 193 | 11.2 |
| 71 | 76 | 16.6 |
| 49 | 152 | 10.7 |
| 105 | 58 | 14.1 |
| 107 | 296 | 8.8 |
| 62 | 241 | 4.9 |
| 75 | 64 | 8.3 |
| 49 | 294 | 5.7 |
| 121 | 140 | 19.4 |
| 127 | 74.6 | 19.1 |
| 125 | 173 | 20.2 |
| 123 | 214 | 26.8 |
| 122 | 56 | 16.2 |
| 58 | 352 | 6.5 |
| 158 | 123 | 9.4 |
| 164 | 458 | 9.2 |
| 151 | 53 | 6.5 |
| 161 | 67 | 6.4 |
| D1 | 528 | 4.6 |

IFN-γ is one of the key cytokines for immune cells to exert anti-tumor effects. The experimental results show that the compound of the present invention can effectively increase the IFN-γ secretion level of human peripheral blood mononuclear cells in vitro and has the activity of promoting immune cells to kill tumors. And compared with the control compound D1, the compound of the present invention has advantages in the induction concentration ($EC_{3x}$) and induction multiple of IFN-γ secretion level.

Pharmacological experiment 5: Jurkat IL-2 secretion detection

(1) Take the cultured Jurkat cells, adjust the density to 1×106cells/mL, and inoculate them in a flat-bottom 96-well plate (Jet Biofil, TCP011096), with 100 μL per well;
(2) Add 50 μL of the test compound to each well and incubate at 37°C for 30 min;
(3) Take human anti-CD3/CD28 magnetic beads (Miltenyi, 130-091-441), wash once with RPMI-1640 complete medium, and adjust the density to 4×106beads/mL for later use;
(4) After the compound incubation, 50 μL of anti-human CD3/CD28 magnetic beads were added to each well and cultured at 37°C for 20 h;
(5) The cell culture supernatant was obtained and the IL-2 secretion was detected using the Human IL-2 DuoSet ELISA kit (R&D, DY008);
(6) The log value of the concentration was used as the X-axis and the IL-2 secretion was used as the Y-axis. The "log

(agonist) vs. response - variable slope" equation of the analysis software GraphPad Prism 7 was used to fit the dose-effect curve, thereby obtaining the $EC_{3x}$ value and the maximum induction factor of the compound for IL-2 secretion from human peripheral blood mononuclear cells to reach 3 times the baseline. The results are shown in Table 4.

Table 4

| Compound | $EC_{3x}$ (nM) | Maximum induction factor |
|---|---|---|
| 2 | 345 | 10 |
| 2B | 90.7 | 10.9 |
| 104 | 172 | 8.3 |
| 108 | 448 | 8.4 |
| 40 | 163 | 11.5 |
| D1 | 544 | 8.1 |

IL-2 is a biomarker of T cell activation and an essential cytokine for T cell proliferation. The experimental results show that the compound of the present invention can effectively increase the IL-2 secretion level of Jurkat cells in vitro and has the activity of promoting T cell activation and proliferation. And compared with the control compound D1, the compound of the present invention has advantages in the induction concentration ($EC_{3x}$) and induction multiple at the IL-2 secretion level.

Pharmacological experiment 6: PK study of compound in dogs

The compound was mixed with a solvent of saline or 10% DMSO/5% Solutol/85% saline or 10% DMSO/10% Solutol/80% saline (specific solvents are shown in the table below), vortexed and sonicated to prepare a clear oral solution. Beagle dogs were orally administered with control compound D1, compound 2B and 158A, respectively, and plasma samples were collected at 15 min, 30 min, 1 h, 2 h, 4 h, 8 h, 12 h, and 24 h. The drug concentration was analyzed by LC-MS/MS method, and the pharmacokinetic parameters were calculated using Phoenix WinNolin software (Pharsight, USA).

The dosage and experimental scheme are shown in Table 5 below:

Table 5

| Group | Example | Route | Dose (mg/kg) | Formulation |
|---|---|---|---|---|
| G1 | D1 | PO | 10 | Saline |
| G2 | 2B | PO | 5 | 10%DMSO/5%Solutol/85% Saline |
| G3 | 158A | PO | 5 | 10%DMSO/10%Solutol/80% Saline |

The PK results of the tested dogs are shown in Table 6:

Table 6

| Group | Example | $T_{1/2}$ (h) | PO Plasma AUC (h*ng/ml) | Bioavailability F% |
|---|---|---|---|---|
| G1 | D1 | 3.19 | 396 | 11.6% |
| G2 | 2B | 1.65 | 1082 | 48.4% |
| G3 | 158A | 6.06 | 2704 | 43% |

It can be seen from the PK results in Table 6 that, in beagle dogs, compared with the control compound D1, the oral exposure and bioavailability of the compound of the present invention are significantly improved.

Pharmacological experiment 7: PK study of compound in monkeys

The compounds were mixed with the vehicle 10% DMSO/5% Solutol/85% saline, vortexed and sonicated to prepare clear intravenous and oral solutions. Crab-eating monkeys were intravenously and orally administered with 1 mg/kg and 5 mg/kg of control compound D1 and compound 2B, respectively, and plasma samples were collected intravenously at 5min, 15min, 30min, 1h, 2h, 4h, 8h, 12h, and 24h, and plasma samples were collected orally at 15min,

30min, 1h, 2h, 4h, 8h, 12h, and 24h. The drug concentration was analyzed by LC-MS/MS method, and the pharmacokinetic parameters were calculated using Phoenix WinNolin software (Pharsight, USA).

The dosage and experimental scheme are shown in Table 7 below:

Table 7

| Group | Example | Rout | Dose (mg/kg) | Formulation |
|---|---|---|---|---|
| G1 | D1 | IV | 1 | 10%DMSO/5%Solutol/85% Saline |
| | | PO | 5 | |
| G2 | 2B | IV | 1 | |
| | | PO | 5 | |

The PK results of the monkeys tested are shown in Table 8:

Table 8

| Group | Example | IV Plasma C1_obs (mL/min/kg) | $T_{1/2}$ (h) | PO Plasma AUC (h*ng/ml) | Bioavailability F% |
|---|---|---|---|---|---|
| G1 | D1 | 17.6 | 4.01 | 1722 | 38.2% |
| G2 | 2B | 6.16 | 8.81 | 6302 | 50.2% |

It can be seen from the PK results in Table 8 that, in monkeys, the compounds of the present invention have the advantages of slower clearance rate, higher oral exposure and better oral absorption compared with the control compound D1.

Pharmacological experiment 8: Study on the efficacy of the compound in CT26CDX model mice

A subcutaneous homologous transplant tumor model of mouse colon cancer cell CT26 was constructed. 2.5*105CT26 cells were inoculated subcutaneously into the right hind limb of each Balb/c mouse, with 8 tumor-bearing mice in each group.

The first group (G1) was a vehicle control group (10% propylene glycol/5% HS15/85% glucose).

The second group (G2) was the control compound D1-administered group.

The third to fifth groups (G3-G5) were the groups administered with compound 2B.

The dosage and experimental schedule are shown in Table 9 below.

Table 9

| Group | Number | Example | Dose (mg/kg) | Concentration (mg/ml) | Volume (ml/kg) | Route |
|---|---|---|---|---|---|---|
| G1 | 8 | Vehicle | (N/A) | (N/A) | 10 | PO, QD 0-21 day |
| G2 | 8 | D1 | 75 | 7.5 | 10 | PO, QD 0-21 day |
| G3 | 8 | 2B | 25 | 2.5 | 10 | PO, QD 0-21 day |
| G4 | 8 | | 75 | 7.5 | 10 | PO, QD 0-21 day |
| G5 | 8 | | 75 | 7.5 | 10 | PO, BID 0-21 day |

Note: PO stands for oral gavage, QD stands for once daily dosing, and BID stands for twice daily dosing.

Tumor size and mouse body weight were measured twice a week during the experiment, and the weight of the animals on that day was used as a reference for each drug administration.

The tumor size was measured by using a vernier caliper to measure the length (a) and width (b) of the tumor. The tumor volume (TV) was calculated using the following formula: TV = a*b2/2

After 21 days of administration, the tumor growth inhibition rate (TGI) of each administration group is shown in Table 10.

Table 10

| Group | Example | TGI |
|---|---|---|
| G1 | Vehicle | / |

(continued)

| Group | Example | TGI |
|---|---|---|
| G2 | D1 | 38% |
| G3 | 2B | 59% |
| G4 | | 70% |
| G5 | | 90% |

It can be seen from the efficacy data in Table 10 that, compared with the control compound D1, the compound of the present invention has a higher TGI and better oral efficacy.

Although the present invention has been fully described by way of embodiments thereof, it is to be noted that various changes and modifications will be apparent to those skilled in the art. Such changes and modifications are intended to fall within the scope of the appended claims.

## Claims

1. A compound represented by general formula (I), or a stereoisomer, tautomer, deuterated substance or pharmaceutically acceptable salt thereof:

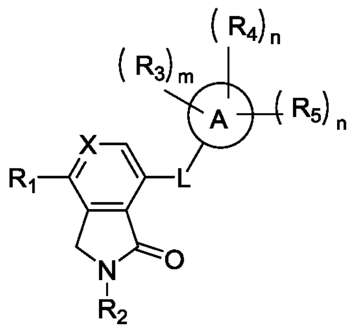

(I)

wherein,

ring A is selected from the group consisting of $C_{5-14}$ bicarbocyclyl, 5-14 membered biheterocyclyl, $C_{10-18}$ bicyclic aryl and 10-18 membered bicyclic heteroaryl;
L is selected from the group consisting of a bond, NH, O and S;
$R_1$ is selected from the group consisting of H, halogen, $C_{1-6}$ alkyl, $C_{1-6}$ alkoxy, haloalkyl, hydroxyalkyl, $C_{2-6}$ alkenyl, $C_{2-6}$ alkynyl, $C_{3-14}$ cycloalkyl, 3-14 membered heterocyclyl, $C_{6-18}$ aryl and 6-18 membered heteroaryl; wherein the $C_{1-6}$ alkyl, $C_{1-6}$ alkoxy, haloalkyl, hydroxyalkyl, $C_{3-14}$ cycloalkyl, 3-14 membered heterocyclyl, $C_{6-18}$ aryl or 6-18 membered heteroaryl can be optionally substituted with one or more $R_a$; $R_a$ is independently selected from the group consisting of H, hydroxyl, cyano, halogen, $C_{1-6}$ alkyl, $C_{1-6}$ haloalkyl, $-C_{0-3}$ alkylene-$OR_b$, -OC(=O) $C_{1-6}$ alkyl, $-C_{0-3}$ alkylene-$SR_b$, $-C_{0-3}$ alkylene-$N(R_b)_2$, $-C_{0-3}$ alkylene-$S(=O)R_b$, $-C_{0-3}$ alkylene-$S(=O)_2R_3$, $-C_{0-3}$ alkylene-$SR_b$, $-C_{0-3}$ alkylene-$S(R_b)_5$, $-C_{0-3}$ alkylene-$C(=O)R_b$, $-C_{0-3}$ alkylene-$C(=O)OR_b$, $-C_{0-3}$ alkylene-C(=O) $N(R_b)_2$, $C_{2-6}$ alkenyl, $C_{2-6}$ alkynyl, $-C_{0-3}$ alkylene-$C_{3-14}$ cycloalkyl, $-C_{0-3}$ alkylene-(3-14 membered heterocyclyl), $-C_{0-3}$ alkylene-$C_{6-18}$ aryl and $-C_{0-3}$ alkylene-(5-18 membered heteroaryl), wherein the $C_{1-6}$ alkyl, $C_{1-6}$ haloalkyl, $C_{2-6}$ alkenyl, $C_{2-6}$ alkynyl, $-C_{0-3}$ alkylene-$C_{3-14}$ cycloalkyl, $-C_{0-3}$ alkylene-(3-14 membered heterocyclyl), $-C_{0-3}$ alkylene-$C_{6-18}$ aryl or $-C_{0-3}$ alkylene-(5-18 membered heteroaryl) can be optionally substituted with one or more $R_b$; each $R_b$ is independently selected from the group consisting of H, halogen, hydroxyl, cyano, $C_{1-6}$ alkyl, $-(CH_2)_{0-3}$ $C_{1-6}$ alkoxy, $C_{3-6}$ cycloalkyl and $C_{1-6}$ haloalkyl;
$R_2$ is selected from the group consisting of H, $C_{1-6}$ alkyl and haloalkyl;
$R_3$ is selected from the group consisting of H, halogen, cyano, $C_{1-6}$ alkyl, $C_{1-6}$ alkoxy, haloalkyl, hydroxyl, hydroxyalkyl, $C_{2-6}$ alkenyl, $C_{2-6}$ alkynyl, $-(CH_2)_{0-3}N(R_b)_2$, $-(CH_2)_{0-3}$-$C_{3-14}$ cycloalkyl, $-(CH_2)_{0-3}$-3-14 membered heterocyclyl, $-(CH_2)_{0-3}$-$C_{6-18}$ aryl and $-(CH_2)_{0-3}$-5-18 membered heteroaryl, wherein the $C_{1-6}$ alkyl, $C_{1-6}$ alkoxy, haloalkyl, hydroxyalkyl, $C_{2-6}$ alkenyl, $C_{2-6}$ alkynyl, $-(CH_2)_{0-3}$-$C_{3-14}$ cycloalkyl, $-(CH_2)_{0-3}$-3-14 membered hetero-

cyclyl, $-(CH_2)_{0-3}-C_{6-18}$ aryl or $-(CH_2)_{0-3}$-5-18 membered heteroaryl can be optionally substituted with one or more $R_c$;

$R_c$ is independently selected from the group consisting of halogen, $C_{1-6}$ alkyl, $C_{1-6}$ alkoxy, $C_{2-6}$ alkenyl, $C_{2-6}$ alkynyl, haloalkyl, cyano, amino, nitro, hydroxyl, hydroxyalkyl, $-C_{0-3}$ alkylene-C(=O)$R_b$, $-C_{0-3}$ alkylene-C(=O) $N(R_b)_2$, $-(CH_2)_{0-3}N(R_b)_2$, $-O(CH_2)_{0-3}-C_{3-14}$ cycloalkyl, $-O(CH_2)_{0-3}$-3-14 membered heterocyclyl, $C_{3-14}$ cycloalkyl, 3-14 membered heterocyclyl, $C_{6-18}$ aryl and 5-18 membered heteroaryl;

$R_4$ or $R_5$ is independently selected from the group consisting of $C_{1-6}$ alkyl, $C_{1-6}$ alkoxy, haloalkyl, hydroxyalkyl, $-(CH_2)_{1-3}N(R_b)_2$, $C_{2-6}$ alkenyl, $C_{3-14}$ cycloalkyl, 3-14 membered heterocyclyl, $C_{6-18}$ aryl and 6-18 membered heteroaryl, wherein the $C_{1-6}$ alkyl, $C_{1-6}$ alkoxy, haloalkyl, hydroxyalkyl, $-(CH_2)_{1-3}N(R_b)_2$, $C_{2-6}$ alkenyl, $C_{3-14}$ cycloalkyl, 3-14 membered heterocyclyl, $C_{6-18}$ aryl and 6-18 membered heteroaryl can be optionally substituted with one or more $R_d$;

$R_4$ or $R_5$, together with directly connected atoms on ring A, respectively form $C_{3-14}$ cycloalkyl, 3-14 membered heterocyclyl, $C_{6-18}$ aryl or 6-18 membered heteroaryl, wherein the $C_{3-14}$ cycloalkyl, 3-14 membered heterocyclyl, $C_{6-18}$ aryl and 6-18 membered heteroaryl can be optionally substituted with one or more $R_d$; or

$R_4$ and $R_5$, together with directly connected atoms on ring A, form $C_{3-14}$ cycloalkyl, 3-14 membered heterocyclyl, $C_{6-18}$ aryl or 6-18 membered heteroaryl; wherein the $C_{3-14}$ cycloalkyl, 3-14 membered heterocyclyl, $C_{6-18}$ aryl and 6-18 membered heteroaryl can be optionally substituted with one or more $R_d$;

$R_d$ is selected from the group consisting of H, halogen, $C_{1-6}$ alkyl, $C_{3-6}$ cycloalkyl, $C_{1-6}$ haloalkyl, cyano, amino, nitro, hydroxy and hydroxyalkyl;

X is $CR_6$ or N;

$R_6$ is selected from the group consisting of H, halogen, $C_{1-6}$ alkyl, $C_{3-6}$ cycloalkyl and $C_{1-6}$ haloalkyl;

m is 0, 1, 2, 3 or 4;

n is 0, 1 or 2.

**2.** The compound, or the stereoisomer, tautomer, deuterated substance or pharmaceutically acceptable salt thereof according to claim 1, wherein the $R_3$ is selected from the group consisting of H, halogen, cyano, $C_{1-6}$ alkyl, $C_{1-6}$ alkoxy, haloalkyl, hydroxyl, hydroxyalkyl, $-(CH_2)_{0-3}N(R_b)_2$, $-(CH_2)_{0-3}-C_{3-14}$ cycloalkyl, $-(CH_2)_{0-3}$-3-14 membered heterocyclyl, $-(CH_2)_{0-3}-C_{6-18}$ aryl and $-(CH_2)_{0-3}$-5-18 membered heteroaryl, wherein the $C_{1-6}$ alkyl, $C_{1-6}$ alkoxy, haloalkyl, hydroxyalkyl, $-(CH_2)_{0-3}-C_{3-14}$ cycloalkyl, $-(CH_2)_{0-3}$-3-14 membered heterocyclyl, $-(CH_2)_{0-3}-C_{6-18}$ aryl or $-(CH_2)_{0-3}$-5-18 membered heteroaryl can be optionally substituted with one or more $R_c$;

$R_c$ is independently selected from the group consisting of halogen, $C_{1-6}$ alkyl, $C_{1-6}$ alkoxy, haloalkyl, cyano, amino, nitro, hydroxyl, hydroxyalkyl, $-C_{0-3}$ alkylene-C(=O)$R_b$, $-C_{0-3}$ alkylene-C(=O)$N(R_b)_2$, $-(CH_2)_{0-3}N(R_b)_2$, $-O(CH_2)_{0-3}C_{3-14}$ cycloalkyl, $-O(CH_2)_{0-3}$-3-14 membered heterocyclyl, $C_{3-14}$ cycloalkyl, 3-14 membered heterocyclyl, $C_{6-18}$ aryl and 5-18 membered heteroaryl.

**3.** The compound, or the stereoisomer, tautomer, deuterated substance or pharmaceutically acceptable salt thereof according to claim 1, wherein each $R_b$ is independently H, halogen, hydroxyl, cyano, $C_{1-6}$ alkyl, $C_{3-6}$ cycloalkyl or $C_{1-6}$ haloalkyl;

$R_3$ is selected from the group consisting of H, halogen, cyano, $C_{1-6}$ alkyl, $C_{1-6}$ alkoxy, haloalkyl, hydroxyl, hydroxyalkyl, $-(CH_2)_{0-3}N(R_b)_2$, $-(CH_2)_{0-3}-C_{3-14}$ cycloalkyl, $-(CH_2)_{0-3}$-3-14 membered heterocyclyl, $-(CH_2)_{0-3}-C_{6-18}$ aryl and $-(CH_2)_{0-3}$-5-18 membered heteroaryl, wherein the $C_{1-6}$ alkyl, $C_{1-6}$ alkoxy, haloalkyl, hydroxyalkyl, $-(CH_2)_{0-3}-C_{3-14}$ cycloalkyl, $-(CH_2)_{0-3}$-3-14 membered heterocyclyl, $-(CH_2)_{0-3}-C_{6-18}$ aryl or $-(CH_2)_{0-3}$-5-18 membered heteroaryl can be optionally substituted with one or more $R_c$;

$R_c$ is independently selected from the group consisting of halogen, $C_{1-6}$ alkyl, $C_{1-6}$ alkoxy, haloalkyl, cyano, amino, nitro, hydroxyl, hydroxyalkyl, $-C_{0-3}$ alkylene-C(=O)$R_b$, $-C_{0-3}$ alkylene-C(=O)$N(R_b)_2$, $-(CH_2)_{0-3}$ $N(R_b)_2$, $-O(CH_2)_{0-3}C_{3-14}$ cycloalkyl, $-O(CH_2)_{0-3}$-3-14 membered heterocyclyl, $C_{3-14}$ cycloalkyl, 3-14 membered heterocyclyl, $C_{6-18}$ aryl and 5-18 membered heteroaryl;

n is 1 or 2.

**4.** The compound, or the stereoisomer, tautomer, deuterated substance or pharmaceutically acceptable salt thereof according to claim 1, wherein each $R_b$ is independently H, halogen, hydroxyl, cyano, $C_{1-6}$ alkyl, $C_{3-6}$ cycloalkyl or $C_{1-6}$ haloalkyl;

$R_3$ is selected from the group consisting of H, halogen, $C_{1-6}$ alkyl, $C_{1-6}$ alkoxy, haloalkyl, hydroxyl, hydroxyalkyl, $-(CH_2)_{0-3}N(R_b)_2$, $-(CH_2)_{0-3}-C_{3-14}$ cycloalkyl, $-(CH_2)_{0-3}$-3-14 membered heterocyclyl, $-(CH_2)_{0-3}-C_{6-18}$ aryl and $-(CH_2)_{0-3}$-5-18 membered heteroaryl; wherein the $C_{1-6}$ alkyl, $C_{1-6}$ alkoxy, haloalkyl, hydroxyalkyl, $-(CH_2)_{0-3}-C_{3-14}$ cycloalkyl, $-(CH_2)_{0-3}$-3-14 membered heterocyclyl, $-(CH_2)_{0-3}-C_{6-18}$ aryl or $-(CH_2)_{0-3}$-5-18 membered heteroaryl

can be optionally substituted with one or more $R_c$;
$R_c$ is independently selected from the group consisting of halogen, $C_{1-6}$ alkyl, $C_{1-6}$ alkoxy, haloalkyl, cyano, amino, nitro, hydroxyl, hydroxyalkyl, $-C_{0-3}$ alkylene-$C(=O)R_b$, $-C_{0-3}$ alkylene-$C(=O)N(R_b)_2$, $-(CH_2)_{0-3}$ $N(R_b)_2$, $-O(CH_2)_{0-3}C_{3-14}$ cycloalkyl, $-O(CH_2)_{0-3}$-3-14 membered heterocyclyl, $C_{3-14}$ cycloalkyl, 3-14 membered heterocyclyl, $C_{6-18}$ aryl and 5-18 membered heteroaryl;
n is 1 or 2.

**5.** The compound, or the stereoisomer, tautomer, deuterated substance or pharmaceutically acceptable salt thereof according to any one of claims 1 to 4, wherein the $R_4$ or $R_5$, together with directly connected atoms on ring A, respectively form $C_{3-14}$ cycloalkyl, 3-14 membered heterocyclyl, $C_{6-18}$ aryl or 6-18 membered heteroaryl; wherein the $C_{3-14}$ cycloalkyl, 3-14 membered heterocyclyl, $C_{6-18}$ aryl and 6-18 membered heteroaryl can be optionally substituted with one or more $R_d$, $R_d$ is selected from the group consisting of H, halogen, $C_{1-6}$ alkyl, $C_{3-6}$ cycloalkyl, $C_{1-6}$ haloalkyl, cyano, amino, nitro, hydroxy and hydroxyalkyl.

**6.** The compound, or the stereoisomer, tautomer, deuterated substance or pharmaceutically acceptable salt thereof according to any one of claims 1 to 5, wherein the L is a bond or NH, preferably NH.

**7.** The compound, or the stereoisomer, tautomer, deuterated substance or pharmaceutically acceptable salt thereof according to any one of claims 1 to 6, wherein the $R_1$ is $C_{6-18}$ aryl or 6-18 membered heteroaryl, wherein the $C_{6-18}$ aryl or 6-18 membered heteroaryl can be optionally substituted with one or more $R_a$; $R_a$ is independently selected from the group consisting of H, hydroxyl, cyano, halogen, $C_{1-6}$ alkyl, $C_{1-6}$ haloalkyl, $-C_{0-3}$ alkylene-$OR_b$, $-OC(=O)C_{1-6}$ alkyl, $-C_{0-3}$ alkylene-$N(R_b)_2$, $-C_{0-3}$ alkylene-$C(=O)R_b$, $-C_{0-3}$ alkylene-$C(=O)OR_b$, $-C_{0-3}$ alkylene-$C(=O)N(R_b)_2$, $C_{2-6}$ alkenyl and $C_{2-6}$ alkynyl, each $R_b$ is independently selected from the group consisting of H, halogen, hydroxyl, cyano, $C_{1-6}$ alkyl, $-(CH_2)_{0-3}C_{1-6}$ alkoxy, $C_{3-6}$ cycloalkyl and $C_{1-6}$ haloalkyl.

**8.** The compound, or the stereoisomer, tautomer, deuterated substance or pharmaceutically acceptable salt thereof according to any one of claims 1 to 7, wherein the $R_1$ is selected from the group consisting of

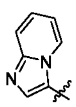, 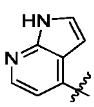, 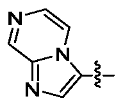, 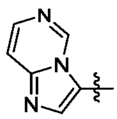, 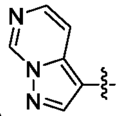, 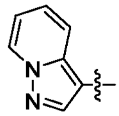, 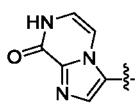,

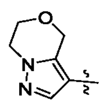, 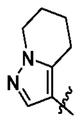, 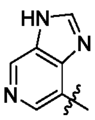, 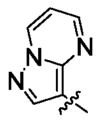 and 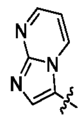;

wherein the

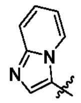, 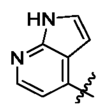, 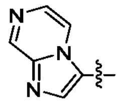,

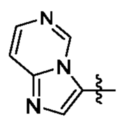, 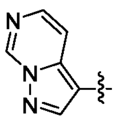, 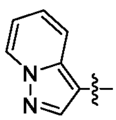, 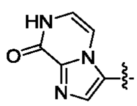, 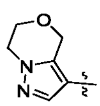, 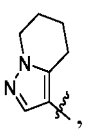, 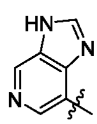, 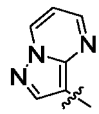 or 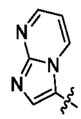

can be optionally substituted with one or more $R_a$, $R_a$ is independently selected from the group consisting of H, hydroxyl, cyano, halogen, $C_{1-6}$ alkyl, $C_{1-6}$ haloalkyl, $-C_{0-3}$ alkylene-$OR_b$, $-OC(=O)C_{1-6}$ alkyl, $-C_{0-3}$ alkylene-$N(R_b)_2$, $-C_{0-3}$ alkylene-$C(=O)R_b$, $-C_{0-3}$ alkylene-$C(=O)OR_b$, $-C_{0-3}$ alkylene-$C(=O)N(R_b)_2$, $C_{2-6}$ alkenyl and $C_{2-6}$ alkynyl, each $R_b$ is independently selected from the group consisting of H, halogen, hydroxyl, cyano, $C_{1-6}$ alkyl, $-(CH_2)_{0-3}C_{1-6}$ alkoxy, $C_{3-6}$ cycloalkyl and $C_{1-6}$ haloalkyl.

**9.** The compound, or the stereoisomer, tautomer, deuterated substance or pharmaceutically acceptable salt thereof

according to any one of claims 1 to 8, wherein the $R_1$ is selected from the group consisting of

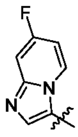, 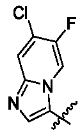, 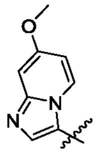, 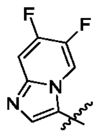, 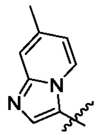, 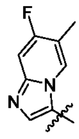, 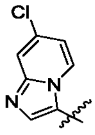, 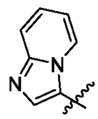,

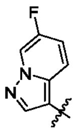, 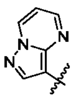, 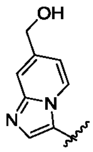, 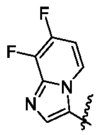, 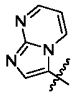, 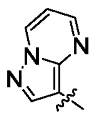, 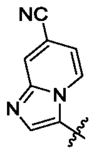, 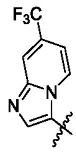, 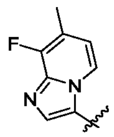, 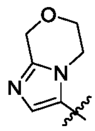, 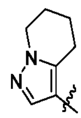,

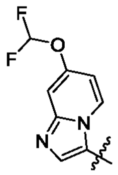, 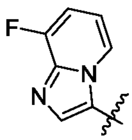, 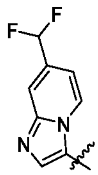, 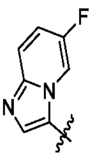, 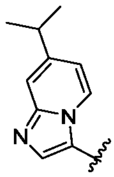, 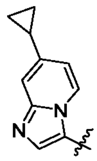, 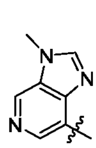, 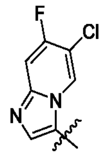 and 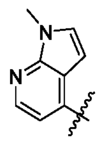.

**10.** The compound, or the stereoisomer, tautomer, deuterated substance or pharmaceutically acceptable salt thereof according to any one of claims 1 to 9, wherein the $R_1$ is

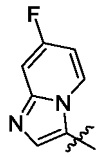

or

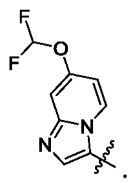.

**11.** The compound, or the stereoisomer, tautomer, deuterated substance or pharmaceutically acceptable salt thereof according to any one of claims 1-10, wherein the $R_2$ is preferably H.

**12.** The compound, or the stereoisomer, tautomer, deuterated substance or pharmaceutically acceptable salt thereof according to any one of claims 1-11, wherein the ring A is selected from the group consisting of $C_{8-10}$ bicarbocyclyl, 8-10 membered biheterocycloalkyl, $C_{10-12}$ bicyclic aryl and 10-12 membered bicyclic heteroaryl, preferably selected from the group consisting of

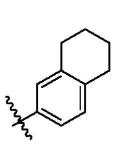, 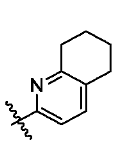, 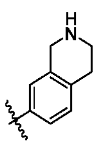, 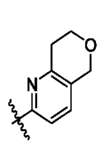, 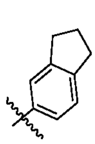, 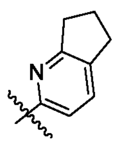, 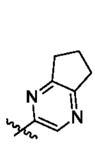, 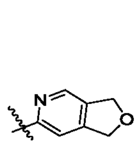, 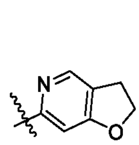

and

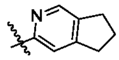.

**13.** The compound, or the stereoisomer, tautomer, deuterated substance or pharmaceutically acceptable salt thereof according to any one of claims 1-12, wherein the ring A is selected from the group consisting of $C_{8-10}$ bicarbocyclyl, 8-10 membered biheterocycloalkyl, $C_{10-12}$ bicyclic aryl and 10-12 membered bicyclic heteroaryl, preferably selected from the group consisting of

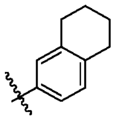, 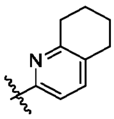, 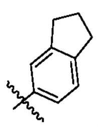, 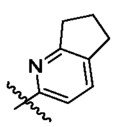, 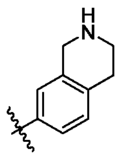 and 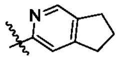.

**14.** The compound, or the stereoisomer, tautomer, deuterated substance or pharmaceutically acceptable salt thereof according to any one of claims 1-13, wherein the $R_3$ is selected from the group consisting of H, halogen, cyano, $C_{1-6}$ alkyl, $C_{1-6}$ alkoxy, haloalkyl, hydroxyl, hydroxyalkyl, $C_{2-6}$ alkenyl, $C_{2-6}$ alkynyl, $-(CH_2)_{0-3}-C_{3-14}$ cycloalkyl, $-(CH_2)_{0-3}$-3-14 membered heterocyclyl and $-(CH_2)_{0-3}N(R_b)_2$, wherein the $C_{1-6}$ alkyl, $C_{1-6}$ alkoxy, $-(CH_2)_{0-3}-C_{3-14}$ cycloalkyl, $-(CH_2)_{0-3}$-3-14 membered heterocyclyl, haloalkyl, hydroxyalkyl, $C_{2-6}$ alkenyl or $C_{2-6}$ alkynyl can be optionally substituted with one or more $R_c$, $R_c$ is independently selected from the group consisting of halogen, $C_{1-6}$ alkyl, $C_{1-6}$ alkoxy, haloalkyl, cyano, amino, nitro, hydroxyl, hydroxyalkyl, $C_{2-6}$ alkenyl, $C_{2-6}$ alkynyl, $-C_{0-3}$ alkylene-$C(=O)R_b$, $-C_{0-3}$ alkylene-$C(=O)N(R_b)_2$, $-(CH_2)_{0-3}N(R_b)_2$, $-O(CH_2)_{0-3}C_{3-14}$ cycloalkyl, $-O(CH_2)_{0-3}$-3-14 membered heterocyclyl, $C_{3-14}$ cycloalkyl, 3-14 membered heterocyclyl, $C_{6-18}$ aryl and 5-18 membered heteroaryl, each $R_b$ is independently selected from the group consisting of H, halogen, hydroxyl, cyano, $C_{1-6}$ alkyl, $-(CH_2)_{0-3}-C_{1-6}$ alkoxy, $C_{3-6}$ cycloalkyl and $C_{1-6}$ haloalkyl.

**15.** The compound, or the stereoisomer, tautomer, deuterated substance or pharmaceutically acceptable salt thereof according to any one of claims 1 to 14, wherein the $R_3$ is selected from the group consisting of H, $-(CH_2)_{0-3}N(R_b)_2$, cyano, $C_{2-6}$ alkenyl, $C_{2-6}$ alkynyl, $-(CH_2)_{0-3}$-3-14 membered heterocyclyl and $C_{1-6}$ alkyl, each $R_b$ is independently selected from the group consisting of H, halogen, hydroxyl, cyano, $C_{1-6}$ alkyl, $C_{3-6}$ cycloalkyl, $-(CH_2)_{0-3}C_{1-6}$ alkoxy and $C_{1-6}$ haloalkyl.

**16.** The compound, or the stereoisomer, tautomer, deuterated substance or pharmaceutically acceptable salt thereof according to any one of claims 1 to 15, wherein the $R_4$, together with directly connected atoms on ring A, form $C_{3-14}$ cycloalkyl, 3-14 membered heterocyclyl, $C_{6-18}$ aryl or 6-18 membered heteroaryl.

**17.** The compound, or the stereoisomer, tautomer, deuterated substance or pharmaceutically acceptable salt thereof according to any one of claims 1-16, wherein the formula (I) is selected from formula (IA):

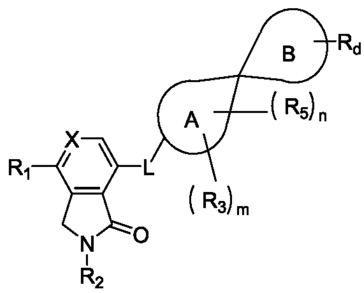

(IA)

wherein,

ring B is selected from the group consisting of $C_{3-14}$ cycloalkyl, 3-14 membered heterocycloalkyl, $C_{6-18}$ aryl and 6-18 membered heteroaryl;

$R_1$, $R_2$, $R_3$, $R_5$, X, L, ring A, $R_d$, m and n have the same definitions as described in any one of claim 1, 2, 3, 4 or 5.

**18.** The compound of formula (IA), or the stereoisomer, tautomer, deuterated substance or pharmaceutically acceptable salt thereof according to claim 17, wherein the ring A is selected from the group consisting of $C_{8-10}$ bicarbocyclyl, 8-10 membered biheterocycloalkyl, $C_{10-12}$ bicyclic aryl and 10-12 membered bicyclic heteroaryl.

**19.** The compound of formula (IA), or the stereoisomer, tautomer, deuterated substance or pharmaceutically acceptable salt thereof according to claim 17 or 18, wherein the ring A is selected from the group consisting of

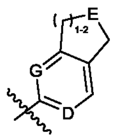, 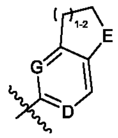 and 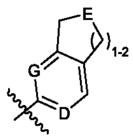;

wherein D, E and G are each independently selected from the group consisting of C, N and O.

**20.** The compound of formula (IA), or the stereoisomer, tautomer, deuterated substance or pharmaceutically acceptable salt thereof according to claim 19, wherein the ring A is selected from the group consisting of

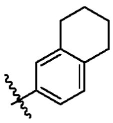, 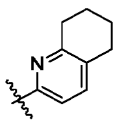, 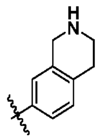, 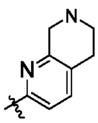, 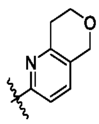, 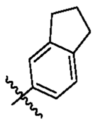,

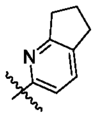, 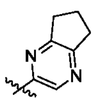, 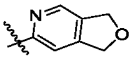, 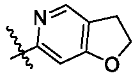, 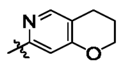 and 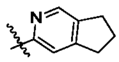.

**21.** The compound of formula (IA), or the stereoisomer, tautomer, deuterated substance or pharmaceutically acceptable salt thereof according to claim 17, wherein

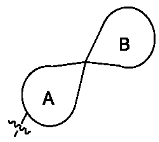

is selected from the group consisting of

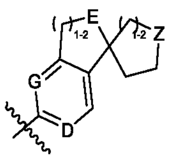, 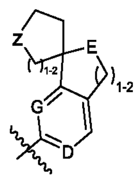, 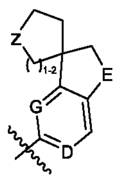, 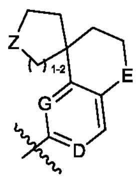,

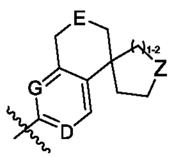 and 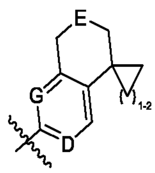,

wherein D, E, G and Z are each independently selected from the group consisting of C, N and O.

**22.** The compound of formula (IA), or the stereoisomer, tautomer, deuterated substance or pharmaceutically acceptable

salt thereof according to claim 21, wherein the

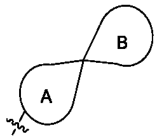

is selected from the group consisting of

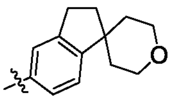, 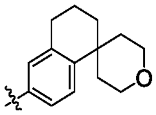, 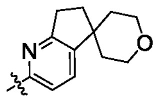, 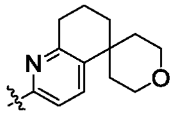,

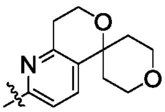, 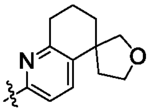, 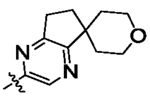, 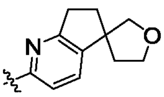, 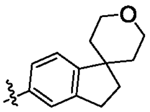, 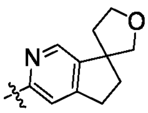,

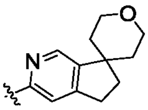, 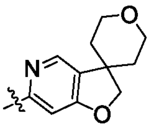, 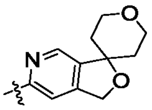, 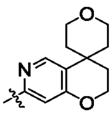, 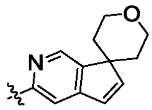, 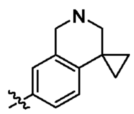, 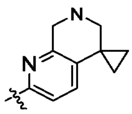,

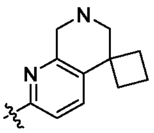, 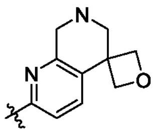, 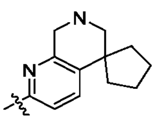, 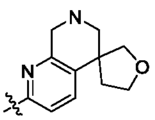, 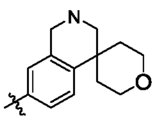, 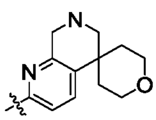

and

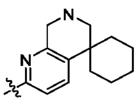.

**23.** The compound of formula (IA), or the stereoisomer, tautomer, deuterated substance or pharmaceutically acceptable salt thereof according to any one of claims 17-22, wherein the $R_3$ is selected from the group consisting of H, halogen, $C_{1-6}$ alkyl, $C_{1-6}$ alkoxy, haloalkyl, $C_{2-6}$ alkenyl, $C_{2-6}$ alkynyl, $-(CH_2)_{0-3}-C_{3-14}$ cycloalkyl, $-(CH_2)_{0-3}$-3-14 membered heterocyclyl and $-(CH_2)_{0-3}N(R_b)_2$, wherein the $C_{1-6}$ alkyl, $C_{1-6}$ alkoxy, $-(CH_2)_{0-3}-C_{3-14}$ cycloalkyl, $-(CH_2)_{0-3}$-3-14 membered heterocyclyl, haloalkyl or hydroxyalkyl can be optionally substituted with one or more $R_c$;

$R_c$ is independently selected from the group consisting of halogen, $C_{1-6}$ alkyl, $C_{1-6}$ alkoxy, haloalkyl, cyano, amino, nitro, hydroxyl, hydroxyalkyl, $-C_{0-3}$ alkylene-C(=O)$R_b$, $-C_{0-3}$ alkylene-C(=O)N$(R_b)_2$, $-(CH_2)_{0-3}N(R_b)_2$, $-O(CH_2)_{0-3}C_{3-14}$ cycloalkyl, $-O(CH_2)_{0-3}$-3-14 membered heterocyclyl, $C_{3-14}$ cycloalkyl, 3-14 membered heterocyclyl, $C_{6-18}$ aryl and 5-18 membered heteroaryl, each $R_b$ is independently selected from the group consisting of H, halogen, hydroxyl, cyano, $C_{1-6}$ alkyl, $-(CH_2)_{0-3}-C_{1-6}$ alkoxy, $C_{3-6}$ cycloalkyl and $C_{1-6}$ haloalkyl;
m is 0, 1, 2 or 3.

**24.** The compound of formula (IA), or the stereoisomer, tautomer, deuterated substance or pharmaceutically acceptable salt thereof according to any one of claims 17-23, wherein the $R_3$ is selected from the group consisting of H, $-(CH_2)_{0-3}N(R_b)_2$, cyano, $C_{2-6}$ alkenyl, $C_{2-6}$ alkynyl, $-(CH_2)_{0-3}$-3-14 membered heterocyclyl and $C_{1-6}$ alkyl, each $R_b$ is independently selected from the group consisting of H, halogen, hydroxyl, cyano, $C_{1-6}$ alkyl, $C_{3-6}$ cycloalkyl, $-(CH_2)_{0-3}-C_{1-6}$ alkoxy and $C_{1-6}$ haloalkyl.

**25.** The compound of formula (IA), or the stereoisomer, tautomer, deuterated substance or pharmaceutically acceptable salt thereof according to any one of claims 17-23, wherein the $R_5$ is $C_{1-6}$ alkyl; n is 0 or 1.

**26.** The compound of formula (IA), or the stereoisomer, tautomer, deuterated substance or pharmaceutically acceptable salt thereof according to any one of claims 17-25, wherein the L is NH.

**27.** The compound of formula (IA), or the stereoisomer, tautomer, deuterated substance or pharmaceutically acceptable salt thereof according to any one of claims 17-25, wherein the $R_1$ is selected from the group consisting of

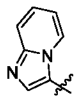, 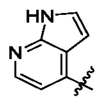, 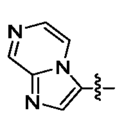, 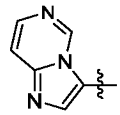, 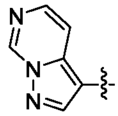,

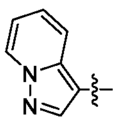, 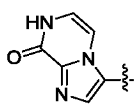, 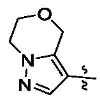, 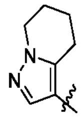, 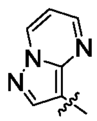 and 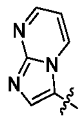;

wherein the

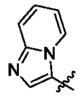, 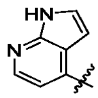,

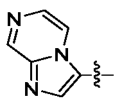, 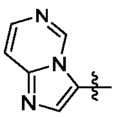, 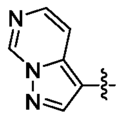, 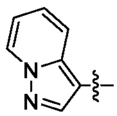, 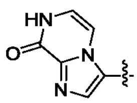, 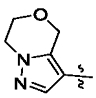, 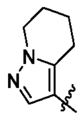, 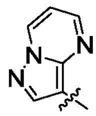 or 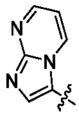

can be optionally substituted with one or more $R_a$, $R_a$ is independently selected from the group consisting of H, hydroxyl, cyano, halogen, $C_{1-6}$ alkyl, $C_{1-6}$ haloalkyl, -$C_{0-3}$ alkylene-$OR_b$, -OC(=O)$C_{1-6}$ alkyl, -$C_{0-3}$ alkylene-N$(R_b)_2$, -$C_{0-3}$ alkylene-C(=O)$R_b$, -$C_{0-3}$ alkylene-C(=O)$OR_b$, -$C_{0-3}$ alkylene-C(=O)N$(R_b)_2$, $C_{2-6}$ alkenyl and $C_{2-6}$ alkynyl, each $R_b$ is independently selected from the group consisting of H, halogen, hydroxyl, cyano, $C_{1-6}$ alkyl, -$(CH_2)_{0-3}C_{1-6}$ alkoxy, $C_{3-6}$ cycloalkyl and $C_{1-6}$ haloalkyl.

**28.** The compound of formula (IA), or the stereoisomer, tautomer, deuterated substance or pharmaceutically acceptable salt thereof according to any one of claims 17-27, wherein the $R_1$ is selected from the group consisting of

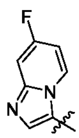, 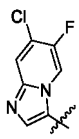, 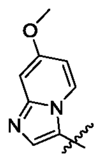, 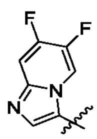, 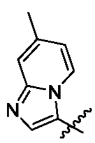, 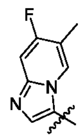,

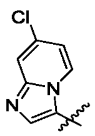, 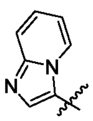, 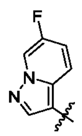, 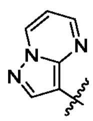, 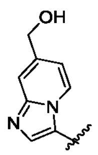, 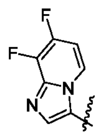, 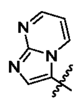, 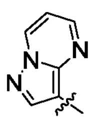, 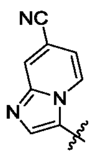, 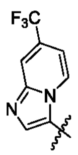,

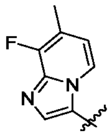, 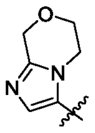, 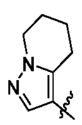, 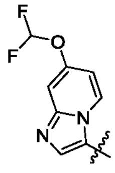, 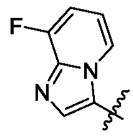, 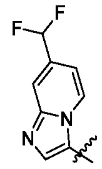, 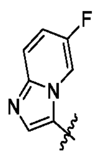, 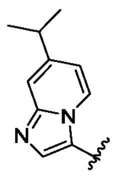, 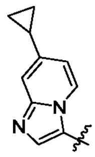,

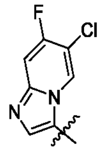 and 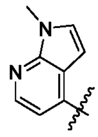.

**29.** The compound of formula (IA), or the stereoisomer, tautomer, deuterated substance or pharmaceutically acceptable salt thereof according to any one of claims 17-28, wherein the $R_2$ is H.

**30.** The compound, or the stereoisomer, tautomer, deuterated substance or pharmaceutically acceptable salt thereof according to any one of claims 1-29, wherein

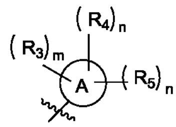

is selected from the group consisting of

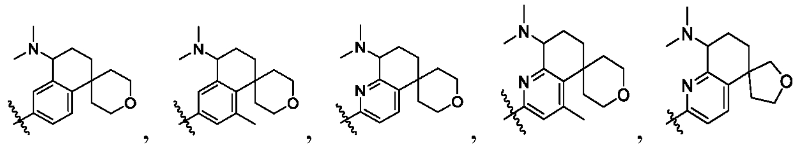

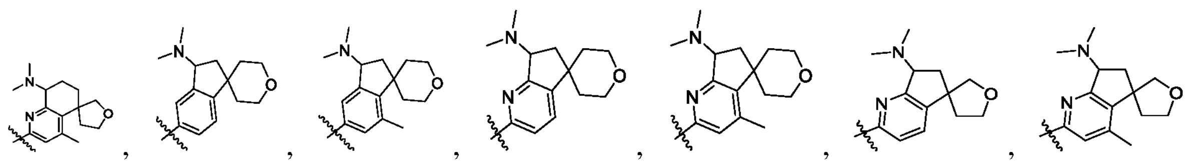

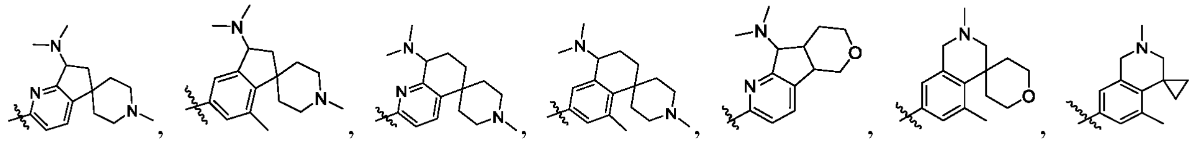

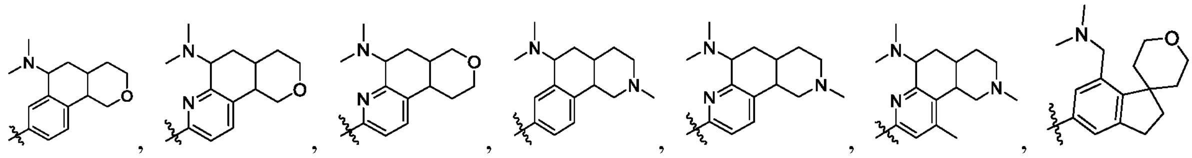

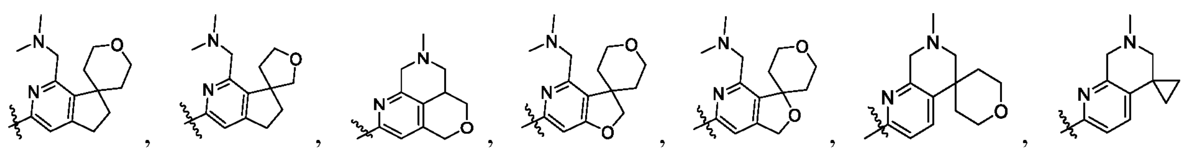

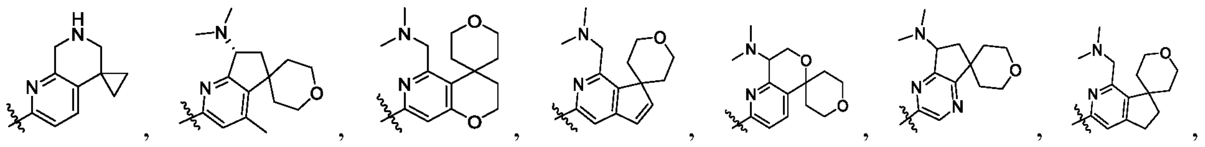

, , , , , , ,

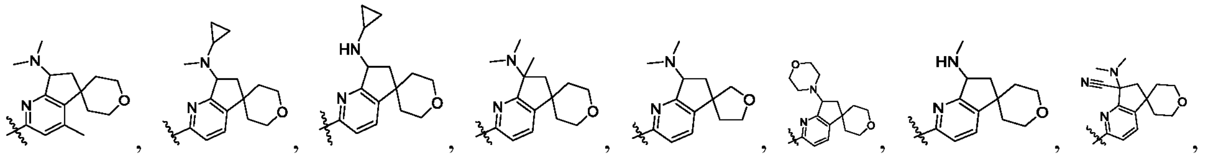

, , , , , , , ,

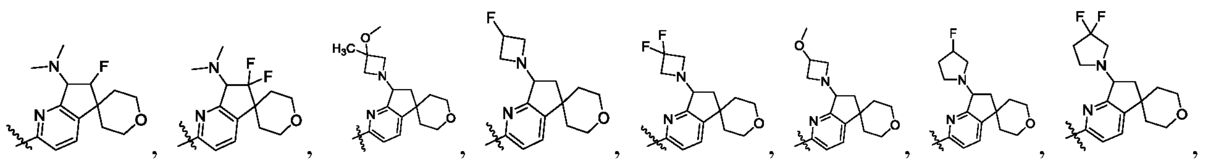

, , , , , , , ,

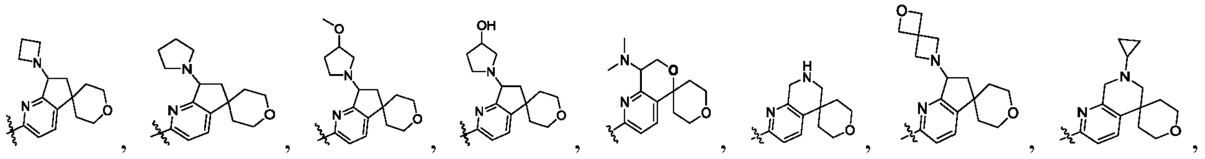

, , , , , , , ,

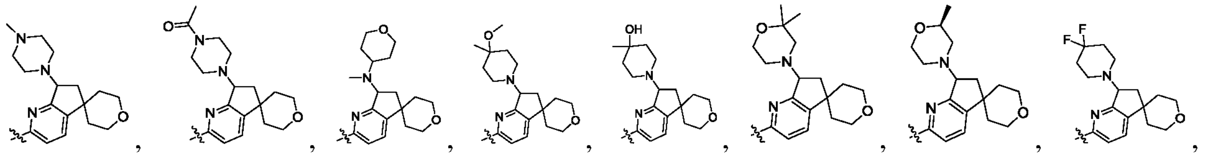

, , , , , , , ,

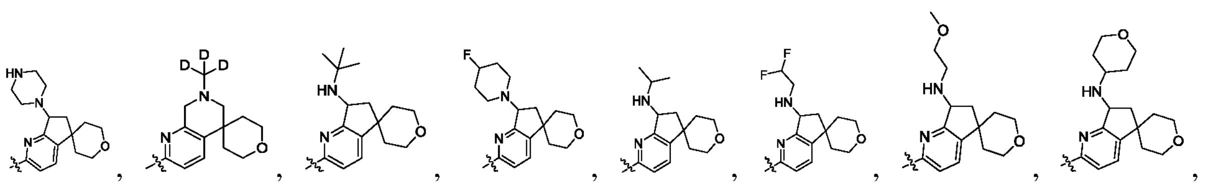

, , , , , , , ,

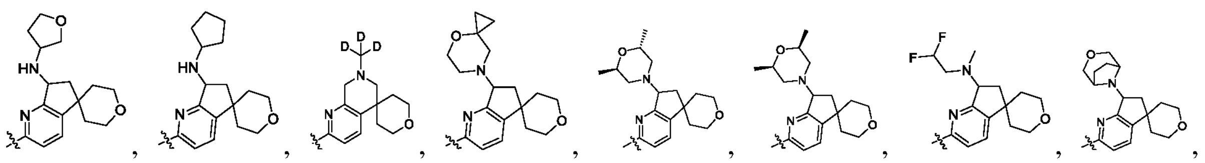

, , , , , , , ,

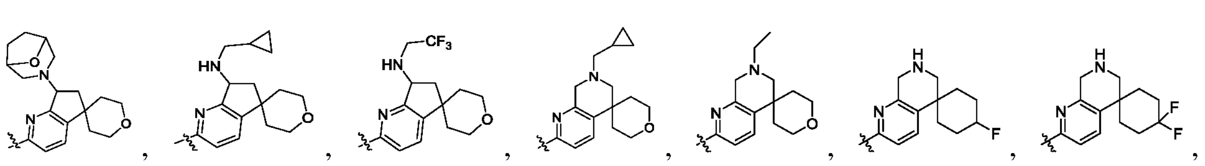

, , , , , , ,

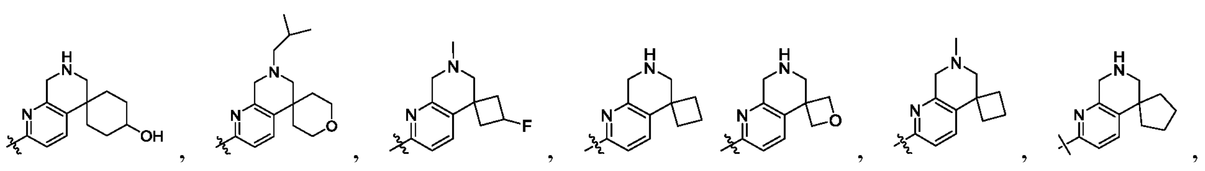

, , , , , ,

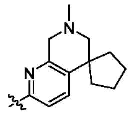, 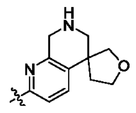, 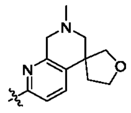, 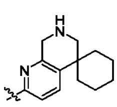, 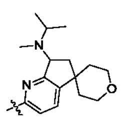, 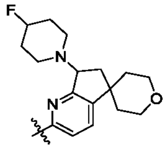, 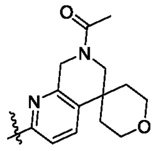,

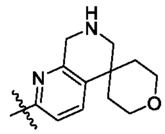, 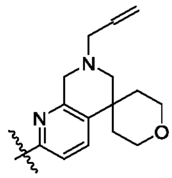, 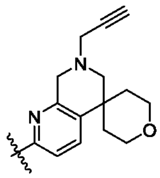, 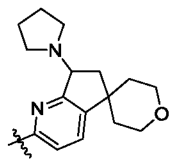 and 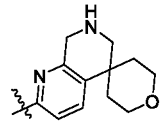.

**31.** The compound, or the stereoisomer, tautomer, deuterated substance or pharmaceutically acceptable salt thereof according to any one of claims 1-30, wherein the formula (I) is selected from the group consisting of:

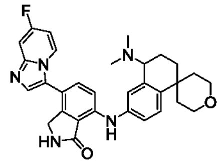
1

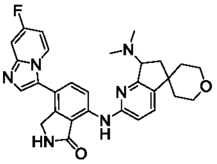
2

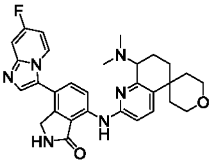
3

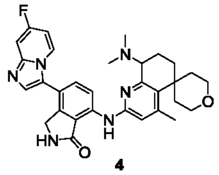
4

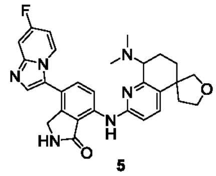
5

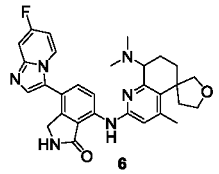
6

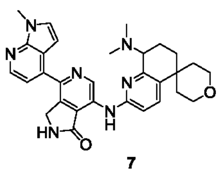
7

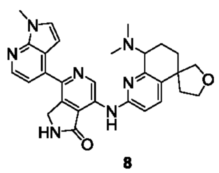
8

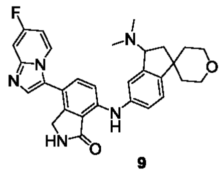
9

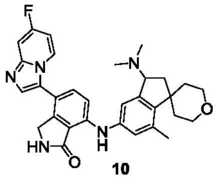
10

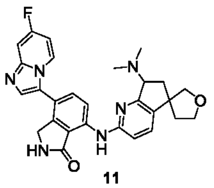
11

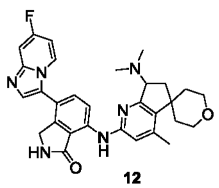
12

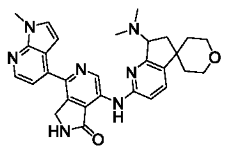
13

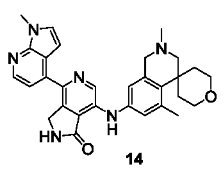
14

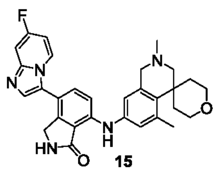
15

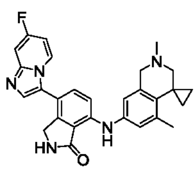
16

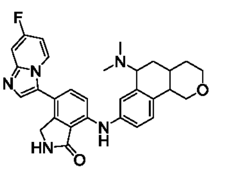
17

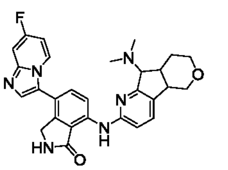
18

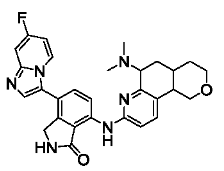
19

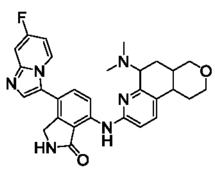
20

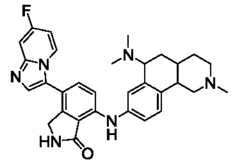
21
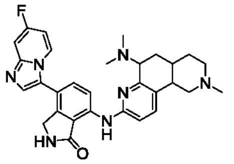
22
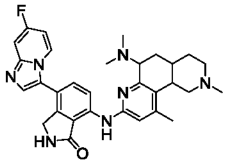
23
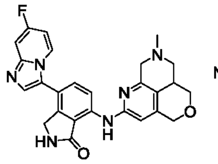
24
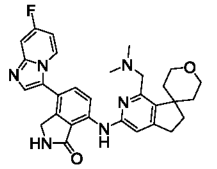
25
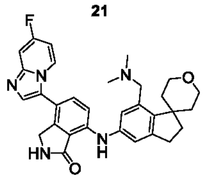
26
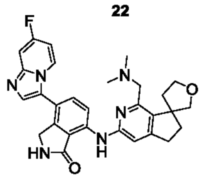
27
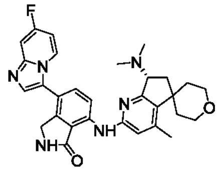
28
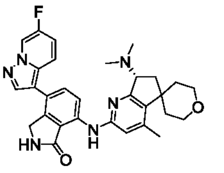
29
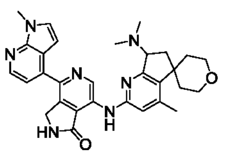
30
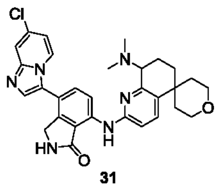
31
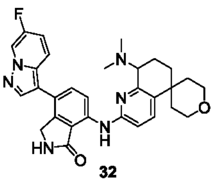
32
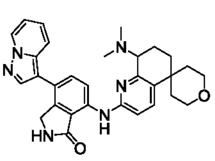
33
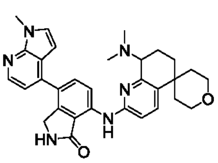
34
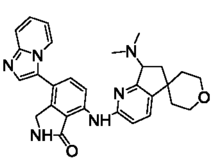
35
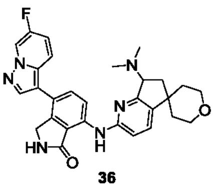
36
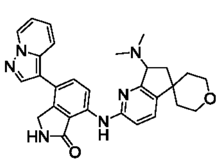
37
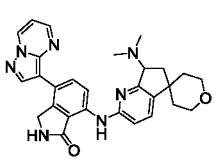
38
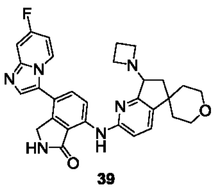
39
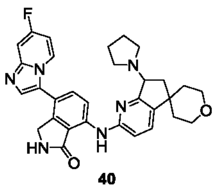
40
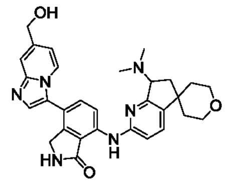
41
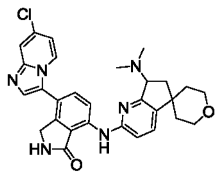
42
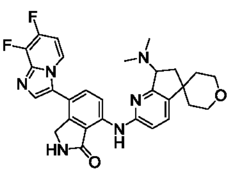
43
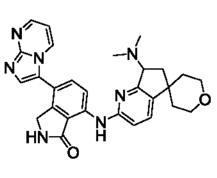
44
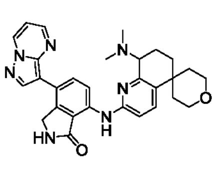
45
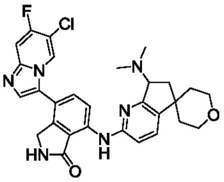
46
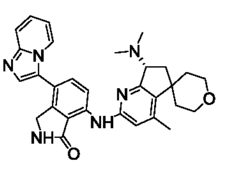
47
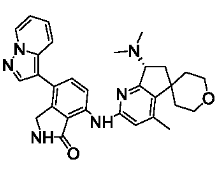
48
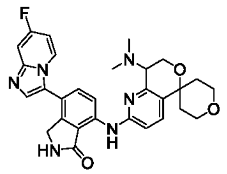
49
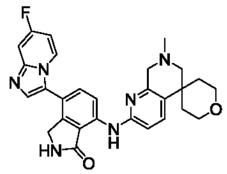
50
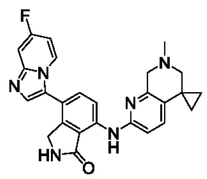
51
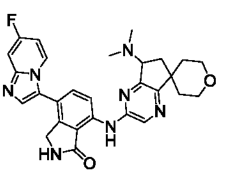
52
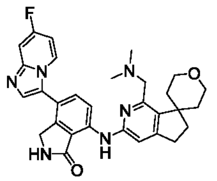
53
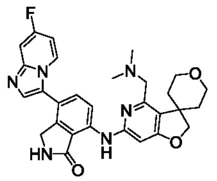
54
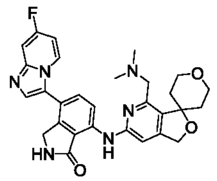
55

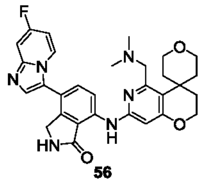
56

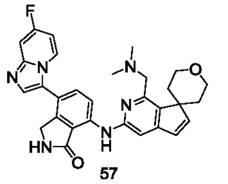
57

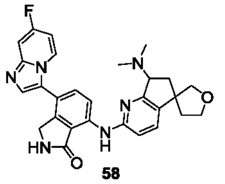
58

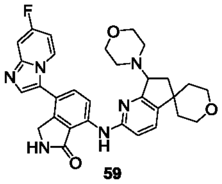
59

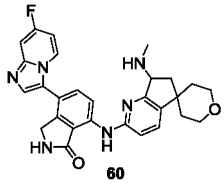
60

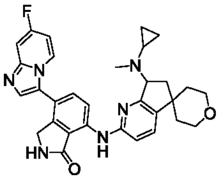
61

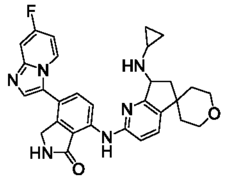
62

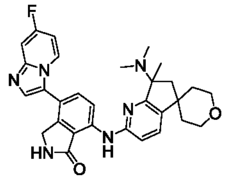
63

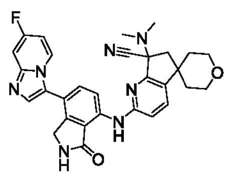
64

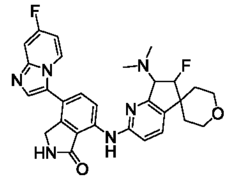
65

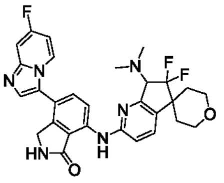
66

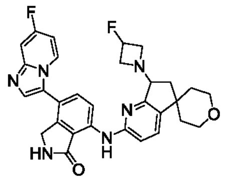
67

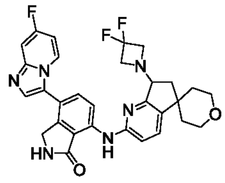
68

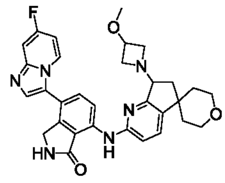
69

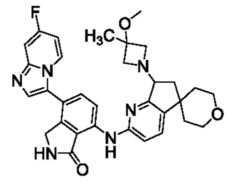
70

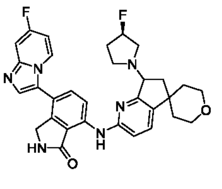
71

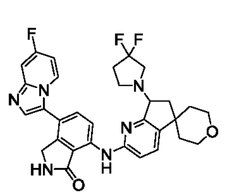
72

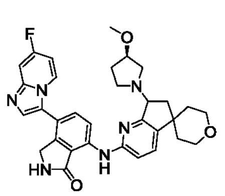
73

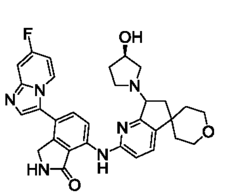
74

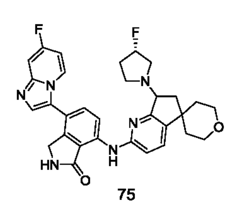
75

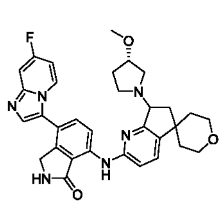
76

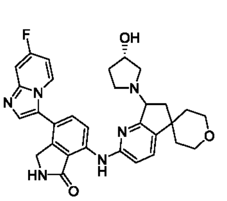
77

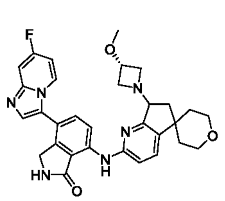
78

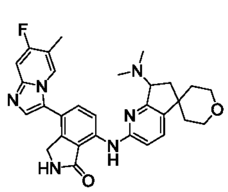
79

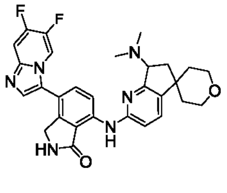
80

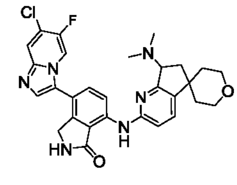
81

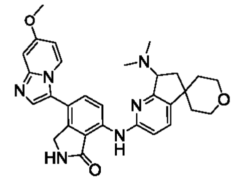
82

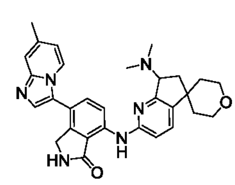
83

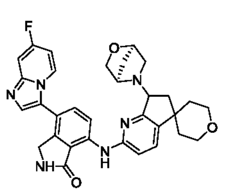
84

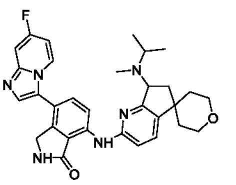
85

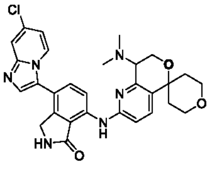
86

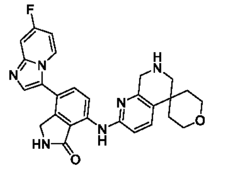
87

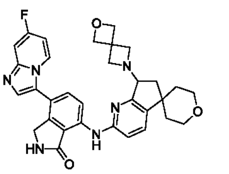
88

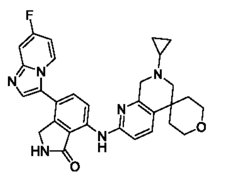
89

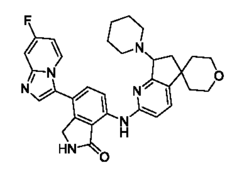
90

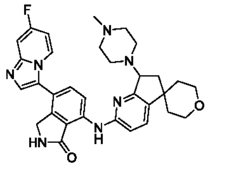
91

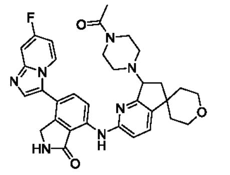
92

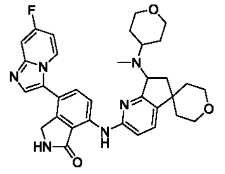
93

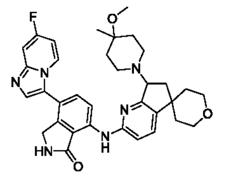
94

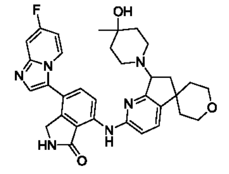
95

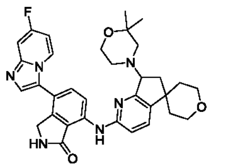
96

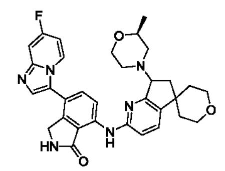
97

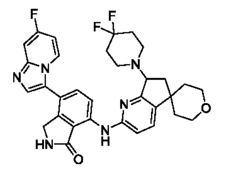
98

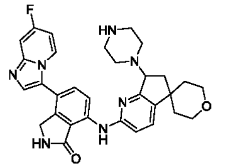
99

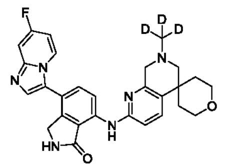
100

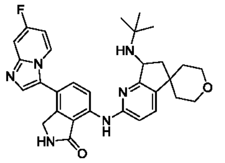
101

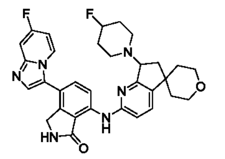
102

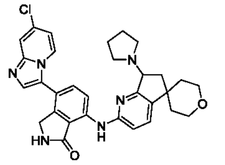
103

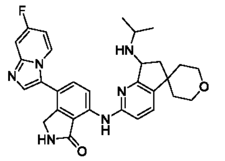
104

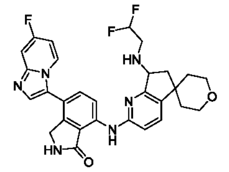
105

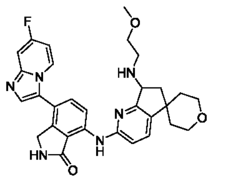
106

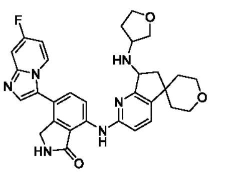
107

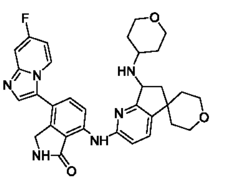
108

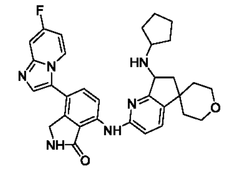
109

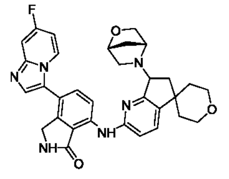
110

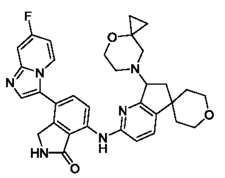
111

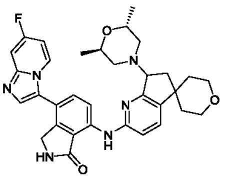
112

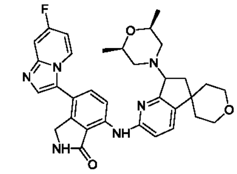
113

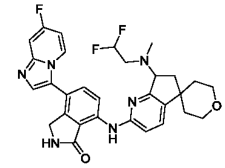
114

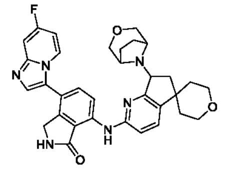
115

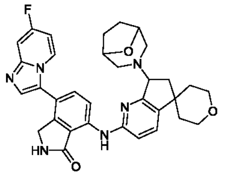
116

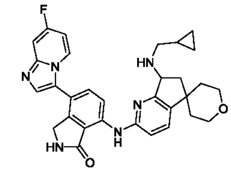
117

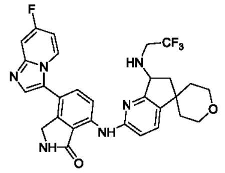
118

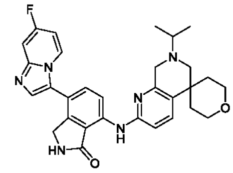
119

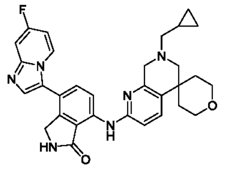
120

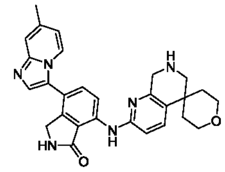
121

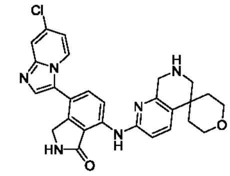
122

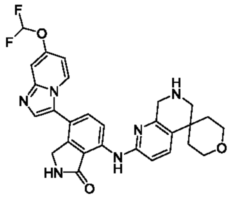
123

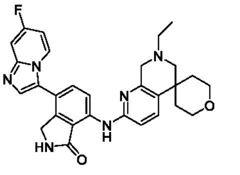
124

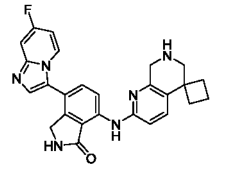
125

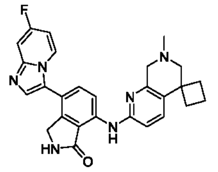
126

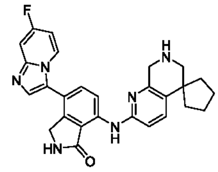
127

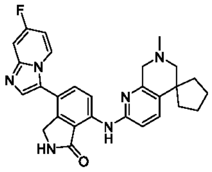
128

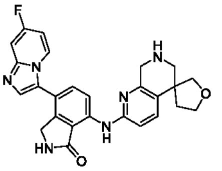
129

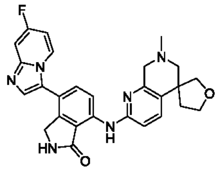
130

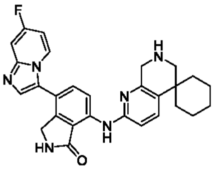
131

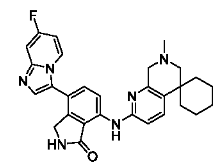
132

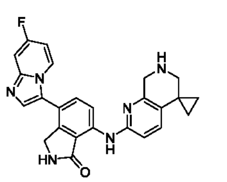
133

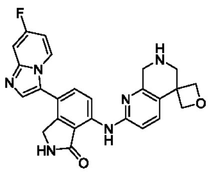
134

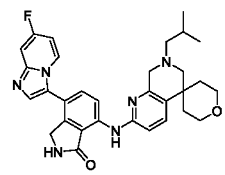
135

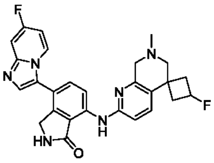
136

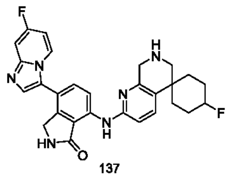
137

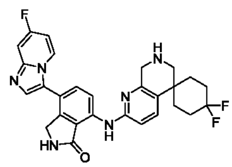
138

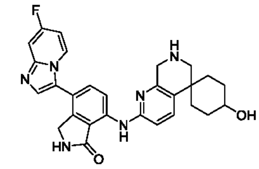
139

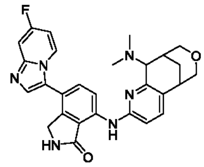
140

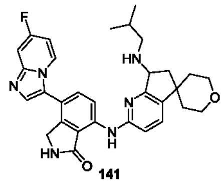
141

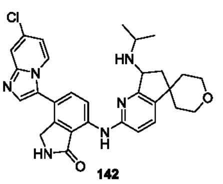
142

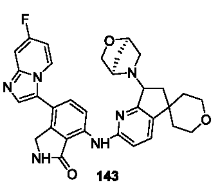
143

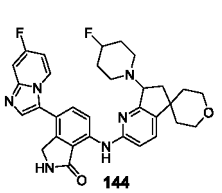
144

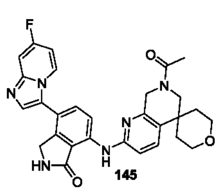
145

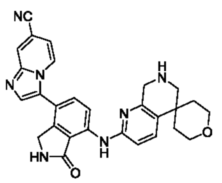
146

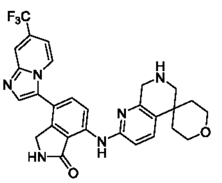
147

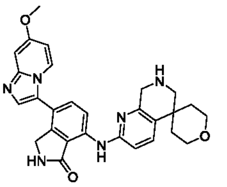
148

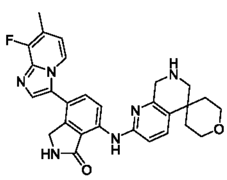
149

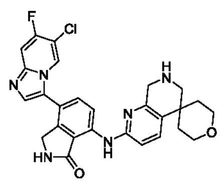
150

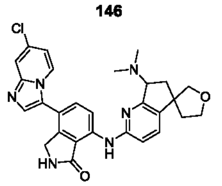
151

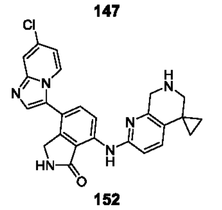
152

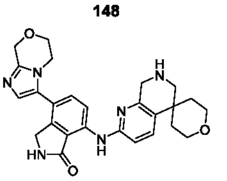
153

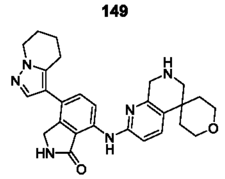
154

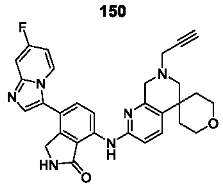
155

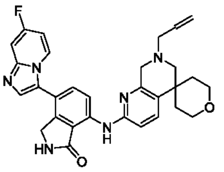
156

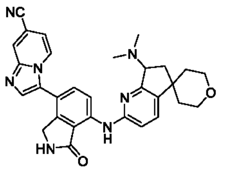
157

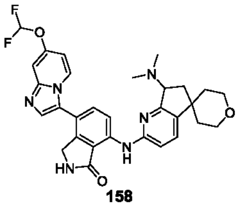
158

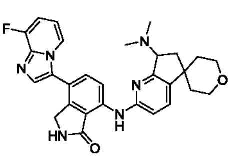
159

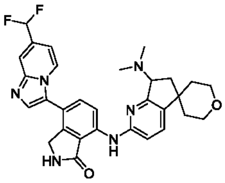
160

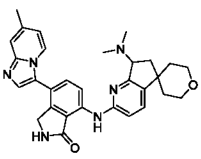
161

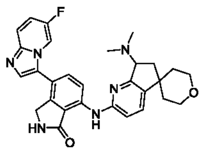
162

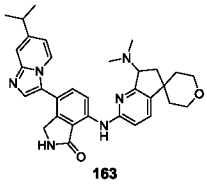
163

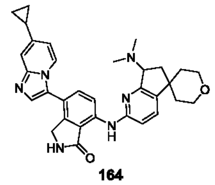
164

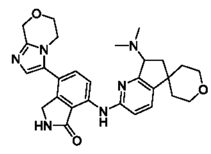
165

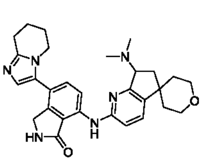
166

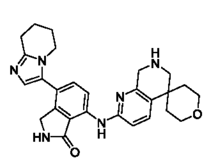
167

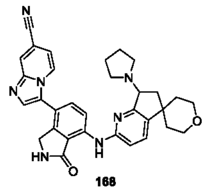
168

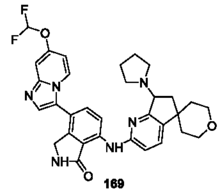
169

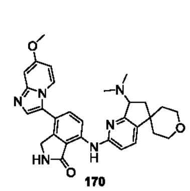
170

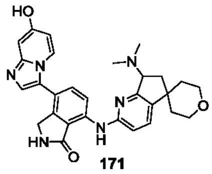
171

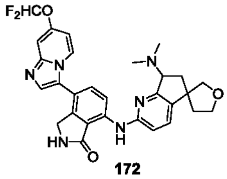
172

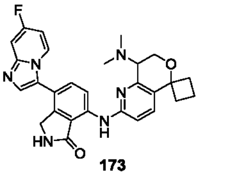
173

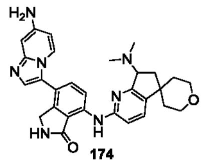
174

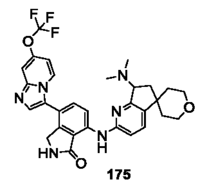
175

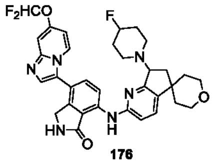
176

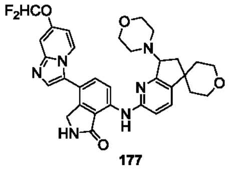
177

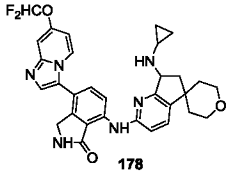
178

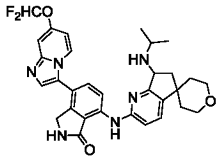
179

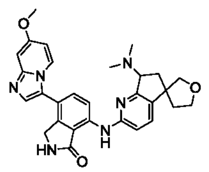
180

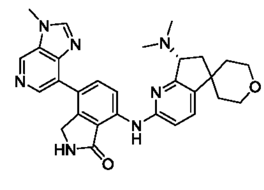
181

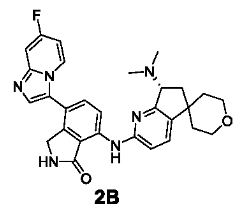
2B

and

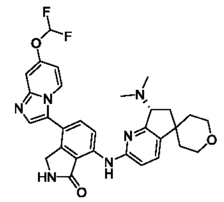
158A

.

**32.** The compound, or the stereoisomer, tautomer, deuterated substance, or pharmaceutically acceptable salt thereof according to any one of claims 1-30, wherein an intermediate compound for synthesizing the compound, or the stereoisomer, tautomer, deuterated substance, or pharmaceutically acceptable salt thereof is selected from the group consisting of:

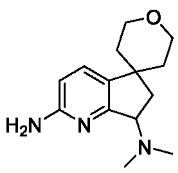
M3 ,

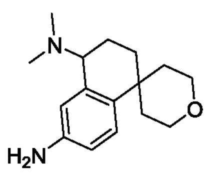
M4 ,

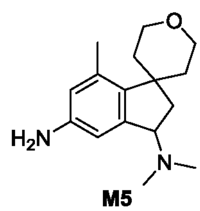
M5 ,

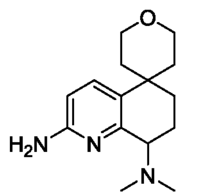
M7 ,

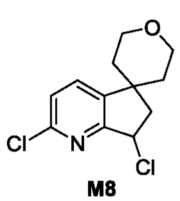
M8 ,

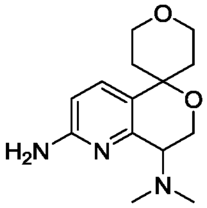
M11
,
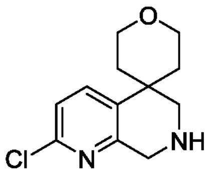
M12
,
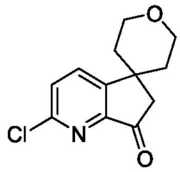
M15-3
,
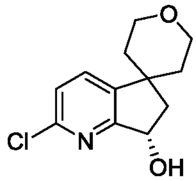
M15-4
and
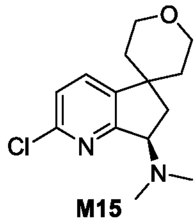
M15
.

**33.** A pharmaceutical composition comprising a therapeutically effective amount of the compound, or the stereoisomer, tautomer, deuterated substance, or pharmaceutically acceptable salt thereof according to any one of claims 1-30.

**34.** Use of the compound, or the stereoisomer, tautomer or pharmaceutically acceptable salt thereof according to any one of claims 1 to 30, or the pharmaceutical composition according to claim 32 in the preparation of a drug.

**35.** The use of claim 33, wherein the drug is for the treatment and/or prevention of cancer.

**36.** The use of claim 33 or 34, wherein the drug is for treating and/or preventing disease mediated by HPK1.

**37.** The use of claim 33 or 34, wherein the said cancer or disease is selected from the group consisting of breast cancer, multiple myeloma, bladder cancer, endometrial cancer, gastric cancer, cervical cancer, rhabdomyosarcoma, non-small cell lung cancer, small cell lung cancer, pleomorphic lung cancer, ovarian cancer, esophageal cancer , melanoma, colorectal cancer, hepatoma, head and neck tumors, hepatic cholangiocarcinoma, myelodysplastic syndrome, glioblastoma, prostate cancer, thyroid cancer, Schwann cell tumor, lung squamous cell carcinoma , lichenoid keratosis, synovial sarcoma, skin cancer, pancreatic cancer, testicular cancer and liposarcoma.

**38.** A method of treating and/or preventing diseases, comprising administering to a subject in need a therapeutically effective amount of the compound, or the stereoisomer, tautomer or pharmaceutically acceptable salt thereof according to any one of claims 1-30, or the pharmaceutical composition of claim 32.

**39.** The method of claim 37, wherein the disease to be treated and/or prevented is cancer.

**40.** The method of claim 37 or 38, wherein the disease to be treated and/or prevented is mediated by HPK1.

**41.** The method of claim 37 or 38, wherein the cancer or disease is selected from the group consisting of breast cancer, multiple myeloma, bladder cancer, endometrial cancer, gastric cancer, cervical cancer, rhabdomyosarcoma, non-small cell lung cancer, small cell lung cancer, polymorphic lung cancer, ovarian cancer, esophageal cancer Carcinoma, melanoma, colorectal cancer, hepatoma, head and neck tumors, cholangiocarcinoma, myelodysplastic syndrome, glioblastoma, prostate cancer, thyroid cancer, Schwann cell tumor, lung squamous cell carcinoma, lichenoid keratosis, synovial sarcoma, skin cancer, pancreatic cancer, testicular cancer, and liposarcoma.

## INTERNATIONAL SEARCH REPORT

International application No.
**PCT/CN2023/099307**

**A. CLASSIFICATION OF SUBJECT MATTER**

C07D 471/04(2006.01)i； C07D 209/44(2006.01)i； A61K 31/4035(2006.01)i； A61P 35/04(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNABS; WPABS; CNTXT; CJFD; VEN; WOTXT; USTXT; EPTXT; CNKI; PATENTICS; 万方, WANFANG; 超星读秀, CHAOXING DUXIU; ISI-Web of Science; STN-registry; STN-caplus: 贝达药业, 吴颢, 吴文茂, 张展, 许艳杰, 袁丁, 卢路, 王维, 朱小惯, 张泳, 张剑, 刘相来, 周伯君, 周全, 兰宏, 王家炳, 丁列明, 造血祖细胞激酶1, 丝裂原活化蛋白激酶1, 异吲哚啉酮, 肿瘤, 癌, HPK1, MAP4K1, +isoindolinone+, cancer, tumor, 结构式(I), structural formula (I)

**C. DOCUMENTS CONSIDERED TO BE RELEVANT**

| Category* | Citation of document, with indication, where appropriate, of the relevant passages | Relevant to claim No. |
|---|---|---|
| A | WO 2021050964 A1 (NIMBUS SATURN, INC.) 18 March 2021 (2021-03-18)<br>claims 1, 26, 28-33, description, table 8 | 1-37 |
| A | US 2005026976 A1 (ABBOTT LAB) 03 February 2005 (2005-02-03)<br>entire document | 1-27 |
| A | WO 2019164847 A1 (INCYTE CORP.) 29 August 2019 (2019-08-29)<br>entire document | 1-37 |
| A | CN 109721620 A (NANJING TRANSTHERA BIOSCIENCES CO., LTD.) 07 May 2019 (2019-05-07)<br>entire document | 1-37 |

☐ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **08 September 2023** | **22 September 2023** |

| Name and mailing address of the ISA/CN | Authorized officer |
|---|---|
| **China National Intellectual Property Administration (ISA/ CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/099307**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **38-41**
because they relate to subject matter not required to be searched by this Authority, namely:

Claims 38-41 set forth a method for treating and/or preventing a disease, which falls within methods for treatment of a living human or animal body, that is, falls within the cases set out in PCT Rule 39.1(iv) for which an international search is not required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/099307**

| Patent document cited in search report | Publication date (day/month/year) | Patent family member(s) | Publication date (day/month/year) |
|---|---|---|---|
| WO 2021050964 A1 | 18 March 2021 | US 2021078998 A1 | 18 March 2021 |
| | | US 11034694 B2 | 15 June 2021 |
| | | US 2021078996 A1 | 18 March 2021 |
| | | US 11548890 B1 | 10 January 2023 |
| | | IL 291244 A | 01 May 2022 |
| | | CA 3150108 A1 | 18 March 2021 |
| | | US 2021087189 A1 | 25 March 2021 |
| | | US 11028085 B2 | 08 June 2021 |
| | | KR 20220105631 A | 27 July 2022 |
| | | EP 4027995 A1 | 20 July 2022 |
| | | EP 4027995 A4 | 23 August 2023 |
| | | BR 112022004451 A2 | 21 June 2022 |
| | | JP 2022547719 A | 15 November 2022 |
| | | US 2021087190 A1 | 25 March 2021 |
| | | US 11021481 B2 | 01 June 2021 |
| | | TW 202126647 A | 16 July 2021 |
| | | AU 2020347274 A1 | 31 March 2022 |
| | | US 2021078997 A1 | 18 March 2021 |
| | | US 11078201 B2 | 03 August 2021 |
| | | AR 119954 A1 | 26 January 2022 |
| | | IN 202217013163 A | 01 July 2022 |
| | | CN 114945366 A | 26 August 2022 |
| | | HK 40070563 A0 | 28 October 2022 |
| | | VN 90775 A | 25 November 2022 |
| US 2005026976 A1 | 03 February 2005 | US 7129260 B2 | 31 October 2006 |
| WO 2019164847 A1 | 29 August 2019 | US 2019256520 A1 | 22 August 2019 |
| | | US 10752635 B2 | 25 August 2020 |
| | | TW 202000669 A | 01 January 2020 |
| | | AR 114328 A1 | 19 August 2020 |
| CN 109721620 A | 07 May 2019 | CN 109721620 B | 13 May 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2021050964 A